(19) Europäisches Patentamt
European Patent Office
Office européen des brevets

(11) **EP 4 595 996 A2**

(12) **EUROPEAN PATENT APPLICATION**

(43) Date of publication:
**06.08.2025 Bulletin 2025/32**

(21) Application number: **25158131.0**

(22) Date of filing: **22.04.2022**

(51) International Patent Classification (IPC):
***A61M 5/00*** *(2006.01)*

(52) Cooperative Patent Classification (CPC):
**C07K 16/2866; A61M 5/142; A61P 29/00; G01N 33/564;** A61K 2039/54; A61K 2039/545; A61M 5/20; C07K 2317/21; C07K 2317/76; G01N 2800/104; G01N 2800/347; G01N 2800/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA TN**

(30) Priority: **23.04.2021 US 202163178745 P**
**15.07.2021 US 202163221986 P**
**21.10.2021 US 202163270091 P**

(62) Document number(s) of the earlier application(s) in accordance with Art. 76 EPC:
**22724712.9 / 4 192 882**

(71) Applicant: **Astrazeneca AB**
**151 85 Södertälje (SE)**

(72) Inventors:
- **CHIA, Yen Lin**
  **Wilmington, 19850-5437 (US)**
- **LINDHOLM, Catharina**
  **Södertälje (SE)**
- **TUMMALA, Rajendra**
  **Gaithersburg (US)**
- **ROSKOS, Lorin**
  **Gaithersburg, 20878 (US)**
- **ALMQUIST, Joachim**
  **Södertälje (SE)**
- **ROUSE, Tomas**
  **Södertälje (SE)**
- **TRASIEVA, Teodora**
  **Gaithersburg, 20878 (US)**
- **WHITE, Wendy**
  **Gaithersburg, 20878 (US)**
- **SINIBALDI, Dominic**
  **Gaithersburg, 20878 (US)**
- **NEWCOMBE, Paul**
  **Cambridge (GB)**
- **RAMASWAMY, Madhu**
  **Gaithersburg, 20878 (US)**

(74) Representative: **AstraZeneca Intellectual Property**
**Eastbrook House**
**Shaftesbury Road**
**Cambridge CB2 8BF (GB)**

Remarks:
- The complete document including Reference Table(s) and the Sequence Listing(s) can be downloaded from the EPO website
- This application was filed on 14-02-2025 as a divisional application to the application mentioned under INID code 62.
- Claims filed after the date of receipt of the divisional application (Rule 68(4) EPC).

# (54) TREATMENT OF LUPUS NEPHRITIS

(57) The disclosure relates to methods and compositions for the treatment of lupus nephritis. Specifically, the disclosure relates to methods comprising administering to a subject a type of I IFN receptor inhibitor.

EP 4 595 996 A2

## Description

### 1. BACKGROUND

Lupus nephritis (LN) is one of the most prevalent severe disease manifestations of lupus, occurring in approximately 40% of SLE patients [1]. LN is more prevalent in African Americans, Hispanics, and Asians compared with patients of European descent [2]. The accumulation of immune complexes and the subsequent inflammatory response in kidney tissue can lead to irreversible glomerular and tubulointerstitial damage [1]. LN is strongly associated with the increased morbidity and pre-mature mortality in SLE with the standardized mortality ratio being about 3-fold higher in LN patients compared with patients with nonrenal SLE and 6- to 9-fold higher compared with the general population. According to the World Health Organization (WHO) histological classification, proliferative LN includes patients with focal Class III and diffuse Class IV proliferative glomerulonephritis [3], a subset of patients with poor prognosis, with up to 45% of patients progressing to end-stage kidney disease within 15 years of diagnosis [4].

The ultimate treatment goal for patients with active, proliferative LN is to prevent end-stage kidney failure and death [5]. Persistent proteinuria and/or acute kidney dysfunction indicate renal inflammation and are risk factors for progressive kidney damage and worse long-term outcomes [1]. Therefore, short-term treatment goals include attenuating proteinuria, measured using the urine protein-creatinine ratio (UPCR), and stabilizing/improving the estimated glomerular filtration rate (eGFR) [5].

Histopathological classes III and IV represent proliferative LN that generally requires intensive immunosuppressive therapy to achieve the treatment goal of renal remission, preserved renal function, and ultimately prevention of end-stage renal disease (ESRD). Such immunosuppressive therapy with Mycophenolate mofetil (MMF) or cyclophosphamide (CYC), in combination with glucocorticoids, is the current recommended off-label standard of care treatments for proliferative LN in international guidelines, for which an unmet therapeutic need clearly remains; the treatment typically consists of an initial intensive immunosuppressive period for 3 to 6 months followed by less intensive therapy for several years to maintain remission [5]. However, not all patients respond to this therapy: only 10% to 40% achieve remission after one year [6] and disease flares are common [7]. Furthermore, the current treatments have significant side effects, such as the risk of pre-mature menopause induced by CYC and organ damage from long-term glucocorticoid use.

The Food and Drug Administration (FDA) approved belimumab in 2020 and voclosporin in 2021 for the treatment of patients with LN based on positive efficacy results beyond standard therapy. However, in phase 3 trials, less than 50% of patients achieved a complete renal response (CRR) following treatment with belimumab (CRR; requiring urine protein-creatinine ratio (UPCR) <0.5 mg/mg) [8-10]. As such, there remains a need for additional treatment options to further increase response rates while reducing glucocorticoid exposure.

LN diagnosis is made by renal biopsy and histopathological classification according to the 2003 ISN/RPS classification criteria [3] and the histopathological classification also guides treatment. Active Class III and Class IV LN generally requires initially intensive immunosuppressive therapy combined with high dose glucocorticoids followed by several years of continued immunosuppressive treatment to achieve the clinically important treatment goals of renal remission, preserved renal function, and ultimately prevention of ESKD. The currently recommended immunosuppressive therapy for Class III and IV LN (used as off-label in most regions) consists of MMF or cyclophosphamide in combination with glucocorticoids [5].

Even if renal outcomes have improved after introduction immunosuppressive treatment only 10% to 40% achieve remission after 1 year [6] and disease flares are common. Importantly, up to 20% of LN patients develop ESKD within 10 years of initial diagnosis despite treatment and therapy is associated with significant side effects including organ damage from long-term glucocorticoid use. Despite recent approvals of belimumab in US and EU and voclosporin in US for treatment of adult patients with active a large unmet need remains since more than half of patients don't respond to these therapies. Thus, new effective and safe therapies targeting novel pathways for treatment of active LN remains in order to achieve clinical treatment goals, i.e. improve renal remission rates, reduce flares, and prevent of ESKD, while reducing the need for glucocorticoids.

The unmet need for LN thus remains substantial, with the need for novel, targeted therapies for improved renal responses, reduced flares, and prevention of ESRD, as well as reduced need for glucocorticoids. LN remission rates remain suboptimal [8], and patients are at high risk of developing end-stage kidney disease [4] and drug-related toxicity, particularly relating to prolonged, high-dose glucocorticoid use [5].

Anifrolumab is a human, monoclonal antibody that targets the type I interferon (IFN) receptor subunit 1 [11]. Two phase 3 randomized controlled trials TULIP-1 and TULIP-2 (NCT02446899 and NCT02962960, respectively) demonstrated that 300 mg intravenous (IV) every four weeks (Q4W) anifrolumab provides therapeutic benefit across multiple clinical endpoints and is well tolerated by patients with moderate to severe SLE. TULIP-1 and TULIP-2 excluded patients with LN. The safety and efficacy of type I IFN receptor inhibitor in patients with LN has not previously been demonstrated, and anifrolumab is not approved for the treatment of LN.

The present invention solves one or more of the above-mentioned problems.

## 2. SUMMARY

The present invention relates to a treatment for lupus nephritis (LN). Particularly, the invention relates to the use of a type I IFN receptor (IFNAR1) inhibitor for use in a method of treating LN. The invention is supported *inter alia* by efficacy and safety data from a phase 2, multicenter, multinational, randomized, double-blind, placebo-controlled clinical trial (NCT02547922), data from which is presented herein for the first time.

In another aspect, the invention also relates to safe and efficacious dosage regimes of a type I IFNAR (also referred to herein as IFNAR1 and IFNR) inhibitor for use in the treatment of LN. Surprisingly, a dose of a IFNAR1 inhibitor that is greater than the IFNAR1 inhibitor dose previous described for SLE patients (as described in WO 2013/188494, incorporated herein by reference) is identified as safe and efficacious in LN patients. The invention is supported *inter alia* by efficacy and safety data from NCT02547922, data and dosage information for which is presented herein for the first time.

The present invention also relates to subcutaneous doses of an IFNAR1 inhibitor and their use in the treatment of LN. The invention is supported *inter alia* by efficacy, safety and PK data from a 2 phase 3, multicenter, multinational, randomized, double-blind, placebo-controlled clinical trials in SLE patients (NCT02446899 and NCT02962960), a Phase 2, multinational, multicenter, randomized, double-blind, placebo controlled, parallel-group clinical trial in SLE patients (NCT02962960), a phase 2, multicenter, multinational, randomized, double-blind, placebo-controlled clinical trial in LN patients (NCT02547922) and a phase I, Randomized, Placebo-Controlled, Double-Blind clinical trial in health subjects (NCT02601625), together with PK/PD modelling data that is presented herein for the first time.

## 3. BRIEF DESCRIPTION OF FIGURES

***Figure 1: IFN scores distribution***
**Figure 1A:** 4-gene IFN score distribution. **Figure 1B:** Distribution of the 21-gene IFNGS in patients with SLE, LN and Sjogren's syndrome. LN: lupus nephritis; SLE: Systemic Lupus Erythematosus; HD: healthy donor.

***Figure 2: Study 7 TULIP-LN trial design and patient disposition.***
**Figure 2A:** Flow Chart of TULIP-LN Trial Design. **Figure 2B:** Patient disposition for the completed 52-week double-blind treatment period. All percentages are based on the 145 patients in the full analysis set. [a]Of patients not randomized, 179 did not meet the screening criteria, 7 withdrew consent, 2 experienced AEs, 1 was lost to follow-up, and 1 patient was not included because of the physician's decision; [b]One patient was assigned to but did not receive at least 1 dose of each of the anifrolumab regimens and therefore was not included in the analysis; [c]Reasons for not entering the second-year extension period included AEs, development of specific trial intervention discontinuation criteria, patient's decision, and lack of therapeutic response.

***Figure 3: Time to Discontinuation of Investigational Product, Kaplan-Meier Plot (mITT Population)***
More patients discontinued trial intervention early in the placebo (42.9%) group than in both anifrolumab groups. BR, basic regimen; IR, intensified regimen; mITT, modified intention-to-treat. At the time of the primary analysis (Week 52), the second-year study period was still ongoing; data from patients who continued into the ongoing second-year study period were censored.

***Figure 4: Key efficacy endpoints over time.***
**Figure 4A.** A geometric mean (GM) change from baseline in 24-hour UPCR over time. GMR of the relative improvement in 24-hour UPCR for anifrolumab groups vs placebo groups, where GMR <1 favors anifrolumab. A P-value $\leq$0.05 for the combined anifrolumab vs placebo group was deemed significant. All other P-values presented are nominal. There was a numerically larger improvement in 24-hour UPCR for the combined anifrolumab group and anifrolumab IR group versus the placebo group from Week 12 to Week 36, and for the anifrolumab IR group versus anifrolumab BR group at all time points. **Figure 4B.** Percentage of patients with CRR over time. Anifrolumab BR responses for all CRR definitions were generally similar to or lower than the placebo group at all time points apart from Week 12. **Figure 4C:** Key efficacy endpoints over time. The time to sustained $CRR_{0.5}$ was numerically shorter with anifrolumab IR than with placebo. Time to $CRR_{0.5}$ sustained through Week 52. BR, basic regimen; CI, confidence interval; CRR, complete renal response; $CRR_{0.5}$, CRR with UPCR $\leq$0.5 mg/mg; GM, geometric mean; GMR, geometric mean ratio; HR, hazard ratio; IR, intensified regimen; UPCR, urine protein-creatinine ratio. Error bars represent 95% CIs. [a]GM of the ratio of the 24-hour UPCR values at each time point over the baseline value for each treatment group (values <1 indicate an improvement); [c]Patients from Italy and France were excluded from the analysis; [d]Time to sustained $CRR_{0.5}$ was analyzed *post hoc.*

***Figure 5: 24-hour UPCR, CRR and sustained steroid reduction***

**Figure 5A:** 24-Hour UPCR Change from Baseline at Week 52 by Subgroup Forest Plot. There were no major differences in 24-hour UPCR across predefined subgroups. **Figure 5B:** CRR and sustained steroid reduction. Anifrolumab IR was associated with a CRR with sustained glucocorticoid reduction.

***Figure 6. Mean Cumulative Proteinuria (UPCR) Over Time***

Both anifrolumab groups had a numerically lower cumulative proteinuria than the placebo group throughout the treatment duration. Mean cumulative proteinuria (area under the curve in UPCR standardized by the expected follow-up time) for anifrolumab IR, anifrolumab BR, and placebo was assessed using analysis of covariance controlling for baseline UPCR and stratification factors. All data after discontinuation are excluded from the analysis. Error bars represent standard error.

***Figure 7: Percentage of Patients with A $CRR_a$ and $CRR_{0.5}$ Over Time***

**Figure 7A:** Percentage of patients with a $CRR_a$ over time. **Figure 7B:** Percentage of patients with a $CRR_{0.5}$ over time. $CRR_a$, complete renal response with inactive urinary sediment requirement; $CRR_{0.5}$, complete renal response with urine protein-creatinine ratio ≤0.5 mg/mg requirement; IR, intensified regimen. Error bars represent 95% confidence intervals.

***Figure 8. IFNGS neutralization and measures* of *disease activity over time.***

Median percentage 21-gene type I IFN PD neutralization among IFNGS test-high patients. A median PD neutralization >80% was observed with anifrolumab IR across all visits (Weeks 12, 24, 36, and 52) and with anifrolumab BR at Weeks 12 and 24 only, after which there was a rebound in IFNGS. BR, basic regimen; IFNGS, interferon gene signature; IR, intensified regimen; LS, least squares, MAD, median absolute deviation; PD, pharmacodynamic; PGA, Physician's Global Assessment, PtGA, Patient's Global Assessment; SE, standard error; SLEDAI-2K, Systemic Lupus Erythematosus Disease Activity Index 2000. Number of patients with non-missing value at visit are presented.

***Figure 9. Plots of Anti-dsDNA Antibodies and C3 Complement Levels***

**Figure 9A:** Compared with the placebo group, patients positive for anti-dsDNA antibodies at baseline had numerically greater reductions in anti-dsDNA antibody levels with anifrolumab IR versus placebo. Data points are median change from baseline and error bars represent median absolute deviation. **Figure 9B:** Patients with low C3 at baseline had an increase in C3 across groups (IR and BR). Data points are median change from baseline and error bars represent median absolute deviation.

***Figure 10. Plot of C4 Complement Levels***

There were no clear differences in C4 increases across groups. Data points are median change from baseline and error bars represent median absolute deviation.

***Figure 11. SLEDAI-2K, PGA and PtGA***

Compared with placebo, anifrolumab IR elicited numerically greater improvements from baseline in measures of disease activity (SLEDAI-2K, PGA, PtGA). **Figure 11A:** Non-renal SLEDAI-2K change from baseline. **Figure 11B:** PGA change from baseline. **Figure 11C:** PtGa from baseline.

***Figure 12. Logarithmic Anifrolumab Serum Concentration-Time Profiles (Pharmacokinetic Analysis Set)***

BR, basic regimen; IR, intensified regimen; LLOQ, lower limit of quantitation.

***Figure 13. PK Modeling of Anifrolumab Concentrations Over Time for Anifrolumab Basic and Intensified Regimens in IFNGS-High Patients With LN and SLE***

**Figure 13A:** In IFNGS-high patients (94.5%), the median Week 12 anifrolumab steady-state concentration was 63.4 μg/mL with anifrolumab BR. **Figure 13B:** The median Week 12 anifrolumab steady-state concentration was 63.4 μg/mL with anifrolumab IR (~50% lower than in nonrenal SLE). Dashed black lines represent median PK concentration at Week 12 steady-state for patients with LN and nonrenal SLE. PK modeling was performed using a nonlinear mixed-effect model with NONMEM 7.3 software (ICON Development Solutions, Ellicott City, MD, United States; 2006). The predicted anifrolumab concentrations for patients with SLE are based on data pooled from 4 clinical trials of anifrolumab in patients with SLE (n=664): the phase 2, multicenter, open-label study in Japanese patients (NCT01559090), the phase 2b global, multicenter MUSE RCT (NCT01438489), and the phase 3 global, multicenter TULIP-1 (NCT02446912) and TULIP-2 (NCT02446899) RCTs.

***Figure 14. Overlay* of *observed concentration profiles for the basic and intensified treatment regimen in study***

***07 and model-projects based on population PK model in patients with SLE***

***Figure 15. Estimated Anifrolumab Clearance (L/day) in Patients Depending on Baseline 24-Hour UPCR Value (≤3 mg/mg vs >3 mg/mg)***

Anifrolumab clearance was higher among patients with baseline UPCR >3 mg/mg than those with UPCR ≤3 mg/mg. BR, basic regimen; IR, intensified regimen; PK, pharmacokinetics; UPCR urine protein-creatinine ratio. Bars represent median anifrolumab clearance ± interquartile range. Using the Population-PK model developed for nonrenal SLE, the estimated individual clearance estimates for baseline 24-hour UPCR subgroups (≤3 mg/mg vs >3 mg/mg) in the combined anifrolumab group were estimated a nonlinear mixed-effect model with NONMEM 7.3 software, fitted to anifrolumab BR and anifrolumab IR datasets. PK data collected from the anifrolumab IR group after tapering to 300 mg were excluded, because of the potential impact of tapering on the change of time-dependent clearance.

***Figure* 16: *Mean anifrolumab serum concentration-time profiles***

**Figure 16A:** Study MI-CP180 in Scleroderma (SSc) - Mean anifrolumab serum concentration-time profiles following a single IV dose. Data represent +/- SD. Mean data below LLOQ are not plotted. IV, intravenous; LLOQ, lower limit of quantification; MEDI 546, anifrolumab; n, number of patients in a subgroup; SSc, systemic sclerosis. **Figure 16B:** Study 06 in healthy volunteers - Mean anifrolumab serum concentration-time profiles following a single SC and IV dose. Samples with actual collection time deviating from nominal collection time by >10% were excluded from the mean. IV, intravenous; N, number of subjects; SC, subcutaneous.

***Figure 17: Study 08 study design and results***

**Figure 17A:** Study design for phase II of SC anifrolumab in SLE patients. Study 08 (NCT02962960) evaluated the effect of two anifrolumab doses every other week. **Figure 17B:** Mean serum concentration of anifrolumab over time. **Figure 17C:** Anifrolumab neutralization of the type I IFN gene signature.

***Figure 18: Type I IFN 21-gene signature neutralization in type I IFN test-high patients in studies 04, 05, and 1013***

***Figure 19: Computed median AUC Ratios (SC/IV)***

**Figure 19A:** Computed median AUC Ratio (SC/IV) between weeks 0-52 for various SC doses. The computed median AUC Ratio (SC/IV), based on the estimated bioavailability from Study 06, between weeks 0-52, where the subcutaneous dose is either 75mg (+ sign), 90 mg (empty squares), 105 mg (circles), 120 mg (triangles), or 135 mg (filled squares). The subcutaneous dose here is administered once every 7 days (QW); the IV dose is administered once every 4 weeks (Q4W) at a dose of 300 mg. Based on the AUC, both 90 and 105 mg SC QW appear similar to 300 mg IV. **Figure 19B:** Computed median AUC ratio (SC/IV) for 90 mg and 105 mg SC QW. The computed median AUC Ratio (SC/IV), based on the estimated bioavailability ~7% lower than the bioavailability calculated from Study 06, between weeks 0-52, where the subcutaneous dose is either 90 mg SC QW or 105 mg SC.

***Figure 20: Anifrolumab concentration over time at different doses***

**Figure 20A:** A plot showing (computed) trough concentrations of plasma anifrolumab in a patient administered either (i) 105 mg of anifrolumab subcutaneously, once every 7 days (straight line); (ii) 300 mg anifrolumab intravenously, once every 4 weeks (lower dotted line); (ii) 1000 mg anifrolumab intravenously, once every 4 weeks (upper dotted line). Shaded area represents the area between 5th and 95th percentiles of the 300 mg IV Q4W dose. **Figure 20B:** Anifrolumab trough concentration in IFNGS high SLE subjects. Computed trough concentrations of anifrolumab in IFNGS high patients' plasma after administration as follows: (i) 300 mg IV Q4W; (ii) 90 mg SC QW; (iii) 105 mg SC QW; (iv) 135 mg SC QW; (v) 1000 mg IV Q4W. SC = subcutaneous. Based on trough, both 90 and 105 mg SC QW were projected to have higher PD suppressions than 300 mg IV.

***Figure 21: Positive Exposure-BICLA relationship observed in TULIP 1 & TULIP 2 in IFNGS high patients***

**Figure 21A:** TULIP I, for placebo, 150 mg and 300 mg anifrolumab. **Figure 21B:** TULIP II, for placebo and 300 mg.

***Figure 22: BICLA dose response***

**Figure 22A:** Dose response curve, for probability of meeting BICLA response criteria (in IFNGS high patients) versus anifrolumab $C_{ave}$ over 52 weeks, showing the predicted mean (grey line) and 95% confidence interval (CI) (dashed area). Patients are grouped by dose (150 mg, n =62; 300 mg, n=242; and 1000 mg). **Figure 22B:** Predicted PK and efficacy for different SC doses. The probability of meeting BICLA (in IFNGS high patients) for weekly subcutaneous doses starting from 105 mg, and up to 150 mg. Assumptions for generating the data include no dose delays/interruptions.

***Figure 23: $C_{troughs}$ following injection at thigh compared to injection* at *abdomen***
**Figure 23A:** 150 mg SC Q2W. **Figure 23B:** 300 mg SC Q2W.

***Figure 24: Exposure prediction based on 81-87% bioavailability and preliminary PK modelling***
Anifrolumab $C_{ave}$ medium ratio predicted for 90-150 mg SC QW to 300 mg Q4W, based on PK preliminary modelling and bioavailability assumptions.

***Figure 25: Anifrolumab $C_{ave}$ over 52 weeks in IFNGS high patients for different SC and IV doses***
**Figure 25A:** 105 mg SC QW. **Figure 25B:** 120 mg SC QW. **Figure 25C:** Overlap with 1000 mg IV Q4W.

***Figure 26: $C_{ave}$ median ratio SC QW to 300 mg IV Q4W***
**Figure 26A:** 81% bioavailability assumed. **Figure 26B:** 70% bioavailability assumed.

***Figure 27: Average anifrolumab concentration versus herpes zoster incidence***
The incidence of Herpes Zoster (%) in patients in the Study 1013 receiving placebo, 300 mg IV anifrolumab or 1000 mg IV anifrolumab.

***Figure 28: Median change in UPCR over time for patients with baseline 24-hour UPCR ≤ 3 mg/mg and magnitude of the change in clearance***
**Figure 28A:** UPCR ≤ 3. **Figure 28B:** UPCR >3. CL, clearance, hr, hour, n, number of patients; UPCR, urine protein-creatinine ratio; yr, year.

***Figure 29: Type I IFN 21-GS in LN patients***
**Figure 29A:** PD suppression over time in study 07 for patients with baseline 24-hour UPCR > 3 mg/mg by treatment regimen. **Figure 29B:** 24-hour UPCR level stratified by percentage steady state PD suppression in study 07 for patients with baseline 24-hour UPCR > 3 mg/mg.

***Figure 30: Visual predicative check of UPCR model***
Plots are showing 95% confidence intervals for the model-predicted median (dark grey) and 10th and 90th percentiles (light grey) of UPCR, together with observed individual data (circles) and its median (solid line) and 10th and 90$^{th}$ percentiles (dashed lines). Model predictions have been corrected for dropout. Binning is indicated by the vertical lines at the top. BR, basic regimen, IR intensified regimen, UPCR, urine protein-creatinine ratio.

***Figure 31: Visual predictive check of PK model***
Plots are showing 95% confidence intervals for the model-predicted median (dark grey) and 10th and 90th percentiles (light grey) of anifrolumab trough concentrations, together with observed individual data (circles) and its median (solid line) and 10th and 90th percentiles (dashed lines). Model predictions have been corrected for dropout. Bottom Straight line indicates LLOQ of 0.02 μg/m. The 10th percentile of the concentration in BR at Week 12 was below the LLOQ. Binning is indicated by the vertical lines at the top. BR, basic regimen, IR, intensified regimen, LLOQ, lower limit of quantification; PK, pharmacokinetic.

***Figure 32: Visual predictive check of dropout model***
Plots are showing 95% confidence intervals for the model-predicted dropout (dark grey), together with observed dropout (black line). BR, basic regimen; IR, intensified regimen.

***Figure 33: Model-predicted impact of the intensified treatment period on UPCR response***
BR, basic regimen; IR, intensified regimen; IV, intravenous; Q4W, once every four weeks; QW, once a week; SC, subcutaneous; UPCR, urine protein-creatinine ratio.

***Figure 34: Model predictions of PK, UPCR, PD, and clearance for the proposed regimen***
Simulations are showing the median (bold line) and the 10th to 90th percentile interval of the population (shaded area), including only patients that has not dropped out. Upper left panel is showing anifrolumab concentrations. Reference lines indicate the median trough concentration at Week 24 for 300 mg IV Q4W as predicted by the previously developed SLE PK model, and the estimated IC80 and IC90 of the PD signature according to the SLE PD model. Upper right panel is showing UPCR. Percentage numbers are showing the proportion of patients below the 0.5 mg/mg (accounting also for the patients that dropped out). Lower left panel is showing PD suppression. The levels of 80% and 90% suppression are shown as references. The lower right panel is showing anifrolumab clearance. CL, clearance; IC80, 80% inhibitory concentration; IC90, 90% inhibitory concentration; IV, intravenous; PD, pharmacodynamics; PK, pharmacokinetic; SLE, systemic lupus erythematosus; UPCR, urine protein-creatinine ratio.

***Figure 35: 1150 mg SC provides similar AUC to 900mg IV in healthy volunteers***

***Figure* 36: *1150 mg SC provides similar AUC to 900mg IV in LN patients***

**Figure 36A:** AUC ratio is close to 1.0 for the entire 6 months of the intensified treatment. **Figure 36B:** SC median $C_{trough}$ at week 24 (72 μg/mL) is lower compared to SLE patients on 1000 mg Q4W. . **Figure 36C:** PD suppression at troughs remains high throughout the intensified treatment.

***Figure 37: Urinary proteins in LN***

**Figure 37A:** Urinary Proteins Associated With High NIH-AI and NIH-CI Scores. Protein associations (FDR <0.1) are color coded by whether their concentrations positively (orange, +) or negatively (blue,-) correlate with the respective outcome. **Figure 37A:** Urinary Proteins Associated With ≥4 Clinical Characteristics. Protein associations (FDR <0.1)) are color coded by whether their concentrations positively (+) or negatively (-) correlate with the respective outcome.

***Figure* 38: *Urinary proteins and IFNGS***

**Figure 38A:** Urinary Proteins Associated With High IFNGS, and Their Correlations With Other Measures. Protein associations (FDR <0.1)) are color coded by whether their concentrations positively (+) or negatively (-) correlate with the respective outcome. **Figure 38B:** Venn Diagram of Overlap in Significant Protein Associations Across Three Renal Measures. Only proteins with statistically significant associations (FDR<0.1) are listed.

***Figure* 39: *Ingenuity Pathway analysis***

**Figure 39A:** Ingenuity Pathway Analysis of the 11 Proteins Commonly Associated With eGFR, SLEDAI-R, and NIH-AI. Top four disease and molecular function categories as scored by Fisher's Exact Test are displayed. Highly redundant categories were removed. Significance threshold (dashed line). **Figure 39B:** Urinary Proteins Unique to All Clinical Features.

***Figure 40. Delivery device***

Anifrolumab is administered by an injection device **[1] [9]** such as a prefilled syringe (PFS) **(Figure 40A**) or an autoinjector (AI) (**Figure 40B**).

***Figure 41. Autoinjector***

The autoinjector for administering anifrolumab of the functional variant thereof in exploded view (**Figure 41A**), assembled (**Figure 41B**) and filled with drug substance (**Figure 41C**).

***Figure 42. Accessorized pre-filled syringe***

The accessorized pre-filled syringe (APFS) for anifrolumab of the functional variant thereof. The primary tube is shown in assembled form (**Figure 42A**) and in exploded view (**Figure 42B**). The APFS with its additional components is shown in assembled form (**Figure 42D**).

***Figure 43. Packaging for the delivery device***

***Figure 44. Anifrolumab Heavy Chain alignment***

***Figure 45. Anifrolumab Light Chain alignment***

## 4. DETAILED DESCRIPTION

### 4.1. Method of treating lupus nephritis (LN)

In a first aspect the invention relates to a method of treating lupus nephritis (LN) in a subject in need thereof, the method comprising administering a type I IFN receptor (IFNAR) inhibitor to the subject, wherein the method reduces lupus nephritis disease activity in the subject. The method may reduce LN disease severity in the subject. The method may prevent worsening of LN disease in the subject. LN diagnosis of the subject may be made by renal biopsy and histopathological classification according to the 2003 ISN/RPS classification criteria [3]. LN may be proliferative LN. LN may be Class III or IV (both with or without Class V) LN. The IFNAR1 inhibitor may be anifrolumab or a functional variant thereof.

The invention also relates to a dosage regime of an IFNAR inhibitor for the treatment of LN in a subject. The data provided herein demonstrate that the dosage regime of an IFNAR previously identified as suitable for treatment of SLE is insufficient to treat LN. The treatment of LN requires an intensified dosage regime (e.g. of 900 mg Q4W IV for at least 3 weeks, or the equivalent subcutaneous dose) followed by a basic dosage regime (e.g. of 300 mg Q4W or the equivalent subcutaneous dose).

Reducing LN disease activity in the subject may comprise treating LN in the subject. Reducing LN disease activity may comprise a complete renal response (CRR) in the subject post-treatment compared to pre-treatment. A CRR may be achieved by week 36 of treatment. Reducing LN disease activity in the subject may comprise a CRR and a UPCR of ≤0.5 mg/mg post-treatment. Reducing LN disease activity in the subject may comprise reduction in proteinuria in the subject post-treatment compared to proteinuria in the subject pre-treatment. The proteinuria may be measured by UPCR. Proteinuria may be measured by 24-hour UPCR (see **Section 5.5.7**). Reducing lupus nephritis disease activity in the subject may comprise an alternative CRR (aCRR) in the subject post-treatment (see **Section 5.5.5**). The method may comprise administering Mycophenolate mofetil (MMF) and/or steroid to the subject. The method may comprise steroid sparing in the subject, wherein the dose of the steroid administered to the subject is tapered from a pre-sparing dose to a post-sparing dose. The post-sparing dose may be ≤7.5 mg/day prednisone or prednisone equivalent dose (see **Section 5.4**). The pre-sparing dose may be 20 mg/day or prednisone equivalent dose. The steroid may comprise a glucocorticoid. The steroid may comprise an oral glucocorticoid. The method may comprise hydrocortisone, mometasone, fluticasone, fluocinolone acetonide, fluocinolone, flurandrenolone acetonide, ciclesonide, budesonide, beclomethasone, deflazacort, flunisolide, beclomethasone dipropionate, betamethasone, betamethasone valerate, methylprednisolone, dexamethasone, prednisolone, cortisol, triamcinolone, clobetasol, clobetasol propionate, clobetasol butyrate, cortisone, corticosterone, clocortolone, dihydroxycortisone, alclometasone, amcinonide, diflucortolone valerate, flucortolone, fluprednidene, fluandrenolone, fluorometholone, halcinonide, halobetasol, desonide, diflorasone, flurandrenolide, fluocinonide, prednicarbate, desoximetasone, fluprednisolone, prednisone, azelastine, dexamethasone 21-phosphate, fludrocortisone, flumethasone, fluocinonide, halopredone, hydrocortisone 17-valerate, hydrocortisone 17-butyrate, hydrocortisone 21-acetate, prednisolone, prednisolone 21-phosphate, clobetasol propionate, triamcinolone acetonide, or a mixture thereof. The steroid may comprise prednisone.

### 4.2. The subject

The subject may be a human subject. The subject may be an adult. The subject may be a patient with an elevated type I IFN gene signature. The subject may be a type I interferon stimulated gene signature (IFNGS)-test high patient pre-administration with the dose or unit dose. The subject may have elevated expression of the genes IFI27, IFI44, IFI44L, and RSAD2 in the whole blood. The subject may have elevated expression of the genes IFI27, IFI44, IFI44L, and RSAD2 in the whole blood compare to a healthy subject. The method may comprise identifying the subject as IFNGS-test high patient pre-treatment with the dose or unit dose. The method may comprise measuring the expression of the genes IFI27, IFI44, IFI44L, and RSAD2 in the whole blood of the subject. The method may comprise measuring the expression of the genes IFI27, IFI44, IFI44L, and RSAD2 in the whole blood of the subject by RT-PCR. The method may comprise measuring the expression of the genes IFI27, IFI44, IFI44L, and RSAD2 in a sample of whole blood from the subject by RT-PCR. The subject may be a type I IFN 21-gene signature high patient. The subject may be a type I IFN 4-gene signature high patient.

The invention also relates to a method of treatment of lupus nephritis comprising analysing the levels of a protein or proteins in the urine of the subject pre-treatment and/or post treatment, optionally in an isolated urine sample from the subject. The protein or proteins may be identified as elevated in the subject's urine pre-treatment compared to the level of the protein in a healthy subject. Post-treatment, the level of the protein or proteins in the urine of the subject may be reduced compared to the pre-treatment level of the protein or proteins in the urine of the subject.

The invention also relates to a method for identifying a subject as suitable for treatment with a IFNAR1 inhibitor, the method comprising identifying or detecting elevated expression of a protein or proteins in an isolated urine sample from the subject compared to expression of the protein or proteins respectively in a healthy subject. The IFNAR1 inhibitor may be an IFNAR1 inhibitor according to the method of the invention. The method of treatment may be method of treatment of the invention.

The subject may have proliferative LN. The subject may have active LN. The subject may have Class III or Class IV LN, with or without co-existing Class V LN.

The protein or proteins may comprise Adiponectin, Alpha-2-Macroglobulin (A2Macro), Antithrombin-III (AT-III), Apolipoprotein A-I (Apo A-I), Apolipoprotein B (Apo B), Apolipoprotein C-I (Apo C-I), Apolipoprotein C-III (Apo C-III), Fatty Acid-Binding Protein, heart (FABP, heart), Lactoferrin (LTF), Neuropilin-1, Omentin, Serum Amyloid P-Component (SAP) and/or von Willebrand Factor (vWF).

The protein or proteins may comprise Apo A-11, Apo B, Apo C-I, Cathepsin D, EN-RAGE, Fibrinogen, LTF, MCP-1, RANTES and/or IL-1β. The protein or proteins may comprise Apo B, Apo C-I, and/or LTF.

### 4.3. Pharmaceutical composition

In another aspect the invention relates to a pharmaceutical composition for use in treating LN in a subject thereof, the method comprising subcutaneously administering the pharmaceutical composition to the subject, wherein the pharmaceutical composition comprises the unit dose of the invention.

In another aspect the invention relates to a pharmaceutical composition for use in treating LN in a subject thereof, the method comprising intravenously or subcutaneously administering the pharmaceutical composition to the subject.

In another aspect the invention relates to a pharmaceutical composition for use in a method of treating LN in a subject thereof, the method comprising subcutaneously administering the pharmaceutical composition to the subject, wherein the pharmaceutical composition comprises a dose of a IFNAR1 inhibitor (e.g. anifrolumab or a functional variant thereof), wherein the dose is more than (>)105 mg and less than (<)150 mg. The dose of the IFNAR1 inhibitor (e.g. anifrolumab or the functional variant thereof) may be a unit dose (unit dose form, pharmaceutical unit dose form, pharmaceutical unit dose). Functional anifrolumab variants include antigen-binding fragments of anifrolumab and antibody and immunoglobulin derivatives of anifrolumab.

In another aspect the invention relates to a pharmaceutical composition for use in a method of treating lupus nephritis in a subject thereof, the method comprising subcutaneously administering the pharmaceutical composition to the subject, wherein the pharmaceutical composition comprises a dose of the IFNAR1 inhibitor (e.g. anifrolumab or functional variant thereof), wherein administering the pharmaceutical composition every week provides a plasma concentration in the subject that is at least equivalent to the plasma concentration provided by intravenous administration of 300 mg of the IFNAR1 inhibitor (e.g. anifrolumab or the functional variant thereof) every 4 weeks. Administering the dose every week may provide a plasma concentration in the subject that is about equivalent to the plasma concentration provided by intravenous administration of 400 mg of the IFNAR1 inhibitor (e.g. anifrolumab or the functional variant thereof) every 4 weeks. The dose may be <150 mg (i.e. less than 150 mg) of the IFNAR1 inhibitor (e.g. anifrolumab or the functional variant thereof). The dose may be >105 mg (i.e. more than 105 mg) of the IFNAR1 inhibitor (e.g. anifrolumab or the functional variant thereof). The dose may be ≤135 mg (i.e. 135 mg or less) of the IFNAR1 inhibitor (e.g. anifrolumab or the functional variant thereof). The dose may be about 120 mg of the IFNAR1 inhibitor (e.g. anifrolumab or the functional variant thereof). The dose may be 120 mg of the IFNAR1 inhibitor (e.g. anifrolumab or the functional variant thereof).

The pharmaceutical composition may comprise about 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 300, 305, 310, 800, 805, 810, 820, 825, 830, 835, 840, 845, 850, 855, 860, 865, 870, 875, 880, 885, 890, 895, 890, 900, 905, 910, 915, 920, 925, 930, 935, 940, 945, 950, 955, 960, 965, 970, 975, 980, 985, 990, 1000, 1050, 1010, 1020, 1025, 1030, 1035, 1040, 1045, 1050, 1055, 1060, or 1065 mg of the IFNAR1 inhibitor (e.g. anifrolumab or the functional variant thereof).

The pharmaceutical composition may be administered to the subject in a single administration step or in multiple administration steps.

The pharmaceutical composition may be administered at intervals of 6-8 days. The pharmaceutical composition may be administered once per week (QW). The pharmaceutical composition may be administered in a single administration step. The dose may be 120 mg of a IFNAR1 inhibitor (e.g. anifrolumab or the functional variant thereof), and the method of treatment may comprise administering the dose in a single administration step once per week (QW). The pharmaceutical composition may be administered once per week for at least about 4 weeks. The pharmaceutical composition may be administered once per week for at least about 8 weeks. The dose or unit dose may be administered once per week for at least about 12 weeks. The pharmaceutical composition may be administered once per week for at least about 16 weeks. The pharmaceutical composition may be administered once per week for at least about 20 weeks. The pharmaceutical composition may be administered once per week for at least about 24 weeks. The pharmaceutical composition may be administered once per week for at least about 28 weeks. The pharmaceutical composition may be administered once per week for at least about 32 weeks. The pharmaceutical composition may be administered once per week for about 8 weeks. The pharmaceutical composition may have a volume permitting delivery to the subject in a single subcutaneous administration step. The pharmaceutical composition may have a volume of 0.5 to 1 ml. The pharmaceutical composition may have a volume of less than 1 ml. The pharmaceutical composition may have a volume of about 0.8 ml.

Many patients with LN receive corticosteroids (glucocorticoids, oral corticosteroids, OCS). However, corticosteroids are associated with organ damage. Anifrolumab permits tapering of the corticosteroids (glucocorticoids) in LN patients (steroid sparing). The method of treatment or method may comprise administering a corticosteroid to the subject, optionally wherein the corticosteroid is an oral corticosteroid. The method may comprise tapering the dose of corticosteroids administered to the subject (steroid sparing). The method may comprise administering a first dose of the corticosteroid and subsequently administering a second dose of the corticosteroid, wherein the second dose of the corticosteroid is lower than the first dose of the corticosteroid. The second dose of the corticosteroid may be about a 7.5 mg prednisone-equivalent dose or less (see **Table 5**). The second dose of the corticosteroid may be a 5 mg prednisone-equivalent dose or less. The method or method of treatment may comprise administrating the second dose of the corticosteroid once per day. The first dose of the corticosteroid may be about a 10 mg prednisone-equivalent dose. The method may comprise tapering the dose of corticosteroid administered to the patient from 10 mg or more per day to less than 10 mg per day. The method or method of treatment may comprise administering the second dose of the corticosteroid once per day. The method may permit administration of a reduced dose of corticosteroids that is sustained for weeks. The second dose of the corticosteroid may be administered for at least 24 weeks. The second dose of the corticosteroid may be administered for at least 28 weeks.

Administration of the pharmaceutical composition may provide a plasma concentration of the IFNAR1 inhibitor (e.g. anifrolumab or the functional variant thereof) in the patient of ≥ 10 μg (i.e. 10 μg or more). Administration of the pharmaceutical composition may provide a plasma concentration of the IFNAR 1 inhibitor (e.g. anifrolumab or the functional variant thereof) in the subject of 10-100 μg/ml. Administration of the pharmaceutical composition may provide a plasma concentration of the IFNAR1 inhibitor (e.g. anifrolumab or the functional variant thereof) in the subject of 20-80 μg/ml. Administration of the pharmaceutical composition may provide a plasma concentration of the IFNAR1 inhibitor (e.g. anifrolumab or the functional variant thereof) in the subject of 30-70 μg/ml. Administration of the pharmaceutical composition may provide a trough concentration of the IFNAR1 inhibitor (e.g. anifrolumab or the functional variant thereof) in the subject of ≥ 20 μg/ml (i.e. 20 μg/ml or more). Administration of the pharmaceutical composition may provide a trough concentration of the IFNAR1 inhibitor (e.g. anifrolumab or the functional variant thereof) in the subject of ≥ 30 μg/ml (i.e. 30 μg/ml or more). Administration of the pharmaceutical composition may provide a trough concentration of the IFNAR1 inhibitor (e.g. anifrolumab or the functional variant thereof) in the subject of ≥ 40 μg/ml (i.e. 40 μg/ml or more). Administration of the pharmaceutical composition may provide a trough concentration of the IFNAR1 inhibitor (e.g. anifrolumab or the functional variant thereof) in the subject of 20-100 μg/ml. Administration of the pharmaceutical composition may provide a trough concentration of the IFNAR1 inhibitor (e.g. anifrolumab or the functional variant thereof) in the subject of 30-80 μg/ml. Administration of the pharmaceutical composition may provide a trough concentration of the IFNAR1 inhibitor (e.g. anifrolumab or the functional variant thereof) in the subject of 40-70 μg/ml.

The subject may be a human subject. The subject may be an adult. The subject may have LN. The subject may be a patient with an elevated type I IFN gene signature. The subject may be a type I interferon stimulated gene signature (IFNGS)-test high patient pre-administration with the dose or unit dose. The subject may have elevated expression of the genes IFI27, IFI44, IFI44L, and RSAD2 in the whole blood. The method may comprise identifying the subject as IFNGS-test high patient pre-treatment with the dose or unit dose. The method may comprise measuring the expression of the genes IFI27, IFI44, IFI44L, and RSAD2 in the whole blood of the subject. The method may comprise measuring the expression of the genes IFI27, IFI44, IFI44L, and RSAD2 in the whole blood of the subject by RT-PCR.

The pharmaceutical composition may provide a therapeutic effect in the subject that is at least equivalent to a therapeutic effect provided by administration of an intravenous dose of 300 mg anifrolumab or the functional variant thereof administered once every (Q4W). The pharmaceutical composition may provide a trough concentration of the IFNAR1 inhibitor (e.g. anifrolumab or the functional variant thereof) in the subject that is greater than a trough concentration of anifrolumab or the functional variant thereof provided by administration of an intravenous dose of 300 mg anifrolumab or the functional variant thereof once every 4 weeks (Q4W). The IFNAR1 inhibitor (e.g. anifrolumab or the functional variant thereof) may be comprised within a pharmaceutical composition. The pharmaceutical composition may comprise 150 to 200 mg/ml of the IFNAR1 inhibitor (e.g. anifrolumab or the functional variant thereof), 25 to 150 mM of lysine salt and an uncharged excipient. The pharmaceutical composition may comprise about 150 mg/mL anifrolumab or the functional variant thereof. The pharmaceutical composition may comprise 50 mM lysine HCl. The pharmaceutical composition may comprise 130 mM trehalose dihydrate. The pharmaceutical composition may comprise 0.05% polysorbate 80 or polysorbate 20. The pharmaceutical composition may comprise 25 mM histidine/histidine HCl. The pharmaceutical composition may comprise 150 mg/mL anifrolumab or the functional variant thereof, 50 mM lysine HCl, 130 mM trehalose dihydrate, 0.05% polysorbate 80 and 25 mM histidine/histidine HCl.

In another aspect, the invention relates to an injection device comprising the unit dose of the invention, or the pharmaceutical composition for the use of the invention.

In another aspect, the invention relates to an injection device comprising a pharmaceutical composition. The pharmaceutical in the injection device may comprise >105 mg (i.e. more than 105 mg) and <150 mg (i.e. less than 150 mg) of the IFNAR1 inhibitor (e.g. anifrolumab or a functional variant thereof). The pharmaceutical composition in the injection device may comprise about 120 mg of the IFNAR1 inhibitor (e.g. anifrolumab or the functional variant thereof). The pharmaceutical composition in the injection device may comprise 120 mg of the IFNAR1 inhibitor (e.g. anifrolumab or the functional variant thereof). The pharmaceutical composition in the injection device may comprise about 1150 mg of the IFNAR1 inhibitor (e.g. anifrolumab or the functional variant thereof). The pharmaceutical composition in the injection device may comprise 1150 mg of the IFNAR1 inhibitor (e.g. anifrolumab or the functional variant thereof). The injection device may comprise 0.8 ml of the pharmaceutical composition. The injection device may comprise 7.7 ml of the pharmaceutical composition. The concentration of the IFNAR1 inhibitor (e.g. anifrolumab or the functional variant thereof) in the pharmaceutical composition in the injection device may be 150 mg/ml. The volume of the pharmaceutical composition in the injection device may be at least about 0.8ml. The volume of the pharmaceutical composition may be about 0.8ml.

The pharmaceutical composition in the injection device may comprise 150 to 200 mg/ml anifrolumab or the functional variant thereof, 25 to 150 mM of lysine salt and an uncharged excipient. The pharmaceutical composition in the injection device may comprise 150 mg/mL anifrolumab or the functional variant thereof. The pharmaceutical composition in the injection device may comprise 50 mM lysine HCl. The pharmaceutical composition may comprise 130 mM trehalose dihydrate. The pharmaceutical composition in the injection device may comprise 150 to 200 mg/ml anifrolumab or the functional variant thereof, 25 to 150 mM of lysine salt and an uncharged excipient. The pharmaceutical composition in the injection device may comprise 150 mg/mL anifrolumab or the functional variant thereof. The pharmaceutical composition may comprise 50 mM lysine HCl. The pharmaceutical composition in the injection device may comprise 130 mM trehalose dihydrate. The pharmaceutical composition in the injection device may comprise 0.05% polysorbate 80 or polysorbate 20. The pharmaceutical composition in the injection device may comprise 25 mM histidine/histidine HCl. The pharmaceutical composition in the injection device may comprise 150 mg/mL anifrolumab or the functional variant thereof, 50 mM lysine HCl, 130 mM trehalose dihydrate, 0.05% polysorbate 80 and 25 mM histidine/histidine HCl.

### 4.4. Device

As well as providing for subcutaneous administration of the antibody, the ability to self-administer (e.g. for home use) may further be enhanced by subcutaneous administration via an accessorized pre-filled syringe (APFS), an autoinjector (AI), or a combination thereof. Such devices have been found to be welltolerated and reliable for administering subcutaneous doses of an antibody and provide further options for optimizing patient care. Indeed, such devices may reduce the burden of frequent clinic visits for patients. An example of a suitable APFS device is described in Ferguson *et. al.* [12], which is incorporated herein by reference in its entirety.

The dose elucidated by the inventors provides yet advantages in the context of APFS-administration, as an APFS device typically administers a maximal volume of 1 ml. A dose in the range of >105 mg to < 155 mg can be readily accommodated by a volume of ~0.8 ml, such that the dose(s) of the present invention are uniquely suited to APFS and AI administration. For comparison, due to viscosity of the anifrolumab, larger doses (particularly doses of >150 mg) would need to be administered within a volume of > 1ml, requiring at least two SC injections, which is inconvenient for the patient, and would require a plurality of pre-filled devices

The delivery device may be single use, disposable system that is designed to enable manual, SC administration of the dose.

In another aspect the invention relates to an injection device comprising a unit dose. The unit dose may comprise >105 mg (i.e. at least 105 mg) and <150 mg (i.e. less than 150 mg) of the IFNAR1 inhibitor, optionally wherein the IFNAR1 inhibitor is anifrolumab or a functional variant thereof. The unit dose may comprise ≤135 mg (i.e. 135 mg or less) of the IFNAR1 inhibitor, optionally wherein the IFNAR1 inhibitor is anifrolumab or the functional variant thereof. The unit dose may comprise about 120 mg of the IFNAR1 inhibitor, optionally wherein the IFNAR1 inhibitor is anifrolumab or the functional variant thereof. The unit dose in the injection device may comprise 120 mg of the IFNAR1 inhibitor, optionally wherein the IFNAR1 inhibitor is anifrolumab or the functional variant thereof. The unit dose in the injection device may consist essentially of >105 mg and <150 mg of the IFNAR1 inhibitor, optionally wherein the IFNAR1 inhibitor is anifrolumab or the functional variant thereof. The unit dose in the injection device may consist essentially of ≤135 mg of the IFNAR1 inhibitor, optionally wherein the IFNAR1 inhibitor is anifrolumab or the functional variant thereof. The unit dose in the injection device may consist essentially of about 120 mg anifrolumab or the or the functional variant thereof. The concentration of anifrolumab or the functional variant thereof in the unit dose in the injection device may be about 150 mg/ml. The volume of the unit dose in the injection device may be less than 1ml. The unit dose in the injection device may have a volume of 0.5 to 1 ml. The concentration of the unit dose may be about 0.8 ml. The volume of the unit dose may be 0.8 ml. The unit dose in the injection device may comprise a formulation of 150 to 200 mg/ml anifrolumab or the functional variant thereof, 25 to 150 mM of lysine salt and an uncharged excipient. The unit dose in the injection device may comprise a formulation of 150 to 200 mg/ml anifrolumab or the functional variant thereof, 25 to 150 mM of lysine salt and an uncharged excipient. The unit dose comprises a formulation of 25 mM histidine-HCL, 130 mM trehalose, and 0.05% w/v polysorbate 80. The formulation may have a pH of about 5.9.

In another aspect the invention relates to an injection device comprising a unit dose. The unit dose may comprise 1150 mg of the IFNAR inhibitor, optionally wherein the IFNAR1 inhibitor is anifrolumab or a functional variant thereof. The concentration of anifrolumab or the functional variant thereof in the unit dose in the injection device may be about 150 mg/ml. The volume of the unit dose in the injection device may be less than 1ml. The unit dose in the injection device may have a volume of 0.5 to 1 ml. The concentration of the unit dose may be about 0.8 ml. The volume of the unit dose may be 0.8 ml. The volume of the unit dose in the injection device may be about 1 ml. The volume of the unit dose in the injection device may be 7.7ml. The unit dose in the injection device may comprise a formulation of 150 to 200 mg/ml anifrolumab or the functional variant thereof, 25 to 150 mM of lysine salt and an uncharged excipient. The unit dose in the injection device may comprise a formulation of 150 to 200 mg/ml anifrolumab or the functional variant thereof, 25 to 150 mM of lysine salt and an uncharged excipient. The unit dose comprises a formulation of 25 mM histidine-HCL, 130 mM trehalose, and 0.05% w/v polysorbate 80. The formulation may have a pH of about 5.9.

The injection device may be a pre-filled syringe (PFS). The injection device may be an accessorized pre-filed syringe (AFPS). The injection device may be an auto-injector (AI).

### 4.5. Kit

In another aspect the invention relates to a kit comprising a unit dose of the invention and instructions for use, wherein the instructions for use comprise instructions for subcutaneous administration of the unit dose to a subject.

In another aspect the invention relates to a kit comprising the pharmaceutical composition for the use of the invention, wherein the instructions for use comprise instructions for subcutaneous administration of the pharmaceutical composition to a subject.

In another aspect the invention relates to a kit comprising the injection device of any of the invention, and instructions for use, wherein the instruction for use comprise instructions for use of the injection device to subcutaneously administer the unit dose or pharmaceutical composition to the subject.

The instructions for use may specify that the injection device, unit dose and/or pharmaceutical composition are for use in the treatment of lupus nephritis. The kit of the invention may comprise packaging, wherein the packaging is adapted to hold the injection device and the instructions for use. The instructions for use may be attached to the injection device. The instruction for use may comprise instructions for administration of >105 mg and <150 mg anifrolumab or functional variant thereof. The instruction for use may comprise instructions for administration of ≤135 mg anifrolumab or the functional variant thereof. The instruction for use may comprise instructions for administration of 120 mg anifrolumab or the functional variant thereof. The instruction for use may comprise instructions for administration of 120 mg anifrolumab or the functional variant thereof every 4 weeks. The instructions for use may define the subject as having a type I IFN mediated disease. The instructions may define the subject as having LN. The instructions for use may be written instructions. The instructions may specify that the unit dose of pharmaceutical composition is for use according a method of the invention. The instruction for use may comprise instructions for subcutaneous administration of anifrolumab or functional variant thereof. The instruction for use may comprise instructions for administration of 1150 mg anifrolumab or functional variant thereof. The instructions for use may define the subject as having a type I IFN mediated disease. The instructions may define the subject as having LN. The instructions for use may be written instructions. The instruction for use may comprise instructions for intravenous administration of anifrolumab or functional variant thereof. The instruction for use may comprise instructions for administration of 900 to 1000 mg anifrolumab or functional variant thereof. The instruction for use may comprise instructions for administration of 900 mg anifrolumab or functional variant thereof. The instructions for use may define the subject as having a type I IFN mediated disease.

### 4.6. Dose regimes

In another aspect the invention relates to an IFNAR1 inhibitor dosage regimen for the treatment of LN. The dosage regimen may comprise a first intensive regime (IR) comprising x3 intravenous 900 mg doses Q4W, followed by basic regime (BR) of a) weekly subcutaneous 120 mg dose, or b) an intravenous 300 mg dose Q4W (or a mixture of a) and b)). The IFNAR1 inhibitor may be anifrolumab or a functional variant thereof.

In another aspect the invention relates to an IFNAR1 inhibitor dosage regimen for the treatment of LN. The dosage regimen may comprise a first intensive regime (IR) comprising x6 intravenous 900 mg doses Q4W, followed by basic regime (BR) of a) weekly subcutaneous 120 mg dose, or b) an intravenous 300 mg dose Q4W (or a mixture of a) and b)). The IFNAR1 inhibitor may be anifrolumab or a functional variant thereof.

In another aspect the invention relates to an IFNAR1 inhibitor dosage regimen for the treatment of LN. The dosage regimen may comprise a first intensive regime (IR) comprising x6 subcutaneous 1150 mg doses Q4W, followed by basic regime (BR) of a) weekly subcutaneous 120 mg dose, or b) an intravenous 300 mg dose Q4W (or a mixture of a) and b)). The IFNAR1 inhibitor may be anifrolumab or a functional variant thereof.

The method may comprise administering intravenously an intravenous dose of an IFNAR1 inhibitor (e.g. anifrolumab or the functional variant thereof to the subject). The intravenous dose may be ≥300 mg of an IFNAR1 inhibitor (e.g. anifrolumab or the functional variant thereof). The intravenous dose may be ≤1000mg of an IFNAR1 inhibitor (e.g. anifrolumab or the functional variant thereof). The intravenous dose may be 900 mg to 1000 mg of an IFNAR1 inhibitor (e.g. anifrolumab or the functional variant thereof). The intravenous dose may be >300 mg of an IFNAR1 inhibitor (e.g. anifrolumab or the functional variant thereof). The intravenous dose may be about 300 mg, about 900 mg or 1000 mg of an IFNAR1 inhibitor (e.g. anifrolumab or the functional variant thereof). The intravenous dose may be 300 mg, 900 mg or 1000 mg of an IFNAR1 inhibitor (e.g. anifrolumab or the functional variant thereof). The intravenous dose may be administered about every four weeks (Q4W). The intravenous dose may be administered about every month. A dose of 300 mg IV dose may be administered using an infusion pump over a minimum of 30 minutes. A dose of 900 mg IV dose may be administered using an infusion pump over a minimum of 60 minutes. An anifrolumab 300 mg IV dose may be supplied as a 2 ml vial, at a concentration of 150 mg/mL.

The method may comprise administering subcutaneously a subcutaneous dose of anifrolumab or the functional variant thereof. The subcutaneous dose may be administered after, before or in between intravenous administration of the intravenous dose. The subcutaneous dose may be >105 mg and <150 mg anifrolumab or the functional variant thereof. The subcutaneous dose may be ≤135 mg anifrolumab or the functional variant thereof. The subcutaneous dose may be about 120 mg anifrolumab or the functional variant thereof. The subcutaneous dose may be administered in a single administration step. The subcutaneous dose may be administered at intervals of 6-8 days. The subcutaneous dose may be administered once per week. The subcutaneous dose may have a volume of 0.5 to 1 ml. The subcutaneous dose may have a volume of 0.5 to 1.0 ml. The subcutaneous dose may have a volume of about 0.8 ml. The subcutaneous dose may have a volume of 0.8 ml. The subcutaneous dose may about 1150 mg (e.g. 1155 or 1150 mg) anifrolumab or the functional variant thereof. The subcutaneous dose may have a volume of about 8 ml. The subcutaneous dose may have a volume of about 7.7ml.

The method may comprise administering to the subject a first dose of a IFNAR1 inhibitor, followed by a second dose of the IFNAR1 inhibitor, wherein the first dose is higher than the second dose. The first dose may be administered intravenously. The first dose may be >300 mg. The first dose may be ≤1000 mg. The first dose may be about 900 mg. The first dose may be administered Q4W. The first dose may be administered to the subject 3 times before the second dose is administered to the subject. The first dose may be administered to the subject 6 times before the second dose is administered to the subject. The first dose may be administered every 4 weeks for 12 weeks before the second dose is administered. The first dose may be administered every 4 weeks for 24 weeks before the second dose is administered. The first dose may be administered subcutaneous. The first dose may be about 1150 mg or 1150 mg. The first dose may be administered Q4W. The first dose may be administered to the subject 3 times before the second dose is administered to the subject. The first dose may be administered to the subject 6 times before the second dose is administered to the subject. The first dose may be administered every 4 weeks for 12 weeks before the second dose is administered. The first dose may be administered every 4 weeks for 24 weeks before the second dose is administered. The intravenous dose may be administered as part of an intensive dosage regime (IR), wherein the total dose of the IFNAR1 inhibitor administered during the IR is 2.7 to 81g, optionally 72.9 g, over a 12 to 24 week period. The IR may comprise administration of a SC dose of the IFNAR1 inhibitor that is equivalent to an IV dose of 900 to 1000 mg Q4W.

The second dose may be administered subcutaneously. The second dose may be >105 mg and ≤135 mg and administer subcutaneously. The second dose may be about 120 mg and administer subcutaneously. The second dose may be administered once per week.

The second dose may be administered intravenously. The second dose may be administered every month. The second dose may be administered Q4W. The second dose may be ≥300 mg. The second dose may be ≤1000mg and administered intravenously. The second dose may be about 300 mg and administered intravenously. The dose may be about 900 mg and administered intravenously Q4W, wherein the second dose is about 120mg administered subcutaneously QW. The dose may be about 900 mg and administered intravenously Q4W, wherein the second dose is about 300 mg administered intravenously Q4W, optionally wherein the first dose is administered to the subject at least 3 times before the second dose is administered to the patient, optionally wherein the first dose is administered to the subject at least 6 times before the second dose is administered to the patient. The second dose may be administered for at least a year.

The method may comprise administering a unit dose or pharmaceutical composition comprising about 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 300, 305, 310, 800, 805, 810, 820, 825, 830, 835, 840, 845, 850, 855, 860, 865, 870, 875, 880, 885, 890, 895, 890, 900, 905, 910, 915, 920, 925, 930, 935, 940, 945, 950, 955, 960, 965, 970, 975, 980, 985, 990, 1000, 1050, 1010, 1020, 1025, 1030, 1035, 1040, 1045, 1050, 1055, 1060, or 1065 mg of an IFNAR1 inhibitor (e.g. anifrolumab or the functional variant thereof).

### 4.7. Unit dose

A unit dose (also referred to as a unit dose form, a pharmaceutical unit dose or a pharmaceutical unit dose form) is a dose formed from a single unit. A unit dose (unit dose form) is suitable for administration to a subject in a single administration step. A unit dose (unit dose form) may be packaged in a single-unit container, for example a single-use pre-filled syringe or autoinjector. Unit doses provide the advantage that they can be ordered, packaged, handled and administered as single dose units containing a pre-determined amount of a drug. Unit doses decrease administration errors and reduce waste.

In another aspect the present invention relates to a unit dose (pharmaceutical unit dose, unit dose form or pharmaceutical unit dose form) for subcutaneous administration comprising >105 mg (i.e. more than 105 mg) and <150 mg (i.e. less than 150 mg) of an IFNAR1 inhibitor (e.g. anifrolumab or a functional variant thereof). The unit dose may comprise 105 mg to 149 mg of an IFNAR inhibitor.

The unit dose may comprise ≤135 mg (i.e. 135 mg or less) of an IFNAR1 inhibitor. The unit dose may comprise 105 mg to 135 mg of an IFNAR inhibitor. The unit dose may comprise about 120 mg of the IFNAR1 inhibitor. The unit dose may comprise 120 mg of the IFNAR1 inhibitor. The unit dose may consist essentially of >105 mg and <150 mg of the IFNAR1 inhibitor. The unit dose may consist essentially of ≤135 mg of the IFNAR1 inhibitor. The unit dose may consist essentially of about of the IFNAR1 inhibitor. The concentration of the IFNAR1 inhibitor in the unit dose may be about 150 mg/ml. The volume of the unit dose may be less than 1ml. The dose or unit dose may have a volume of 0.5 to 1 ml. The concentration of the unit dose may be about 0.8 ml. The volume of the unit dose may be 0.8 ml. The unit dose may comprise a formulation of 150 to 200 mg/ml of the IFNAR1 inhibitor, 25 to 150 mM of lysine salt and an uncharged excipient. The unit dose may comprise a formulation of 150 to 200 mg/ml anifrolumab or the functional variant thereof, 25 to 150 mM of lysine salt and an uncharged excipient. The unit dose comprises a formulation of 25 mM histidine-HCL, 130 mM trehalose, and 0.05% w/v polysorbate 80. The formulation may have a pH of about 5.9.

The unit dose may comprise ≤135 mg (i.e. 135 mg or less) of an IFNAR1 inhibitor (e.g. anifrolumab or the functional variant thereof). The unit dose may comprise about 120 mg of an IFNAR1 inhibitor (e.g. anifrolumab or the functional variant thereof). The unit dose may comprise 120 mg of an IFNAR1 inhibitor (e.g. anifrolumab or the functional variant thereof. The unit dose may consist essentially of >105 mg and <150 mg anifrolumab or the functional variant thereof). The unit dose may consist essentially of ≤135 mg of an IFNAR1 inhibitor (e.g. anifrolumab or the functional variant thereof). The unit dose may consist essentially of about 120 mg of an IFNAR1 inhibitor (e.g. anifrolumab or the or the functional variant thereof). The concentration of an IFNAR1 inhibitor (e.g. anifrolumab or the functional variant thereof) in the unit dose may be about 150 mg/ml. The volume of the unit dose may be less than 1ml. The dose or unit dose may have a volume of 0.5 to 1 ml. The concentration of the unit dose may be about 0.8 ml. The volume of the unit dose may be 0.8 ml. The unit dose may comprise a formulation of 150 to 200 mg/ml anifrolumab or the functional variant thereof, 25 to 150 mM of a lysine salt and an uncharged excipient. The unit dose may comprise a formulation of 150 to 200 mg/ml anifrolumab or the functional variant thereof, 25 to 150 mM of a lysine salt and an uncharged excipient. The unit dose comprises a formulation of 25 mM histidine-HCL, 130 mM trehalose, and 0.05% w/v polysorbate 80. The formulation may have a pH of about 5.9. The unit dose may comprise 1150 or 1155mg of anifrolumab or the functional variant thereof.

The dose or unit dose may be 105 mg, 106 mg, 107 mg, 108 mg, 109 mg, 110 mg, 111 mg, 112 mg, 113 mg, 114 mg, 115 mg, 116 mg, 117 mg, 118 mg, 119 mg, 120 mg, 121 mg, 122 mg, 123 mg, 124 mg or 125 mg, 126 mg, 127 mg, 128 mg, 129 mg, 130 mg, 131 mg, 132 mg, 133 mg, 134 mg, 135 mg, 136 mg, 137 mg, 138 mg, 139 mg, 140 mg, 141 mg, 142 mg, 143 mg, 144 mg, 145 mg, 146 mg, 147 mg, 148 mg, or 149 mg, 150 mg, 151 mg, 152 mg, 153 mg, 154 mg, 155 mg, 156 mg, 157 mg, 158 mg, 159 mg, 160 mg, 161 mg, 162 mg, 163 mg, 164 mg, 165 mg, 166 mg, 167 mg, 168 mg, 167 mg 168 mg, 169 mg, 170 mg, 171 mg, 172 mg, 173 mg, 174 mg, 175 mg, 176 mg, 177 mg, 178 mg, 179 mg, 180 mg, 181, mg, 182 mg, 183 mg, 184 mg, 185 mg, 186 mg, 187 mg, 188 mg, 189 mg, 190 mg, 191 mg, 192 mg, 193 mg, 194 mg, 195 mg, 196 mg, 197 mg, 198 mg, 199 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 300 mg, 305 mg, 310 mg, 800 mg, 805 mg, 810 mg, 820 mg, 825 mg, 830 mg, 835 mg, 840 mg, 845 mg, 850 mg, 855 mg, 860 mg, 865 mg, 870 mg, 875 mg, 880 mg, 885 mg, 890 mg, 895 mg, 890 mg, 900 mg, 905 mg, 910 mg, 915 mg, 920 mg, 925 mg, 930 mg, 935 mg, 940 mg, 945 mg, 950 mg, 955 mg, 960 mg, 965 mg, 970 mg, 975 mg, 980 mg, 985 mg, 990 mg, 1000 mg, 1050 mg, 1010 mg, 1020 mg, 1025 mg, 1030 mg, 1035 mg, 1040 mg, 1045 mg, 1050 mg, 1051 mg, 1052 mg, 1053 mg, 1054 mg, 1055 mg, 1056 mg, 1057 mg, 1058 mg, 1059 mg, 1060 mg, 1061 mg, 1062 mg, 1063 mg, 1064 mg, or 1065 mg of an IFNAR1 inhibitor (e.g. anifrolumab or the functional variant thereof).

In another aspect, the invention relates to a method for treating LN in a subject, the method of treatment comprising subcutaneously administering the unit dose of the invention to a subject having LN. In another aspect the invention relates to a method of treating a LN in a subject, the method comprising subcutaneously administering a dose of anifrolumab or a functional variant thereof, wherein the dose is >105 mg and <150 mg.

In another aspect, the invention relates to a method of treating LN in a subject, the method comprising subcutaneously administering a dose of anifrolumab or a functional variant thereof, wherein administering the dose every week provides a plasma concentration in the subject that is at least equivalent to the plasma concentration provided by intravenous administration of 300 mg of anifrolumab or the functional variant thereof every 4 weeks. Administering the dose every week may provide a plasma concentration in the subject that is more than the plasma concentration provided by intravenous administration of 300 mg of anifrolumab or the functional variant thereof every 4 weeks. Administering the dose every week may provide a plasma concentration in the subject that is at least equivalent to the plasma concentration provided by intravenous administration of 400 mg of anifrolumab or the functional variant thereof every 4 weeks. The dose may be administered in a single-administration step. The dose administered to the subject may be <150 mg (i.e. less than 150 mg) of an IFNAR1 inhibitor (e.g. anifrolumab or the functional variant thereof). The dose administered to the subject may be >105 mg (i.e. more than 105 mg) of an IFNAR1 inhibitor (e.g. anifrolumab or the functional variant thereof). The dose administered to the subject may be ≤135 mg (i.e. 135 mg or less) of an IFNAR1 inhibitor (e.g. anifrolumab or the functional variant thereof). The dose administered to the subject may be about 120 mg of an IFNAR1 inhibitor (e.g.

anifrolumab or the functional variant thereof).

The methods of the invention may comprise administering the dose or unit dose at intervals of 6-8 days. The dose or unit dose may be administered once per week (QW). The dose or unit dose may be 120 mg anifrolumab or the functional variant thereof, wherein the method comprises administering the dose in a single administration step once per week (QW). In other words, the method comprises administering 120 mg QW of anifrolumab of the functional variant thereof. The dose or unit dose may be administered once per week for at least about 4 weeks. The dose or unit dose may be administered once per week for at least about 8 weeks. The dose or unit dose may be administered once per week for at least about 12 weeks. The dose or unit dose may be administered once per week for at least about 16 weeks. The dose or unit dose may be administered once per week for at least about 20 weeks. The dose or unit dose may be administered once per week for at least about 24 weeks. The dose or unit dose may be administered once per week for at least about 28 weeks. The dose or unit dose may be administered once per week for at least about 32 weeks. The dose or unit dose may be administered once per week for about 8 weeks. The dose or unit dose may have a volume suitable for delivery in a single subcutaneous administration step. The dose or unit dose may have a volume of 0.5 to 1 ml. The dose or unit dose may have a volume of less than 1 ml. The dose or unit dose may have a volume of about 0.8 ml.

Administration of the dose or unit dose may provide a plasma concentration of anifrolumab or the functional variant thereof in the patient of ≥ 10 μg (i.e. 10 μg or more) anifrolumab or the functional variant thereof per ml of plasma (i.e. a plasma concentration of ≥ 10 μg/ml). Administration of the dose or unit dose may provide a plasma concentration of anifrolumab or the functional variant thereof in the subject of about 10-100 μg/ml. Administration of the dose or unit dose may provide a plasma concentration of anifrolumab or the functional variant thereof in the subject of 20-80 μg/ml. Administration of the dose or unit dose may provide a plasma concentration of anifrolumab or the functional variant thereof in the subject of 30-70 μg/ml. Administration of the dose or unit dose may provide a trough concentration of anifrolumab or the functional variant thereof in the subject of ≥ 20 μg/ml (i.e. 20 μg/ml or more). Administration of the dose or unit dose may provide a trough concentration of an IFNAR1 inhibitor (e.g. anifrolumab or the functional variant thereof in the subject of ≥ 30 μg/ml (i.e. 30 μg/ml or more). Administration of the dose or unit dose may provide a trough concentration of an IFNAR1 inhibitor (e.g. anifrolumab or the functional variant thereof in the subject of ≥ 40 μg/ml (i.e. 40 μg/ml or more)). Administration of the dose or unit dose may provide a trough concentration of anifrolumab or the functional variant thereof in the subject of 20-100 μg/ml. Administration of the dose or unit dose may provide a trough concentration of anifrolumab or the functional variant thereof in the subject of about 30-80 μg/ml. Administration of the dose or unit dose may provide a trough concentration of anifrolumab or the functional variant thereof in the subject of 40-70 μg/ml.

The dose or unit dose may provide a therapeutic effect in the subject that is at least equivalent to a therapeutic effect provided by administration of an intravenous dose of 300 mg of an IFNAR1 inhibitor (e.g. anifrolumab or the functional variant thereof) administered once every (Q4W). The dose or unit dose may provide a trough concentration of anifrolumab or the functional variant thereof in the subject that is greater than a trough concentration of anifrolumab or the functional variant thereof provided by administration of an intravenous dose of 300 mg anifrolumab or the functional variant thereof once every 4 weeks (Q4W).

### 4.8. IFNAR1 inhibitor

A "type I interferon receptor inhibitor" refers to a molecule that is antagonistic for the receptor of type I interferon ligands such as interferon-a and interferon-β (IFNAR, IFNAR1). Such inhibitors, subsequent to administration to a patient, preferably provide a reduction in the expression of at least 1 (preferably at least 4) pharmacodynamic (PD) marker genes selected from the group consisting of IFI6, RSAD2, IFI44, IFI44L, IFI27, MX1, IFIT1, HERC5, ISG15, LAMP3, OAS3, OAS1, EPST1, IFIT3, LY6E, OAS2, PLSCR1, SIGLECI, USP18, RTP4, and DNAPTP6. The at least 4 genes may suitably be IFI27, IFI44, IFI44L, and RSAD2. The "type I interferon receptor" is preferably interferon-α/β receptor (IFNAR).

For example, the type I interferon receptor inhibitor may be an antibody or antigen-binding fragment thereof that inhibits type I IFN activity (by inhibiting the receptor). An example of a suitable antibody or antigen-binding fragment thereof (that inhibits type I IFN activity) is an interferon-α/β receptor (IFNAR) antagonist. The type I interferon receptor inhibitor may be an antibody or antigen-binding fragment thereof that inhibits type I IFN activity. Additionally or alternatively, the type I interferon receptor inhibitor may be a small molecule inhibitor of a type I interferon receptor (e.g. for pharmacological inhibition of type I interferon receptor activity).

The IFNAR1 inhibitor may be a human monoclonal antibody specific for IFNAR1. The IFNAR1 inhibitor may be a modified IgG1 class human monoclonal antibody specific for IFNAR1.

The antibody may comprise a heavy chain variable region complementarity determining region 1 (HCDR1) comprising the amino acid sequence of SEQ ID NO: 3. The antibody may comprise a heavy chain variable region complementarity determining region 2 (HCDR2) comprising the amino acid sequence of SEQ ID NO: 4. The antibody may comprise a heavy chain variable region complementarity determining region 3 (HCDR3) comprising the amino acid sequence of SEQ ID NO: 5. The antibody may comprise a light chain variable region complementarity determining region 1 (LCDR1) comprising the amino acid sequence SEQ ID NO: 6 The antibody may comprise a light chain variable region complementarity determining region 2 (LCDR2) comprising the amino acid sequence SEQ ID NO: 7. The antibody may comprise a light chain variable region complementarity determining region 3 (LCDR3) comprising the amino acid sequence SEQ ID NO: 8.

The antibody may comprise a human heavy chain variable region comprising the amino acid sequence of SEQ ID NO: 1. The antibody may comprise a human light chain variable region comprising the amino acid sequence of SEQ ID NO: 2. The antibody may comprise a human light chain constant region comprising the amino acid sequence of SEQ ID NO: 9. The antibody may comprise a human heavy chain constant region comprising the amino acid sequence of SEQ ID NO: 10. The antibody may comprise in the Fc region an amino acid substitution of L234F, as numbered by the EU index as set forth in Kabat and wherein said antibody exhibits reduced affinity for at least one Fc ligand compared to an unmodified antibody. The antibody may comprise a human heavy chain comprising the amino acid sequence of SEQ ID NO: 11. The antibody may comprise a human light chain comprising the amino acid sequence of SEQ ID NO: 12.

The antibody may comprise: (a) a heavy chain variable region complementarity determining region 1 (HCDR1) comprising the amino acid sequence of SEQ ID NO: 3;(b) a heavy chain variable region complementarity determining region 2 (HCDR2) comprising the amino acid sequence of SEQ ID NO: 4; c) a heavy chain variable region complementarity determining region 3 (HCDR3) comprising the amino acid sequence of SEQ ID NO: 5; (d) a light chain variable region complementarity determining region 1 (LCDR1) comprising the amino acid sequence SEQ ID NO: 6; (e) a light chain variable region complementarity determining region 2 (LCDR2) comprising the amino acid sequence SEQ ID NO: 7; and f) a light chain variable region complementarity determining region 3 (LCDR3) comprising the amino acid sequence SEQ ID NO: 8.

The antibody may comprise (a) a human heavy chain comprising the amino acid sequence of SEQ ID NO: 11; and (b) a human light chain comprising the amino acid sequence of SEQ ID NO: 12.

The IFNAR1 inhibitor may be anifrolumab or a functional variant thereof.

### 4.9. Formulations

The anifrolumab or the functional variant thereof may be comprised within a pharmaceutical composition. The pharmaceutical composition may comprise 150 to 200 mg/ml anifrolumab or the functional variant thereof, 25 to 150 mM of a lysine salt and an uncharged excipient. The pharmaceutical composition may comprise 150 mg/mL anifrolumab or the functional variant thereof. The pharmaceutical composition may comprise 50 mM lysine HCl. The pharmaceutical composition may comprise 130 mM trehalose dihydrate. The pharmaceutical composition may comprise 0.05% polysorbate 80 or polysorbate 20. The pharmaceutical composition may comprise 25 mM histidine/histidine HCl. The pharmaceutical composition may comprise 150 mg/mL anifrolumab or the functional variant thereof, 50 mM lysine HCl, 130 mM trehalose dihydrate, 0.05% polysorbate 80 and 25 mM histidine/histidine HCl.

Stable formulations suitable for administration to subjects and comprising anifrolumab are described in detail in US patent 10125195 B1, which is incorporated herein in its in entirety.

## 5. DEFINITIONS

### 5.1. IFNAR inhibitors

Anifrolumab (MEDI-546, anifro, ANI) is a human immunoglobulin G1 kappa (IgG1κ) monoclonal antibody (mAb) directed against subunit 1 of the type I interferon receptor (IFNAR1, IFN-αR1, IFNAR). Anifrolumab downregulates IFNAR signaling and suppresses expression of IFN-inducible genes. Disclosures related to anifrolumab can be found in U.S. Patent No. 7662381 and U.S. Patent No. 9988459, which are incorporated herein by reference in their entirety. The sequence information for anifrolumab is provided in the WHO recommended INN: List 71 (WHO Drug information, Vol. 28, No. 1, 2014) and Peng *et al.* [13]. Sequence information for anifrolumab is also provided in **Table 1** and **Figure 44** and **Figure 45.**

**Table 1: Sequences**

| | |
|---|---|
| Anifrolumab VH (SEQ ID NO: 1) | EVQLVQSGAEVKKPGESLKISCKGSGYIFT**NYWIA**WVRQMPGKGLESMG**IIYPGDSDIRYSPSFQG**QVTISADKSITTAYLQWSSLKASDTAMYYCAR**HDIEGFDY**WGRGTLVTVSS |
| Anifrolumab VK (SEQ ID NO: 2) | EIVLTQSPGTLSLSPGERATLSC**RASQSVSSSFFA**WYQQKPGQAPRLLIY**GASSRAT**GIPDRLSGSGSGTDFTLTITRLEPEDFAVYYC**QQYDSSAIT**FGQGTRLEIK |
| HCDR1 (SEQ ID NO: 3) | NYWIA |

(continued)

| | |
|---|---|
| HCDR2 (SEQ ID NO: 4) | IIYPGDSDIRYSPSFQG |
| HCDR3 (SEQ ID NO: 5) | HDIEGFDY |
| LCDR1 (SEQ ID NO: 6) | RASQSVSSSFFA |
| LCDR2 (SEQ ID NO: 7) | GASSRAT |
| LCDR3 (SEQ ID NO: 8) | QQYDSSAIT |
| Light chain constant region (SEQ ID NO: 9) | RTVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC |
| Heavy chain constant region (SEQ ID NO: 10) | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKRVEPKSCDKTHTCP PCPAPEFEGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPASIEKTIS KAKGQPREPQVYTLPPSREEMTKNQVSLTCLVKGFYPSDIAVEWESNGQPENNYK TTPPVLDSDGSFFLYSKLTVDKSRWQQGNVFSCSVMHEALHNHYTQKSLSLSPGK |
| Heavy chain (SEQ ID NO: 11) | EVQLVQSGAEVKKPGESLKISCKGSGYIFTNYWIAWVRQMPGKGLESMGIIYPGD SDIRYSPSFQGQVTISADKSITTAYLQWSSLKASDTAMYYCARHDIEGFDYWGRG TLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKRVEPKSC DKTHTCPPCPAPEFEGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPA SIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQKS LSLSPGK |
| Light chain (SEQ ID NO: 12) | EIVLTQSPGTLSLSPGERATLSCRASQSVSSSFFAWYQQKPGQAPRLLIYGASSR ATGIPDRLSGSGSGTDFTLTITRLEPEDFAVYYCQQYDSSAITFGQGTRLEIKRT VAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |

Anifrolumab is an immunoglobulin comprising an HCDR1, HCDR2 and HCDR3 of SEQ ID NO: 3, SEQ ID NO: 4, and SEQ ID NO: 5, respectively (or functional variant thereof); and an LCDR1, LCDR2 and LCDR3 of SEQ ID NO: 6, SEQ ID NO: 7, and SEQ ID NO: 8, respectively (or functional variant thereof). Anifrolumab is an immunoglobulin comprising a VH of SEQ ID NO: 1 and a VL of SEQ ID NO: 2.

The constant region of anifrolumab has been modified such that anifrolumab exhibits reduced affinity for at least one Fc ligand compared to an unmodified antibody. Anifrolumab is a modified IgG class monoclonal antibody specific for IFNAR1 comprising in the Fc region an amino acid substitution of L234F, as numbered by the EU index as set forth in Kabat (1991, NIH Publication 91-3242, National Technical Information Service, Springfield, Va.). Anifrolumab is a modified IgG class monoclonal antibody specific for IFNAR1 comprising in the Fc region an amino acid substitution of L234F, L235E and/or P331S, as numbered by the EU index as set forth in Kabat (1991, NIH Publication 91-3242, National Technical Information Service, Springfield, Va.). Anifrolumab is an antibody comprising a light chain constant region of SEQ ID NO: 9. Anifrolumab is an antibody comprising a heavy chain constant region of SEQ ID NO: 10. Anifrolumab is an antibody comprising a light chain constant region of SEQ ID NO: 9 and a heavy chain constant region of SEQ ID NO: 10. Anifrolumab is an antibody comprising a heavy chain of SEQ ID NO: 11. Anifrolumab is an antibody comprising a light chain of SEQ ID NO: 12. Anifrolumab is an antibody comprising a heavy chain of SEQ ID NO: 11 and a light chain of SEQ ID NO: 12.

Functional variants of anifrolumab are sequence variants that perform the same function as anifrolumab. Functional variants of anifrolumab are variants that bind the same target as anifrolumab and have the same effector function as anifrolumab. Functional variants of anifrolumab are variants that bind the same target as anifrolumab and have the same effector function as anifrolumab with the same or greater affinity. Functional anifrolumab variants include antigen-binding fragments of anifrolumab and antibody and immunoglobulin derivatives of anifrolumab. Functional variants include biosimilars and interchangeable products. The terms biosimilar and interchangeable product are defined by the FDA and EMA. The term biosimilar refers to a biological product that is highly similar to an approved (e.g. FDA approved) biological product (reference product, e.g. anifrolumab) in terms of structure and has no clinically meaningful differences in terms of pharmacokinetics, safety and efficacy from the reference product. The presence of clinically meaningful differences of a biosimilar may be assessed in human pharmacokinetic (exposure) and pharmacodynamic (response) studies and an assessment of clinical immunogenicity. An interchangeable product is a biosimilar that is expected to produce the same clinical result as the reference product in any given patient.

For example, a variant of the reference (anifrolumab) antibody may comprise: a heavy chain CDR1 having at most 2 amino acid differences when compared to SEQ ID NO: 3; a heavy chain CDR2 having at most 2 amino acid differences when compared to SEQ ID NO: 4; a heavy chain CDR3 having at most 2 amino acid differences when compared to SEQ ID NO: 5; a light chain CDR1 having at most 2 amino acid differences when compared to SEQ ID NO: 6; a light chain CDR2 having at most 2 amino acid differences when compared to SEQ ID NO: 7; and a light chain CDR3 having at most 2 amino acid differences when compared to SEQ ID NO: 8; wherein the variant antibody binds to the target of anifrolumab (e.g. IFNAR) and preferably with the same affinity.

A variant of the reference (anifrolumab) antibody may comprise: a heavy chain CDR1 having at most 1 amino acid difference when compared to SEQ ID NO: 3; a heavy chain CDR2 having at most 1 amino acid difference when compared to SEQ ID NO: 4; a heavy chain CDR3 having at most 1 amino acid difference when compared to SEQ ID NO: 5; a light chain CDR1 having at most 1 amino acid differences when compared to SEQ ID NO: 6; a light chain CDR2 having at most 1 amino acid difference when compared to SEQ ID NO: 7; and a light chain CDR3 having at most 1 amino acid difference when compared to SEQ ID NO: 8; wherein the variant antibody binds to the target of anifrolumab (e.g. IFNAR) optionally with the same affinity.

A variant antibody may have at most 5, 4 or 3 amino acid differences total in the CDRs thereof when compared to a corresponding reference (anifrolumab) antibody, with the proviso that there is at most 2 (optionally at most 1) amino acid differences per CDR. A variant antibody may have at most 2 (optionally at most 1) amino acid differences total in the CDRs thereof when compared to a corresponding reference (anifrolumab) antibody, with the proviso that there is at most 2 amino acid differences per CDR. A variant antibody may have at most 2 (optionally at most 1) amino acid differences total in the CDRs thereof when compared to a corresponding reference (anifrolumab) antibody, with the proviso that there is at most 1 amino acid difference per CDR.

A variant antibody may have at most 5, 4 or 3 amino acid differences total in the framework regions thereof when compared to a corresponding reference (anifrolumab) antibody, with the proviso that there is at most 2 (optionally at most 1) amino acid differences per framework region. Optionally a variant antibody has at most 2 (optionally at most 1) amino acid differences total in the framework regions thereof when compared to a corresponding reference (anifrolumab) antibody, with the proviso that there is at most 2 amino acid differences per framework region. Optionally a variant antibody has at most 2 (optionally at most 1) amino acid differences total in the framework regions thereof when compared to a corresponding reference (anifrolumab) antibody, with the proviso that there is at most 1 amino acid difference per framework region.

A variant antibody may comprise a variable heavy chain and a variable light chain as described herein, wherein: the heavy chain has at most 14 amino acid differences (at most 2 amino acid differences in each CDR and at most 2 amino acid differences in each framework region) when compared to a heavy chain sequence herein; and the light chain has at most 14 amino acid differences (at most 2 amino acid differences in each CDR and at most 2 amino acid differences in each framework region) when compared to a light chain sequence herein; wherein the variant antibody binds to the same target antigen as the reference (anifrolumab) antibody (e.g. IFNAR) and preferably with the same affinity.

The variant heavy or light chains may be referred to as "functional equivalents" of the reference heavy or light chains. A variant antibody may comprise a variable heavy chain and a variable light chain as described herein, wherein: the heavy chain has at most 7 amino acid differences (at most 1 amino acid difference in each CDR and at most 1 amino acid difference in each framework region) when compared to a heavy chain sequence herein; and the light chain has at most 7 amino acid differences (at most 1 amino acid difference in each CDR and at most 1 amino acid difference in each framework region) when compared to a light chain sequence herein; wherein the variant antibody binds to the same target antigen as the reference (anifrolumab) antibody (e.g. IFNAR) and preferably with the same affinity.

Functional variants of anifrolumab include the antibodies described in WO 2018/023976 A1, incorporated herein by reference (**Table 2**).

**Table 2: anti-IFNAR antibody sequences**

| | | | | |
|---|---|---|---|---|
| H15D10 | | VH | EVQLVQSGAE VKKPGESLRISC KGSGYTFT NYWVAWVRQM PGKGLESMGI IYPGDSDTRY SPSFQGHVTI SADKSISTAY | SEQ ID NO: 13 |

(continued)

| | | | | |
|---|---|---|---|---|
| L8C3 | | VL | DIQMTQSPSSLSASLGDRVTITCRASQN VGNYLNWYQQKPGKAPKLLIYRASNLAS GVPSRFSGSGSGTDFTLTISSLQPEDFA TYYCQQMEHAPPTFGQGTKVEIKR | SEQ ID NO: 14 |
| L16C11 | | VL | EIVLTQSPGTLSLSPGERATLSCRASQS VIGYYLAWYQQKPGQAPRLLIYSVSTLA SGIPDRFSGSGSGTDFTLTISRLEPEDF AVYYCQQYYRFPITFGQGTKVEIK | SEQ ID NO: 15 |
| H19B7 | | VH | EVQLVQSGAE VKKPGESLRI SCKGSGYTFT NYWMAWVRQM PGKGLESMGI IYPSDSDTRY SPSFQGHVTI SADKSISTAY LQWSSLKASD TAMYYCARHD VEGYDYWGQG TLVTVSS | SEQ ID NO: 16 |

Functional variants include antibodies comprising the VH amino acid sequence SEQ ID NO: 13. Functional variants include antibodies comprising the VH amino acid sequence SEQ ID NO: 16. Functional variants include antibodies comprising the VL amino acid sequence SEQ ID NO: 14. Functional variants include antibodies comprising the VL amino acid sequence SEQ ID NO: 15. Functional variants include antibodies comprising the VL amino acid sequence SEQ ID NO: 16. Functional variants include antibodies comprising the VH sequence SEQ ID NO: 13 and VL amino acid sequence SEQ ID NO: 16. Functional variants include antibodies comprising the VH sequence SEQ ID NO: 13 and VL amino acid sequence SEQ ID NO: 15. Functional variants include antibodies comprising the VH sequence SEQ ID NO: 16 and VL amino acid sequence SEQ ID NO: 15. Functional variants include antibodies comprising the VH sequence SEQ ID NO: 16 and VL amino acid sequence SEQ ID NO: 14.

IFNAR inhibitors may be a monoclonal antibody comprising the VH amino acid sequence SEQ ID NO: 13. The anti-IFNAR antibodies may comprise the VH amino acid sequence SEQ ID NO: 16. The anti-IFNAR antibodies may comprise the VL amino acid sequence SEQ ID NO: 14. The anti-IFNAR antibodies may comprise the VL amino acid sequence SEQ ID NO: 15. The anti-IFNAR antibodies may comprise the VL amino acid sequence SEQ ID NO: 16. The anti-IFNAR antibodies may comprise the VH sequence SEQ ID NO: 13 and VL amino acid sequence SEQ ID NO: 16. The anti-IFNAR antibodies may comprise the VH sequence SEQ ID NO: 13 and VL amino acid sequence SEQ ID NO: 15. The anti-IFNAR antibodies may comprise the VH sequence SEQ ID NO: 16 and VL amino acid sequence SEQ ID NO: 15. The anti-IFNAR antibodies may comprise the VH sequence SEQ ID NO: 16 and VL amino acid sequence SEQ ID NO: 14.

Functional variants of anifrolumab and anti-IFNAR antibodies include the QX006N antibody described in CN 11327807, incorporated herein by reference.

**Table 3: QX006N antibody sequences**

| | |
|---|---|
| VH (SEQ ID NO: 17) | EVQLVESGGGLVQPGGSLRLSCAASGFSLSSYYMTWVRQAPGKGLEWVSV INVYGGTYYASWAKGRFTISRDNSKNTLYLQMNSLRAEDTAVYYCAREDV AVYMAIDLWGQGTLVTVSS |
| VL (SEQ ID NO: 18) | AIQMTQSPSSLSASVGDRVTITCQASQSISNQLSWYQQKPGKAPKLLIYD ASSLASGVPSRFSGSRSGTKFTLTISSLQPEDFATYYCLGIYGDGADDGI AFGGGTKVEIK |
| HCDR1 (SEQ ID NO: 19) | SYYMT |
| HCDR2 (SEQ ID NO: 20) | VINVYGGTYYASWAKG |
| HCDR3 (SEQ ID NO: 21) | EDVAVYMAIDL |
| LCDR1 (SEQ ID NO: 22) | QASQSISNQLS |
| LCDR2 (SEQ ID NO: 23) | DASSLAS |
| LCDR3 (SEQ ID NO: 24) | LGIYGDGADDGIA |

IFNAR inhibitors may be a monoclonal antibody comprising the VH amino acid sequence SEQ ID NO: 17. The anti-IFNAR antibodies may comprise the VL amino acid sequence SEQ ID NO: 18.

QX006N is an immunoglobulin comprising an HCDR1, HCDR2 and HCDR3 of SEQ ID NO: 19, SEQ ID NO: 20, and SEQ ID NO: 21, respectively (or functional variant thereof); and an LCDR1, LCDR2 and LCDR3 of SEQ ID NO: 22, SEQ ID NO: 23, and SEQ ID NO: 23, respectively (or functional variant thereof). QX006N is an immunoglobulin comprising a VH amino acid sequence SEQ ID NO: 17 the VL amino acid sequence SEQ ID NO: 18.

### 5.2. Anifrolumab clinical studies

| Study ID | Status | Study design and type of control | Objectives | Subjects | Doses |
|---|---|---|---|---|---|
| **Phase I** | | | | | |
| MI-CP180 | Completed | Open-label, dose escalation (SAD/-MAD) | **Primary:** Safety and tolerability<br>**Secondary:** PK, immunogenicity, PD | Patients with SSc | IV single dose: 0.1 mg/kg - 20 mg/kg<br>IV multiple dose: 0.3 mg/kg - 5.0 mg/kg |
| study 06 | Completed | Randomized, double-blind, placebo controlled | **Primary:** PK, safety, and tolerability<br>**Secondary:** Immunogenicity | Healthy volunteers | SC single dose 300 mg or placebo (for 300 mg injection)<br>600 mg or placebo (for 600 mg injection)<br>IV single dose 300 mg or placebo (for 300 mg injection) |
| **Phase II** | | | | | |
| study 1013 | Completed | Randomized (1:1:1), double-blind, placebo controlled | **Primary:** SRI(4) at Week 24<br>**Secondary:** SRI(4) at Week 52, OCS reduction, safety, PK, immunogenicity, PD | Patients with SLE who are receiving standard of care | IV, Q4W 300 mg, 1000 mg, or placebo |
| study 07 | Completed | Randomized (1:1:1), double-blind, placebo controlled | **Primary:** UPCR at Week 52<br>**Secondary:** CRR at Week 52 safety and tolerability | Patients with active proliferative LN while receiving SOC | IV, Q4W 300 mg, 900 mg for first 3 doses followed by 300 mg, or placebo |
| study 08 | Completed | Randomized (3:1:3:1), double-blind (through week 12) placebo controlled | **Primary:** PK and PD<br>**Secondary:** Safety and tolerability, immunogenicity | Type I IFNGS test-high SLE patients with active skin manifestations while receiving SOC | SC, Q2W 150 mg, 300 mg, or placebo |

(continued)

| Phase III | | | | | |
|---|---|---|---|---|---|
| study 05 | Completed | Randomized (1:2:2), double-blind, placebo controlled | **Primary:** SRI(4) at Week 52 **Secondary:** SRI(4) in type I IFNGS test-high patients, OCS reduction, CLASI reduction, SRI(4) at Week 24, annualized flare rate Safety and tolerability | Patients with moderate to severe SLE who were receiving SOC | IV, Q4W 150 mg, 300 mg, or placebo |
| study 04 | Completed | Randomized (1:1), double-blind, placebo controlled | **Primary:** BICLA at Week 52<br><br>**Secondary:** BICLA in type I IFNGS test-high patients, OCS reduction, CLASI reduction, joint count reduction, annualized flare rate Safety and tolerability | Patients with moderate to severe SLE who were receiving SOC) | IV, Q4W 300 mg or placebo |
| study 09 | Ongoing | Randomized (~ 4:1), double-blind, placebo controlled | **Primary:** Safety and tolerability | Patients with SLE who completed study 04 or study 05 | IV, Q4W 300 mg or placebo Patients on anifrolumab in studies 04 and 05 continued on 300 mg, placebo patients from studies 04 and 05 randomized 1:1 to anifrolumab 300 mg or placebo |
| (TULIP SC) | Ongoing | Randomized (1:1), double blind, placebo controlled | **Primary:** BICLA at Week 52 **Secondary:** time to BICLA, sustained OCS reduction, annualized flare rate, BICLA at Week 16 Safety and tolerability | Adult patients with moderatetosevere systemic SLE who were receiving standard of care | SC, QW 120 mg or placebo |

### 5.3. Lupus nephritis (LN)

Presence of proteinuria, haematuria or urine granular casts, or unexplained decrease in renal function are all clinical signs of renal involvement in SLE patients. LN diagnosis is made by renal biopsy and histopathological classification according to the 2003 ISN/RPS classification criteria [3] which are the gold standard for renal assessment in LN.

LN is histologically classified as one of Classes I-V [3,14] (**Table 4**).

**Table 4: The 2003 ISN/RPS classification of LN**

| Class | Definition | Clinical findings | Subclasses |
|---|---|---|---|
| I | Minimal mesangial LN | Normal glomeruli by LM, but mesangial immune deposits by IF | None |
| II | Mesangial proliferative LN | Purely mesangial hypercellularity of any degree or mesangial matrix expansion by LM, with mesangial immune deposits. A few isolated subepithelial or subendothelial deposits may be visible by IF or EM, but not by LM | None |
| III | Focal LN | Active or inactive focal, segmental or global endocapillary or extracapillary glomerulonephritis involving <50% of all glomeruli, typically with focal subendothelial immune deposits, with or without mesangial alterations | III (A): active lesions; focal proliferative LN |
| | | | III (A/C): active and chronic lesions; focal proliferative and sclerosing LN |
| | | | III (C): chronic inactive lesions with glomerular scars; focal sclerosing LN |
| IV | Diffuse LN | Active or inactive diffuse, segmental or global endocapillary or extracapillary glomerulonephritis involving ≥50% of all glomeruli, typically with diffuse subendothelial immune deposits, with or without mesangial alterations | IV-S[a] (A): active lesions; diffuse segmental proliferative LN |
| | | | IV-G[b] (A): active lesions; diffuse global proliferative LN |
| | | | IV-S[a] (A/C): active and chronic lesions; diffuse segmental proliferative and sclerosing LN |
| | | | IV-G[b] (A/C): active and chronic lesions; diffuse global proliferative and sclerosing LN |
| | | | IV-S[a] (C): chronic inactive lesions with scars; diffuse segmental sclerosing LN |
| | | | IV-G[b] (C): chronic inactive lesions with scars; diffuse global sclerosing LN |
| V | Membranous LN | Global or segmental subepithelial immune deposits or their morphological sequelae by LM and by IF or EM, with or without mesangial alterations. May occur in combination with class III or IV, in which case both classes are diagnosed. May show advanced sclerosis | None |

(continued)

| Class | Definition | Clinical findings | Subclasses |
|---|---|---|---|
| VI | Advanced sclerotic LN | ≥90% of glomeruli globally sclerosed without residual activity | None |
| IF, immunofluorescence; LM, light microscopy; LN, lupus nephritis. [a]Diffuse segmental LN indicates that 50% or more of the involved glomeruli have segmental lesions (that is, glomerular lesions involving less than 50% of the glomerular tuft). [b]Diffuse global LN indicates that 50% or more of the involved glomeruli have global lesions (that is, glomerular lesions involving 50% or more of the glomerular tuft). | | | |

Until very recently there has not been any approved treatment for Lupus Nephritis (LN). The 2 newly approved treatments (Benlysta™ and Lupkynis™-approved in US, MAA under review by EMA) are both approved for use as add on therapy to background Standard of Care (SoC) with mycophenolate mofetil (MMF) which in itself is not approved for LN in most regions, but is widely used as SoC in accordance with international guidelines. The standard treatment for proliferative LN consists of 6 to 12 months of intensive immunosuppressive therapy (usually cyclophosphamide or mycophenolate mofetil [MMF] with high dose corticosteroids) followed by a longer period of less intensive maintenance therapy with MMF or azathioprine and low dose steroids. MMF is the recommended by American College of Rheumatology (ACR) and the 2019 Update of the Joint European League Against Rheumatism and European Renal Association European Dialysis and Transplant Association (EULAR/ERA-EDTA) recommendations for the management of lupus nephritis with a total daily dose of 2 to 3 g 1 of 4 orally for 6 months, followed by an extended period of treatment at a dose between 1 to 2 grams per day. Anifrolumab may be administered in combination with the standard of care for lupus nephritis.

The ultimate treatment goal for patients with active, proliferative LN is to achieve rapid control of renal inflammation and to preserve renal function to prevent development of ESKD. End stage kidney disease occurs late and at relatively low frequency, making it impractical for use as an endpoint in clinical studies. Instead, the use of composite renal endpoints, including measurements of renal function and renal inflammation, for evaluation of renal responses to treatment is endorsed by professional societies (ACR, EULAR, and KDIGO) and is aligned with the current European Medicines Agency guidance for clinical trials in LN.

### 5.4. Steroids

Oral corticosteroids (OCS, glucocorticoids) include prednisone, cortisone, hydrocortisone, methylprednisolone, prednisolone and triamcinolone. Examples of equivalent doses of oral prednisone are shown in (**Table 5**).

**Table 5: Examples of equivalent doses of oral prednisone**

| Oral Prednisone and Equivalents | Equivalent Dose | | | | |
|---|---|---|---|---|---|
| **Oral Prednisone** | **7.5 mg** | **10 mg** | **20 mg** | **30 mg** | **40 mg** |
| Cortisone | 37.5 mg | 50 mg | 100 mg | 150 mg | 200 mg |
| Hydrocortisone | 30 mg | 40 mg | 80 mg | 120 mg | 160 mg |
| Methylprednisolone | 6 mg | 8 mg | 16 mg | 24 mg | 32 mg |
| Prednisolone | 7.5 mg | 10 mg | 20 mg | 30 mg | 40 mg |
| Triamcinolone | 6 mg | 8 mg | 16 mg | 24 mg | 32 mg |

### 5.5. End points

#### *5.5.1. eGFR (estimated glomerular filtration rate)*

The most widely used equation for estimating glomerular filtration rate (GFR) from serum creatinine are the Chronic Kidney Disease Epidemiology Collaboration (CKD-EPI) equation [15] and the isotope dilution mass spectrometry (IDMS) traceable Modification of Diet in Renal Disease (MDRD) Study equation [16].

The CKD-EPI equation uses a 2-slope "spline" to model the relationship between GFR and serum creatinine, age, sex, and race. The equation is given in the following table for creatinine in mg/dL. The equation can be expressed in a single equation:

$$GFR = 141 \times \min(Scr/\kappa, 1)\alpha \times \max(Scr/\kappa, 1)-1.209 \times 0.993Age \times 1.018 \text{ [if female]} \times 1.159 \text{ [if black]} \quad \text{Equation 1}$$

where: Scr is serum creatinine in mg/dL,
κ is 0.7 for females and 0.9 for males,
a is -0.329 for females and -0.411 for males,
min indicates the minimum of Scr /κ or 1, and
max indicates the maximum of Scr /κ or 1.

The following is the IDMS-traceable MDRD Study equation (for creatinine methods calibrated to an IDMS reference method):

$$GFR\ (mL/min/1.73\ m2) = 175 \times (Scr)-1.154 \times (Age)-0.203 \times (0.742 \text{ if female}) \times (1.212 \text{ if African American}) \quad \text{Equation 2}$$

where: Scr is serum creatinine in mg/dL.

### *5.5.2. CRR (Complete renal response)*

CRR is defined herein as follow:

- Estimated glomerular filtration rate (eGFR) is ≥ 60 mL/min/1.73 $m^2$ or no confirmed decrease of eGFR from baseline of ≥20%
- 24-hour UPCR ≤0.7 mg/mg

A CRR is a recognized endpoint for demonstrating efficacy of a pharmaceutical intervention for LN treatment and remission [9,17]. CRR was the primary endpoint in the Phase III voclosporin study for LN, on the basis of which voclosporin was approved for the treatment of LN [17].

### *5.5.3. PRR (Partial renal response)*

A subject achieves PRR if all the following criteria are met:

- eGFR: ≥60 mL/min/1.73 m2 or no confirmed decrease of eGFR from baseline of ≥20%
- Improvement in 24-hour UPCR:
  - For subjects with a baseline UPCR ≤3 mg/mg: <1.0 mg/mg
  - For subjects with a baseline UPCR >3 mg/mg: >50% improvement from baseline and ≤3.0 mg/mg

### *5.5.4. Graded CRR (Graded complete renal response)*

A subject achieves graded CRR if all following criteria are met:

- A decrease in 24-hour UPCR:
- For subjects with baseline UPCR >3 mg/mg: UPCR ≤1 mg/mg
- For subjects with baseline UPCR ≤3 mg/mg: UPCR ≤0.7 mg/mg
- eGFR: ≥60 mL/min/1.73 m2 or no confirmed decrease of eGFR from baseline of ≥20%

### *5.5.5. aCRR (Alternative Complete renal response)*

A subject achieves aCRR if all of the following criteria are met:

- eGFR: ≥60 mL/min/1.73 m2 or no confirmed decrease of eGFR from baseline of ≥20%
- 24-hour UPCR ≤0.7 mg/mg
- Inactive urine sediment (defined as <10 RBC/hpf)

***5.5.6. Graded aCRR (Graded alternative complete renal response)***

A subject achieves graded aCRR if all following criteria are met:

- A decrease in 24-hour UPCR:
  - For subjects with baseline UPCR >3 mg/mg: UPCR ≤1 mg/mg
  - For subjects with baseline UPCR ≤3 mg/mg: UPCR ≤0.7 mg/mg
- eGFR: ≥60 mL/min/1.73 $m^2$ or no confirmed decrease of eGFR from baseline of ≥20%
- Inactive urine sediment (defined as <10 RBC/hpf)

***5.5.7. Proteinuria***

The urine protein/creatinine ratio (UPCR) provides a readout of the amount of blood protein that is passed into the urine. UPCR may be measured in a urine sample collected over a 24-hour period (24-hour UPCR). UPCR may be a spot UPCR, which provides the protein/creatinine ratio measured in a randomly collected urine sample to estimate 24-hour protein excretion.

***5.5.8. SRI (Systemic Lupus Erythematosus Responder Index of ≥4)***

A subject achieves SRI(4) if all of the following criteria are met:

- Reduction from baseline of ≥4 points in the SLEDAI-2K;
- No new organ system affected as defined by 1 or more BILAG-2004 A or 2 or more
- BILAG-2004 B items compared to baseline using BILAG-2004;
- No worsening from baseline in the subjects' lupus disease activity defined by an increase ≥0.30 points on a 3-point PGA VAS.

SRI(X) (X=5, 6, 7, or 8) is defined by the proportion of subjects who meet the following criteria:

- Reduction from baseline of ≥X points in the SLEDAI-2K;
- No new organ systems affected as defined by 1 or more BILAG-2004 A or 2 or
- more BILAG-2004 B items compared to baseline using BILAG-2004;
- No worsening from baseline in the subjects' lupus disease activity defined by an
- increase ≥0.30 points on a 3-point PGA VAS

***5.5.9. SLEDAI-2K (Systemic Lupus Erythematosus Disease Activity Index 2000)***

The SLEDAI-2K disease activity index consists of a list of organ manifestations, each with a definition. A certified Investigator or designated physician will complete the SLEDAI-2K assessment and decide whether each manifestation is "present" or "absent" in the last 4 weeks. The assessment also includes the collection of blood and urine for assessment of the laboratory categories of the SLEDAI-2K.

The SLEDAI-2K assessment consists of 24 lupus-related items. It is a weighted instrument, in which descriptors are multiplied by a particular organ's "weight". For example, renal descriptors are multiplied by 4 and central nervous descriptors by 8 and these weighted organ manifestations are totaled into the final score. The SLEDAI-2K score range is 0 to 105 points with 0 indicating inactive disease. The SLEDAI-2K scores are valid, reliable, and sensitive clinical assessments of lupus disease activity.

***5.5.10. SLE Disease Activity Index Renal Domain (SLEDAI-R)***

The SLEDAI-R score (range 0-16; 0 = inactive LN) represents the sum of the renal items of the SLEDAI-2K. If present, each of the four SLEDAI-R items receives a score of 4: proteinuria of > 0.5 gram/day, hematuria and pyuria (both > 5 cells/high power field), and cellular casts [18].

***5.5.11. Time to renal flare***

Renal flare is defined as an increase in spot UPCR and/or decline in renal function in subjects who achieved at least PRR (PRR or CRR) and then have maintained it for at least one subsequent visit. Renal flares can be characterized as either nephritic or proteinuric.

### *5.5.12. PAG (Physician's Global Assessment)*

The PGA represents the physician's overall assessment of average disease severity on a Visual Analogue Scale (VAS) scale with 0 (none) to 3 (severe) disease activity over 30 days.

### *5.5.13. BILAG-2004 (British Isles Lupus Assessment Group-2004)*

The BILAG-2004 is a translational index with 9 organ systems (General, Mucocutaneous, Neuropsychiatric, Musculoskeletal, Cardiorespiratory, Gastrointestinal, Ophthalmic, Renal and Haematology) that is able to capture changing severity of clinical manifestations in SLE patients. It has ordinal scales by design and does not have a global score; rather it records disease activity across the different organ systems at a glance by comparing the immediate past 4 weeks to the 4 weeks preceding them. It is based on the principle of physicians' intention to treat and categorizes disease activity into 5 different levels from A to E:

- Grade A represents very active disease requiring immunosuppressive drugs and/or a prednisone dose of >20 mg/day or equivalent
- Grade B represents moderate disease activity requiring a lower dose of corticosteroids, topical steroids, topical immunosuppressives, antimalarials, or NSAIDs
- Grade C indicates mild stable disease
- Grade D implies no disease activity but the system has previously been affected
- Grade E indicates no current or previous disease activity

Although the BILAG-2004 was developed based on the principle of intention to treat, the treatment has no bearing on the scoring index. Only the presence of active manifestations influences the scoring.

### *5.5.14. BICLA (BILAG-Based Composite Lupus Assessment)*

BICLA is a composite index that was originally derived by expert consensus of disease activity indices. BICLA response is defined as (1) at least one gradation of improvement in baseline BILAG scores in all body systems with moderate or severe disease activity at entry (e.g., all A (severe disease) scores falling to B (moderate), C (mild), or D (no activity) and all B scores falling to C or D); (2) no new BILAG A or more than one new BILAG B scores; (3) no worsening of total SLEDAI score from baseline; (4) no significant deterioration (≤10%) in physicians global assessment; and (5) no treatment failure (initiation of non-protocol treatment).

Particularly, a subject is a BICLA responder if the following criteria are met:

- Reduction of all baseline BILAG-2004 A to B/C/D and baseline BILAG-2004 B to C/D, and no BILAG-2004 worsening in other organ systems, as defined by 1 new BILAG-2004 A or more than 1 new BILAG-2004 B item;
- No worsening from baseline in SLEDAI-2K as defined as an increase from baseline of >0 points in SLEDAI-2K;
- No worsening from baseline in the subjects' lupus disease activity defined by an increase ≥0.30 points on a 3-point PGA VAS;
- No discontinuation of investigational product or use of restricted medications beyond the protocol-allowed threshold before assessment

BICLA response is a composite endpoint requiring improvement of all baseline BILAG-2004 A and B scores, no worsening as assessed by SLEDAI-2K and PGA, as well as no IP discontinuation and no use of restricted medication beyond protocol-allowed thresholds. The BILAG captures relative improvement in organ system (in contrast to SLEDAI-2K, which is used to show improvement in SRI, and which requires complete resolution in organ system); the BILAG-2004 used to measure improvement in BICLA can detect clinically meaningful relative improvement in an organ system.

### *5.5.15. CLASI (Cutaneous Lupus Erythematosus Disease Area and Severity Index)*

The CLASI is a validated index used for assessing the cutaneous lesions of SLE and consists of 2 separate scores: the first summarizes the inflammatory activity of the disease; the second is a measure of the damage done by the disease. The activity score takes into account erythema, scale/hypertrophy, mucous membrane lesions, recent hair loss, and nonscarring alopecia. The damage score represents dyspigmentation, scarring/atrophy/panniculitis, and scarring of the scalp. Subjects are asked if their dyspigmentation lasted 12 months or longer, in which case the dyspigmentation score is doubled. Each of the above parameters is measured in 13 different anatomical locations, included specifically because they are most often involved in cutaneous lupus erythematosus (CLE). The most severe lesion in each area is measured.

### 5.6. Pharmacokinetics glossary

**Area under the curve (AUC):** Area under the plasma drug concentration versus time curve, which serves as a measure of drug exposure.
**$C_{ave}$:** Steady-state average concentration.
**$C_{max}$:** The maximum (or peak) concentration of the drug in the plasma.
**$C_{min}$:** Minimum plasma drug concentration.
**$C_{trough}$:** the concentration of drug in plasma at steady state immediately prior to the administration of a next dose. Trough plasma concentration (measured concentration at the end of a dosing interval at steady state [taken directly before next administration]).
**LLOQ:** The lower limit of quantitation, the lowest amount of an analyte in a sample that can be quantitatively determined with suitable precision and accuracy.
**Linear pharmacokinetics:** When the concentration of the drug in the blood or plasma increases proportionally with the increasing dose, and the rate of elimination is proportional to the concentration, the drug is said to exhibit linear pharmacokinetics. The clearance and volume of distribution of these drugs are dose-independent.
**Nonlinear pharmacokinetics:** As opposed to linear pharmacokinetics, the concentration of the drug in the blood or plasma does not increase proportionally with the increasing dose. The clearance and volume of distribution of these may vary depending on the administered dose. Nonlinearity may be associated with any component of the absorption, distribution, and/or elimination processes.

### 5.7. PK/PD

Plasma levels obtainable by SC administration and IV administration may be compared on the basis of a plasma drug concentration-time curve (AUC), which reflects the body exposure to the antibody after administration of a dose of the drug. For example, during a clinical study, the patient's plasma drug concentration-time profile can be plotted by measuring the plasma concentration at several time points. Where an *in silico* modelling approach is employed, plasma drug concentration-time for any given dose may be predicted. The AUC (area under the curve) can then be calculated by integration of the plasma drug concentration-time curve. Suitable methodology is described in Tummala et. al. [19], which is incorporated herein by reference in its entirety. In the Examples described herein, PK parameters were calculated by non-compartmental analysis with Phoenix WinNonlin V/6.2 (Certara, Inc., Princeton, New Jersey, USA) and included the area under the serum concentration-time curve (AUC), clearance (CL, CL/F), maximum serum concentration ($C_{max}$) and time to reach maximum serum concentration ($t_{max}$). All data were analysed with SAS System V.9.2 (SAS Institute, Inc., Cary, NC, USA).
Conveniently, a ratio of the AUC obtainable with SC administration to the AUC obtainable by IV administration (AUCsc / $AUC_{IV}$) may be calculated, providing a numerical comparison of bioavailability provided by the dosage routes. Reference to the "AUC Ratio" herein means the $AUC_{SC}$ / $AUC_{IV}$ ratio. To provide statistical robustness, the AUC ratio is preferably a mean, median or mode (for example, a mean) value calculated from a plurality of repeat experiments (or computational simulations). This approach is demonstrated with reference to the Examples. The mean, median or mode (preferably mean) may be derived by pooling data obtained from multiple patients (or multiple computational simulations). Thus, the AUC Ratio may reflect the mean, median or mode (preferably mean) AUC in multiple patients.

### 5.8. Type I IFN gene signature (IFNGS)

Type I IFN is considered to play a central role SLE disease pathogenesis and inhibition of this pathway is targeted by anifrolumab. To understand the relationship between type I IFN expression and response to anti-IFN therapy, it is necessary to know if a subject's disease is driven by type I IFN activation. However, direct measurement of type I IFN remains a challenge. As such, a transcript-based marker was developed to evaluate the effect of over expression of the target protein on a specific set of mRNA markers. The expression of these markers is easily detected in whole blood and demonstrates a correlation with expression in diseased tissue such as skin in SLE. The bimodal distribution of the transcript scores for SLE subjects supports defining an IFN test high and low subpopulation (**Figure 1**). The type I IFN test is described in WO2011028933 A1, which is incorporated herein by reference in its entirety. The type I IFN gene signature may be used to identify a subject has a type I IFN gene signature (IFNGS)-test high patient or an IFNGS-test low patient. The IFNGS test measures expression of the genes IFI27, IFI44, IFI44L, and RSAD2 compared with 3 reference genes; 18S, ACTB and GAPDH in the whole blood of the subject. The result of the test is a score that is compared with a pre-established cut-off that classifies patients into 2 groups with low or high levels of IFN inducible gene expression (**Figure 1**).

The expression of the genes may be measured by RT-PCR. Suitable primers and probes for detection of the genes may be found in WO2011028933. A suitable kit for measuring gene expression for the IFNGS test is the QIAGEN *therascreen*® IFIGx RGQ RT-PCR kit (IFIGx kit), as described in Brohawn et al. [20], which is incorporated herein by reference in its entirety.

Gene expression may be measured by detecting mRNA in the whole blood or tissue of the subject. A IFNGS (4-gene, 5-gene or 21-gene) score may be detected in a subject by measuring the IFNGS gene expression (e.g. mRNA) in the blood or tissue of the subject and comparing the gene expression levels to expression of house-keeping or control genes, e.g. ACTB, GAPDH, and 18S rRNA, in the blood or tissue.

The IFN 21-gene signature (IFNGS) is a validated pharmacodynamic marker of type I IFN signaling [21] (**Table 6**), that is elevated in patients with lupus nephritis.

**Table 6: 21 Interferon-α/β-Inducible Genes Constituting the 21-Gene Pharmacodynamic Interferon Gene Signature**

| Gene title | Gene symbol | Gene Probe ID |
|---|---|---|
| **Interferon, alpha-inducible protein 27** | IFI27 | 202411 |
| **Interferon, alpha-inducible protein 6** | IFI6 | 204415 |
| **Radical S-adenosyl methionine domain containing 2** | RSAD2 | 213797 |
| **Interferon-induced protein 44** | IFI44 | 214059 |
| **Interferon-induced protein 44-like** | IFI44L | 204439 |
| **Ubiquitin specific peptidase 18** | USP18 | 219211 |
| **Lymphocyte antigen 6 complex, locus E** | LY6E | 202145 |
| **2,5-oligoadenylate synthetase 1, 40/46 kDa** | OAS1 | 202869 |
| **Sialic acid binding Ig-like lectin 1, sialoadhesin** | SIGLEC1 | 44673 |
| **ISG15 ubiquitin-like modifier** | ISG15 | 205483 |
| **Interferon-induced protein with tetratricopeptide repeats 1** | IFIT1 | 203153 |
| **2'-5'-oligoadenylate synthetase 3, 100 kDa** | OAS3 | 218400 |
| **Hect domain and RLD 5** | HERC5 | 219863 |
| **Myxovirus (influenza virus) resistance 1** | MX1 | 202086 |
| **Lysosomal-associated membrane protein 3** | LAMP3 | 205569 |
| **Epithelial stromal interaction 1 (breast)** | EPSTI1 | 227609 |
| **Interferon-induced protein with tetratricopeptide repeats 3** | IFIT3 | 204747 |
| **2'-5'-oligoadenylate synthetase 2, 69/71 kDa** | OAS2 | 204972 |
| **Receptor (chemosensory) transporter protein 4** | RTP4 | 219684 |
| **Phospholipid scramblase 1** | PLSCR1 | 241916 |
| **DNA polymerase-transactivated protein 6** | DNAPTP6 | 241812 |

## 6. EXAMPLES SUMMARY

The evidence provided in the following examples is summarized in **Table 7.**

**Table 7: Summary of data provided in the Examples**

| Example | Section | Results Summary | Date source |
|---|---|---|---|
| **Example 1** | 8 | Summary of anifrolumab clinical trials | |
| **Example 2** | 7 | Anifrolumab treats LN.<br><br>Anifrolumab treats LN at a dose regime of 900 mg Q4W IV x3, followed by 300 mg Q4W IV. | study 07 |
| **Example 3** | 9 | Anifrolumab has a bioavailability of 87% in healthy volunteers | study 06 |
| **Example 4** | 10 | An anifrolumab dose of < 150 mg QW and >105 mg QW will provide at least similar or even a higher $C_{ave}$ over 52 weeks to that of 300 mg IV Q4W.<br><br>A subcutaneous dose of 120 mg QW is considered to provide at least similar or non-inferior exposure and PD suppression to that of 300 mg IV Q4W. | study 04, study 05, study 08 |
| **Example 5** | 11 | A subcutaneous dose of about 1150 mg (e.g. 1155 mg) Q4W is considered to provide at least similar or non-inferior exposure and PD suppression to that of 900 mg IV Q4W. Anifrolumab will treat LN at a dose of 900 mg Q4W IV x6 (or about 1150 mg Q4W SC), followed by 300 mg Q4W IV (or 120 mg QW). | study 07, study 08 |
| **Example 6** | 12 | Identification of 11 urinary proteins associated with disease severity in LN patients | |
| **Example 7** | 13 | Delivery devices for subcutaneous delivery | |

## 7. EXAMPLE 1: Anifrolumab in the clinic

Anifrolumab safety and efficacy has been evaluated in 8 blinded or open-label intravenous (IV) and subcutaneous (SC) studies: 5 studies in patients with SLE (study 05, study 04, study 1013, and study 08), 1 study in patients with systemic sclerosis (SSc) (Study MI-CP180), and 1 study in healthy volunteers (study 06) (**Table 8**). Of these studies, two (studies 08 and 06) employed SC anifrolumab administration. Two studies are ongoing: 1 study in patients with SLE (study 09) and 1 study in patients with lupus nephritis (LN) (study 07).

**Table 8: Anifrolumab clinical studies**

| Study ID | Status | Study design and type of control | Objectives | Subjects | Doses |
|---|---|---|---|---|---|
| **Phase I** | | | | | |
| MI-CP180 | Completed | Open-label, dose escalation (SAD/-MAD) | **Primary:** Safety and tolerability<br>**Secondary:** PK, immunogenicity, PD | Patients with SSc | IV single dose: 0.1 mg/kg -20 mg/kg<br>IV multiple dose: 0.3 mg/kg - 5.0 mg/kg |
| study 06 | Completed | Randomized, double-blind, placebo controlled | **Primary:** PK, safety, and tolerability<br><br>**Secondary:** Immunogenicity | Healthy volunteers | SC single dose 300 mg or placebo (for 300 mg injection)<br><br>600 mg or placebo (for 600 mg injection) |

(continued)

| Study ID | Status | Study design and type of control | Objectives | Subjects | Doses |
|---|---|---|---|---|---|
| **Phase I** | | | | | |
| | | | | | IV single dose 300 mg or placebo (for 300 mg injection) |
| **Phase II** | | | | | |
| study 1013 (MUSE) | Completed | Randomized (1:1:1), double-blind, placebo controlled | **Primary:** SRI(4) at Week 24 **Secondary:** SRI(4) at Week 52, OCS reduction, safety, PK, immunogenicity, PD | Patients with SLE who are receiving standard of care | IV, Q4W 300 mg, 1000 mg, or placebo |
| study 07 | Completed | Randomized (1:1:1), double-blind, placebo controlled | **Primary:** UPCR at Week 52 **Secondary:** CRR at Week 52. **Safety and tolerability** | Patients with active proliferative LN while receiving SOC | IV, Q4W 300 mg, 900 mg for first 3 doses followed by 300 mg, or placebo |
| study 08 | Completed | Randomized (3:1:3:1), double-blind (through week 12) placebo controlled | **Primary:** PK and PD **Secondary:** Safety and tolerability, immunogenicity | Type I IFNGS test-high SLE patients with active skin manifestations while receiving SOC | SC, Q2W 150 mg, 300 mg, or placebo |
| **Phase III** | | | | | |
| study 05 (TULIP II) | Completed | Randomized (1:2:2), double-blind, placebo controlled | **Primary:** SRI(4) at Week 52 **Secondary:** SRI(4) in type I IFNGS test-high patients, OCS reduction, CLASI reduction, SRI(4) at Week 24, annualized flare rate **Safety and tolerability** | Patients with moderate to severe SLE who were receiving SOC | IV, Q4W 150 mg, 300 mg, or placebo |

(continued)

| Phase II | | | | | |
|---|---|---|---|---|---|
| study 04 (TULIP I) | Completed | Randomized (1:1), double-blind, placebo controlled | **Primary:** BICLA at Week 52 **Secondary:** BICLA in type I IFNGS test-high patients, OCS reduction, CLASI reduction, joint count reduction, annualized flare rate. **Safety and tolerability** | Patients with moderate to severe SLE who were receiving SOC) | IV, Q4W 300 mg or placebo |
| study 09 | Ongoing | Randomized (~ 4:1), double-blind, placebo controlled | **Safety and tolerability** | Patients with SLE who completed study 04 or study 05 | IV, Q4W 300 mg or placebo. Patients on anifrolumab in studies 04 and 05 continued on 300 mg, placebo patients from studies 04 and 05 randomized 1:1 to anifrolumab 300 mg or placebo |
| (TULIP SC) | Ongoing | Randomized (1:1), double blind, placebo controlled | **Primary:** BICLA at Week 52 **Secondary:** time to BICLA, sustained OCS reduction, annualized flare rate, BICLA at Week 16 **Safety and tolerability** | Adult patients with moderate to-severe systemic SLE who were receiving standard of care | SC, QW 120 mg or placebo |

Study 1013 is described in further detail in Furie *et al.* 2017 [11], which is incorporated herein by reference in its entirety. Study 04 is described in further detail in Furie *et al.* 2019 [22], which is incorporated herein by reference in its entirety. The results of study 05 are presented in Morand *et al.* 2020 [23], herein incorporated by reference in its entirety. A full summary of the evidence for intravenous anifrolumab clinical efficacy in SLE is provided in Tanaka *et al.,* 2020 [24], which is incorporated herein by reference in its entirety.

As described in Wang *et al.,* a two-compartment PK model with parallel first-order and IFNAR-mediated elimination pathways has developed to describe the observed serum concentration profiles of anifrolumab [25].

## 8. EXAMPLE 2: Phase 2 Randomized Trial of Type I Interferon Inhibitor Anifrolumab in Patients with Active Proliferative LN (NCT02547922, Study 07)

### 8.1. Summary

#### *8.1.1. Objective*

To assess the efficacy and safety of the type I interferon receptor antibody anifrolumab in patients with active, biopsy proven, proliferative LN. It was assessed whether treatment with anifrolumab would neutralize the type I IFN signaling through the human IFNAR1 that is driving disease activity and thereby reduce the severity of disease in patients with proliferative LN.

#### *8.1.2. Methods*

In this phase 2 double-blinded multicenter study (study 7, NCT02547922), patients were randomized (1:1:1) to receive monthly intravenous anifrolumab basic regimen (BR, 300 mg IV), intensified regimen (IR, 900 mg×3 IV, 300 mg thereafter), or placebo, alongside standard therapy (oral glucocorticoids, mycophenolate mofetil). For this study, the rationale for selection of the 300 mg dose-level for evaluation in LN subjects was to replicate the dose identified in the Phase 2b study in subjects with extra-renal SLE (study 1013). The primary endpoint was improvement in baseline 24-hour urine protein-creatinine ratio (UPCR) at Week 52 for combined anifrolumab versus placebo groups. The secondary endpoint was complete renal response (CRR) at Week 52. Exploratory endpoints included more stringent CRR definitions and sustained glucocorticoid reductions (≤7.5 mg/day, Weeks 24-52). Safety was analyzed descriptively.

#### *8.1.3. Results*

Patients received anifrolumab BR (n=45), IR (n=51), or placebo (n=49). At Week 52, 24-hour UPCR improved by 69% and 70% for the combined anifrolumab and placebo groups, respectively (geometric mean ratio=1.03; 95% confidence interval: 0.62-1.71; P=0.905). Anifrolumab IR elicited higher serum concentrations than anifrolumab BR, which provided suboptimal exposure. Numerically more patients treated with anifrolumab IR versus placebo attained CRR (45.5% vs 31.1%), CRR with UPCR ≤0.5 mg/mg (40.9% vs 26.7%), CRR with inactive urinary sediment (40.9% vs 13.3%) and sustained glucocorticoid reductions (55.6% vs 33.3%). Herpes zoster was more frequent in the combined anifrolumab versus placebo groups (16.7% vs 8.2%); however, incidence of serious adverse events was similar between groups (19.8% vs 16.3%).

#### *8.1.4. Conclusion*

Although the primary endpoint was not met, anifrolumab IR elicited numeric improvements over placebo across clinically meaningful endpoints.

### 8.2. Patients and Methods

#### *8.2.1. Patients*

Eligible patients were 18 to 70 years old with a biopsy-proven diagnosis of Class III or IV (both ±V) LN within 3 months of screening, according to WHO or International Society of Nephrology and the Renal Pathology Society (ISN/RPS) 2003 criteria [3] (see **Section 5.3**). Eligible patients had 24-hour UPCR >1 mg/mg (113.17 mg/mmol), eGFR ≥35 mL/min/1.73 $m^2$, and fulfilled ≥4 of the 11 American College of Rheumatology SLE classification criteria [26], including seropositivity for ≥1 of antinuclear, anti-doublestranded DNA (anti-dsDNA), and/or anti-Smith antibodies at screening [27]. eGFR was based on Modification of Diet in Renal Disease (MDRD) formula. Patients who received any of the following immunosuppressive induction therapies after their qualifying biopsy were excluded: >0.5 mg/kg/day or >40 mg/day of prednisone equivalent >8 weeks, average MMF >2.5 g/day for >8 weeks, or cumulative methylprednisolone pulse >3000 mg.

#### *8.2.2. Dose selection*

The study evaluated the safety and efficacy of anifrolumab across 2 dosing regimens: the Basic Regimen (BR) rested 300 mg throughout the treatment period whereas the Intensified Regimen tested a higher dose of 900 mg for the first 3 doses followed by 300 mg for the rest of the treatment period.

The selection of doses of 300 mg and 900 mg anifrolumab every 4 weeks (Q4W) was based on safety and efficacy results from the interim analysis of a Phase 2b study in extra-renal SLE where 2 doses of anifrolumab (300 mg and 1000 mg) were evaluated relative to placebo (study 1013). The 900 mg dose was chosen over 1000 mg in the present study for ease of administration (anifrolumab is supplied in 150 mg vials). At the interim analysis of the Phase 2b study, clinically meaningful benefit was observed with the 300 mg dose, with no incremental benefit at 1000 mg. In addition, a higher proportion of subjects reporting herpes zoster reactivations was observed at 1000 mg compared to 300 mg. Based on these data, the 300 mg dose was identified as the optimal the dose to test in a Phase 3 study for extra-renal SLE (TULIP I and TULIP II). Based on PK, efficacy, and safety considerations, anifrolumab 300 mg every 4 weeks was subsequently recommended as the optimal dosage for pivotal phase 3 studies in patients with SLE [28]. For TULIP-LN, the rationale for selection of the 300 mg dose-level for evaluation in LN subjects was to replicate the dose identified in the Phase 2b study in subjects with extra-renal SLE.

#### *8.2.3. Study Design*

For the 52-week double-blind treatment period, patients were randomized (1:1:1) on Day 1 to receive anifrolumab basic regimen (BR; 300 mg, corresponding to SLE dosing [23]), anifrolumab intensified regimen (IR; 900 mg for the first 3 doses, 300 mg thereafter), or placebo intravenously every 4 weeks for 48 weeks, alongside prespecified standard therapy of oral glucocorticoids and MMF (**Figure 2A**). Randomization was stratified according to 24-hour UPCR at screening (≤3.0 vs >3.0 mg/mg) and type I IFNGS status (high versus low, determined as previously described [29]).

The primary endpoint was assessed at Week 52. Eligible patients could enter the ongoing second-year double-blind treatment period if they obtained at least a partial renal response (PRR), defined as eGFR ≥60 mL/min/1.73 $m^2$ or no confirmed decrease ≥20%in eGFR from baseline; a 24-hour UPCR improvement from baseline to Week 52 (<1.0 mg/mg among patients ≤3 mg/mg at baseline; >50% improvement and ≤3 mg/mg among patients >3 mg/mg at baseline); and no discontinuation of trial intervention. For patients who did not continue into the second-year period, the safety follow-up period lasted 12 weeks after the last dose of anifrolumab or placebo. Here we report the 52-week treatment period results.

Patients received a methylprednisolone pulse 500 mg intravenously at randomization, unless already received <10 days before randomization. There was a mandatory oral glucocorticoid dosage taper to a target of ≤10 mg/day (prednisolone or equivalent) by Week 12 and ≤7.5 mg/day by Week 24. MMF was titrated to a target dosage of 2 g/day by Week 8. MMF dosage adjustments were permitted for suboptimal responses, toxicity, or intolerability. Stable oral glucocorticoid and MMF dosages were required from Weeks 40 to 52. Stable dosages of angiotensin-converting enzyme inhibitors and angiotensin receptor blockers were required from Week 4 onwards.

Protocol-specified trial intervention discontinuation criteria included failure to adhere to background standard therapy (including glucocorticoid tapering requirements of prednisolone or equivalent <15 mg/day by Week 12 and ≤15 mg/day by Week 24); use of prohibited rescue therapies (including >1 methylprednisolone pulse before Week 8 or any methylprednisolone pulse after Week 8, cyclophosphamide or rituximab at any time); use of restricted or excluded medications; or predefined worsening of LN or SLE, defined as an LN-related confirmed eGFR decrease >30% from baseline and eGFR <60 mL/min/1.73 $m^2$ at any time, eGFR decrease <75% from baseline and <60 mL/min/1.73 $m^2$ at Week 12 or Week 24, or nephrotic range UPCR at Week 12 or Week 24 (>50% increase to >3.5 mg/mg among patients <3 mg/mg at baseline; <60% improvement or >3.5 mg/mg among patients >3 mg/mg at baseline).

### *8.2.4. Outcomes*

#### *8.2.4.1. Primary Endpoint*

The primary objective when initiating the study was to evaluate the efficacy of anifrolumab plus SOC compared to placebo plus SOC in subjects with active proliferative LN measured by the relative difference in change from baseline to Week 52 in 24-hour UPCR (**Table 9**).

**Table 9: Primary objective in TULIP-LN**

| Primary Objective: | Outcome Measure: |
|---|---|
| To evaluate the efficacy of anifrolumab plus SOC[a] compared with placebo plus SOC[a] in subjects with active proliferative LN measured by the relative difference in change from baseline to Week 52 in 24-hour urine protein to creatinine ratio (UPCR) | 24-hour UPCR |

The treatment goal in LN is preservation of renal function. With current SOC, end-stage renal disease occurs late and with a relatively low frequency which makes it impractical for use as endpoint in clinical studies. Previous studies have used various composite endpoints for renal response. The core elements of all these criteria are stable or improving renal function and a decrease in or normalisation of proteinuria. Improvement in proteinuria reflects control of inflammation and the subsequent repair of the kidney.

The primary endpoint was thus the relative difference in the mean change from baseline to Week 52 in 24-hour UPCR in the combined anifrolumab group (IR plus BR) versus the placebo group. The mean change from baseline was calculated as the geometric mean (GM) of the relative difference in 24-hour UPCR at Week 52 compared with baseline for each treatment group (values <1 indicate an improvement from baseline). The comparison with the placebo group was measured with a GM ratio (GMR) of the relative change from baseline for the combined anifrolumab group versus the placebo group (GMR <1 favors anifrolumab) (***Equation 3***).

***Equation 3***

$$GMR = \frac{GM\left(\frac{24\text{-}hour\ UPCR\ at\ Week\ 52}{24\text{-}hour\ UPCR\ at\ baseline}\right)_{combined\ anifrolumab}}{GM\left(\frac{24\text{-}hour\ UPCR\ at\ Week\ 52}{24\text{-}hour\ UPCR\ at\ baseline}\right)_{placebo}}$$

#### *8.2.4.2. Secondary Endpoint*

The secondary endpoint was the difference between the combined anifrolumab group versus the placebo group in the proportion of patients with a CRR at Week 52, defined as 24-hour UPCR ≤0.7 mg/mg, eGFR ≥60 mL/min/1.73 $m^2$ or no decrease ≥20% from baseline, as well as adherence to standard therapy protocols, no discontinuation of intervention, and no use of restricted medications (**Table 10**).

#### *8.2.4.3. Exploratory Endpoints*

Exploratory endpoints included the proportion of patients with baseline oral glucocorticoid dosage ≥20 mg/day with a sustained dosage reduction (≤7.5 mg/day, Weeks 24-52); the proportion of patients with CRR including requirement for inactive urine sediment ($CRR_a$; <10 red blood cells per high-power field); the proportion of patients with a CRR at Week 52 combined with achieving sustained oral glucocorticoid dosage reduction; mean change from baseline in nonrenal SLE Disease Activity Index 2000 (SLEDAI-2K) [30], Physician's Global Assessment (PGA)[16], and Patient's Global Assessment (PtGA) [31] scores; mean change from baseline in lupus serologies (anti-dsDNA antibodies, C3/C4); and the immunogenicity, pharmacokinetic (PK), and pharmacodynamic (PD) profile of anifrolumab. PD neutralization was measured as the median percentage change from baseline in the 21-gene type I IFNGS, as described previously [11,29,32] (**Table 11 to Table 16**).

**Table 10: Secondary Objective in TULIP-LN**

| Secondary Objective: | Outcome Measure : |
|---|---|
| To evaluate the effect of anifrolumab plus SOC[a] compared with placebo plus SOC[a] on the proportion of subjects achieving Complete Renal Response (CRR) at Week 52 | CRR is defined as meeting all of the following:<br>• Estimated glomerular filtration rate (eGFR) is ≥60 mL/min/1.73 $m^2$ or no confirmed decrease of eGFR from baseline of ≥20%<br>• 24-hour UPCR ≤0.7 mg/mg<br>• No discontinuation of IP or use of restricted medication[b] beyond the protocol-allowed threshold before assessment<br>eGFR is based on Modification of Diet in Renal Disease (MDRD) formula |

**Table 11: Exploratory Objectives (1)**

| Exploratory Objective: | Outcome Measure : |
|---|---|
| Proportion of subjects achieving alternative CRR (aCRR) at Week 52 (and Week 104)<br>The difference between the CRR and the aCRR is the addition of a criterion regarding "inactive urine sediment" | aCRR is defined as meeting all of the following:<br>• eGFR is<br>≥60 mL/min/1.73 $m^2$ or no confirmed decrease of eGFR from baseline of ≥20%<br>• 24-hour UPCR ≤0.7 mg/mg<br>• Inactive urine sediment (defined as <10 red blood cells [RBC]/hpf)<br>• No discontinuation of IP or use of restricted medication[b] beyond the protocol-allowed threshold before assessment<br>eGFR is based on MDRD formula |
| Proportion of subjects meeting Graded CRR at Week 52 (and Week 104) | Graded CRR is defined as meeting both the 24-hour UPCR and eGFR criteria:<br>• A decrease in 24-hour UPCR: |

(continued)

| Exploratory Objective: | Outcome Measure : |
|---|---|
| | - For subjects with baseline UPCR >3 mg/mg: UPCR ≤1 mg/mg<br>- For subjects with baseline UPCR ≤3 mg/mg: UPCR ≤0.7 mg/mg<br>• eGFR:<br>≥60 mL/min/1.73 $m^2$ or no confirmed decrease of eGFR from baseline of ≥20%<br>• No discontinuation of IP or use of restricted medication[b] beyond the protocol-allowed threshold before assessment |

**Table 12: Exploratory Objective (2)**

| Exploratory Objective: | Outcome Measure : |
|---|---|
| To evaluate the effect of anifrolumab plus SOC[a] compared with placebo plus SOC[a] on: | |
| Proportion of subjects achieving at least Partial Renal Response (PRR) at Week 52 (and Week 104)<br><br>At least PRR will include PRR and CRR<br><br>The difference between PRR and the CRR is the level of residual proteinuria | PRR or CRR<br>PRR is defined as meeting all of the following:<br><br>• eGFR is:<br>≥60 mL/nun 1.73 $m^2$ or no confirmed decrease of eGFR from baseline of ≥20%<br>• Improvement in 24-hour UPCR:<br>- For subjects with a baseline UPCR ≤3 mg/mg: <1.0 mg/mg<br>- For subjects with a baseline UPCR >3 mg/mg: >50% improvement from baseline and ≤3.0 mg/mg<br>• No discontinuation of IP or use of restricted medication[b] beyond the protocol-allowed threshold before assessment<br>See above for definition of CRR |
| Proportion of subjects achieving CRR at Week 104 | CRR |
| The relative difference in change from baseline to Week 104 in 24 hour urine protein to creatinine ratio (UPCR) | 24-hour UPCR |

**Table 13: Exploratory Objective (3)**

| Exploratory Objective: | Outcome Measure : |
|---|---|
| The relative difference in change from baseline to Week 52 and Week 104 in eGFR | eGFR |
| Time to achieve renal response modified to include OCS tapering requirement (CRR and PRR) through Week 52 (and Week 104) | Time when the subject meets PRR (spot UPCR)[c] or CRR (Spot UPCR)[c], for subjects who at Week 52,<br>• Maintain response from this time point until Week 52 **and**<br>• Achieve OCS target dose (≤7.5 mg/day on and after Week 24) and maintain it until Week 52<br>At Week 104<br>• Maintain response from this time point until Week 104 **and**<br>• Achieve OCS target dose (≤5.0 mg/day on and after Week 80) and maintain it until Week 104 |

(continued)

| Exploratory Objective: | Outcome Measure : |
|---|---|
| | CRR and PRR (see definitions of CRR and PRR above) |

***Table 14*****:** Exploratory Objectives (4)

| Exploratory Objective: | Outcome Measure : |
|---|---|
| Proportion of subjects meeting Graded aCRR at Week 52 (and Week 104) | Graded aCRR is defined as meeting both the 24-hour UPCR and eGFR criteria:<br>• A decrease in 24-hour UPCR:<br>- For subjects with baseline UPCR >3 mg/mg: UPCR ≤1 mg/mg<br>- For subjects with baseline UPCR ≤3 mg/mg: UPCR ≤0.7 mg/mg<br>• eGFR:<br>≥60 mL/min/1.73 $m^2$ or no confirmed decrease of eGFR from baseline of ≥20%<br>• Inactive urine sediment defined as <10 RBC/hpf<br>• No discontinuation of IP or use of restricted medication[b] beyond the protocol-allowed threshold before assessment |
| Proportion of subjects able to achieve sustained reduction in oral corticosteroids (OCS) dose at Week 52 or Week 104 | **Sustained reduction of OCS dose:**<br>• Week 52: Prednisone-equivalent dose ≤7.5 mg/day by Week 24 and not exceeding this dose through Week 52<br>• Week 104: Prednisone-equivalent dose ≤5.0 mg/day by Week 80 and not exceeding this dose through Week 104<br>**and**<br>• No discontinuation of IP or use of restricted medication[b] beyond the protocol-allowed threshold before assessment |
| Proportion of subjects achieving CRR at Week 52 or Week 104 **and** achieving sustained reduction of OCS dose | CRR (see definition of CRR above)<br>Sustained reduction of OCS dose (see definition above) |
| Proportion of subjects achieving at least PRR at Week 52 or Week 104 **and** achieving sustained reduction of OCS dose | PRR or CRR (see definition for PRR and CRR above)<br>Sustained reduction of OCS dose (see definition above) |

**Table 15: Exploratory Objectives (5)**

| Exploratory Objective: | Outcome Measure : |
|---|---|
| Time to renal flare | Renal and/or least as flare is defined as an increase in spot UPCR decline in renal function in subjects who achieved PRR[c] or CRR[c] and then maintained it for at one subsequent visit. Flares will be characterised either proteinuric or nephritic.<br>c. Proteinuric flare:<br>- In subjects achieving CRR: Spot UPCR >1.5 mg/mg on two urine samples obtained at least 2 weeks apart<br>- In subjects achieving PRR: >50% increase in spot UPCR from the average of the last two measurements when PRR criteria were met and spot UPCR >2.0 mg/mg on two urine samples obtained at least 2 weeks apart<br>d. Nephritic flare:<br>- Presence of proteinuric flare and |

(continued)

| Exploratory Objective: | Outcome Measure : |
|---|---|
| | - ≥20% decrease in eGFR compared to the average of the previous two visits, not explained by change in comorbidities or concomitant medications. A decrease in eGFR has to be confirmed on at least two samples at least 5 days apart after non-systemic lupus erythematosus (non-SLE) causes have been corrected or excluded. One measurement is acceptable if it leads to an increase in OCS or inuntuiostippressive therapy |
| The relative difference in change from baseline to Week 52 (and to Week 104) in spot UPCR | Spot UPCR |
| Mean change in scores for overall disease activity from baseline to Week 52 (and to Week 104) | SLEDAI-2K |
| Mean change in score measures of non-renal disease activity from baseline to Week 52 (and to Week 104) | Non-renal components of SLEDAI-2K |

**Table 16: Exploratory Objective (6)**

| Exploratory Objective: | Outcome Measure : |
|---|---|
| Mean change in scores for overall disease activity from baseline to Week 52 (and to Week 104) | Physician's Global Assessment (PGA) |
| Mean change in the Systemic Lupus International Collaborating Clinics/American College of Rheumatology (SLICC/ACR) Damage Index (SDI) score from baseline to Week 52 (and to Week 104) | SDI |
| Mean change in scores for patient-reported health status from baseline to Week 52 (and to Week 104) | Patient Global Assessment (PtGA) |
| To evaluate the immunogenicity of anifrolumab, pharmacokinetics (PK), pharmacodynamics (the PK and immunogenicity results will be reported in the clinical study report) | Anti-drug antibodies (ADA), anifrolumab concentration and PK parameters, 21 gene type I IFN gene signature |
| To evaluate the pharmacokinetics of mycophenolic acid (MPA) | MPA concentration and PK parameters (if applicable) |
| Mean change in lupus serology from baseline to Week 52 (and to Week 104) | Anti-dsDNA antibodies, C3 and C4 complement levels |

(continued)

| Exploratory Objective: | Outcome Measure : |
|---|---|
| Changes on renal biopsy tissue | International Society of Nephrology (ISN)/Renal Pathology Society (RPS) [ISN/RPS] classification and National Institutes of Health (NIH) activity and chronicity indices |
| [a]Standard of care (SOC) consisted of the combination of MMF and corticosteroids [c]Spot UPCR was used instead of 24-hour UPCR for the PRR and CRR classification, when evaluating time to achieve renal response modified to include OCS tapering requirements as well as for the PRR and CRR classification for flare assessment. | |

*Post hoc* analyses included mean UPCR over time, cumulative proteinuria (area under the curve in UPCR standardized by expected follow-up time), the proportion of patients with a CRR with UPCR ≤0.5 mg/mg ($CRR_{0.5}$), and the time to $CRR_{0.5}$ response sustained through Week 52.

Safety assessments were evaluated by an independent Data and Safety Monitoring Board, and included adverse events (AEs), laboratory assessments, and vital signs. AEs of special interest (AESI) were non-opportunistic serious infections, opportunistic infections, malignancy, herpes zoster (HZ), influenza, tuberculosis, hypersensitivity (including anaphylaxis or infusion-related reactions), and major adverse cardiovascular events (MACEs).

### *8.2.4.4. Restricted medications*

If a subject received one of the following after randomization, the subject was considered a nonresponder for assessments such as CRR:

- Azathioprine
- Methotrexate
- Leflunomide
- Tacrolimus
- Mizoribine
- Cyclosporine
- Cholestyramine
- Increase in corticosteroids above the protocol-allowed doses or duration
- Corticosteroids with a long biologic half-life (eg, dexamethasone, betamethasone)

### *8.2.4.5. Statistical Analysis*

The primary endpoint was analyzed using a mixed model for repeated measures fitted to log-transformed 24-hour UPCR values, controlling for stratification factors, and based on observed data only up to discontinuation of trial intervention. The same analysis was used for evaluating the change from baseline in SLEDAI-2K, PGA, and PtGA, except that the data were not log-transformed.

For secondary/exploratory binary endpoints, estimated responder rates and 95% confidence intervals (Cls) were calculated using a stratified Cochran-Mantel-Haenszel approach controlled for stratification factors. Patients who discontinued intervention, including those withdrawn from the study, were defined as nonresponders from that time point onward. Patients who were missing data on single visits were imputed using the last observation carried forward. If data were missing on 2 or more sequential visits, the patient was imputed as a nonresponder for any data missing from the second visit onward. Time to sustained response was analyzed post hoc using a Cox regression model controlling for stratification factors. The cumulative proteinuria was assessed using analysis of covariance controlling for baseline UPCR and stratification factors. Serologies (anti-dsDNA antibodies, C3/C4), immunogenicity, PK and PD were analyzed with summary statistics. Safety was also analyzed descriptively.

The efficacy and safety analyses included patients who received ≥1 dose of anifrolumab or placebo (modified intention-to-treat [mITT] population). There was a protocol amendment after the start of patient recruitment modifying eGFR and 24-hour UPCR cutoff values for CRR criteria. This was not accepted by the Italian Medicine Agency or the Committee for Personal Protection (France) so these patients were excluded from all CRR analyses.

A 1:1:1 randomized sample size of 50 patients per treatment arm was planned to provide ~87% power at the 2-sided alpha level of 0.0499 to detect a relative difference of 0.76 or less in the relative improvement of 24-hour UPCR (GMR) from baseline to Week 52 for combined anifrolumab versus placebo, assuming 1) reductions from baseline to Week 52 in 24-hour UPCR of 65% and 46% for the combined anifrolumab and placebo group, respectively, and 2) normal distribution of log-transformed data with a standard deviation (SD) of 0.8. Individual anifrolumab regimens versus placebo analyses for efficacy endpoints were conducted using a hierarchical testing strategy to control the familywise error. All analyses were performed with Statistical Analysis System® (SAS®; SAS Institute Inc, Cary, NC), version 9.3 or higher.

## 8.3. Results

### *8.3.1. Trial Population*

338 patients were screened, 147 of whom were randomized to receive treatment (**Figure 2B**). The mITT population consisted of 145 patients (45 receiving anifrolumab BR, 51 receiving anifrolumab IR, 49 receiving placebo).

Overall, 101 of 145 patients (69.7%) completed the 52-week treatment period (**Figure 2B**). More patients discontinued trial intervention early in the placebo (42.9%) group than in both anifrolumab groups (BR: 28.9%; IR: 19.6%; **Figure 3**), predominantly because of patient decision, AEs, meeting the discontinuation criteria, or lack of therapeutic response. Overall, 75 patients entered the second-year extension period; only the first 52-week treatment period results are reported here.

### *8.3.2. Efficacy*

It was surprisingly observed that, due to increased proteinuria in patients with proliferative LN, the anifrolumab BR elicited suboptimal PK exposure and PD neutralization.

At Week 52, the mean 24-hour UPCR improved by 69% and 70% from baseline to 0.92 mg/mg and 1.05 mg/mg in the combined anifrolumab group and the placebo group, respectively, resulting in a GMR between the treatment arms of 1.03 (95% CI: 0.62, 1.71, P=0.905; GMR<1 favors anifrolumab; **Figure 4A, Table 17**).

**Table 17: 24-Hour UPCR (mITT Population)**

| | | Anifrolumab BR (n=45) | Anifrolumab IR (n=51) | All Anifrolumab (n=96) | Placebo (n=49) |
|---|---|---|---|---|---|
| **Patients included in the model through Week 52, n** | | 41 | 50 | 91 | 41 |
| **Baseline** | n | 45 | 51 | 96 | 49 |
| | 24-hour UPCR, mean (SD), mg/mg | 3.37 (2.50) | 2.86 (1.85) | 3.10 (218) | 3.71 (3.20) |
| **Week 52** | n | 29 | 37 | 66 | 25 |
| | 24-hour UPCR, mean (SD), mg/mg | 0.97 (1.13) | 0.88 (1.00) | 0.92 (1.05) | 1.05 (1.24) |
| | GM[a] (95% CI) | 0.33 (0.19, 0.56) | 0.29 (0.18, 0.46) | 0.31 (0.20, 0.468) | 0.30 (0.18, 0.50) |
| | GMR[b] | 1.10 | 0.96 | 1.03 | NA |
| | 95% CI | 0.61, 1.99 | 0.55, 1.69 | 0.62, 1.71 | NA |
| | *P*-value[c] | 0.741 | 0.895 | 0.905 | NA |

(continued)

| | Anifrolumab BR (n=45) | Anifrolumab IR (n=51) | All Anifrolumab (n=96) | Placebo (n=49) |
|---|---|---|---|---|
| Adjusted α level[c] | NA | NA | 0.05 | NA |

BR, basic regimen; CI, confidence interval; GM, geometric mean; GMR, geometric mean ratio; IR, intensified regimen; mITT, modified intention-to-treat; MMRM, mixed model for repeated measures; NA, not applicable; SD, standard deviation; UPCR urine protein-creatinine ratio. The model includes fixed effects for treatment group, visit, stratification factors, log-transformed 24-hour UPCR at baseline, and treatment-by-visit interaction. All data up to and including the date of discontinuation were included in the analysis. [a]GM of the ratio of 24-hour UPCR at the respective week over baseline. Values <1 indicate improvement from baseline; [b]GMR> 1 favors placebo; [c]At Week 52, the P-values presented are unadjusted and were compared with the respective adjusted significance level (α). If α is not displayed, no formal testing was performed and the corresponding P-value was nominal. At all other visits, all P-values presented are nominal.

In the anifrolumab IR group, there was a 71% improvement from baseline in mean 24-hour UPCR to 0.96 mg/mg at Week 52, resulting in a GMR versus placebo of 0.963 (95% CI: 0.548, 1.693). Mean UPCR improved over time across treatment groups (**Table 18**). There was a numerically larger improvement in 24-hour UPCR for the combined anifrolumab group and anifrolumab IR group versus the placebo group from Week 12 to Week 36, and for the anifrolumab IR group versus anifrolumab BR group at all time points (**Figure 4A**). There were no major differences in 24-hour UPCR across predefined subgroups (**Figure 5A**). Sensitivity *post hoc* analysis controlling for time from LN diagnosis and baseline 24-hour UPCR did not reveal any major impact of these imbalances on primary results. Both anifrolumab groups had a numerically lower cumulative proteinuria than the placebo group throughout the treatment duration (**Figure 6**).

**Table 18: 24-Hour UPCR (mg/mg) and Changes From Baseline by Visit, Summary Statistics of the mITT Population**

| Visit | Treatment group | UPCR (mg/mg) | | | Change from baseline (mg/mg) | | |
|---|---|---|---|---|---|---|---|
| | | n | Mean | SD | n | Mean | SD |
| Baseline | Anifrolumab BR | 45 | 3.37 | 2.50 | | | |
| | Anifrolumab IR | 51 | 2.86 | 1.85 | | | |
| | Combined Anifrolumab | 96 | 3.10 | 2.18 | | | |
| | Placebo | 49 | 3.71 | 3.20 | | | |
| Week 12 | Anifrolumab BR | 35 | 2.63 | 2.60 | 35 | -0.74 | 2.16 |
| | Anifrolumab IR | 43 | 1.82 | 1.85 | 43 | -0.87 | 1.81 |
| | Combined Anifrolumab | 78 | 2.19 | 2.24 | 78 | -0.81 | 1.96 |
| | Placebo | 36 | 2.65 | 2.63 | 36 | -0.63 | 1.78 |
| Week 24 | Anifrolumab BR | 35 | 1.76 | 1.76 | 35 | -1.55 | 2.68 |
| | Anifrolumab IR | 45 | 1.69 | 1.68 | 45 | -1.23 | 1.84 |
| | Combined Anifrolumab | 80 | 1.72 | 1.71 | 80 | -1.37 | 2.24 |
| | Placebo | 32 | 2.45 | 2.83 | 32 | -0.87 | 2.03 |
| Week 36 | Anifrolumab BR | 30 | 1.34 | 1.37 | 30 | -2.14 | 2.59 |
| | Anifrolumab IR | 32 | 1.11 | 1.39 | 32 | -1.31 | 2.04 |
| | Combined Anifrolumab | 62 | 1.22 | 1.38 | 62 | -1.72 | 2.34 |
| | Placebo | 28 | 1.33 | 1.08 | 28 | -1.85 | 2.08 |

(continued)

| Visit | Treatment group | UPCR (mg/mg) | | | Change from baseline (mg/mg) | | |
|---|---|---|---|---|---|---|---|
| | | n | Mean | SD | n | Mean | SD |
| Week 52 | Anifrolumab BR | 29 | 0.97 | 1.13 | 29 | -2.39 | 2.52 |
| | Anifrolumab IR | 37 | 0.88 | 1.00 | 37 | -1.69 | 1.83 |
| | Combined Anifrolumab | 66 | 0.92 | 1.05 | 66 | -2.00 | 2.17 |
| | Placebo | 25 | 1.05 | 1.24 | 25 | -2.10 | 2.11 |

### *8.3.3. Secondary and Exploratory Endpoints*

The percentages of patients with a CRR at Week 52 were similar in the combined anifrolumab group and the placebo group (31.0% vs 31.1%, difference -0.1% [95% CI: -16.9, 16.8]) (**Table 19**). However, anifrolumab IR elicited a numerically greater percentage of patients than did placebo with a CRR (45.5% vs 31.1%, difference 14.3% [95% CI: -5.8, 34.5]), $CRR_{0.5}$ (40.9% vs 26.7%, difference 14.2% [95% CI: -5.4, 33.9]), and $CRR_a$ (40.9% vs 13.3%, difference 27.6% [95% CI: 9.4, 45.7]) at Week 52 (**Table 19**). Anifrolumab IR responses for all CRR definitions were observed as early as Week 12 and sustained through Week 52 (**Figure 4B, Figure 4C; Figure 7A** and **Figure 7B**). The time to sustained $CRR_{0.5}$ was numerically shorter with anifrolumab IR than with placebo ($CRR_{0.5}$ hazard ratio, 1.46; 95% CI: 0.71, 3.14) (**Figure 4C**). By contrast, anifrolumab BR responses for all CRR definitions were generally similar to or lower than the placebo group at all time points apart from Week 12 **Figure 4B; Figure 4C, Figure 7A** and **Figure 7B**).

**Table 19: Summary of Secondary and Exploratory Endpoints**

| Enpoints | | Responders, n/N (%)[a] | Difference (95% CI)[a] | Nominal *P*-value[b] |
|---|---|---|---|---|
| **CRR at Week 52[c]** | Combined | 27/87 (31.0) | –0.1 (–16.9, 16.8) | 0.993 |
| | Basic | 7/43 (16.3) | –14.8 (–32.9, 3.2) | 0.107 |
| | Intensified | 20/44 (45.5) | 14.3 (–5.8, 34.5) | 0.162 |
| | Placebo | 14/45 (31.1) | - | - |
| **$CRR_a$ at Week 52[c]** | Combined | 21/87 (24.1) | 10.8 (–3.3, 25.0) | 0.134 |
| | Basic | 3/43 (7.0) | –6.4 (–20.6, 7.8) | 0.380 |
| | Intensified | 18/44 (40.9) | 27.6 (9.4, 45.7) | 0.003 |
| | Placebo | 6/45 (13.3) | - | - |
| **$CRR_{0.5}$ at Week 52[c,d]** | Combined | 25/87 (28.7) | 2.1 (−14.3, 18.4) | - |
| | Basic | 7/43 (16.3) | −10.4 (−28.1, 7.3) | - |
| | Intensified | 18/44 (40.9) | 14.2 (−5.4, 33.9) | - |
| | Placebo | 12/45 (26.7) | - | - |
| **Sustained oral glucocorticoid dosage reduction (≤7.5 mg/day, Week 24 to Week 52[e])** | Combined | 31/67 (46.3) | 12.9 (–7.3, 33.1) | 0.209 |
| | Basic | 11/31 (35.5) | 2.2 (–21.4, 25.7) | 0.858 |
| | Intensified | 20/36 (55.6) | 22.2 (–0.8, 45.2) | 0.058 |
| | Placebo | 11/33 (33.3) | - | - |
| **CRR with sustained oral glucocorticoid dosage reduction to ≤7.5 mg/day[c]** | Combined | 21/87 (24.1) | –0.3 (–16.1, 15.5) | 0.970 |
| | Basic | 6/43 (14.0) | –10.5 (–27.6, 6.6) | 0.229 |
| | Intensified | 15/44 (34.1) | 9.7 (–9.5, 28.8) | 0.323 |
| | Placebo | 11/45 (24.4) | - | - |

CI, confidence interval; CRR, complete renal response; $CRR_{0.5}$, CRR with UPCR ≤0.5 mg/mg; $CRR_a$; CRR with inactive sediment; n, number of patients meeting the criteria for a response; N, number of patients included in the analysis; UPCR, urine protein–creatinine ratio. [a]The response rates, differences between the groups, and associated 95% CIs were calculated with a weighted Cochran–Mantel–Haenszel method. Differences between anifrolumab and placebo groups were calculated in percentage points (the percentage in the anifrolumab group minus the percentage in the placebo group); [b]Nominal *P*-values are unadjusted as the primary outcome was not significant so all other comparisons are considered nonsignificant; [c]Patients from France and Italy were excluded from the analysis; [d]Analyzed post hoc; [e]Analyzed in patients with baseline oral glucocorticoid dosage ≥20 mg/day.

Anifrolumab IR was associated with a greater percentage of patients versus placebo with sustained glucocorticoid dosage reduction to ≤7.5 mg/day (55.6% vs 33.3%, difference 22.2% [95% CI: -0.8, 45.2]) and a CRR with sustained glucocorticoid reduction (34.1% vs 24.4%, difference 9.7% [95% CI: -9.5, 28.8], **Figure 5B**).

Compared with placebo, anifrolumab IR elicited numerically greater improvements from baseline in measures of disease activity (SLEDAI-2K, PGA, PtGA) (**Figure 11**) and lupus serologies (**Figure 9**). Compared with the placebo group, patients positive for anti-dsDNA antibodies at baseline had numerically greater reductions in anti-dsDNA antibody levels with anifrolumab IR versus placebo (**Figure 9A**). Patients with low C3 at baseline had an increase in C3 levels across groups (**Figure 9B**). There were no clear differences in C4 increases across groups (**Figure 10**). Compared with the placebo group, the C3 increase was numerically larger with anifrolumab IR from Week 36 and with anifrolumab BR from Week 44 (**Figure 9B**). Overall, the incidence of antidrug antibody positivity at any time during the study was low and similar across groups (anifrolumab BR, 6.7%; anifrolumab IR, 3.9%; placebo, 4.1%).

### *8.3.4. Pharmacokinetics*

The PK analysis included 95 patients who received anifrolumab and had at least 1 quantifiable serum PK observation after the first dose. Anifrolumab exhibited nonlinear PK between the BR and IR groups (**Figure 12**). In IFNGS-high patients (94.5%), the median Week 12 anifrolumab steady-state concentration was 63.4 μg/mL with anifrolumab IR (**Figure 13B**) and 8.2 μg/mL with anifrolumab BR (~50% lower than in nonrenal SLE) (**Figure 13A**). When anifrolumab IR was tapered to 300 mg at Week 16, the median trough concentrations at Weeks 24 and 36 were lower than in patients with nonrenal SLE. Anifrolumab clearance was higher among patients with baseline UPCR >3 mg/mg than those with UPCR ≤3 mg/mg (**Figure 15**).

### *8.3.5. Pharmacodynamics*

The PD analysis included 137 IFNGS-high patients. A median PD neutralization >80% was observed with anifrolumab IR across all visits (Weeks 12, 24, 36, and 52) and with anifrolumab BR at Weeks 12 and 24 only, after which there was a rebound in IFNGS (**Figure 8**). Minimal PD neutralization was observed in the placebo group.

### *8.3.6. Safety and Tolerability*

Safety was evaluated in the mITT population (**Table 20**).

The incidence of any AE was similar across groups. AEs that were more common (≥5% difference) in the combined anifrolumab group versus the placebo group were HZ (16.7% vs 8.2%), urinary tract infection (16.7% vs 10.2%), and influenza (8.3% vs 2.0%). Serious AEs occurred in 22.2%, 17.6%, and 16.3% of the anifrolumab BR, anifrolumab IR, and placebo groups, respectively. The only serious AE reported in >1 patient per treatment group was HZ. There were no deaths during the treatment period; however, there was 1 fatal vascular neurologic AE in the anifrolumab BR group during follow-up. AEs leading to discontinuation of trial intervention occurred in 11% to 12% of patients across groups.

Slightly more patients in the combined anifrolumab versus the placebo group developed any infection or infestation (72.9% vs 63.3%) and any serious infection or infestation (10.4% vs 8.2%). However, the incidences of protocol-specified AESI infections, apart from HZ, were low and similar for the combined anifrolumab group versus the placebo group, including non-opportunistic serious infections (1.0% vs 6.1%), opportunistic infections (1.0% vs 2.0%), influenza (5.2% vs 2.0%), and tuberculosis (no patients). HZ occurred in 20.0% and 13.7% patients in the anifrolumab BR and IR groups, respectively. Of these 16 cases, 6 were serious, 5 were severe and 11 were of mild to moderate intensity, and all were cutaneous (13 localized, 3 disseminated). Most HZ events tended to occur early in the trial and were resolved with treatment. Other AESIs, including malignancy and MACEs, occurred in <1% of patients across anifrolumab groups. There were no reports of anaphylaxis, and there was 1 serious infusion-related reaction in the anifrolumab BR group. No clinically important worsening was observed in hematology or chemistry panels, urinalysis, vital signs, or electrocardiograms.

*Table 20: Anifrolumab safety*

| AEs during treatment, mITT population | Anifrolumab combined (n=96) | Anifrolumab BR (n=45) | Anifrolumab IR (n=51) | Placebo (n=49) |
|---|---|---|---|---|
| **Any AE** | 90 (93.8) | 43 (95.6) | 47 (92.2) | 44 (89.8) |
| **Any AE with outcome of death** | 0 | 0 | 0 | 0 |
| **Any SAE** | 19 (19.8) | 10 (22.2) | 9 (17.6) | 8 (16.3) |
| **Any AE leading to discontinuation of trial intervention** | 11 (11.5) | 5 (11.1) | 6 (11.8) | 6 (12.2) |
| **Adverse events of special interest** | 23 (24.0) | 12 (26.7) | 11 (21.6) | 8 (16.3) |
| Non-opportunistic serious infections[a] | 1 (1.0) | 0 | 1 (2.0) | 3 (6.1) |
| Opportunistic infections[b] | 1 (1.0) | 1 (2.2) | 0 | 1 (2.0) |
| Anaphylaxis | 0 | 0 | 0 | 0 |
| Malignancy | 1 (1.0) | 0 | 1 (2.0) | 0 |
| Herpes zoster[c] | 16 (16.7) | 9 (20.0) | 7 (13.7) | 4 (8.2) |
| Tuberculosis/LTB | 0 | 0 | 0 | 0 |
| Influenza | 5 (5.2) | 2 (4.4) | 3 (5.9) | 1 (2.0) |
| Vasculitis (non-SLE) | 0 | 0 | 0 | 0 |
| Major adverse cardiovascular events according to the CV-EAC | 0 | 0 | 0 | 1 (2.0) |
| **Any AEs ≥5% in the combined anifrolumab group** | | | | |
| Urinary tract infection | 16 (16.7) | 10 (22.2) | 6 (11.8) | 5 (10.2) |
| Herpes zoster | 16 (16.7) | 9 (20.0) | 7 (13.7) | 4 (8.2) |
| Nasopharyngitis | 15 (15.6) | 6 (13.3) | 9 (17.6) | 9 (18.4) |
| Upper respiratory tract infection | 15 (15.6) | 8 (17.8) | 7 (13.7) | 8 (16.3) |
| Bronchitis | 11 (11.5) | 4 (8.9) | 7 (13.7) | 6 (12.2) |
| Influenza | 8 (8.3) | 2 (4.4) | 6 (11.8) | 1 (2.0) |
| Diarrhea | 7 (7.3) | 3 (6.7) | 4 (7.8) | 10 (20.4) |
| Cough | 7 (7.3) | 4 (8.9) | 3 (5.9) | 4 (8.2) |
| Pharyngitis | 7 (7.3) | 3 (6.7) | 4 (7.8) | 2 (4.1) |
| Oral herpes | 6 (6.3) | 3 (6.7) | 3 (5.9) | 2 (4.1) |
| Headache | 5 (5.2) | 2 (4.4) | 3 (5.9) | 4 (8.2) |
| Herpes simplex | 5 (5.2) | 3 (6.7) | 2 (3.9) | 2 (4.1) |
| Nausea | 5 (5.2) | 1 (2.2) | 4 (7.8) | 2 (4.1) |

AE, adverse event; BR, basic regimen; CV-EAC, Cardiovascular Event Adjudication Committee; IR, intensified regimen; LTB, latent tuberculosis; MedDRA, Medical Dictionary for Regulatory Activities; mITT, modified intention-to-treat; SAE, serious adverse event; SLE, systemic lupus erythematosus. AEs are coded using MedDRA version 22.1. Percentages are based upon the 145 patients in the mITT who received at least 1 dose of anifrolumab or placebo. Any AE occurring from the day of the first dose to 28 days after the last dose was included. [a]Excludes tuberculosis/latent tuberculosis and influenza; [b]Excludes herpes zoster and visceral disseminated herpes zoster; [c]Includes visceral disseminated herpes zoster.

### 8.4. Discussion

These data describe the results from a phase 2 TULIP-LN trial, which explored the safety and efficacy of adding 2 different anifrolumab dosages to standard therapy for patients with active, proliferative LN. The primary endpoint (UPCR improvement in the combined anifrolumab group versus the placebo group) was not met; however, anifrolumab IR was associated with numeric efficacy across a range of clinically meaningful renal endpoints, including proteinuria, multiple stringent CRR definitions (requiring UPCR improvement and inactive urinary sediment), and sustained glucocorticoid dosage reductions.

Surprisingly, in contrast to previous observation in SLE from study 1013, study 05 and study 04, intensified anifrolumab dosing was required to elicit clinical efficacy in patients with LN, likely because anifrolumab IR yielded similar serum exposure and PD neutralization to that seen with anifrolumab BR (SLE dosing) in nonrenal SLE. In contrast, because of the proteinuria in LN leading to increased clearance, anifrolumab BR elicited suboptimal serum exposure that was ~50% lower in patients with LN than in patients with nonrenal SLE. Indeed, anifrolumab BR elicited limited PD neutralization and clinical responses.

Reduction of proteinuria is strongly associated with reduced risk of end-stage kidney disease [33]. As such, attenuation of proteinuria is an appropriate, objective surrogate endpoint for a phase 2 proof-of-concept trial. Here, 24-hour UPCR improved by approximately 70% from baseline across groups. Furthermore, anifrolumab IR yielded a 30% numeric improvement over placebo at Week 52 in cumulative proteinuria, which is a unique endpoint in LN trials that adds clinical value because it represents overall proteinuria improvement over time, making it less susceptible to short-term confounders, such as collection errors, diet, and exercise.

Anifrolumab IR was also associated with efficacy over placebo across all CRR definitions as early as Week 12. Anifrolumab IR yielded the strongest response (treatment difference 28%) for $CRR_a$, a highly stringent criterion requiring no hematuria (a marker of glomerular inflammation and acute kidney injury [34]). Additionally, more patients with high baseline glucocorticoid dosages in the anifrolumab IR group versus the placebo group attained a sustained glucocorticoid dosage reduction or a CRR with a sustained dosage reduction.

Overall, the safety profile observed with anifrolumab in patients with LN was consistent with that observed in patients with nonrenal SLE, including numerically higher incidence of influenza and cutaneous HZ with anifrolumab versus placebo [11]. Most AEs were mild or moderate in intensity, were nonserious, and did not lead to discontinuation of the trial intervention. Notably, anifrolumab IR was not associated with higher incidence of AEs or AESIs, including HZ, compared with anifrolumab BR.

Patients with LN are nearly double as likely to develop serious infections and are at higher risk of HZ infection than patients with nonrenal SLE [35]. In line with this, the incidence of cutaneous HZ was higher among patients with LN than those with nonrenal SLE, perhaps owing to LN disease severity requiring more potent background immunosuppressive treatments [5]. This theory is supported by the observation that cutaneous HZ tended to occur early in the trial when glucocorticoid dosages were high.

Overall, the TULIP-LN results supports the efficacy and safety of anifrolumab IR in LN. Anifrolumab IR was superior over placebo for several clinically relevant endpoints and was generally well tolerated over a 52-week period.

### 8.5. Summary

This Phase II LN study 07 was a 2-year global, multicenter, exploratory, double-blind placebo-controlled, study to explore the efficacy and safety of anifrolumab in patients with active, Class III or IV (both with or without Class V) LN. The study evaluated 2 anifrolumab dosing regimens as compared with placebo: a basic regimen using the proposed dose for SLE patients (300 mg IV Q4W) and an intensified regimen (first 3 doses of 900 mg IV Q4W followed by 300 mg IV Q4W for the remainder of the study). All patients received MMF plus glucocorticoids as background SOC therapy. The 147 randomized patients were allocated at a 1:1:1 ratio to the anifrolumab intensified, anifrolumab basic or placebo group, respectively. Despite the study not meeting its primary objective, relative difference in UPCR change from baseline to Week 52 for the anifrolumab groups combined vs placebo, exploratory and post-hoc efficacy analyses suggested a beneficial effect of anifrolumab intensified regimen vs placebo on a range of clinically meaningful endpoints at Week 52. The proportion of patients achieving CRR with ≤ 0.7 mg/mg UPCR threshold was numerically larger in anifrolumab vs placebo group (treatment difference 14.3%; 95% CI: -5.8, 34.5). In addition, more anifrolumab-treated patients achieved CRR using a more stringent UPCR threshold (≤ 0.5 mg/mg) as compared with placebo (treatment difference 14.2%; 95% CI: -5.4, 33.9).

One important treatment goal in LN is to achieve rapid and maintained control of the renal disease; in this study the time to achieve CRR (≤ 0.5 mg/mg UPCR threshold) that was sustained over time through Week 52 was numerically shorter with anifrolumab intensified dosing than with placebo (hazard ratio, 1.46; 95% CI: 0.71, 3.14). Moreover, numerically more patients in anifrolumab group compared with placebo achieved a sustained reduction of oral glucocorticoids, i.e., to a dose of ≤ 7.5 mg/day of prednisolone or equivalent by Week 24 and maintaining this dose through Week 52 (treatment difference 22.2%; 95% CI: -0.8, 45.2).

In terms of anifrolumab exposure, this was likely suboptimal in the basic dosing regimen due to higher clearance associated with proteinuria in LN, whereas the exposure in intensified regimen was more comparable to exposure for 300 mg IV Q4W in non-renal SLE.

## 9. EXAMPLE 3: Subcutaneous administration of anifrolumab

### 9.1. Phase I study MI-CP180 of IV anifrolumab in patients with SSc

Mean anifrolumab serum concentrations after a single-dose administration based on body weight are presented in **Figure 16A.** After a single-dose administration, anifrolumab exhibited nonlinear-linear PK at lower dose levels (<10.0 mg/kg) in both IFNGS high and IFNGS low patients. A dose-proportional increase in $C_{max}$ was observed, but an increase in AUC was more than dose proportional between 0.1 and 10.0 mg/kg. Anifrolumab t1/2 was more prolonged in higher dose cohorts. At the highest dose level investigated (20.0 mg/kg), the terminal t1/2 was approximately 12 days.

### 9.2. Phase I of IV and SC anifrolumab in healthy volunteers (study 06)

In this Phase I randomized, placebo-controlled study, 30 healthy adults were assigned to three treatment cohorts (anifrolumab 300 mg SC (n=6), anifrolumab 300 mg intravenous (n=6), anifrolumab 600 mg SC (n=6)) and placebo (n=4/cohort). After SC administration, exposure to anifrolumab increased dose proportionally from 300 mg to 600 mg based on area under the serum concentration-time curve. Arithmetic mean serum anifrolumab concentration-time profiles following single IV and SC administration are shown in **Figure 16B.** As reported in Tummala *et al.* 2018 [19], which is incorporated herein by reference in its entirety, this study estimated the bioavailability to anifrolumab in healthy volunteers to be 87% of the intravenous exposure.

### 9.3. Phase II of SC anifrolumab in SLE patients (study 08)

This study was designed to characterize the pharmacokinetics and pharmacodynamics of subcutaneously administered anifrolumab (**Figure 17A**).

The study explored the clinical pharmacology, safety, and exploratory efficacy of subcutaneous anifrolumab. Pharmacokinetics in study 08 were consistent with the high bioavailability in study 06 (healthy volunteers) and high CL in IFNGS high patients with SLE. Anifrolumab, administered subcutaneously every 2 weeks to patients with SLE and moderate-to-severe skin manifestations had non-linear pharmacokinetics that were more than dose proportional, and neutralized the type I interferon gene signature in a dose-dependent manner (**Figure 17B** and **Figure 17C**). In particular, 150 mg or 300 mg of subcutaneous anifrolumab administered every 2 weeks for 50 weeks had non-linear pharmacokinetics, whereby $C_{trough}$ concentrations were more than dose proportional. The number of adverse events with subcutaneous anifrolumab was similar to the numbers observed following intravenous administration in larger studies of patients with SLE.

*The results of study 08 are fully described in Bruce et al.* [36], *which is incorporated herein by reference in its entirety.*

Study 08 was limited by small samples sizes, and no conclusions could be drawn about the biological effects of the study drug (e.g., on complement C3 or C4 concentrations) or its clinical efficacy. The inclusion of only patients with high type I interferon gene signatures and active skin disease also limited the generalizability of the study to patients with similar disease characteristics. The study was further limited by the increasing frequency of missing values with time.

### 9.4. Conclusion

The PK of anifrolumab consistently exhibited target mediated drug disposition where the concentrations or exposures decreased more than dose-proportional at lower dose levels. High bioavailability of anifrolumab administered via SC injection was observed in study 06 (healthy volunteers); the ratio of the AUC of anifrolumab SC to anifrolumab IV under 300 mg was approximately 87%.

## 10. EXAMPLE 4: Determination of the optimal subcutaneous unit dose

### 10.1. Aim

In order to detect an optimal dosage regimen for subcutaneous administration of anifrolumab, the inventors developed a population PK and a PK/PD model, designed to utilize existing human clinical trial. The PK data from phase III studies 04 and 05 and phase II study 1013 were used to assist the development of the population PK model.

In studies 1013, 04, and 05, in SLE patients, treatment with a ≥ 300 mg Q4W anifrolumab dose resulted in a rapid and sustained neutralization of type I IFN 21-gene signature of larger than 80% (**Figure 18**). Patients who received anifrolumab 150 mg showed a sub-optimal neutralization of the type I IFN gene signature and patients who received placebo did not show any neutralization. Patients with higher PD suppressions were associated with higher BICLA and SRI(4) response at Week 52.

An initial goal of the inventors was to detect a subcutaneous dose providing an equivalent exposure as a standard 300 mg IV (Q4W) dose, while concomitantly allowing more regular dosing that could be provided in a lower volume. This was based on the understanding that 300 mg IV Q4W provides optimal clinical PK profiles and clinical efficacy (e.g. in terms of achieving BICLA response in SLE patients) as reported e.g. in Furie *et. al.* 2017 [11] which is incorporated herein by reference in its entirety.

**10.2. 5.3: Results**

***10.2.1. Initial selection of the subcutaneous dose for anifrolumab***

In an initial analysis, the inventors determined specific dosage regimens predicted to provide equivalent exposure to that achievable with 300 mg Q4W IV. A dosage regimen of 105 mg subcutaneous weekly (QW) was initially found to provide an AUC ratio close to (or slightly greater than) 1 (**Figure 19A**), even where projected bioavailability was reduced by ~7% relative to that reported in Tummal *et. al.* 2018 [19] (incorporated herein by reference in its entirety) to account for inter-individual variance in bioavailability (**Figure 19B**). 105 mg subcutaneous QW appeared to provide comparable or improved median trough concentrations and IFNGS suppression as the comparative 300 Q4W mg IV dose **(Figure 20A, Figure 20B).** From these initial analyses it appeared that SC 105 mg QW dose of anifrolumab should be selected as equivalent to a 300 mg Q4W and thus as having the optimal efficacy/risk profile for the treatment of SLE patients. Importantly these analyses assumed that the 300 mg IV dose was on of close to the plateau of the dose response curve for anifrolumab, i.e. that increasing the dose beyond 300 mg IV Q4W would provide not provide any meaningful benefit to patients, particularly when taking into account the increased risk of herpes zoster infection for higher doses.

***10.2.2. Amended selection of the subcutaneous dose for anifrolumab***

The inventors therefore first considered 105 mg QW to be the optimal SC dose of anifrolumab for the treatment of type I IFN mediated disease based on the data available from the MUSE study, study 06 and study 08. However, to confirm the selection of the 105 mg SC dose, the inventors conducted further analysis of the data from the TULIP I (study 04) and TULIP II (study 05) clinical trials.

Using the additional data, a positive-exposure-BICLA relationship in IFNGS high patients was demonstrated. Surprisingly, this relationship was observed even within the 300 mg IV Q4W group **(Figure 21A** and **Figure 21B**). BICLA response within the 300 mg IV Q4W patient group was therefore variable. Logistic regression of the week 52 BILCA response in patients confirmed that PK exposure was a significant covariate in both TULIP I and TULIP II. $C_{ave}$ was found to be statistically significant in both the analysis of all-comers and IFNGS high completed the treatments in both TULIP I and TULIP II independently and pooled TULIP I and TULIP II analysis. Exposure-response demonstrating higher $C_{ave}$ were correlated with higher BICLA and SRI(4) in pooled data from the TULIP I and TULIP II studies. In other words, there was exposure-dependent variability in response to anifrolumab within lupus patients administered 300 mg Q4W IV (**Figure 21A** and **Figure 21B**).

Surprisingly, the 300 mg IV Q4W dose was thus found to reside on the onset of the plateau of exposure response, whilst the suboptimal 150 mg IV dose resided in the step region of the exposure-response curve (**Figure 22A**). As a consequence of these analyses, the inventors determined that a 105 mg QW subcutaneous dose (previously considered equivalent to a 300 mg IV Q4W dose) would not provide the optimal balance of efficacy and safety in lupus patients. The inventors thus determined to select another dose for SC administration that would mitigate the impact of variability in response a population of lupus patients.

In summary, from initial analysis, it appeared that administration of a subcutaneous dose of 105 mg QW anifrolumab would achieve at least a similar efficacy as 300 mg IV Q4W. However, surprisingly, following further analysis by the inventors of newly available data from further studies, it was found that the concentration of this weekly (QW) dose could be increased without reaching a maximum threshold in terms of bioavailability and efficacy. In other words, the QW dose could be increased beyond 105 mg to provide even greater blood plasma concentrations and IFNGS suppression, and to mitigate the observed variability in response in SLE patients. A dose of 105 mg would therefore be sub-optimal.

The surprising additional dose-response curve data were further validated by demonstrating that the probability of meeting a relevant BICLA response (in IFNGS high patients) was increased for weekly subcutaneous administration with concentrations higher than the 105 mg dose (**Table 21**). These data demonstrate the unexpected position of the dose-response plateau (e.g. under subcutaneous administration), which shifts to the right for doses increasing above 105 mg (**Figure 22B**), showing the maximal BICLA response is in fact achievable with a dose of greater than 105 mg and that a higher dose would be preferable (**Table 21**).

**Table 21: SC Efficacy Projection assuming no dose delays/interruptions.**

| | 90 mg SC QW | 105 mg SC QW | 120 mg SC QW | 135 mg SC QW | 150 mg SC QW |
|---|---|---|---|---|---|
| Equivalent IV dose | ~ 300 mg IV Q4W | | ~<400 mg IV Q4W | ~<450 mg IV Q4W | < 500 mg IV Q4W<br>~300 mg SC Q2W |
| Median Cave ratio to 300 mg IV | 0.92 | 1.14 | 1.36 | 1.59 | 1.81 |
| % exceeded 95th percentile of 300 mg IV | 3.3% | 9.4% | 20.1% | 33.5% | 48.9% |
| % overlapped with ≥5th percentile of 1000 mg IV | 0.3% | 1.8% | 5% | 11% | 21% |
| % IFNGS high pts with 55% chance of BICLA response | ~86% | ~94% | ~98% | ~99% | ~100% |
| % IFNGS high pts with 60% chance of BICLA response | ~10% | ~23% | ~38% | ~55% | ~68% |

### *10.2.3. The bioavailability of anifrolumab is highly variable*

Upon further investigation as to the bioavailability of anifrolumab, the inventors elucidated that a surprisingly high level of variability in anifrolumab bioavailability subsequent to subcutaneous administration may exist amongst different patients. The high level of variability in anifrolumab bioavailability was not appreciated in previous studies reporting >80% bioavailability for subcutaneous administration (see **Example 3**) [19]. The bioavailability (F1) of anifrolumab in Study 08 (SLE patients, SC) was found to be 81% in healthy volunteers using the population PK model (**Table 22**).

The bioavailability of a typical monoclonal antibody via subcutaneous injection ranged from 52-80% [37]. The inventors conducted external validation of Study 08, Ph2 SC in SLE, using a PPK model developed with healthy volunteers and SLE patients from IV studies to determine the bioavailability in lupus population.

***Table 22:*** Anifrolumab bioavailability based on healthy volunteers

| Parameters ± SE | Final IV SLE model (including 6 subjects from 06 IV arm) | Study 06 HVs (300 mg IV: 6, 300 mg SC: 6, 600 mg SC: 6) |
|---|---|---|
| CL (IFNGS high) | 0.193 L/day | - |
| CL (IFNGS low/HVs) | 0.153 L/day | 0.146±0.036 L/day |
| IIV: CL | 0.109 (CV: 33.1%) | 0.0431 (CV: 20.8%) |
| F1 | - | 0.812±0.12 |
| Ka | - | 0.274±0.124 /day |
| IIV: Ka | - | 0.221 (CV: 47%) |

In-depth analysis of the data from study 08 revealed that bioavailability was affected by SC administration site. In particular, when the bioavailability of 300 mg at the abdomen was estimated versus IV, the bioavailability (F1) was estimated to be 85.4% compared to 81% when the sites of injection was not taken into consideration. As such, $C_{troughs}$ following injection at thigh trended downward compared to injection at abdomen (**Figure 23A** and **Figure 23B**). As such, it was surprisingly concluded that bioavailability may, in fact, be as low as 70%, taking into account variability due to injection site and the higher variability in bioavailability for lupus (SLE) patients compared to healthy volunteers. Importantly, if a bioavailability (F1) of 81-87% was assumed, 105 mg was initially projected to provide a comparable $C_{ave}$ to that of 300 mg IV (**Figure 24**). By contrast, when the estimated bioavailability was reduced to ~70% or less, the median $C_{ave}$ of the 105 mg QW subcutaneous dose fell to below 1 (**Figure 25A, Figure 25B** and **Table 23**).

**Table 23: Anifrolumab bioavailability**

| Bioavailability | 90 mg SC QW | 105 mg SC QW | 120 mg SC QW | 135 mg SC QW | 150 mg SC QW |
|---|---|---|---|---|---|
| 82% | 0.92 | 1.14 | 1.36 | 1.59 | 1.81 |
| ~70% | 0.73 | 0.92 | 1.11 | 1.31 | 1.49 |
| ~60% | 0.57 | 0.73 | 0.89 | 1.06 | 1.22 |
| Values = median $C_{ave}$ to 300 mg IV; SC= subcutaneous | | | | | |

Furthermore, there was an undesirable 30% overlap in $C_{ave}$ between 105 mg SC QW and the suboptimal IV dose, 150 mg Q4W versus the only 16% overlap observed when the bioavailability was assumed to be 81% (**Figure 25A**). However, when a SC 120 mg dose was used, the $C_{ave}$ overlap with the 150 mg IV dose was less than the overlap with the optimal IV dose of 300 mg IV, even when a low bioavailability of 70% was assumed (**Figure 25B**). Furthermore, the 120 mg SC QW dose had minimal overlap with the undesirable 1000 mg IV dose (**Figure 25C**), at which the risk of herpes zoster infection is increased (**Figure 27**). A 150 mg SC QW dose had an undesirable overlap with the 1000 mg IV Q4W dose. Even more surprisingly, a SC dose of 120 mg or more was projected to have better PD suppression (**Table 24**) than the assumed optimal 300 mg IV dose (**Table 25**).

Selection of a dose higher than 105 mg, preferably 120 mg or higher, therefore optimizes the exposure-response by minimizing the impact of the variability of the onset of response and bioavailability in patients with SLE and LN (**Table 24, Figure 26A** and **Figure 26B**). A SC dose of below 150 mg QW is also desirable to reduce the risk of herpes zoster infection.

**Table 24: Calculated % PD suppression at week 24, SC dose**

| SC | WK24 Suppression (%) | | |
|---|---|---|---|
| Dose (mg) | 75% | 80% | 90% |
| 90 | 89.0 | 84.6 | 63.8 |
| 105 | 92.9 | 89.8 | 69.2 |
| 120 | 94.8 | 91.9 | 74.2 |
| 135 | 96.0 | 93.9 | 75.8 |
| 150 | 96.5 | 94.6 | 80.2 |

**Table 25: Calculated % PD suppression at week 24, IV dose**

| IV | WK24 Suppression (%) | | |
|---|---|---|---|
| **Dose (mg)** | **75%** | **80%** | **90%** |
| **300** | 74.2 | 68.3 | 42.5 |
| **400** | 82.9 | 77.9 | 54.7 |
| **450** | 85.9 | 80.8 | 56.4 |
| **500** | 88.7 | 84.8 | 62.5 |
| **600** | 92.7 | 88.8 | 68.9 |
| **1000** | 96.9 | 94.5 | 80.2 |

Doses of 120 mg and 135 mg QW particularly provide reasonable benefit-risk profiles. At doses at 150 mg QW or above, there is an increase in safety risk e.g. an increase in the risk of herpes zoster in patients, given that a SC dose of 150 mg QW is equivalent to a 1000 mg IV Q4W (**Figure 25C, Figure 27**). A subcutaneous dose of less than 150 mg QW and more than 105 mg QW was therefore determined as the preferred dose. A subcutaneous dose of less than 150 mg QW and less or equal to 135 mg was determined as the more preferred dose. A subcutaneous dose of 120 mg was determined as optimal dose.

To summarize, the inventors have surprisingly found that the optimal subcutaneous dose of anifrolumab may first appear to be 105 mg QW given the preliminary data that was previously available (**FIG. 12**). However, further data and analyses surprisingly revealed that a dose of 105 mg QW or lower would under-dose a significant proportion of patients (**Figure 22B, Table 23**). Thus, a particularly advantageous dosing regimen demonstrated by the inventors was doses higher than 105 mg QW. A particularly optimal dose was determined to be 120 mg subcutaneous QW, which is equivalent to approximately 400 mg IV Q4W, depending on estimated bioavailability. The optimal SC dose is therefore surprisingly >30% higher than what would be considered optimal based solely on a comparison with 300 mg IV Q4W and the previously understood bioavailability of anifrolumab.

The inventors have thus surprisingly demonstrated that a dose of greater than 105 mg SC QW and less than 150 mg SC QW, and in particular a dose of 120 mg QW (a) maximizes efficacy whilst maintaining an acceptable safety profile, (b) mitigates the impact of variability in bioavailability and (c) mitigates the impact of variability in the onset of response. Thus, dosing at greater than 105 mg QW advantageously accounts for the variance in bioavailability, leading to improved therapeutic outcome. A dose of less than 150 mg QW mitigates the risk of herpes zoster infection.

Pharmacokinetic data in healthy volunteers (study 06 [IV arm only]) and in patients with SLE (studies 1013, 02, 04, and 05) were also pooled to evaluate the impacts of covariates, such as demographics and renal/liver function tests, on PK exposure. Higher body weight and type I IFN test high patients were found to have significantly higher clearance (CL) and lower concentrations. However, surprisingly there was no clinically relevant impact of these covariates on efficacy and safety. Surprisingly, other covariates pertaining to specific populations evaluated in population PK modeling were not found to be significant including race/ethnicity/region, age, gender, renal/hepatic function tests, standard of care therapy (e.g., OCS, anti-malarial, azathioprine, methotrexate, mycophenolate mofetil, mycophenolic acid, mizoribine, and NSAIDs), and commonly used medications in SLE patients (ACE inhibitors and HMG-CoA reductase inhibitors).

### 10.3. Conclusion

The present inventors have demonstrated that an anifrolumab dose of <150 mg QW and >105 mg QW will provide at least similar or even a higher $C_{ave}$ over 52 weeks to that of 300 mg IV Q4W. A 120 mg SC QW dose will particularly provide an efficacy at least equivalent to that demonstrated for a 300 mg IV Q4W dose in LN and SLE patients. It is further plausibly demonstrated that a 120 mg SC QW dose will provide an efficacy greater than that demonstrated for a 300 mg IV Q4W dose.

A dosing regimen of 900 mg anifrolumab IV Q4W for 6 doses followed by 120 mg anifrolumab SC QW was thus selected based on a combination of PK/PD data and modelling of data from the Phase II LN study (Study 07, see **Section 8.** and **Table 8**) and data from the anifrolumab IV and SC clinical program in SLE as described **Section 10.2.**

Study 07, assessed 2 dosing regimens: a basic regimen using the proposed dose for SLE patients (300 mg IV Q4W) and an intensified regimen, which commenced with 3 doses of 900 mg IV Q4W followed by 300 mg IV Q4W for the remainder of the study. The intensified regimen showed results suggesting a greater treatment benefit over the basic regimen.

However, the exposure in the initial phase 300 mg IV Q4W of both regimens was suboptimal when compared with the 300 mg IV Q4W dose in SLE without active renal disease. Therefore, a more intensive dosing regimen was selected, with an initial dose of 900 mg IV Q4W for 6 doses, followed by 120 mg SC QW or 300 mg IV Q4W. This regimen will provide sustained anifrolumab exposure/PD suppression and improved UPCR outcome compared with the previously evaluated dose regimens in study 07. The 120 mg SC QW dose will provide at least similar or non-inferior exposure and PD suppression to that of 300 mg IV Q4W in patients with LN.

In summary, the primary objective of study 07 was to evaluate the efficacy of anifrolumab as assessed by 2 different dosing regimens in addition to standard therapy, measured by the relative difference between the combined anifrolumab treatment group and the placebo group in the change from baseline to Week 52 in 24-hour UPCR. The study did not meet this primary endpoint, but the results showed efficacy of the intensified anifrolumab dosing regimen compared with placebo across a range of clinically meaningful endpoints including CRR at Week 52 and sustained OCS tapering. In contrast, anifrolumab exposure was suboptimal with basic dosing regimen due to higher clearance associated with proteinuria in LN.

The IV route of administration has primarily been used in the anifrolumab clinical program in SLE without active renal disease, but a more convenient SC route of administration is also being developed. Both the IV and SC routes of administration have been shown to be safe and well tolerated in the anifrolumab clinical development program in patients with SLE (IV and SC) and in LN (IV). The SC route of administration using aPFS for anifrolumab is expected to provide increased convenience and dosing flexibility and reduced exposure to infection risk related to clinic visits for dosing (including but not limited to influenza or COVID-19) for patients and/or caregivers and to improve treatment accessibility and compliance compared to IV dosing.

## 11. EXAMPLE 5: Selection of dosage regime for lupus nephritis

### 11.1. Introduction

Study 07 did not meet its primary and secondary endpoints for the combined anifrolumab dosing regimens compared with placebo, but the efficacy results for the intensified dosing regimen plausibly indicated a clinical benefit with an acceptable safety profile. By contrast, the basic dosing regimen, whilst providing a treatment effect, resulted in suboptimal anifrolumab exposure in patients with active proliferative LN as compared with SLE without active renal disease.

An aim of study 07 was to achieve the same exposure to anifrolumab as for the proposed dose of 300 mg IV Q4W for SLE without active renal disease. Study 07 (NCT02547922, Section 8) assessed 2 anifrolumab dosing regimens: a basic regimen using the proposed dose for SLE patients (300 mg IV Q4W) and an intensified regimen, which commenced with 3 doses of 900 mg IV Q4W followed by 300 mg IV Q4W for the remainder of the study. As described in Section 8, the intensified regimen showed results suggesting a greater treatment benefit over the basic regimen. However, the exposure in both regimens was suboptimal when compared with the 300 mg IV Q4W dose in SLE without active renal disease (**Figure 13** and **Figure 14**).

### *11.1.1. Pharmacokinetics*

Anifrolumab binds to IFN-αR1 with high specificity and affinity and prevents the formation of a complex with IFN-αR2, leading to blockade of downstream signaling activities. The antibody-receptor complex is then rapidly internalized. Thus, the PK of anifrolumab exhibits target receptor-mediated clearance, with a more rapid clearance observed at lower concentration levels.

In study 07, consistent with earlier findings, nonlinear PK was observed between patients in the basic regimen (300 mg IV Q4W) and patients in the intensified regimen (900 mg IV Q4W for the first 3 doses followed by 300 mg IV Q4W), all with active, proliferative LN. However, lower concentrations were observed during the maintenance phase of 300 mg IV Q4W in both the basic and intensified regimens compared with those of SLE (**Figure 13** and **Figure 14**).

Initial population PK analysis of data from LN patients in study 07, revealed a higher typical clearance of anifrolumab in LN patients than that of SLE patients, 0.245 L/day versus 0.193 L/day. In addition to the previously identified covariates, patients with lower baseline albumin and higher UPCR 24-hour measurements had higher clearance. Prior to the subsequent addition of a time-dependent UPCR covariate, the initial model showed that the patients with larger decrease in clearance over time showed greater improvement of UPCR (**Figure 28A** and **Figure 28B**).

The change in clearance was estimated via a sigmoidal time-dependent function that was multiplied with the linear clearance of the final model presented in Section 3.5.

*Equation 4* $$CL = \theta_{CL} exp\left(\frac{TMAX \cdot time}{TC50 + tim}\right) f(X) e^{\eta CL}$$

Where:

$\theta_{CL}$ is typical clearance,
*f*(*X*) is covariate model *X*;
$\eta CL$ is inter-subject variability of clearance;
*TMAX* is the maximal possible change in the log of clearance; and
*TC50* is the time to reach half the maximal change in log clearance.

### *11.1.2. Pharmacodynamics*

Anifrolumab targets the IFN pathway. To follow the biologic effect of anifrolumab on its target, a 21-gene assay was used to measure the expression of type I IFN-inducible genes in whole blood in both SLE and LN studies. Type I IFN gene signature test-low patients do not have elevated gene signature, so neutralization of the type I IFN PD signature is relevant for test-high patients only. In studies 1013, 04, and 05, in SLE patients, treatment with a ≥ 300 mg Q4W anifrolumab dose resulted in a rapid and sustained neutralization of type I IFN 21-gene signature of larger than 80% (**Figure 18**).

For patients with LN, both the BR and IR showed a rapid and pronounced neutralization of the type I IFN PD signature following the first dose of anifrolumab, in common with the SLE patients who received ≥ 300 mg dose. The patients in the intensified regimen maintained a ≥ 80% median neutralization of the type I IFN PD signature across all study visits to Week 52 as per the SLE patients, but lower PD suppression was observed in patients from basic regimen (**Figure 8**). LN patients with high proteinuria had higher anifrolumab clearance. Thus, a numerically lower PD suppression was observed in the patients with high proteinuria, baseline UPCR > 3 mg/mg, that received basic regimen (300 mg IV Q4W), compared with that in intensified regimen (900 mg IV Q4W for the first 3 doses followed by 300 mg IV Q4W) (**Figure 29A**). Furthermore, for the patients with high proteinuria that were able to attain an approximate PD suppression of > 90%, a much larger UPCR improvement was observed (**Figure 29B**). This suggests a higher PD suppression as a result of higher dose could improve the likelihood of UPCR outcome.

### *11.1.3. Pharmacokinetics/Pharmacodynamics relationship*

A longitudinal population model was developed to characterize the relationship between anifrolumab PK and UPCR in LN patients. Anifrolumab was found to improve proteinuria in an exposure-dependent manner, and the improvement in proteinuria was, in turn, found to lower the anifrolumab clearance. After completion of the PK-UPCR model, a dropout model was developed sequentially, and was integrated with the PK-UPCR model. The resulting PK-UPCR dropout model was used for simulation to evaluate different doses and dosing regimens. The UPCR and treatment discontinuation are variables of particular interest since these are the 2 major drivers of the CRR endpoint.

#### *11.1.3.1. PK-UPCR model*

The UPCR dynamics model was described by a modified turnover model defined by the differential equation:

*Equation 5* $$\frac{dU(t)}{dt} = E_a(t) \times \frac{log2}{k} \times \left(\left((1 - E_s) \times U_b\right)^x - U(f)^x\right)$$

Where:

$U(t)$ is the time-dependent UPCR;
$E_a(t)$ is the time-dependent effect of anifrolumab on the UPCR dynamics;
$k$ a characteristic time parameter;
$E_s$ the constant effect of standard of care treatment on the UPCR dynamics;
$U_b$ is the baseline UPCR; and
$x$ is a shape parameter.

The shape parameter $x$ introduces flexibility that allows the UPCR dynamics to deviate from a perfect exponential behavior. The time-dependent effect of anifrolumab on the UPCR dynamics was further defined as:

*Equation 6* $$E_\alpha(t) = 1 + \alpha \times c(t)$$

Where:

$\alpha$ is a parameter determining the strength of the time-dependent effect of anifrolumab on the UPCR dynamics; and
$c(t)$ is the anifrolumab serum concentration measured in nM.

A parameter scaling factor was introduced into *Equation 6* to avoid a small value of the parameter $\alpha$. When $x = 1$, and in the absence of anifrolumab so that $E_\alpha(t) = 1$, the parameter $k$ becomes the half-life of the equilibration process of U(t). Thus, according to this model, the effect of anifrolumab can be interpreted as the decrease of the UPCR equilibration half-life, or, when $x \neq 1$, the decrease of a more general characteristic time for onset of effect. An additional effect of anifrolumab on the UPCR steady state, i.e., modulating the term $((1 - E_s) \times U_b)^x$, was explored but did not improve the model significantly.

Lognormal inter-individual variability was defined for the parameters $k$, $U_b$, and the standard deviation of the residual error. The inter-individual variability for $E_s$ was defined according to $E_s = 1 - (1 - E_{sTV})e^\eta$, where $E_{STV}$ is the typical value of $E_s$ and $\eta$ is a random effect parameter. This maneuver effectively makes $(1 - E_s)$ log normal but has the purpose of having parameter $E_S$ in the model with the interpretation of reduction of the UPCR baseline due standard of care treatment.

The PK part of the PK-UPCR model was based a population PK model in patients with SLE, but with the following changes. The parameters $Q$ and $K_{SS}$ were fixed to previously estimated values due to the relatively more limited and sparse PK data in LN patients compared with the pooled PK data in SLE patients. In addition, inter-individual variability of the parameter $V_2$ was omitted. Finally, and most importantly, the anifrolumab linear clearance was redefined to include a dependence on the time-dependent model state variable describing UPCR, $U(t)$, according to:

*Equation 7* $$CL = CL_{TV} \times F_{IFN} \times F_{WT} \times F_{UPCR} \times e^\eta$$

Here, $CL_{TV}$ is the typical value of the clearance and $\eta$ is a random effect parameter. The factor $F_{IFN}$ is capturing the effect of the IFN test on clearance, with $F_{IFN} = 1$ for IFN high patients and $F_{IFN} = CL_{IFNLOW}$ for IFN low patients, where

$CL_{IFNLOW}$ is parameter to be estimated. The factor $F_{WT}$ is capturing the effect of body weight on clearance according to:

*Equation 8* $$F_{WT} = \left(\frac{BW}{63.1}\right)^{CL_{WT}}$$

Where:

$CL_{WT}$ is a parameter to be estimated; and
the reference value 63.1 is the median body weight in study 07.

$F_{UPCR}$ is capturing the time-dependent change in clearance due to UPCR dynamics, according to:

*Equation 9* $$F_{UPCR} = 1 + CL_{UPCR} \times (U(t) - 2.53)$$

Where:

$CL_{UPCR}$ is a parameter to be estimated; and
the reference value 2.53 is the observed median UPCR baseline.

The interpretation of the parameter $CL_{UPCR}$ is the relative change in anifrolumab clearance per mg/mg change in UPCR.

A significant effect of anifrolumab on the onset of UPCR improvement was estimated ($\alpha$ = 1.11, CI 0.251-2.50). Furthermore, it was estimated that each mg/mg reduction in UPCR would result in a 21.0% (CI 18.9 - 23.0) decrease of the linear anifrolumab clearance, using the median observed UPCR baseline as a reference. The PK-UPCR model adequately described the UPCR and PK data, as assessed by dropout-corrected visual predictive check plots (**Figure 30** and **Figure 31**).

**11.2. Dropout model**

After developing the PK-UPCR model, its population parameters were fixed and the model was expanded to account for dropout based on a time to event analysis. The purpose of this analysis was to identify whether anifrolumab exposure as such, or its impact on UPCR, could be associated with the risk of discontinuation. The following proportional hazards model was developed:

*Equation 10* $$\beta_0/365) \times e^{\left(\left(\frac{\beta_{PK}}{10^3}\right) \times c(t) + \beta_{UPCR} \times U(t)\right)}$$

Where:

$\beta_0$ is the constant base hazard; and
$\beta_{PK}$ and $\beta_{UPCR}$ are parameters describing the association of risk with the time-dependent concentrations of anifrolumab and UPCR.

Parameter scaling factors were introduced to avoid small values of the parameters $\lambda_0$ and $\beta_{PK}$.

The parameter estimates of the dropout model are shown in **Table 26.** This model adequately described the dropout data (**Figure 32**). The dropout model suggested that patients with higher UPCR levels, as well as lower average anifrolumab concentration, were significantly more likely to discontinue from the treatment.

**Table 26: Parameter estimates of the PK-UPCR dropout model**

| Parameter | Unit | Point estimate | 95% confidence interval |
|---|---|---|---|
| Constant base hazard, $\beta_0$ | 1/year | 0.253 | 0.0978 to 0.496 |
| Association to anifrolumab concentration, $\beta_{PK}$ | 1/µM | -4.94 | -10.7 to -2.05 |

(continued)

| Parameter | Unit | Point estimate | 95% confidence interval |
|---|---|---|---|
| Association to UPCR concentration, $\beta_{PK}$ | mg/mg | 0.304 | 0.128 0.595 |
| PK, pharmacokinetic; UPCR, urine protein-creatinine ratio | | | |

***11.2.1. Simulations with the PK-UPCR-dropout model***

The PK-UPCR-dropout model was used to simulate different dosing regimens. For each simulated scenario, the placebo-adjusted proportion of UPCR responders was computed as a function of time. A UPCR responder was defined as patient with UPCR 24-hour ≤ 0.5 mg/mg and not discontinued from treatment.

First, the impact of extending the intensified treatment period beyond three 900 mg doses was simulated. The PK-UPCR-dropout model found that increasing the duration of the intensified phase of 900 mg doses to 24 weeks (six doses), from the 12 weeks (three doses) in used in study 07, would speed up the early onset of response with respect to placebo, and maintain that response until Week 52 (**Figure 33**). A further extension of the intensive phase to 36 or 52 weeks (nine or 13 doses) was however projected to only result in an incremental benefit per additional dose.

Second, a more detailed simulation was performed for the proposed regimen of an initial dose of 900 mg IV Q4W for 6 doses, followed by 120 mg SC QW (**Figure 34**). On top of the PK-UPCR-dropout model explained above, a previously developed model for the gene signature PD marker in SLE was incorporated to the model. The simulation shows that the initial 900 mg doses are driving a reduction of UPCR and thereby also a reduction of anifrolumab clearance. The largest part of the change in clearance occurs in the first half year and is reflected back in the PK profile, which is showing a gradual increase in concentration during this period. The weekly dosing after the switch from IV to SC administration at Week 24 provides a sustained exposure that is resulting in a sustained PD suppression. The median PD suppression is projected to be greater than 90% and more than 9 out of 10 patients are projected to have a PD suppression greater than 80%.

**11.3. Subcutaneous loading dose for lupus nephritis**

As described in above, an anifrolumab dosage regime of 900 mg IV Q4 loading dose (x3) followed by 300 mg IV Q4 has demonstrated efficacy in lupus nephritis patients. A intensified phase of 900 mg (x6) followed by 300 mg IV Q4 is expected from PK/PD modelling to provide additional benefit. Based on the analysis above (see **Section 10**), the 300 mg IV dose for lupus nephritis may be substituted with a subcutaneous dose of greater than 105 mg and less than 150 mg QW, and optionally with a subcutaneous dose of 120 mg QW. Particularly, given 120 mg SC will provide similar exposure and is expected to be efficacious in SLE, a dose of 120 mg SC QW after the intensified phase in LN is expected to provide at least similar efficacy to the 300 mg IV dose in lupus nephritis patients.

It would also be advantageous for the loading dose for LN to be provided to the patient by subcutaneous delivery. The inventors employed modelling techniques to determine the subcutaneous dose corresponding to the 900 mg IV dose of anifrolumab in lupus nephritis patients. Based on the modelling described above for the PK/PD of anifrolumab in LN patients from study 07, a dose of about 1150 mg was found to provide a similar AUC to 900 mg IV in healthy volunteers (**Figure 35**). The predicted subcutaneous to IV for $AUC_{0\text{-}inf}$ and $C_{trough}$ are shown in **Figure 36.**

**Table 27: Predicted SC to IV ratio for $AUC_{0\text{-}inf}$ and $C_{trough}$**

| Bioavailability | $AUC_{0\text{-}inf}$ | $C_{trough}$ (week 4) |
|---|---|---|
| 70 | 0.84 | 0.98 |
| 81 | 1.02 | 1.17 |
| 85 | 1.09 | 1.25 |

1150 mg SC was also found to provide similar AUC to 900 mg IV in LN patients (**Figure 36**). To take account of volume constraints of a 150 mg/ml anifrolumab formulation (e.g. the need to round the volume to the nearest $10^{th}$ of a ml), a dose of 1155 mg SC anifrolumab may be used for practical reasons, and assumed to be equivalent to a 1150 mg dose.

**11.4. Conclusion**

A population model was developed based on study 07 data of longitudinal anifrolumab exposure, proteinuria (UPCR) levels, and IP discontinuation (dropout). Simulations with this model suggested that increasing the number of initial 900 mg IV Q4W doses from 3 to 6 doses would result in a faster onset of renal response as assessed by a decrease in UPCR. In addition, the model also projected slightly lower discontinuation rates for the proposed regimen of 6 initial IV Q4W doses of anifrolumab as compared with the intensified and basic dosing regimens studied in study 07. The projected lower likelihood of discontinuation of treatment could be due to better improvement in UPCR and renal inflammation.

The inventors thus proposed a more intensive dosing regimen than study 07 of an initial dose of 900 mg IV Q4W for 6 doses (or about 1150 mg SC), followed by 300 mg IV Q4W (or 120 mg SC QW) in patients with active, proliferative LN. This regimen is projected to provide sustained anifrolumab exposure/PD suppression, and improved UPCR outcome compared with the previously evaluated dose regimens in study 07 in patients with active, proliferative LN. The proposed 1150 mg SC and 120 mg SC QW in patients with LN will provide at least similar or non-inferior exposure and PD suppression to that of 900 mg IV Q4W and 300 mg IV Q4W respectively. The doses of the invention are summarized in **Table 28** and **Table 29.**

**Table 28: Lupus nephritis doses for BR**

| Administration route | | Dose (mg) | Frequency |
|---|---|---|---|
| IV | lower limit | 150 | Q4W |
| | selected | 300 | Q4W |
| | upper limit | 1000 | Q4W |
| SC | lower limit | 105 | QW |
| | selected | 120 | QW |
| | upper limit | 150 | QW |

**Table 29: Lupus nephritis doses for IR**

| Administration route | | Dose (mg) | Frequency | Dose no. |
|---|---|---|---|---|
| IV | lower limit | 900 | Q4W | 3 |
| | selected | 900 | Q4W | 6 |
| | upper limit | 1000 | Q4W | 6 |
| SC | lower limit | 1150 | Q4W | 3 |
| | selected | About 1150 (e.g. 1155) | Q4W | 6 |
| | Optional upper limit | 1155 | Q4W | 6 |

The subcutaneous and intravenous dosage regimes of anifrolumab for the treatment of LN are interchangeable. A SC IR regime may be followed by an IV BR regime. Similarly, an IV IR may be followed by a SC BR.

## 12. EXAMPLE 6: LN urinary proteomics reveals common biological pathways identified by distinct disease measures

### 12.1. Background

LN is a severe consequence of SLE and there is a huge unmet need for discovery of urine protein biomarkers that provide non-invasive surrogates of disease activity and response to therapy. The purpose of this study was to measure protein biomarkers in urine samples from a diverse cohort of LN patients and to assess their correlations with patient demographics and clinical characteristics including the renal measures of estimated glomerular filtration rate (eGFR), SLEDAI-renal (SLEDAI-R) and National Institutes of Health Activity (NIH-AI) and chronicity (NIH-CI) indices.

### 12.2. Aim

To measure protein biomarkers in urine samples from a diverse cohort of LN patients and to assess correlations between the biomarkers and patient demographics and clinical characteristics.

### 12.3. Methods

The demographics and characteristics of the cohort of 112 patients is described in **Table 30.** All patients fulfilled the 1997 ACR criteria for SLE and all had biopsy-proven LN. Urine samples from patients and 16 healthy donors (HD) were analyzed for 192 proteins by Luminex and 5 by Simoa™. Protein concentrations were normalized to urinary creatinine levels. Log-concentrations of each protein was assessed for correlation with each clinical feature using linear regression adjusted for age, gender, ethnicity, disease duration, and treatment. eGFR was assessed using the cutoff of >60. The Benjamini-Hochberg procedure was used to calculate false discovery rates, with a cut-off of 0.1 used to determine significance. Statistical significance of intersections of protein lists was assessed via permutation. Pathway assessments were conducted using Ingenuity core analysis.

### 12.4. Results

#### *12.4.1. Summary*

Pre-filtering proteins for LN normalized mean concentrations to be greater than the HD mean + 1.5 SD yielded 97 distinct upregulated proteins. The largest numbers of protein-outcome associations were confounded by age (but not disease duration), gender, and MMF dose. After removing the influence of all confounders, numerous proteins showed statistically significant differential expression with respect to eGFR, SLEDAI-2K, SLEDAI-R, serum C3 and C4, NIH-AI, and NIH-CI. Conversely, no proteins were significantly correlated with LN class, race, or SDI (SLICC/ACR disease Index). The highest numbers of significant proteins were found for eGFR (55 proteins) followed by SLEDAI-R (36) and NIH-AI (20). There was a significant overlap of 11 proteins (**Table 31**) across these three lists (intersection $p=4.1 \times 10^{-5}$), indicating that these clinical renal measures share common biological processes. Pathway analysis of the 11 common proteins indicated enrichment for functions known to be associated with fatty acid/lipid metabolism, cardiovascular disease, cellular trafficking and immune cell infiltration.

#### *12.4.2. Proteomic Urinary Biomarkers Upregulated in LN*

Of the 197 proteins assessed, 97 were distinctly upregulated in LN patients compared with healthy donors. Of the five proteins analyzed by Simoa, three (BLC, IL-1β and IL-6) were detectable in >70% of patients and dysregulated in LN.

#### *12.4.3. Association With Demographics and Clinical Characteristics*

The strongest confounders of protein-outcome associations were age (but not disease duration), sex, and MMF dosage. Adjustment for oral corticosteroid dose, angiotensin-converting enzyme inhibitor, and angiotensin receptor blocker made no substantive difference to any of the associations presented. No proteins were significantly correlated with LN class, race, or SDI. After controlling for the influence of all confounders, numerous proteins showed significant differential expression with respect to eGFR, SLEDAI-2K, SLEDAI-R, serum C3 and C4, NIH-AI, NIH-CI, and the IFNGS.

##### *12.4.3.1. C3 and C4*

Many more proteins were associated with serum C4 than serum C3. Of the 4 proteins associated with serum C3, C3, EN-RAGE, and IL-1β were also associated with serum C4. Myeloperoxidase was uniquely associated with serum C3, whereas 15 proteins including hemopexin (Renal Activity Index for Lupus [RAIL] biomarker), apolipoproteins, IL-6, APRIL, and CRP were unique to serum C4.

##### *12.4.3.2. NIH-AI and NIH-CI:*

Proteins associated with high NIH-AI or NIH-CI scores were largely distinct (**Figure 37A**). 4 proteins associated with both NIH-AI and NIH-CI were associated with cellular senescence (cathepsin D, MCP-1) and atherosclerotic risk (OPG, TIMP-1). Unique proteins (NIH-AI and NIH-CI) were in the RAIL (adiponectin and KIM-1) or associated with T-cell cytokines and apolipoproteins.

##### *12.4.3.3. ≥4 clinical characteristics*

Several proteins were associated with ≥4 of the clinical characteristics (**Figure 37B**).

##### *12.4.3.4. IFNGS*

IFNGS associated significantly with inflammatory immune activator proteins (EN-RAGE, ICAM-1, VCAM-1); activation markers and cytokine activators of B cells (PEACAM-1, BAFF); T cells, chemokines and cytokines (RANTES, IP-10, IL-18); and innate cell activators (CD163, M-CSF) (**Figure 38A**). Although most associations were unique, IFNGS most commonly overlapped with SLEDAI-R.

#### *12.4.3.5. eGFR, SLEDAI-R and NIH-AI*

The most significant protein associations were found for eGFR (55 proteins) followed by SLEDAI-R (36) and NIH-AI (20).

### *12.4.4. Proteins Overlapping With eGFR, SLEDAI-R, and High NIH-AI Score*

11 proteins associated with eGFR, SLEDAI-R, and NIH-AI overlapped significantly (intersection P=4.1 x10-5), indicating that these clinical renal measures share common biological processes (**Figure 38B**). Of the 11 proteins common to eGFR, SLEDAI-R, and high NIH-AI score, three were associated with ≥4 clinical characteristics: Apo B, Apo C-I, and LTF (**Figure 37B**). Pathway analysis of the 11 common proteins indicated enrichment for functions known to be associated with fatty acid/lipid metabolism, cardiovascular disease, cellular trafficking, and immune cell infiltration (**Figure 39A**). Many of the disease and molecular function categories and subcategories are associated with vascular disease in SLE, including lipid metabolism and cardiovascular disease, adhesion of immune cells, accumulation of leukocytes, and the degranulation of cells.

### *12.4.5. Unique Proteins Across All Clinical Features*

Serum C3, C4, NIH-CI, and SLEDAI-2K did not associate with any proteins that were unique. eGFR had the most unique proteins, followed by IFNGS (**Figure 39B**). SLEDAI-R and NIH-AI were associated with two unique proteins

## 12.5. Conclusions

By analyzing LN urinary proteomics, the inventors revealed that 3 clinical renal measures: eGFR, SLEDAI-R and NIH-AI are commonly associated with various proteins and pathways, most of which support the emerging importance of renal vascular pathology in LN.

Luminex (192 proteins) and Simoa (5 proteins) can be successfully used to measure dysregulated urine protein in LN. This approach yields quantitative protein outcomes of more than 100 proteins with no special sample preparation and minimal sample volume. These results indicate that multiple inflammatory mediators and pathways contribute to LN pathophysiology. Three clinical renal measures: eGFR, SLEDAI-R, and NIH-AI were commonly associated with various LN proteins and pathways, and may share common biological processes. 11 proteins common to eGFR, SLEDAI-R, and NIH-AI were associated with vascular pathology in lupus, suggesting that alterations to renal vasculature may be important to kidney damage in LN. IFNGS was significantly associated with immune inflammatory pathways including B-cell activation and most commonly overlapped with SLEDAI-R associated proteins. Low serum C4 was associated with more dysregulated urine proteins than C3 despite a lower number of patients with low serum C4. This suggests serum C4 accumulates in the kidney in patients with low serum C4.

***Table 30:*** *Demographics, treatment and disease characteristics*

| Demographics, treatment and disease characteristics | | |
|---|---|---|
| Age, years | Median (range) | 34.0 (18, 67) |
| Sex, n (%) | Male | 19 (17.0) |
| Race, n (%) | Asian | 18 (16.1) |
| | Black/African American | 7 (6.2) |
| | American Indian/Alaska Native | 2 (1.8) |
| | Other | 35 (31.2) |
| | Native Hawaiian/Pacific Islander | 1 (0.9) |
| | White | 49 (43.8) |
| Time from initial LN diagnosis, months | Median (range) | 8.3 (0, 307) |
| MMF before randomization, n (%) | Yes | 80 (71.4) |
| MMF Dosage, mg/day | Mean (SD) | 1.77 (0.46) |
| OCS Dosage, n (%) | >20 mg/day | 109 (97.3) |
| OCS Dosage, mg/day | Mean (SD) | 22.39 (10.71) |

(continued)

| Demographics, treatment and disease characteristics | | |
|---|---|---|
| Concomitant ACEI/ARB treatment, n (%) | Yes | 81 (72.3) |
| Baseline 24-hour UPCR, mg/mg, Threshold, n (%) | >3.0 | 46 (41.1) |
| Baseline 24-hour UPCR, mg/mg, Continuous | Mean (SD) | 3.34 (2.65) |
| Clinical outcomes | | |
| Baseline eGFR mL/min/1.73m2, Threshold, n (%) | >60 | 86 (76.8) |
| Baseline eGFR mL/min/1.73m2, Continuous | Mean (SD) | 94.58 (42.86) |
| LN Class, n (%) | Class II | 2 (2.0) |
| | Class III | 22 (21.8) |
| | Class III and Class V | 11 (10.9) |
| | Class IV | 59 (58.4) |
| | Class IV and Class V | 6 (5.9) |
| | Class V | 1 (1.0) |
| SLEDAI-2K score, Continuous | Mean (SD) | 10.76 (4.74) |
| SLEDAI-R score, Continuous | Mean (SD) | 6.11 (3.32) |
| SDI score, n (%) | 0 | 70 (63.6) |
| | 1 | 29 (26.4) |
| | 2 | 9 (8.2) |
| | 3 | 1 (0.9) |
| | 4 | 1 (0.9) |
| NIH Lupus Nephritis Activity Index, n (%) | High (>8) | 28 (27.7) |
| NIH Lupus Nephritis Chronicity Index, n (%) | High(>4) | 31 (30.7) |
| Serum C3, n (%) | Low C3 | 75 (67.0) |
| Serum C4, n (%) | Low C4 | 32 (28.6) |

***Table 31**: 11 urinary proteins which reached statistical significance (false discovery rate <0.1) for association with eGFR, SLEDAI-R and NIH-AI*

| **Urinary Protein** |
|---|
| Adiponectin |
| Alpha-2-Macroglobulin (A2Macro) |
| Antithrombin-III (AT-III) |
| Apolipoprotein A-I (Apo A-I) |
| Apolipoprotein B (Apo B) |
| Apolipoprotein C-I (Apo C-I) |
| Apolipoprotein C-III (Apo C-III) |
| Fatty Acid-Binding Protein, heart (FABP, heart) |
| Lactoferrin (LTF) |
| Neuropilin-1 |
| Omentin |
| Serum Amyloid P-Component (SAP) |
| von Willebrand Factor (vWF) |

## 13. EXAMPLE 7: Delivery devices

Anifrolumab is administered by an injection device **[1] [9]** such as a prefilled syringe (PFS) **(Figure 40A)** or an autoinjector (AI) (**Figure 40B**). Higher doses of anifrolumab may be administered by an onboard-delivery system.

### 13.1. Autoinjector

Anifrolumab may be administered by an autoinjector **[1].** The autoinjector is shown in exploded view (**Figure 41A**) and in an assembled form (**Figure 41B**). A label **[4]** is wrapped around and attached to the autoinjector **[1]** (**Figure 41C**). The autoinjector has an autoinjector housing **[3],** cap and cap remover **[2]** and drive unit **[5].** The liquid anifrolumab formulation unit dose **[6]** is contained in the autoinjector housing **[3].** The unit dose **[6]** can be viewed through the viewing window **[7].**

### 13.2. Accessorized pre-frilled syringe

Anifrolumab may be administered by accessorized pre-filled syringe (APFS) **[8].** The APFS **[8]** includes the unit dose of anifrolumab **[6]** contained in a primary container **[9]** shown in an assembled state in **Figure 42A** and in an exploded view in **Figure 42B.** The primary container **[9]** has a plunger stopper **[16].** The primary container has a nominal fill volume **[17]** of 0.8 ml but may contain slightly more than 0.8 ml. The remainder of the space in the primary container **[9]** is taken up by an air bubble **[18].** The air bubble **[18]** may have a size of 3-5mm, optionally, 4 mm. The primary container **[9]** has a defined stopper position **[19].**

The accessorized pre-filled syringe (APFS) primary container **[9]** is provided in a PFS assembly **[8]** including a needle guard **[12],** a finger flange **[11]** and a plunger rod **[13]** (**Figure 42C** and **Figure 42D**). A label **[14]** is provided with the primary container **[9]** in the PFS assembly **[8].** The label **[14]** is wrapped around the syringe **[9]** in the label placement position **[15].**

### 13.3. Packaging

The injection device **[1] [8]** is provided in a kit **[20]** (**Figure 43**). A label **[4] [14]** is provided with the APFS or autoinjector in the packaging. The label includes instruction for the use of the injection device **[1], [8].** The packaging includes a tamper seal.

## REFERENCES

All publications mentioned in the specification are herein incorporated by reference.


[1] J. G. Hanly et al., Rheumatol. Oxf. Engl. 55, 252 (2016).
[2] E. C. Baechler et al., Proc. Natl. Acad. Sci. U. S. A. 100, 2610 (2003).
[3] J. J. Weening et al., Kidney Int. 65, 521 (2004).
[4] N. Maroz and M. S. Segal, Am. J. Med. Sci. 346, 319 (2013).
[5] A. Fanouriakis et al., Ann. Rheum. Dis. 79, 713 (2020).
[6] E. M. Ginzler, A. J. Bollet, and E. A. Friedman, Annu. Rev. Med. 31, 463 (1980).
[7] H.-J. Anders et al., Nat. Rev. Dis. Primer 6, 7 (2020).
[8] R. Furie et al., N. Engl. J. Med. (2020).
[9] C. Arriens et al., Ann. Rheum. Dis. 79, 172 (2020).
[10] B. H. Rovin et al., Kidney Int. 95, 219 (2019).
[11] R. Furie et al., Arthritis Rheumatol. Hoboken Nj 69, 376 (2017).
[12] G. T. Ferguson et al., J. Asthma Allergy 11, 63 (2018).
[13] L. Peng et al., mAbs 7, 428 (2015).
[14] I. M. Bajema et al., Kidney Int. 93, 789 (2018).
[15] A. S. Levey et al., Ann. Intern. Med. 150, 604 (2009).
[16] A. S. Levey et al., Ann. Intern. Med. 145, 247 (2006).
[17] B. H. Rovin et al., Lancet Lond. Engl. 397, 2070 (2021).
[18] M. Petri et al., Arthritis Rheum. 58, 1784 (2008).
[19] R. Tummala et al., Lupus Sci. Med. 5, e000252 (2018).
[20] ACR Meeting Abstracts (n.d.).
[21] B. W. Higgs et al., Ann. Rheum. Dis. 73, 256 (2014).
[22] R. A. Furie et al., Lancet Rheumatol. 1, e208 (2019).

[23] E. F. Morand et al., N. Engl. J. Med. 382, 211 (2020).
[24] Y. Tanaka and R. Tummala, Mod. Rheumatol. 0, 1 (2020).
[25] B. Wang et al., Clin. Pharmacol. Ther. 93, 483 (2013).
[26] M. Aringer et al., Arthritis Rheumatol. Hoboken NJ 71, 1400 (2019).
[27] M. C. Hochberg, Arthritis Rheum. 40, 1725 (1997).
[28] Y. Lin Chia et al., Rheumatol. Oxf. Engl. (2021).
[29] Y. Yao et al., Arthritis Res. Ther. 12, S6 (2010).
[30] D. D. Gladman, D. Ibañez, and M. B. Urowitz, J. Rheumatol. 29, 288 (2002).
[31] M. Petri et al., Arthritis Rheum. 34, 937 (1991).
[32] Y. Yao et al., Hum. Genomics Proteomics HGP 2009, (2009).
[33] J. L. Gorriz and A. Martinez-Castelao, Transplant. Rev. Orlando Fla 26, 3 (2012).
[34] J. A. Moreno et al., Int. J. Mol. Sci. 20, (2019).
[35] J.-Y. Jung et al., Sci. Rep. 9, 9704 (2019).
[36] I. N. Bruce et al., Lancet Rheumatol. 0, (2020).
[37] J. T. Ryman and B. Meibohm, CPT Pharmacomet. Syst. Pharmacol. 6, 576 (2017).

## CLAUSES

The following clauses define embodiments of the disclosure.

Clause 1) A method of treating lupus nephritis (LN) in a subject in need thereof, the method comprising administering a type I IFN receptor (IFNAR1) inhibitor to the subject, wherein the method reduces lupus nephritis (LN) disease activity in the subject.

Clause 2) The method of clause 1, wherein the IFNAR1 inhibitor is a human monoclonal antibody specific for IFNAR1, optionally a modified IgG1 class human monoclonal antibody.

Clause 3) The method of clause 2, wherein the antibody comprises:

a heavy chain variable region complementarity determining region 1 (HCDR1) comprising the amino acid sequence of SEQ ID NO: 3;

a heavy chain variable region complementarity determining region 2 (HCDR2) comprising the amino acid sequence of SEQ ID NO: 4;

a heavy chain variable region complementarity determining region 3 (HCDR3) comprising the amino acid sequence of SEQ ID NO: 5;

a light chain variable region complementarity determining region 1 (LCDR1) comprising the amino acid sequence SEQ ID NO: 6;

a light chain variable region complementarity determining region 2 (LCDR2) comprising the amino acid sequence SEQ ID NO: 7; and

a light chain variable region complementarity determining region 3 (LCDR3) comprising the amino acid sequence SEQ ID NO: 8.

Clause 4) The method of clause 2, wherein the antibody comprises:

a heavy chain variable region complementarity determining region 1 (HCDR1) comprising the amino acid sequence of SEQ ID NO: 19;

a heavy chain variable region complementarity determining region 2 (HCDR2) comprising the amino acid sequence of SEQ ID NO: 20;

a heavy chain variable region complementarity determining region 3 (HCDR3) comprising the amino acid sequence of SEQ ID NO: 21;

a light chain variable region complementarity determining region 1 (LCDR1) comprising the amino acid sequence SEQ ID NO: 22;

a light chain variable region complementarity determining region 2 (LCDR2) comprising the amino acid sequence SEQ ID NO: 23; and

a light chain variable region complementarity determining region 3 (LCDR3) comprising the amino acid sequence SEQ ID NO: 24.

Clause 5) The method of clause 2 or 3, wherein the antibody comprises: (a) a human heavy chain variable region comprising the amino acid sequence of SEQ ID NO: 1; and (b) a human light chain variable region comprising the amino acid sequence of SEQ ID NO: 2.

Clause 6) The method of clause 2 or 4, wherein the antibody comprises: (a) a human heavy chain variable region comprising the amino acid sequence of SEQ ID NO: 17; and (b) a human light chain variable region comprising the amino acid sequence of SEQ ID NO: 18.

Clause 7) The method of any of clauses 2 to 6, wherein the antibody comprises in the Fc region an amino acid substitution of L234F, as numbered by the EU index as set forth in Kabat and wherein said antibody exhibits reduced affinity for at least one Fc ligand compared to an unmodified antibody, optionally wherein the antibody comprises in the Fc region an amino acid substitution of L235E and/or P331S, as numbered by the EU index as set forth in Kabat.

Clause 8) The method of any of clause s 2, 3 or 5, wherein the antibody comprises: (a) a human heavy chain comprising the amino acid sequence of SEQ ID NO: 11; and (b) a human light chain comprising the amino acid sequence of SEQ ID NO: 12.

Clause 9) The method of clause 2, wherein the IFNAR1 inhibitor is anifrolumab or a functional variant thereof.

Clause 10) The method of clause 2, wherein the IFNAR1 inhibitor is QX006N or a functional variant thereof.

Clause 11) The method of any preceding clause, comprising administering intravenously an intravenous dose of the IFNAR1 inhibitor thereof to the subject.

Clause 12) The method of clause 11, wherein the intravenous dose is equal to or greater than (≥)300 mg.

Clause 13) The method of clause 11 or 12, wherein the intravenous dose is equal to or less than (≤)1000mg.

Clause 14) The method of any of clauses 11 to 13, wherein the intravenous dose is greater than (>)300 mg.

Clause 15) The method of any of clauses 11, 12 or 14, wherein the intravenous dose is about 900 mg or about 1000 mg.

Clause 16) The method of any of clauses 11 to 15, wherein the intravenous dose is administered every four weeks (Q4W), optionally wherein the intravenous dose is administered at least 3 times, optionally wherein the intravenous dose is administered at least 6 time.

Clause 17) The method of any of clauses 1 to 10, comprising administering subcutaneously a subcutaneous dose of the IFNAR1 inhibitor.

Clause 18) The method of clause 17, wherein the subcutaneous dose is greater than (>)105 mg and less than (<) 150 mg.

Clause 19) The method of clause 17, wherein the subcutaneous dose is ≤135 mg.

Clause 20) The method of clause 17, wherein the subcutaneous dose is about 120 mg.

Clause 21) The method of any of clauses 17 to 20, wherein the subcutaneous dose is administered in a single administration step.

Clause 22) The method of any of clauses 17 to 21, wherein the subcutaneous dose is administered at intervals of 6-8 days.

Clause 23) The method of any of clauses 17 to 22, wherein the subcutaneous dose is administered once per week.

Clause 24) The method of any of clauses 17 to 23, wherein the subcutaneous dose has a volume of about 0.5 to about 1 ml.

Clause 25) The method of clause 24, wherein the subcutaneous dose has a volume of about 0.8 ml.

Clause 26) The method of clause 17, wherein the subcutaneous dose is >1000 mg, optionally wherein the subcutaneous dose is 1050 to 1200 mg, optionally wherein the subcutaneous dose is 1100 to 1190 mg, optionally wherein the subcutaneous dose is 1150 to 1160 mg, optionally wherein the subcutaneous dose is about 1150 mg or 1155 mg.

Clause 27) The method of clause 17, wherein the subcutaneous dose is administered in a volume of about 8 ml, optionally about 7.7 ml.

Clause 28) The method of any one of clauses 1 to 10, comprising administering to the subject a first dose of the IFNAR1 inhibitor, followed by a second dose of the IFNAR1 inhibitor, wherein the first dose is higher than the second dose.

Clause 29) The method of clause 28, wherein the first dose is administered intravenously.

Clause 30) The method of clause 29, wherein the first dose is >300 mg.

Clause 31) The method of clause of clauses 29 or 30, wherein the first dose is ≤1000 mg.

Clause 32) The method of any of clauses 29 to 31, wherein the first dose is about 900 mg.

Clause 33) The method of clause 28, wherein the first dose is administered subcutaneously, optionally wherein the first dose is >1000 mg, optionally wherein the first dose is 1050 to 1200 mg, optionally wherein the first dose is 1100 to 1190 mg, optionally wherein the first dose is 1150 to 1160 mg, optionally wherein the first dose is about 1150 mg or 1155 mg.

Clause 34) The method of any of clauses 28 to 33, wherein the first dose is administered Q4W.

Clause 35) The method of any of clauses 28 to 34, wherein the first dose is administered to the subject at least 3 times before the second dose is administered to the subject, optionally wherein the first dose is administered 3, 4, 5 or 6 times before the second dose is administered to the subject.

Clause 36) The method of clause 35, wherein the first dose is administered to the subject at least 6 times before the second dose is administered to the subject.

Clause 37) The method of any of clauses 28 to 36, wherein the second dose is administered subcutaneously.

Clause 38) The method of clause 37, wherein the second dose is >105 mg and ≤135 mg.

Clause 39) The method of clause 38, wherein the second dose is about 120 mg.

Clause 40) The method of any of clauses 37 to 39, wherein the second dose is administered once per week.

Clause 41) The method of any of clauses 28 to 36, wherein the second dose is administered intravenously.

Clause 42) The method of clause 41, wherein the second dose is administered Q4W.

Clause 43) The method of clause 41 or 42, wherein the second dose is ≥300 mg.

Clause 44) The method of any of clauses 41 to 43, wherein the second dose is ≤1000mg.

Clause 45) The method of any of clauses 41 to 44, wherein the second dose is about 300 mg.

Clause 46) The method of any of clauses 41 to 45, wherein the second dose is administered Q4W.

Clause 47) The method of clause 28, wherein:

the first dose is about 900 mg and is administered intravenously Q4W, and the second dose is about 120mg and is administered subcutaneously QW, or

the first dose is about 900 mg and administered intravenously Q4W, and the second dose is about 300 mg and is administered intravenously Q4W, or

the first dose is about 1150 or 1155 mg and administered subcutaneously Q4W, and the second dose is about 300 mg and administered intravenously Q4W, or

the first dose is about 1150 mg or 1155 mg and administered subcutaneously, and the second dose is about 120 mg and administered subcutaneous QW;

optionally wherein the first dose is administered for at least 3 months before administration of the second dose, optionally wherein the first dose is administered for at least 6 months before administration of the second dose.

Clause 48) The method of any preceding clause, wherein reducing lupus nephritis disease activity in the subject comprises a complete renal response (CRR) in the subject post-treatment compared to pre-treatment.

Clause 49) The method of clause 48, wherein a CRR is achieved by week 36 of treatment.

Clause 50) The method of clause 49 or 50, wherein reducing LN disease activity in the subject comprises a CRR and a UPCR of ≤0.5 mg/mg post-treatment.

Clause 51) The method of any preceding clauses, wherein reducing LN disease activity in the subject comprises reduction in proteinuria in the subject post-treatment compared to proteinuria in the subject pre-treatment.

Clause 52) The method of clause 51, wherein proteinuria is measured by UPCR

Clause 53) The method of clause 52, wherein proteinuria is measured by 24-hour UPCR.

Clause 54) The method of any preceding claim, wherein reducing LN disease activity in the subject comprises an alternative CRR (aCRR) in the subject post-treatment compared to baseline.

Clause 55) The method of any preceding clause, wherein reducing LN disease activity in the subject comprises treating LN in the subject, optionally wherein the subject is in LN remission post-treatment.

Clause 56) The method of any preceding clause, comprising administering mycophenolate mofetil (MMF) and/or a steroid to the subject.

Clause 57) The method of any preceding clause, the method comprising steroid sparing in the subject, wherein the dose of the steroid administered to the subject is tapered from a pre-sparing dose to a post-sparing dose.

Clause 58) The method of clause 57, wherein the post-sparing dose is ≤7.5 mg/day prednisone or prednisone equivalent dose.

Clause 59) The method of clause 57 or 58, wherein the pre-sparing dose is 20 mg/day prednisone or prednisone equivalent dose.

Clause 60) The method of any of clauses 57 to 59, wherein the steroid comprises a glucocorticoid.

Clause 61) The method of clause 60, wherein the steroid comprises an oral glucocorticoid.

Clause 62) The method of any of claims 57 to 61, wherein the steroid is hydrocortisone, mometasone, fluticasone, fluocinolone acetonide, fluocinolone, flurandrenolone acetonide, ciclesonide, budesonide, beclomethasone, deflazacort, flunisolide, beclomethasone dipropionate, betamethasone, betamethasone valerate, methylprednisolone, dexamethasone, prednisolone, cortisol, triamcinolone, clobetasol, clobetasol propionate, clobetasol butyrate, cortisone, corticosterone, clocortolone, dihydroxycortisone, alclometasone, amcinonide, diflucortolone valerate, flucortolone, fluprednidene, fluandrenolone, fluorometholone, halcinonide, halobetasol, desonide, diflorasone, flurandrenolide, fluocinonide, prednicarbate, desoximetasone, fluprednisolone, prednisone, azelastine, dexamethasone 21-phosphate, fludrocortisone, flumethasone, fluocinonide, halopredone, hydrocortisone 17-valerate, hydrocortisone 17-butyrate, hydrocortisone 21-acetate, prednisolone, prednisolone 21-phosphate, clobetasol propionate, triamcinolone acetonide, or a mixture thereof.

Clause 63) The method of any of clauses 57 to 62, wherein the steroid comprises prednisone.

Clause 64) The method of any preceding clause, comprising analysing the levels of a protein or proteins in the urine of the subject pre-treatment and/or post treatment, optionally in an isolated urine sample from the subject.

Clause 65) The method of any preceding clause, wherein the level of the protein or proteins is identified as elevated in the subject's urine pre-treatment compared to the level of the protein in a healthy subject.

Clause 66) The method of any preceding clause, wherein post-treatment, the level of the protein or proteins in the urine of the subject is reduced compared to the pre-treatment level of the protein or proteins in the urine of the subject.

Clause 67) A method for identifying a subject as suitable for treatment with a IFNAR1 inhibitor, the method comprising identifying elevated expression of a protein or proteins in an isolated urine of the subject compared to expression of the protein or proteins respectively in a healthy subject, wherein the subject is a patient with lupus nephritis.

Clause 68) The method of clause 67, wherein the protein or proteins comprise Adiponectin, Alpha-2-Macroglobulin (A2Macro), Antithrombin-III (AT-III), Apolipoprotein A-I (Apo A-I), Apolipoprotein B (Apo B), Apolipoprotein C-I (Apo C-I), Apolipoprotein C-III (Apo C-III), Fatty Acid-Binding Protein, heart (FABP, heart), Lactoferrin (LTF), Neuropilin-1, Omentin, Serum Amyloid P-Component (SAP) and/or von Willebrand Factor (vWF).

Clause 69) The method of clause 67 or 68, wherein the protein or proteins comprise Apo A-11, Apo B, Apo C-I, Cathepsin D, EN-RAGE, Fibrinogen, LTF, MCP-1, RANTES and/or IL-1β.

Clause 70) The method of clause 69, wherein the protein or proteins comprise Apo B, Apo C-I, and/or LTF.

Clause 71) The method of any preceding clause, wherein the lupus nephritis is proliferative lupus nephritis, optionally wherein the lupus nephritis is Class III or Class IV, with or without co-existing Class V.

Clause 72) A unit dose comprising >105 mg and ≤150 mg anifrolumab or a functional variant thereof.

Clause 73) The unit dose of clause 72, comprising ≤135 mg anifrolumab or the functional variant thereof.

Clause 74) The unit dose of clause 72 or 73, comprising about 120 mg anifrolumab or the functional variant thereof.

Clause 75) The unit dose of clause 72, wherein the unit dose consists essentially of >105 mg and <150 mg anifrolumab or the functional variant thereof.

Clause 76) The unit dose of clause 75, consisting essentially of ≤135 mg anifrolumab or the functional variant thereof.

Clause 77) The unit dose of clause 76, consisting essentially of about 120 mg anifrolumab or the functional variant thereof.

Clause 78) The unit dose of any of clauses 72 to 77, wherein the concentration of anifrolumab or the functional variant thereof in the unit dose is about 150 mg/ml.

Clause 79) The unit dose of any of clauses 72 to 78, wherein the volume of the unit dose is about 0.8 ml.

Clause 80) A unit dose comprising >1000 mg anifrolumab or a functional variant thereof.

Clause 81) The unit dose of clause 80, wherein the unit dose comprises 1050 to 1200 mg anifrolumab or the functional variant thereof, optionally wherein the unit dose is 1100 to 1190 mg anifrolumab or the functional variant thereof, optionally wherein the unit dose is 1150 to 1160 mg anifrolumab or the functional variant thereof, optionally wherein the unit dose is about 1150 mg or 1155 mg anifrolumab or the functional variant thereof.

Clause 82) The unit dose of clause 81, comprising about 1150 mg or 1155 mg anifrolumab in a volume of 7.7 ml, wherein the anifrolumab concentration is 150 mg/ml.

Clause 83) The unit dose of clause 80, wherein the unit dose consists essentially of >1000 mg anifrolumab or the functional variant thereof.

Clause 84) The unit dose of clause 81, consisting essentially of 1050 to 1200 mg anifrolumab or a functional variant thereof, 1100 to 1190 mg anifrolumab or a functional variant thereof, 1150 to 1160 mg anifrolumab or a functional variant thereof, about 1150 mg or 1155 mg anifrolumab or the functional variant thereof.

Clause 85) The unit dose of any of clauses 72- 84, wherein the unit dose comprises a formulation of about 150 to 200 mg/ml anifrolumab or the functional variant thereof, 25 to 150 mM of lysine salt and an uncharged excipient.

Clause 86) The unit dose of clause 85, wherein the unit dose comprises a formulation of about 25 mM histidine-HCL, about 130 mM trehalose, and about 0.05% w/v polysorbate 80 or polysorbate 20.

Clause 87) The unit dose of clause 85 or 86, wherein the formulation has a pH of about 5.9.

Clause 88) The unit dose of any of clauses 72 to 87, for use in the method of any of claims 1 to 71.

Clause 89) The unit dose of clause 72, wherein the volume of the unit dose is about 7.7 ml.

Clause 90) The unit dose of clause 72 or 73, wherein the unit dose comprises a formulation of 150 to 200 mg/ml anifrolumab or the functional variant thereof, 25 to 150 mM of lysine salt and an uncharged excipient.

Clause 91) The unit dose of clause 90, wherein the unit dose comprises a formulation of about 25 mM histidine-HCL, about 130 mM trehalose, and about 0.05% w/v polysorbate 80 or polysorbate 20.

Clause 92) The unit dose of clause 90 or 91, wherein the formulation has a pH of about 5.9.

Clause 93) The unit dose of any of clause 2 to 92, wherein the unit dose is suitable for subcutaneous administration to a subject.

Clause 94) The unit dose of any of clauses 72 to 93, for use in the methods of any of claims 1 to 71.

Clause 95) A pharmaceutical composition for use in the methods of any of clauses 1 to 71, wherein the pharmaceutical composition comprises anifrolumab or a functional variant thereof or the unit dose of any of claims 72 to 94.

Clause 96) An injection device comprising the unit dose of any of clauses 72 to 94 or the pharmaceutical composition of claim 95.

Clause 97) The injection device of clause 96, wherein the injection device is a pre-filled syringe (PFS).

Clause 98) The injection device of clause 97, wherein the injection device is an accessorized pre-filed syringe (AFPS).

Clause 99) The injection device of clause 96, wherein the injection device is an auto-injector.

Clause 100) A kit comprising the injection device of any of clauses 96 to 99, and instructions for use.

Clause 101) The kit of clause 100, wherein the instructions for use comprise instructions for subcutaneous administration of the pharmaceutical composition or unit dose to a subject.

Clause 102) The kit of clause 100 or 101, wherein the instructions for use specify that the injection device, unit dose and/or pharmaceutical composition are for use in the treatment of LN.

Clause 103) The kit of any of clauses 100-101, comprising packaging, wherein the packaging is adapted to hold the injection device and the instructions for use.

Clause 104) The kit of any of clauses 100-103, wherein the instructions for use are attached to the injection device.

Clause 105) The kit of any of clauses 100-104, wherein the instructions for use comprise instructions for subcutaneous administration of anifrolumab or the functional variant thereof.

Clause 106) The kit of clause 105, wherein the instructions for use comprise instructions for subcutaneous administration of >105 mg anifrolumab or the functional variant thereof.

Clause 107) The kit of clause 106, wherein the instructions for use comprise instructions for administration of ≤135 mg anifrolumab or the functional variant thereof.

Clause 108) The kit of clause 10, wherein the instructions for use comprise instructions for administration of 120 mg anifrolumab or the functional variant thereof.

Clause 109) The kit of clause 105, wherein the instructions for use comprise instructions for administration of about 1150 mg or 1155 mg anifrolumab or the functional variant thereof.

Clause 110) The kit of clause 109, wherein the instructions for use comprise instructions for administration of about 1150 mg or 1155 mg anifrolumab or the functional variant thereof every month.

Clause 111) The kit of any of clauses 100-110, wherein the instructions for use comprise instructions for intravenous administration of a 900 mg dose of anifrolumab or instructions for subcutaneous administration of about 1150 mg or 1155 mg dose of anifrolumab, optionally comprising instructions that the intravenous or subcutaneous dose is administered at least 3 times Q4W, optionally at least 6 times Q4W.

Clause 112) The kit of any of clauses 100-111, wherein the instruction for use comprise instructions for the use according to the method of any of clauses 1 to 71.

## Claims

**1.** A type I IFN receptor (IFNAR1) inhibitor for use in a method of treating lupus nephritis (LN) in a subject in need thereof, the method comprising administering a type I IFN receptor (IFNAR1) inhibitor to the subject, wherein the method reduces lupus nephritis (LN) disease activity in the subject, wherein the IFNAR1 inhibitor is a modified IgG1 class human monoclonal antibody specific for IFNAR1 comprising:

a. a heavy chain variable region complementarity determining region 1 (HCDR1) comprising the amino acid sequence of SEQ ID NO: 3;

b. a heavy chain variable region complementarity determining region 2 (HCDR2) comprising the amino acid sequence of SEQ ID NO: 4;
c. a heavy chain variable region complementarity determining region 3 (HCDR3) comprising the amino acid sequence of SEQ ID NO: 5;
d. a light chain variable region complementarity determining region 1 (LCDR1) comprising the amino acid sequence SEQ ID NO: 6;
e. a light chain variable region complementarity determining region 2 (LCDR2) comprising the amino acid sequence SEQ ID NO: 7; and
f. a light chain variable region complementarity determining region 3 (LCDR3) comprising the amino acid sequence SEQ ID NO: 8;

wherein the method comprises administering to the subject a first dose of the IFNAR1 inhibitor, followed by a second dose of the IFNAR1 inhibitor, wherein the first dose is higher than the second dose.

**2.** The type I IFN receptor (IFNAR1) inhibitor for use according to claim 1, wherein the antibody comprises: (a) a human heavy chain variable region comprising the amino acid sequence of SEQ ID NO: 1; and (b) a human light chain variable region comprising the amino acid sequence of SEQ ID NO: 2.

**3.** The type I IFN receptor (IFNAR1) inhibitor for use according to claim 1 or 2, wherein the antibody comprises in the Fc region an amino acid substitution of L234F, as numbered by the EU index as set forth in Kabat and wherein said antibody exhibits reduced affinity for at least one Fc ligand compared to an unmodified antibody, optionally wherein the antibody comprises in the Fc region an amino acid substitution of L235E and/or P331S, as numbered by the EU index as set forth in Kabat.

**4.** The type I IFN receptor (IFNAR1) inhibitor for use according to claim 1, wherein the IFNAR1 inhibitor is anifrolumab or a functional variant thereof.

**5.** The type I IFN receptor (IFNAR1) inhibitor for use according to claim 1-4, wherein the first dose is administered intravenously and the second dose is administered intravenously.

**6.** The type I IFN receptor (IFNAR1) inhibitor for use according to 5, wherein the first and second doses are administered Q4W.

**7.** The type I IFN receptor (IFNAR1) inhibitor for use according to claim 1-4, wherein the first dose is administered subcutaneously and the second dose is administered subcutaneously.

**8.** The type I IFN receptor (IFNAR1) inhibitor for use according to claim 7, wherein the second dose is administered once per week.

**9.** The type I IFN receptor (IFNAR1) inhibitor for use according to of claim 1, wherein the first dose is administered to the subject at least 3 times before the second dose is administered to the subject.

**10.** The type I IFN receptor (IFNAR1) inhibitor for use according to of claim 9, wherein the first dose is administered to the subject at least 6 times before the second dose is administered to the subject.

## Figure 1A

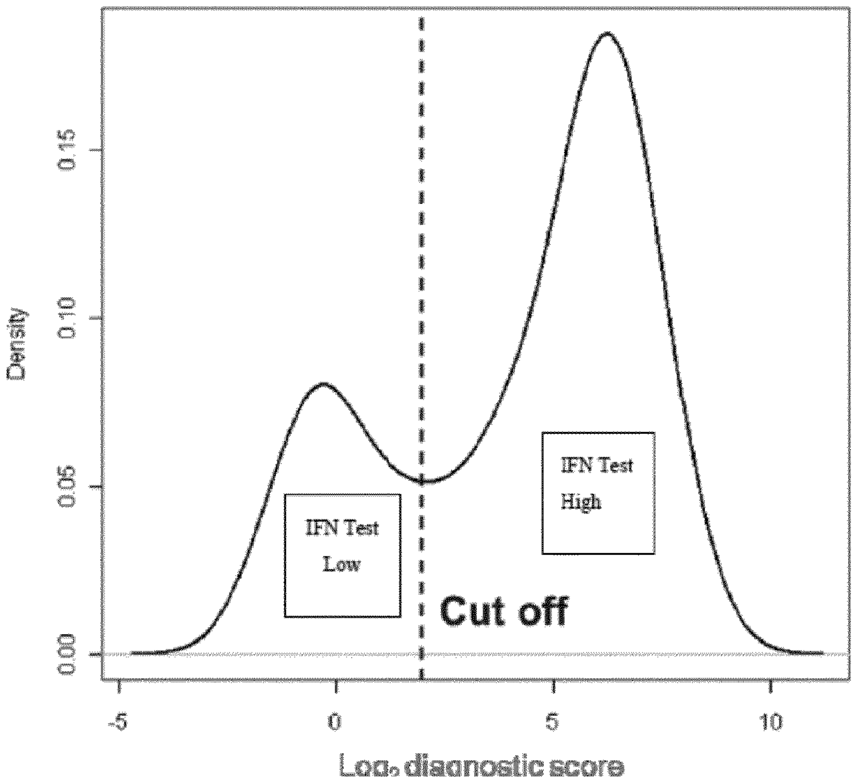


## Figure 1B

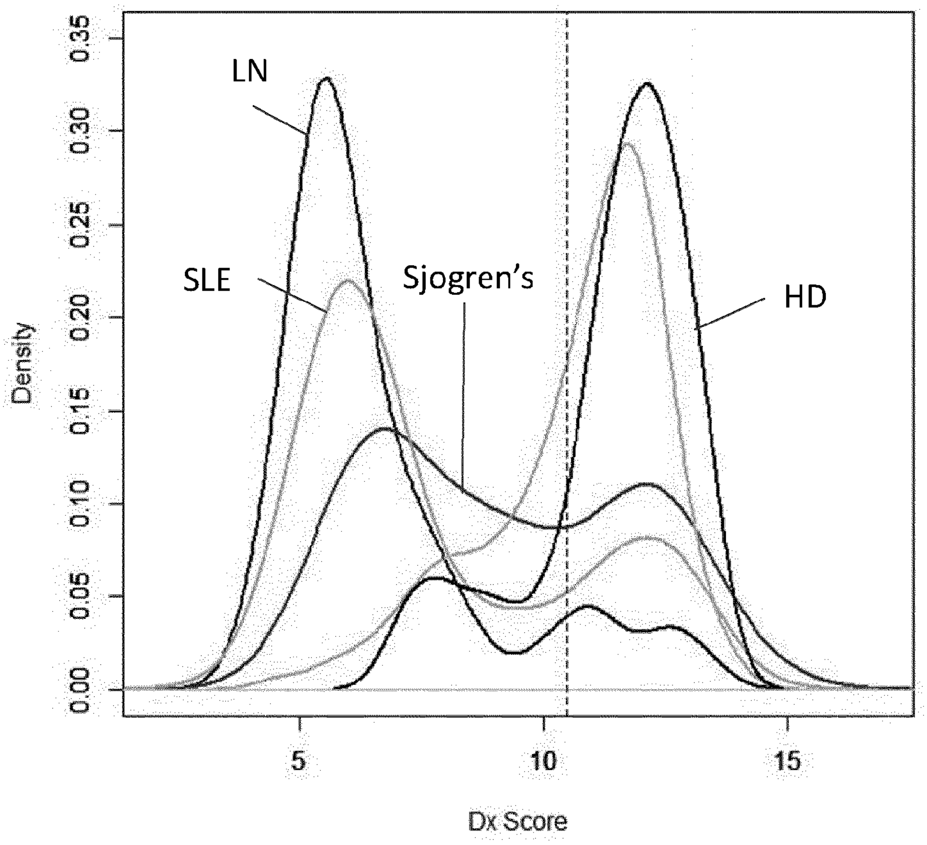

# Figure 2A

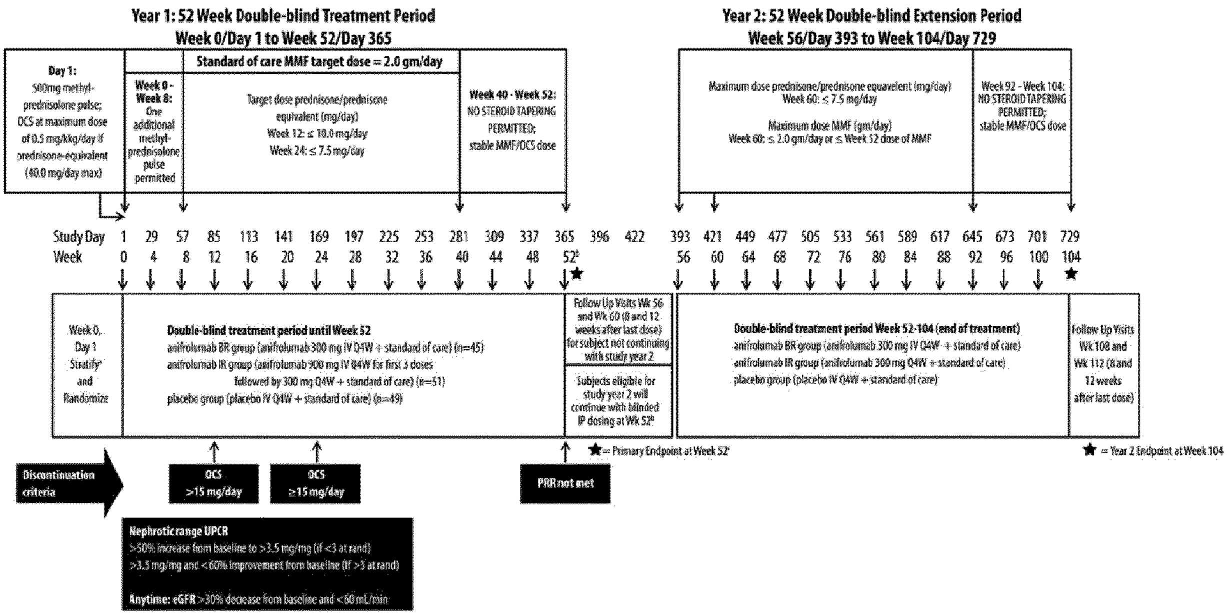


# Figure 2B

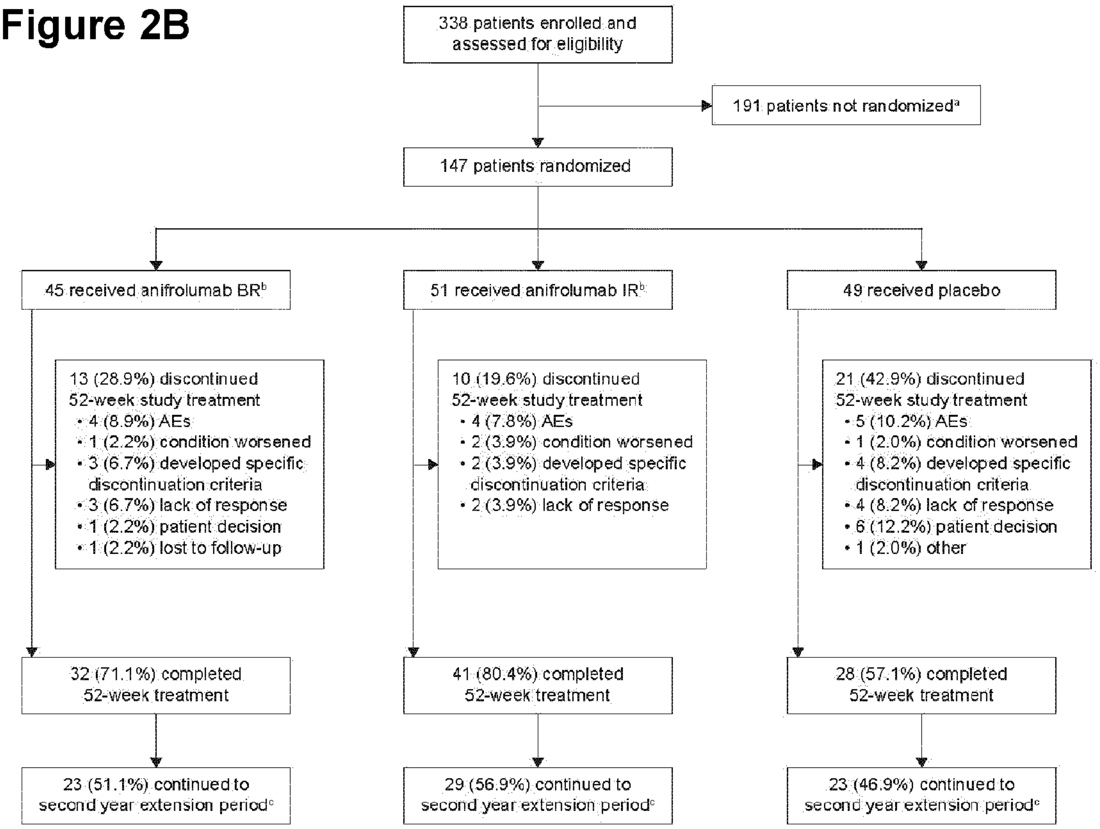

Figure 3

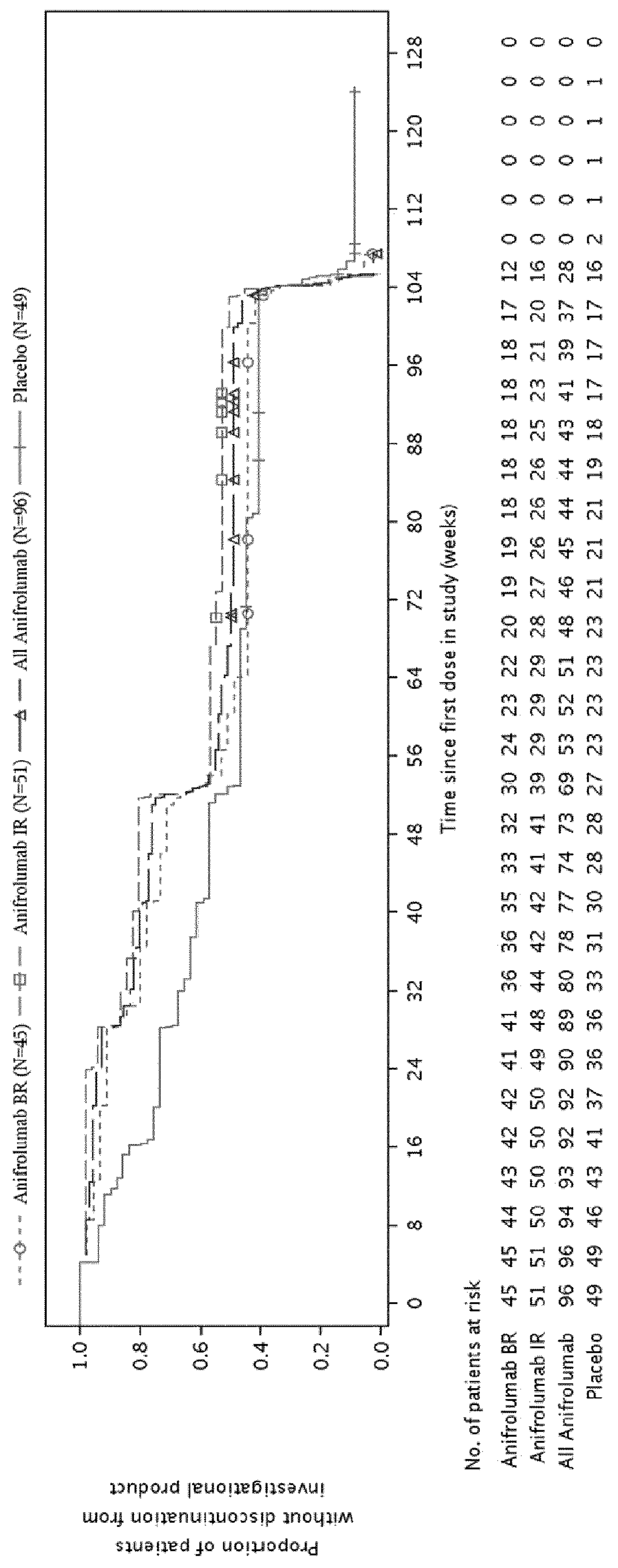
Anifrolumab BR (N=45)
Anifrolumab IR (N=51)
All Anifrolumab (N=96)
Placebo (N=49)
Proportion of patients without discontinuation from investigational product
1.0
0.8
0.6
0.4
0.2
0.0
0 8 16 24 32 40 48 56 64 72 80 88 96 104 112 120 128
Time since first dose in study (weeks)
No. of patients at risk
Anifrolumab BR 45 45 44 43 42 42 41 41 36 36 35 33 32 30 24 23 22 20 19 19 18 18 18 18 17 12 0 0 0 0 0
Anifrolumab IR 51 51 50 50 50 50 49 48 44 42 42 41 41 39 29 29 29 28 27 26 26 26 25 23 21 20 16 0 0 0 0 0
All Anifrolumab 96 96 94 93 92 92 90 89 80 78 77 74 73 69 53 52 51 48 46 45 44 44 43 41 39 37 28 0 0 0 0 0
Placebo 49 49 46 43 41 37 36 36 33 31 30 28 28 27 23 23 23 23 21 21 21 19 18 17 17 17 16 2 1 1 1 1 0

# Figure 4A

A GM change from baseline in 24-hour UPCR over time

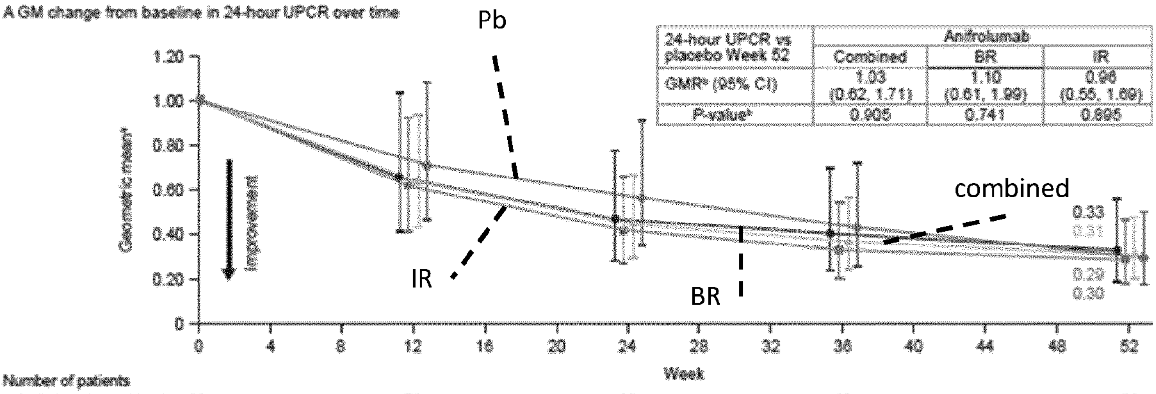


| Number of patients | 0 | 12 | 24 | 36 | 52 |
|---|---|---|---|---|---|
| Anifrolumab combined | 96 | 78 | 80 | 62 | 66 |
| Anifrolumab BR | 45 | 35 | 35 | 30 | 29 |
| Anifrolumab IR | 51 | 43 | 45 | 32 | 37 |
| Placebo | 49 | 38 | 32 | 28 | 25 |

# Figure 4B

B Percentage of patients with a CRR over time[a]

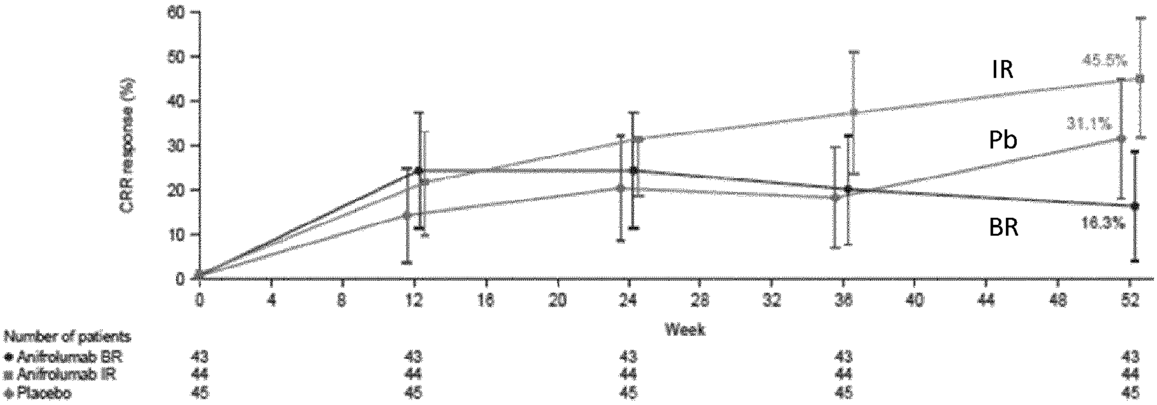


| Number of patients | 0 | 12 | 24 | 36 | 52 |
|---|---|---|---|---|---|
| Anifrolumab BR | 43 | 43 | 43 | 43 | 43 |
| Anifrolumab IR | 44 | 44 | 44 | 44 | 44 |
| Placebo | 45 | 45 | 45 | 45 | 45 |

# Figure 4C

C Time to $CRR_{0.5}$ sustained through Week 52[c,d]

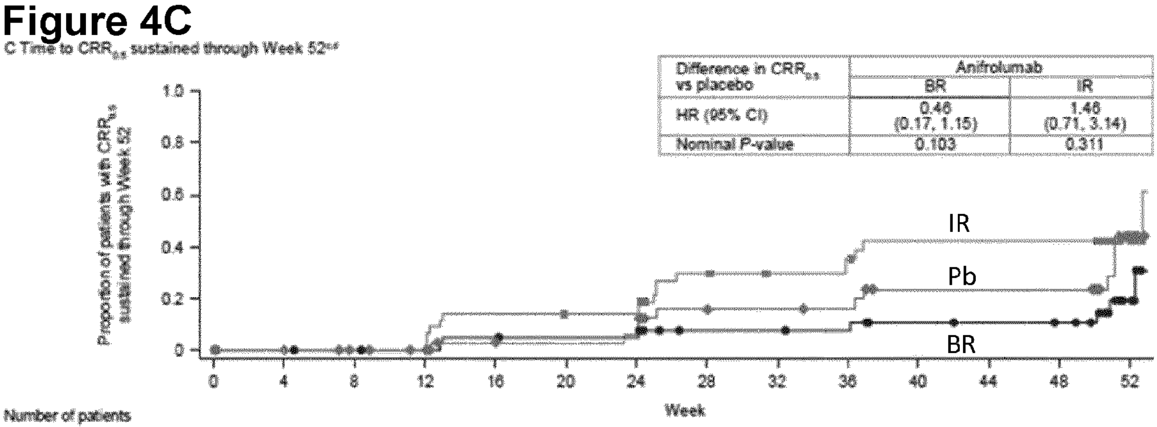


| Number of patients | 0 | 4 | 8 | 12 | 16 | 20 | 24 | 28 | 32 | 36 | 40 | 44 | 48 | 52 |
|---|---|---|---|---|---|---|---|---|---|---|---|---|---|---|
| Anifrolumab BR | 43 | 42 | 41 | 40 | 38 | 37 | 37 | 31 | 31 | 30 | 27 | 26 | 25 | 11 |
| Anifrolumab IR | 44 | 43 | 43 | 43 | 37 | 36 | 36 | 26 | 24 | 22 | 19 | 19 | 19 | 11 |
| Placebo | 45 | 42 | 39 | 37 | 32 | 31 | 30 | 24 | 23 | 22 | 18 | 18 | 18 | 7 |

# Figure 5A

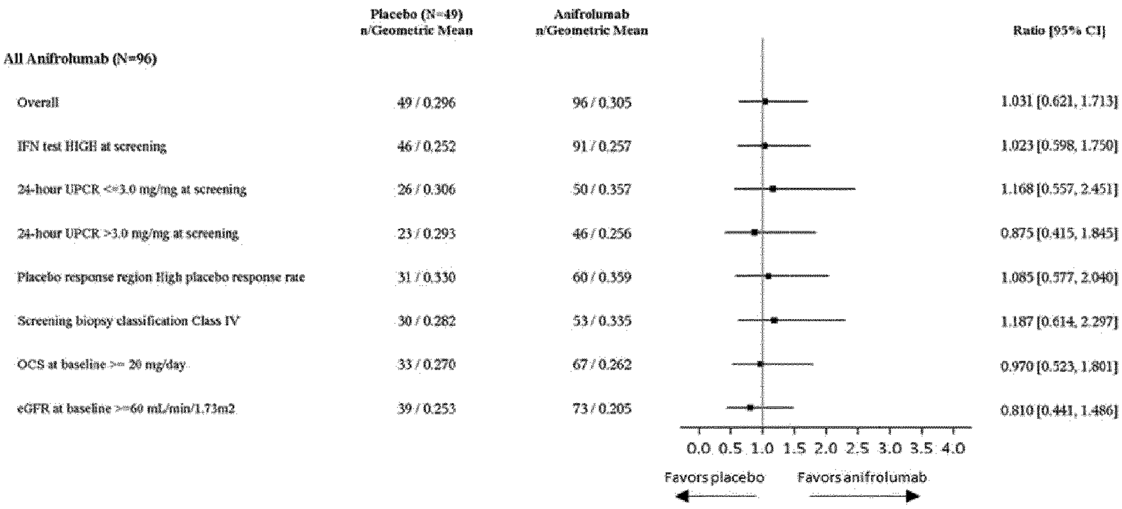


# Figure 5B

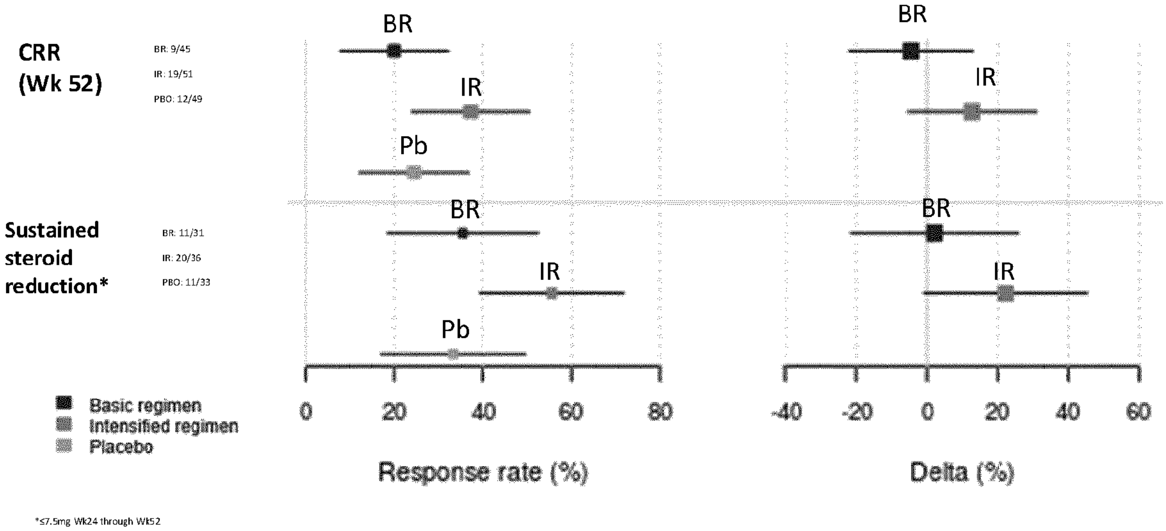

# Figure 6

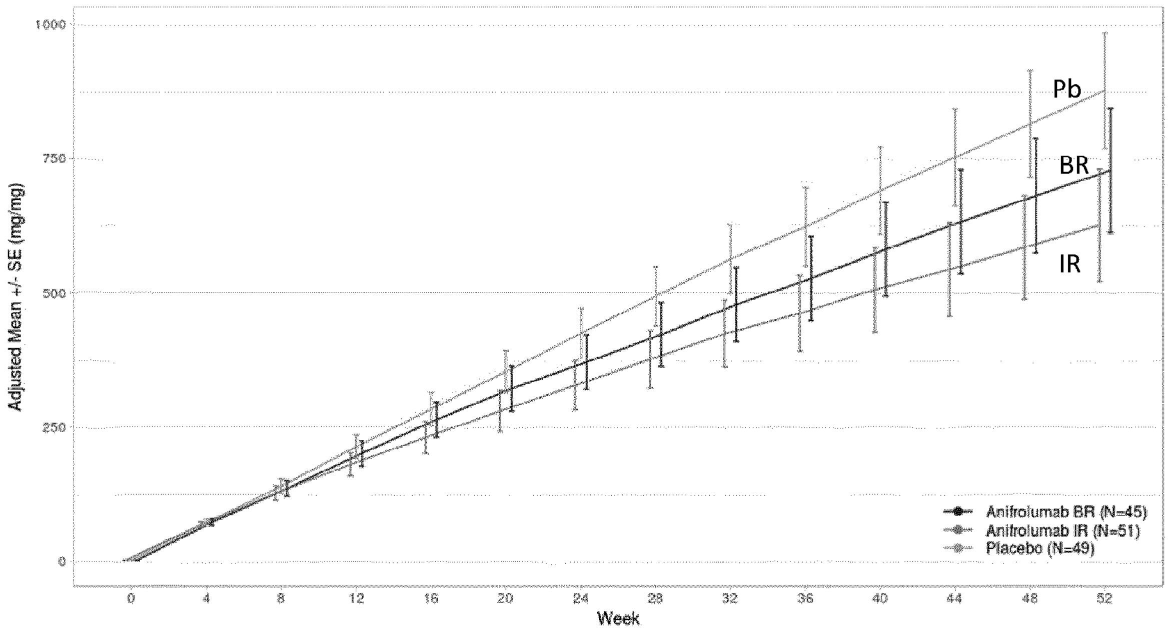
Adjusted Mean +/- SE (mg/mg)
1000
750
500
250
0
0
4
8
12
16
20
24
28
32
36
40
44
48
52
Week
Pb
BR
IR
Anifrolumab BR (N=45)
Anifrolumab IR (N=51)
Placebo (N=49)

# Figure 7A

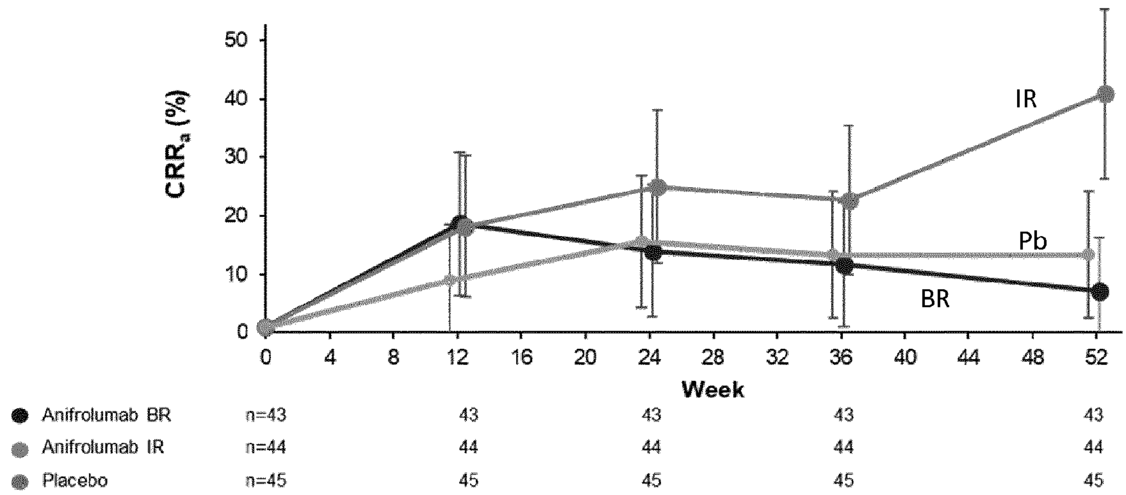


# Figure 7B

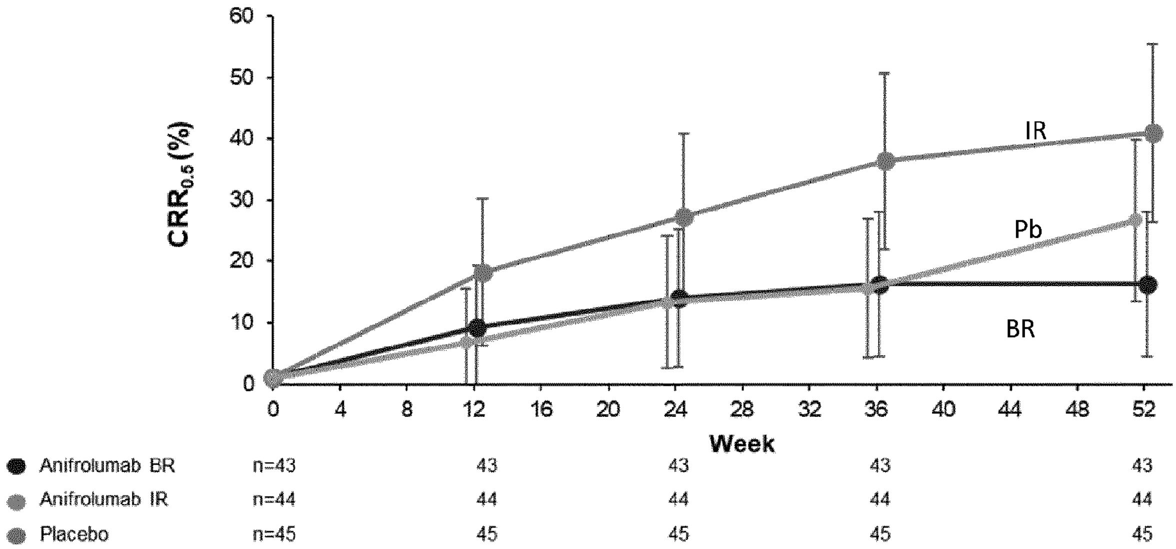

# Figure 8

A Median percentage 21-gene type I IFN PD neutralization among IFNGS test-high patients

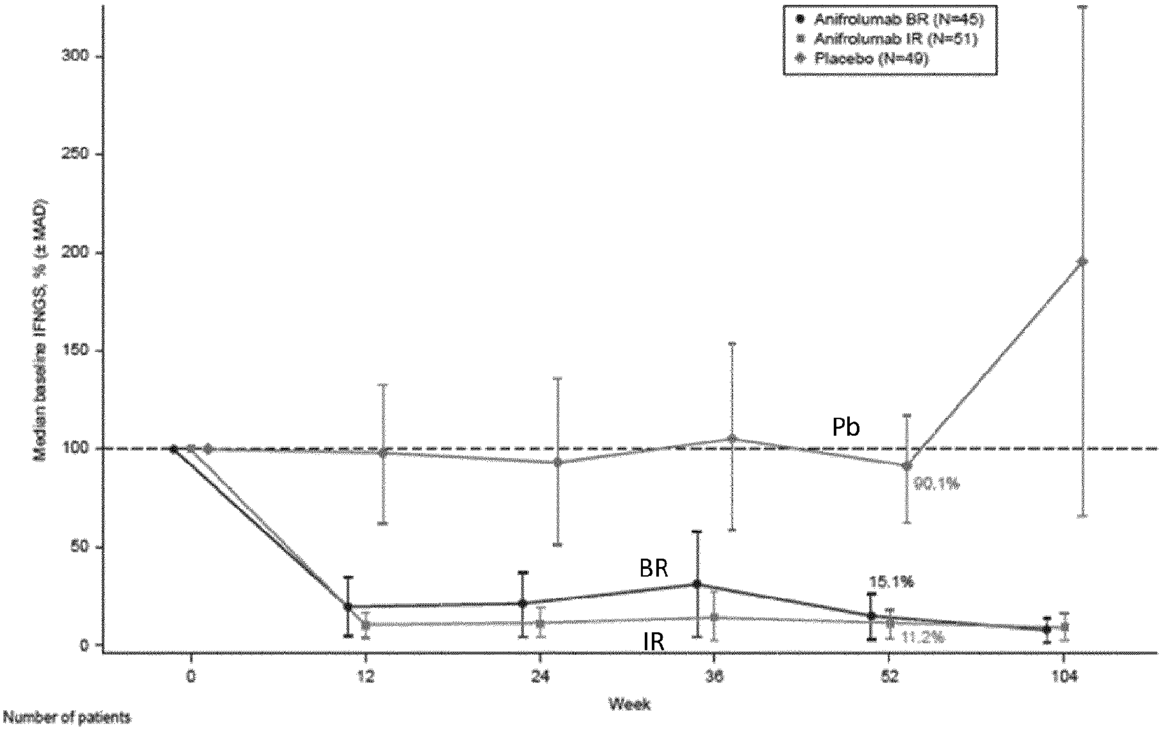


| Number of patients | | | | | |
|---|---|---|---|---|---|
| ● Anifrolumab BR | 39 | 35 | 38 | 30 | 13 |
| ■ Anifrolumab IR | 45 | 43 | 40 | 35 | 21 |
| ◆ Placebo | 37 | 35 | 34 | 31 | 10 |

# Figure 9A

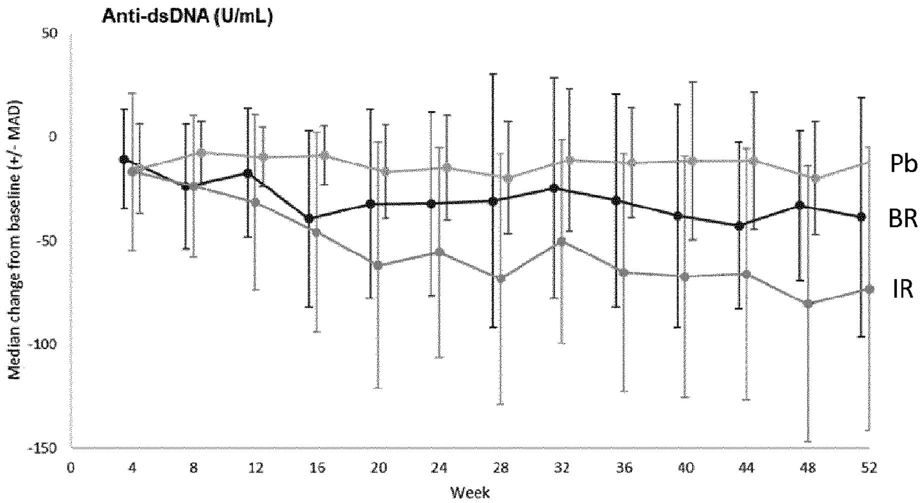


| Number of patients | | | | | | | | | | | | | | |
|---|---|---|---|---|---|---|---|---|---|---|---|---|---|---|
| Anifrolumab BR | 37 | 34 | 35 | 36 | 35 | 34 | 33 | 34 | 34 | 34 | 31 | 29 | 31 | 29 |
| Anifrolumab IR | 39 | 34 | 38 | 36 | 36 | 35 | 35 | 36 | 37 | 37 | 39 | 38 | 37 | 33 |
| Placebo | 39 | 32 | 36 | 34 | 37 | 36 | 33 | 35 | 32 | 33 | 34 | 32 | 31 | 30 |

# Figure 9B

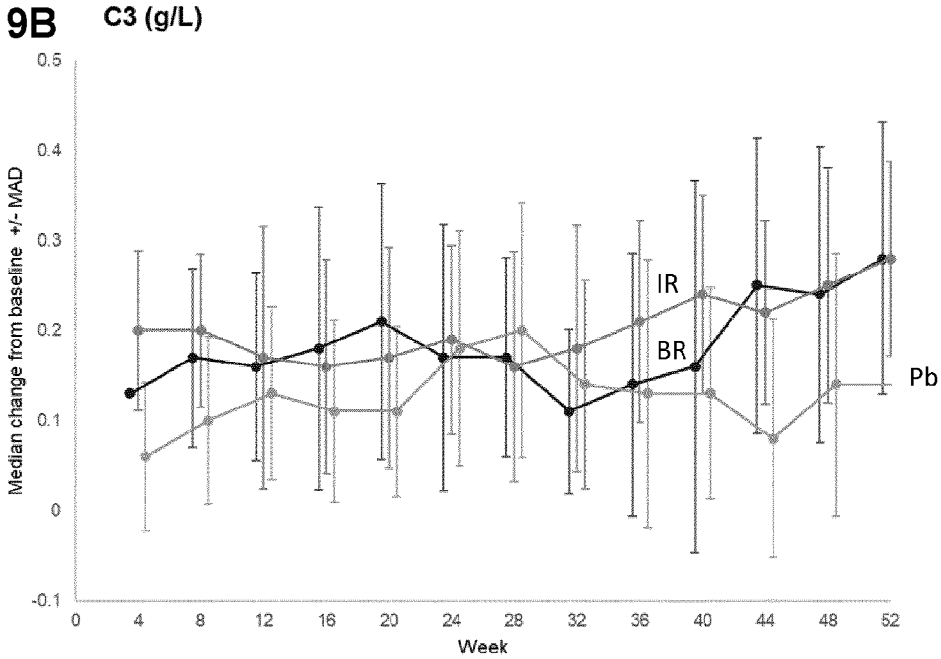


| Number of patients | | | | | | | | | | | | | | |
|---|---|---|---|---|---|---|---|---|---|---|---|---|---|---|
| Anifrolumab BR | 30 | 30 | 29 | 28 | 28 | 29 | 23 | 28 | 25 | 27 | 25 | 23 | 25 | 23 |
| Anifrolumab IR | 27 | 26 | 27 | 27 | 26 | 25 | 26 | 25 | 25 | 26 | 27 | 26 | 25 | 24 |
| Placebo | 42 | 40 | 39 | 37 | 38 | 38 | 36 | 36 | 35 | 34 | 36 | 32 | 32 | 33 |

# Figure 10

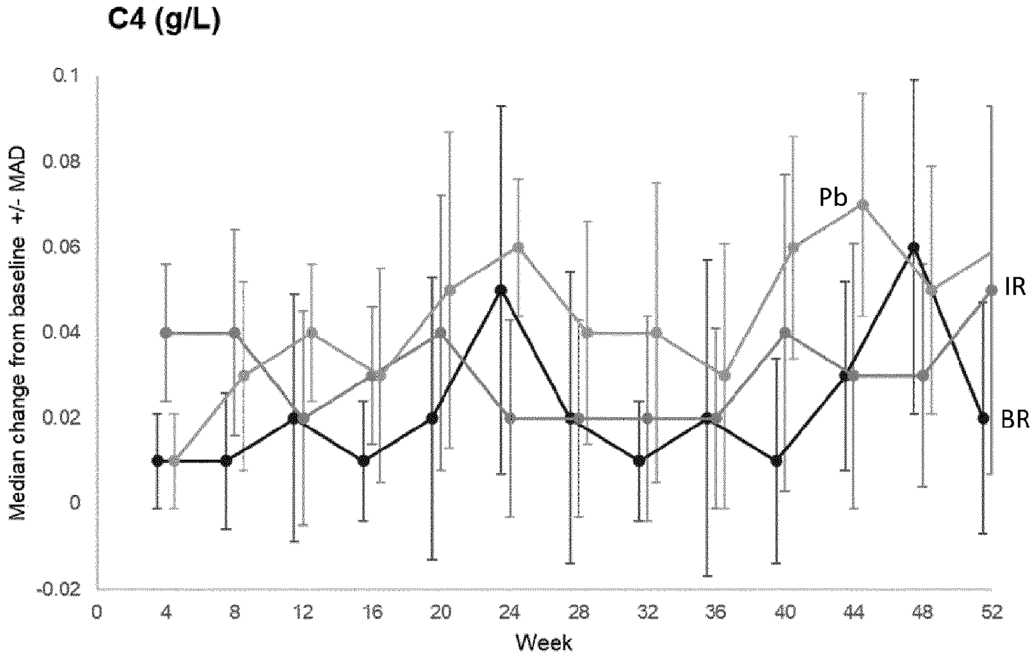


**Number of patients**

| | | | | | | | | | | | | | | |
|---|---|---|---|---|---|---|---|---|---|---|---|---|---|---|
| Anifrolumab BR | 10 | 10 | 10 | 10 | 9 | 10 | 7 | 10 | 8 | 9 | 10 | 9 | 9 | 8 |
| Anifrolumab IR | 14 | 13 | 13 | 14 | 13 | 13 | 13 | 13 | 14 | 13 | 14 | 13 | 13 | 12 |
| Placebo | 20 | 20 | 18 | 16 | 18 | 17 | 16 | 16 | 16 | 15 | 16 | 14 | 13 | 14 |

FIG. 11A

Figure 11B

Figure 11C

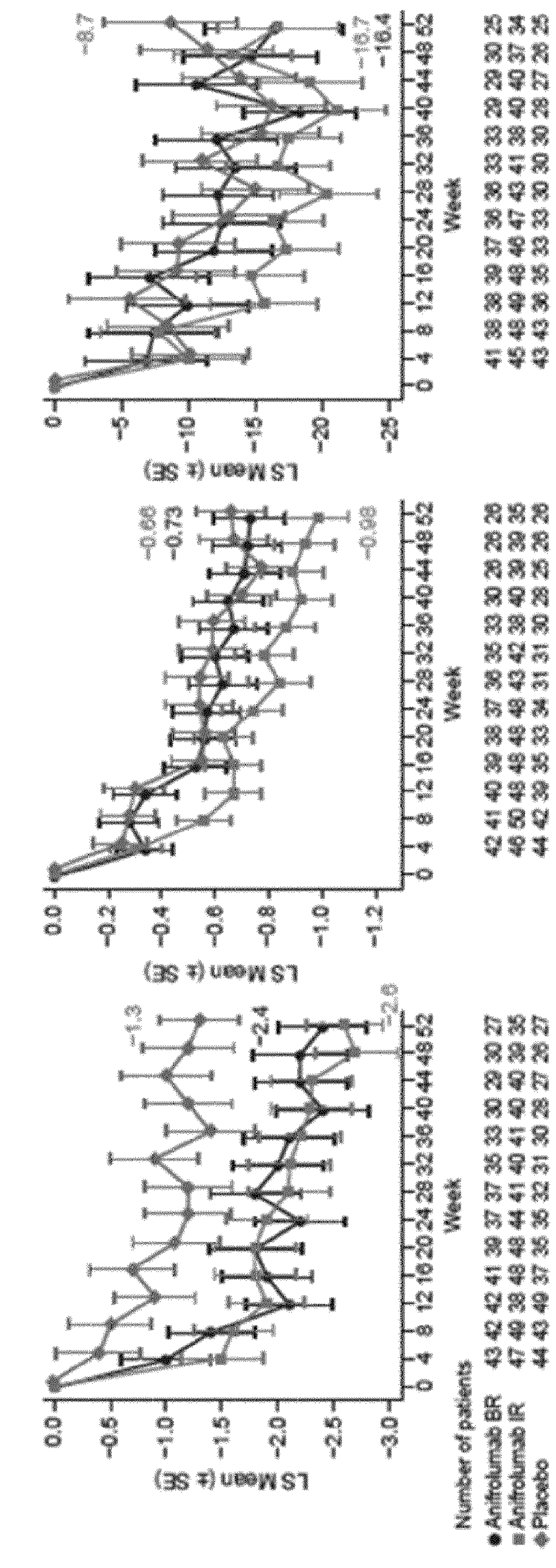

Fligure 12

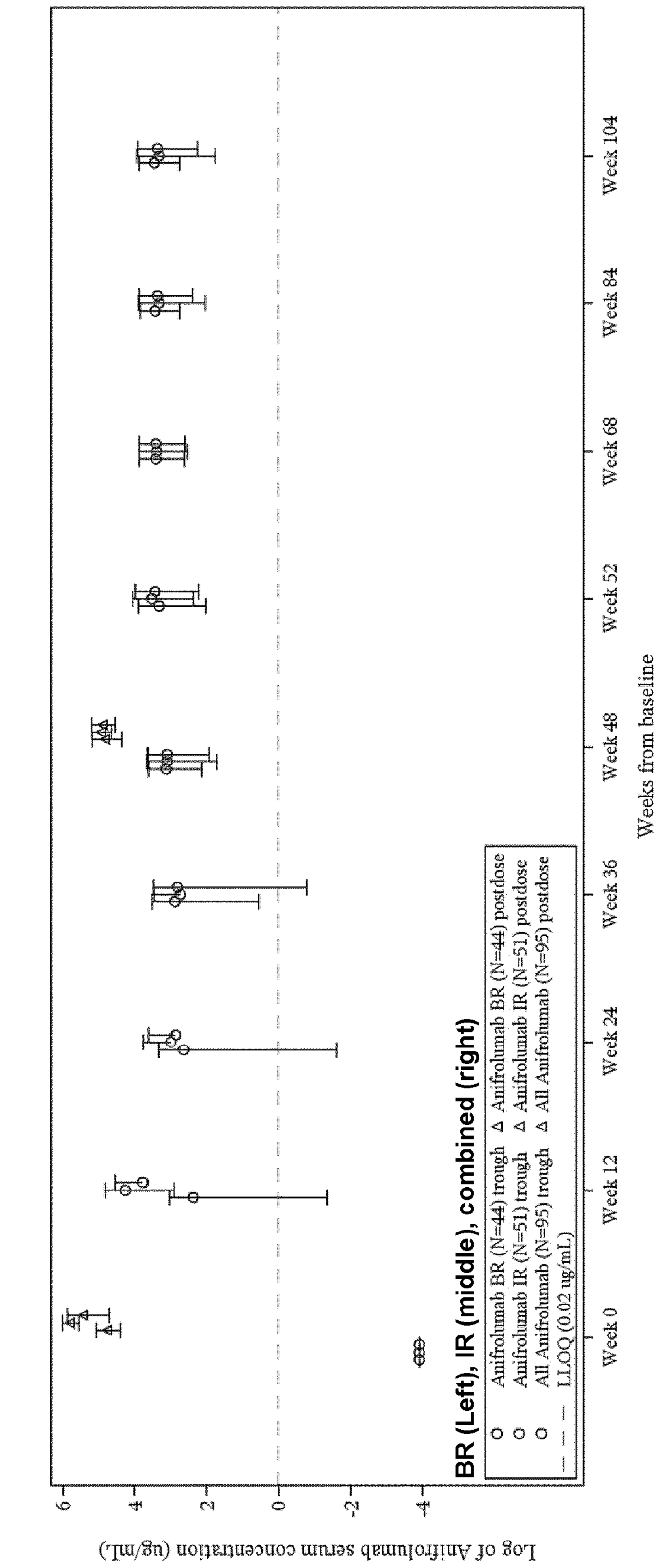
BR (Left), IR (middle), combined (right)
Anifrolumab BR (N=44) trough
Anifrolumab BR (N=44) postdose
Anifrolumab IR (N=51) trough
Anifrolumab IR (N=51) postdose
All Anifrolumab (N=95) trough
All Anifrolumab (N=95) postdose
LLOQ (0.02 ug/mL)
Log of Anifrolumab serum concentration (ug/mL)
6
4
2
0
-2
-4
Week 0
Week 12
Week 24
Week 36
Week 48
Week 52
Week 68
Week 84
Week 104
Weeks from baseline

# Figure 13A

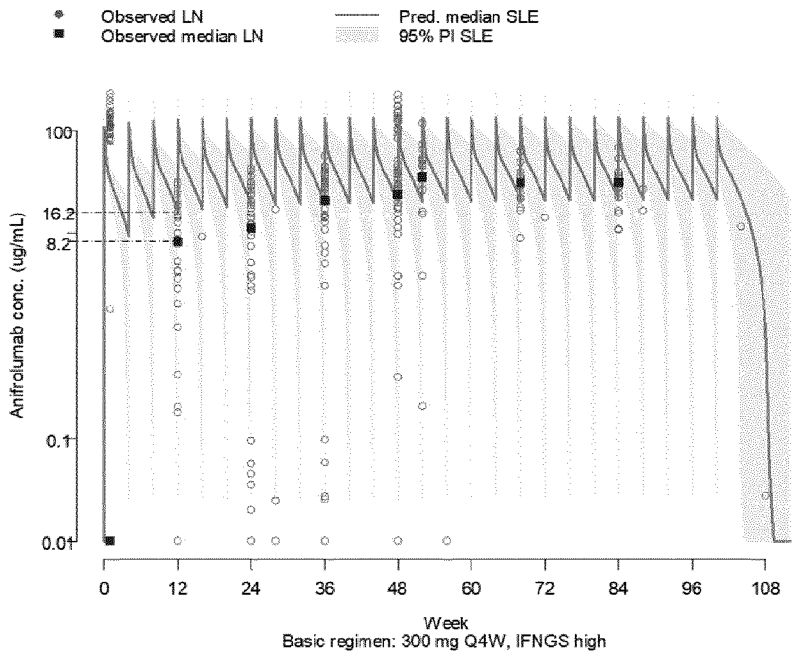


# Figure 13B

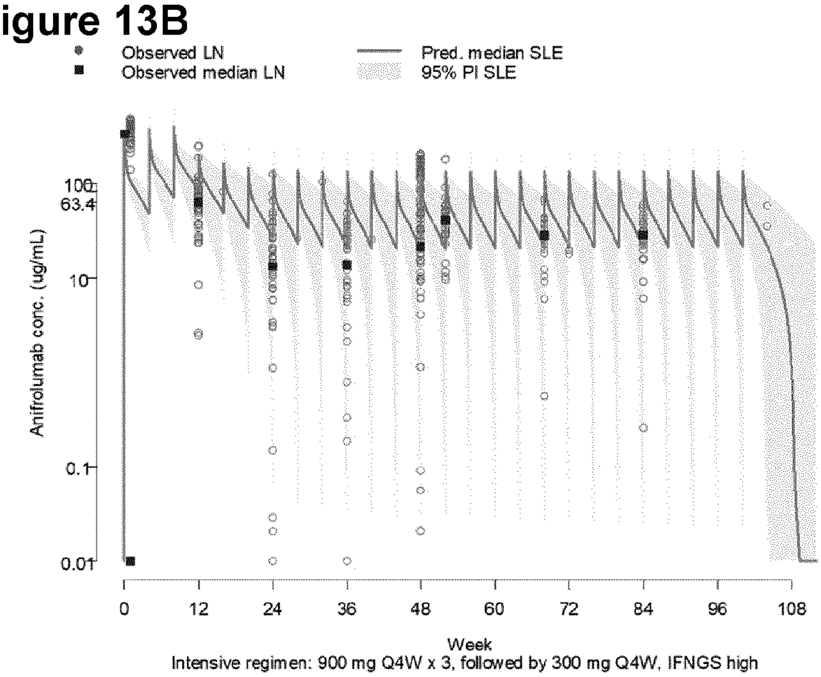

# Figure 14

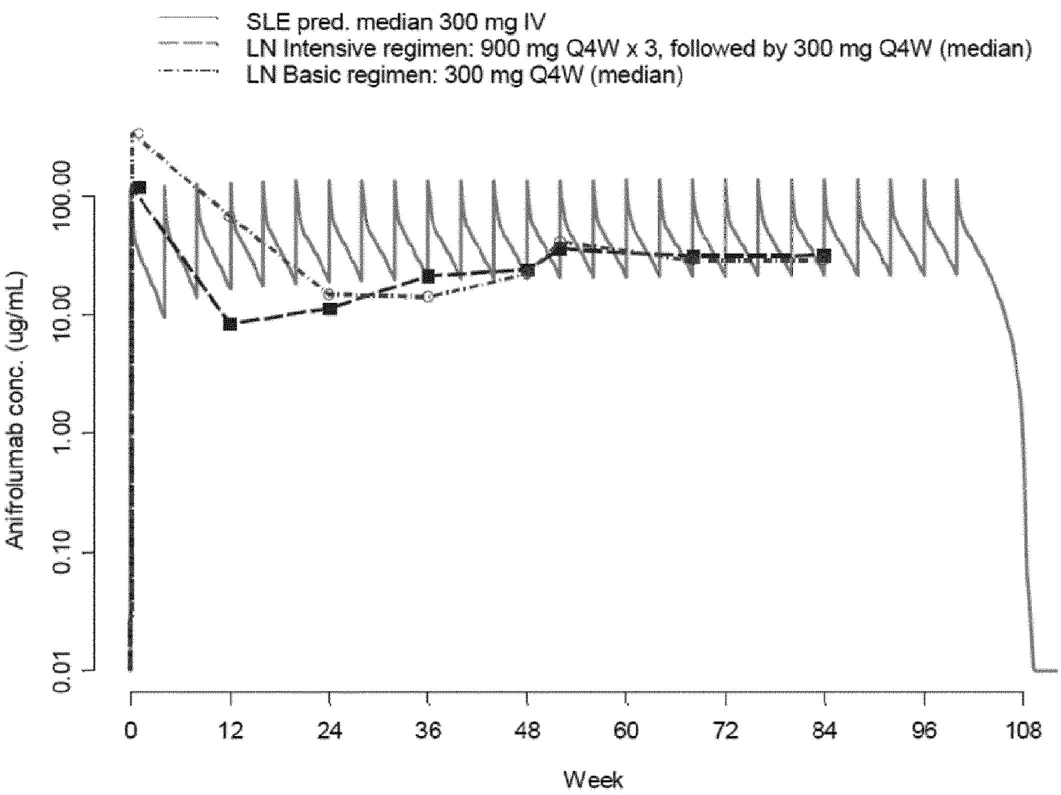
SLE pred. median 300 mg IV
LN Intensive regimen: 900 mg Q4W x 3, followed by 300 mg Q4W (median)
LN Basic regimen: 300 mg Q4W (median)
Anifrolumab conc. (ug/mL)
100.00
10.00
1.00
0.10
0.01
0
12
24
36
48
60
72
84
96
108
Week

Figure 15

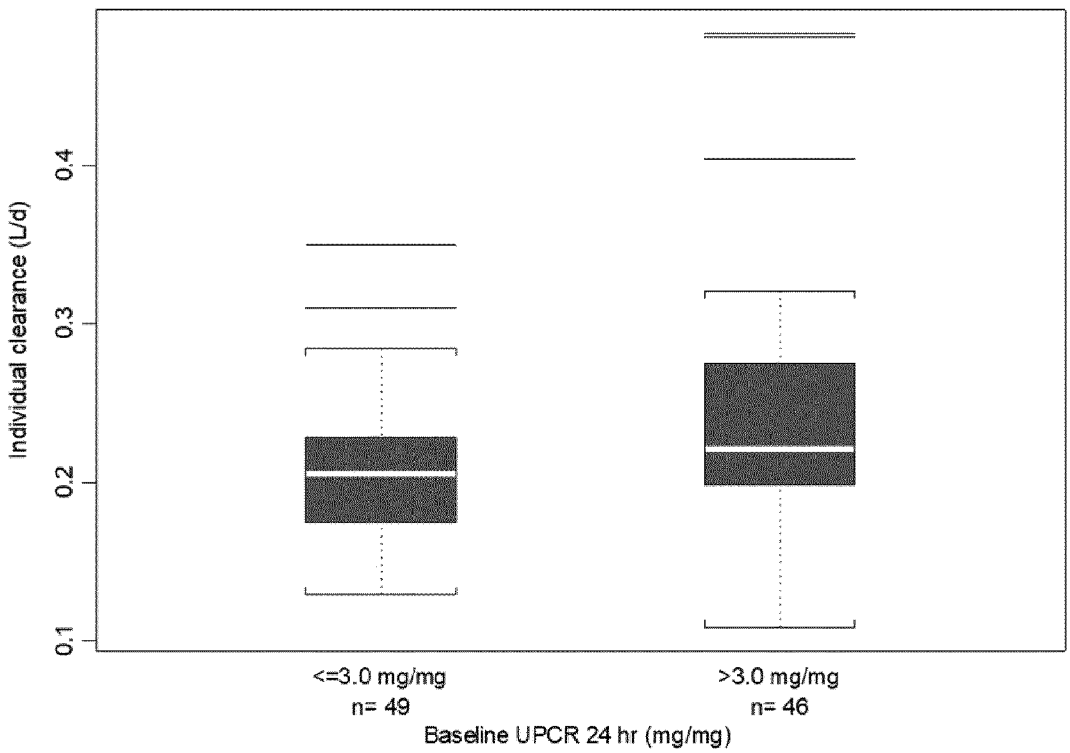
Individual clearance (L/d)
0.1
0.2
0.3
0.4
<=3.0 mg/mg
n= 49
>3.0 mg/mg
n= 46
Baseline UPCR 24 hr (mg/mg)

Figure 16A

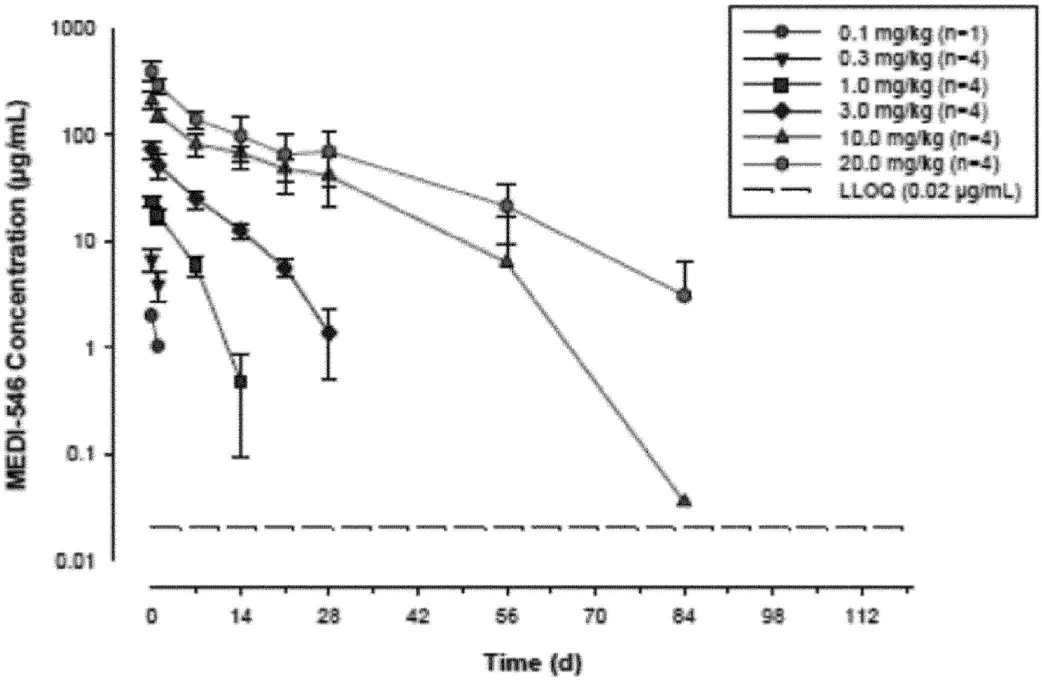


Figure 16B

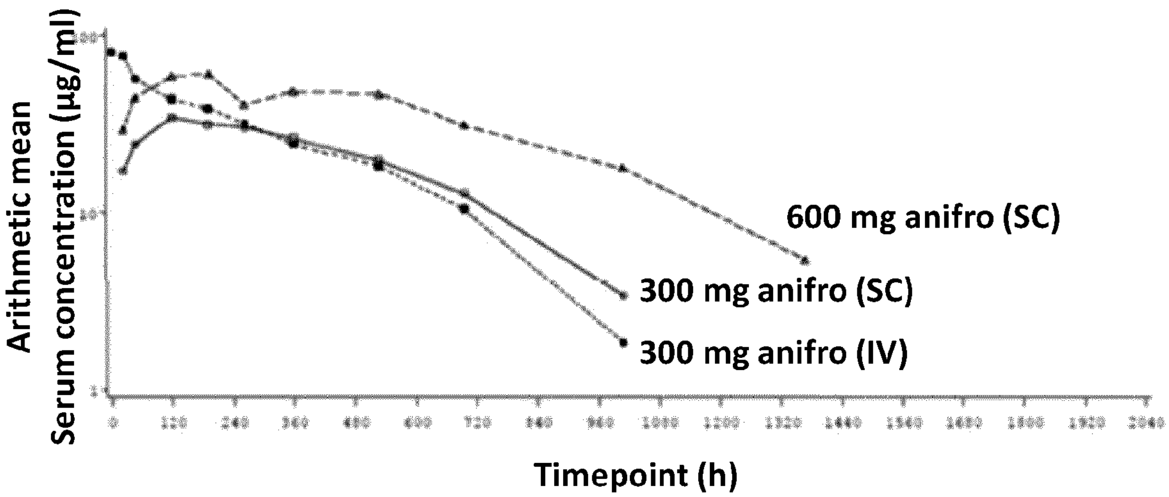

# Figure 17A

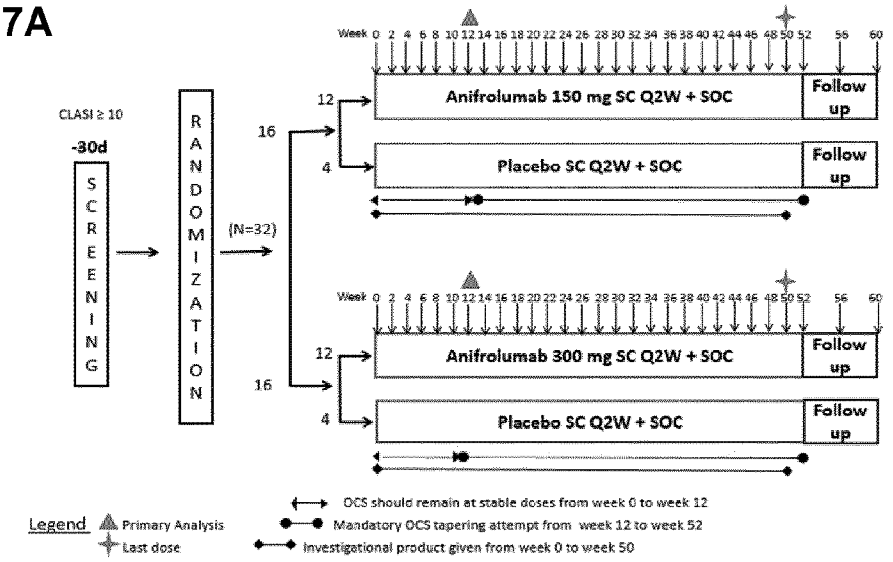


# Figure 17B

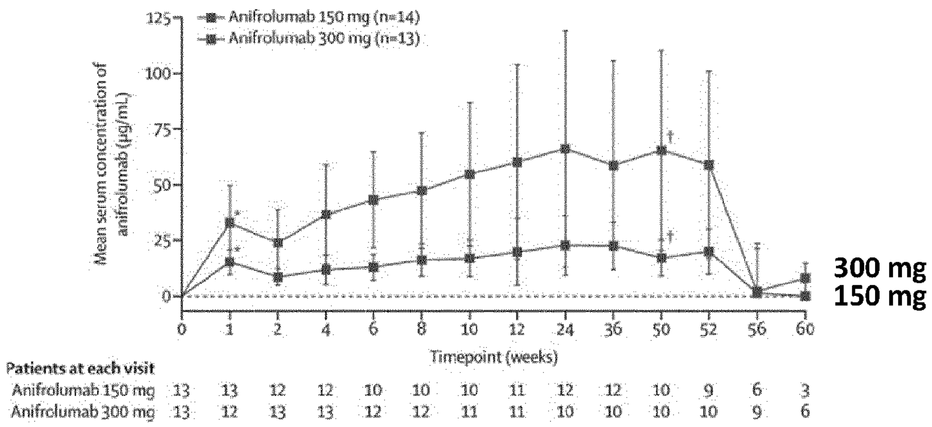


# Figure 17C

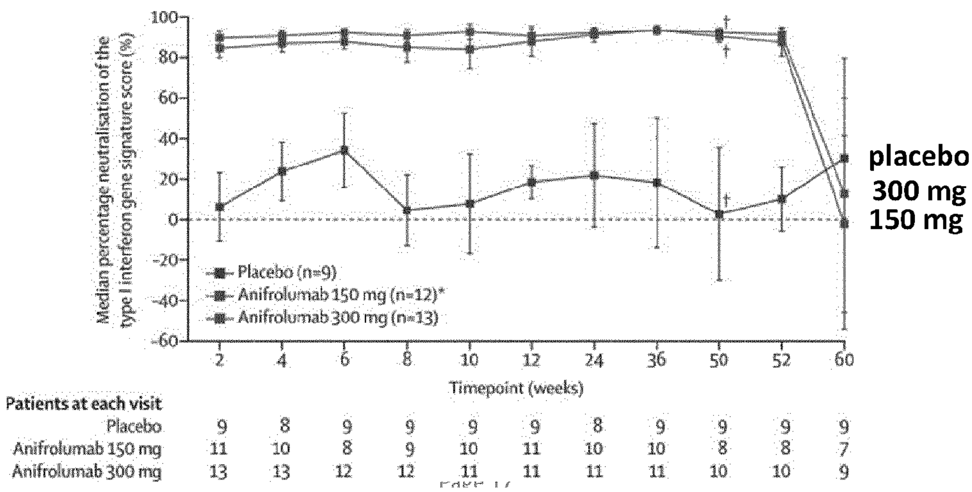

# Figure 18

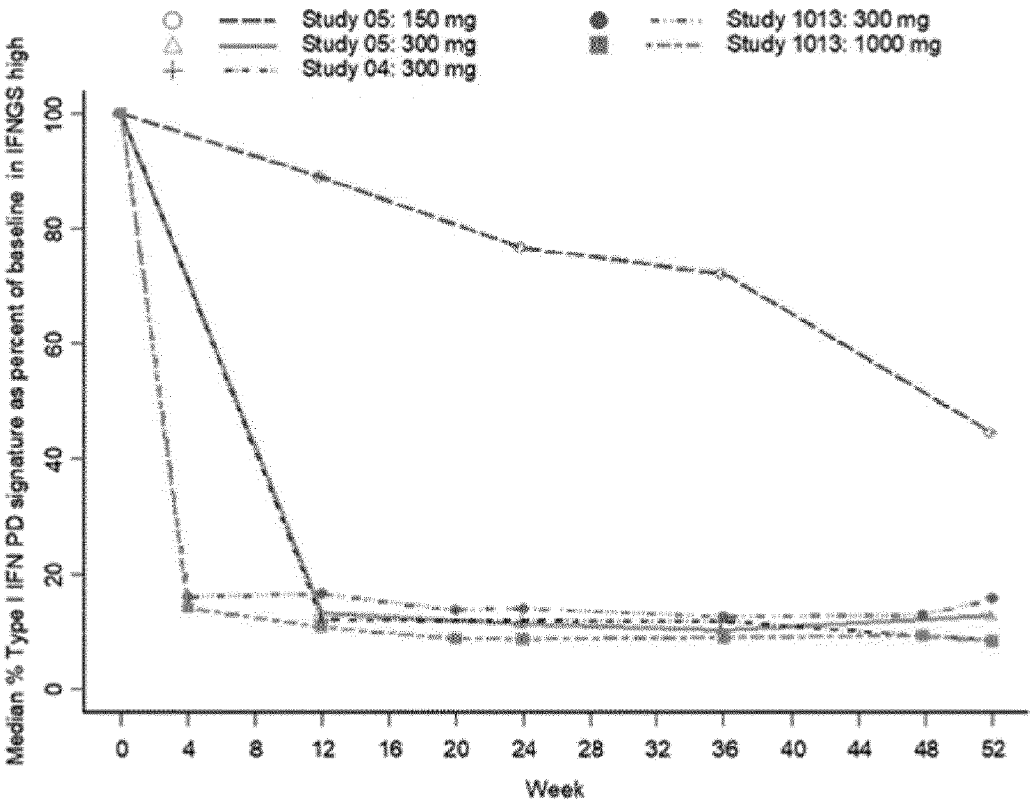
Study 05: 150 mg
Study 05: 300 mg
Study 04: 300 mg
Study 1013: 300 mg
Study 1013: 1000 mg
Median % Type I IFN PD signature as percent of baseline in IFNGS high
0
20
40
60
80
100
0
4
8
12
16
20
24
28
32
36
40
44
48
52
Week

# Figure 19A

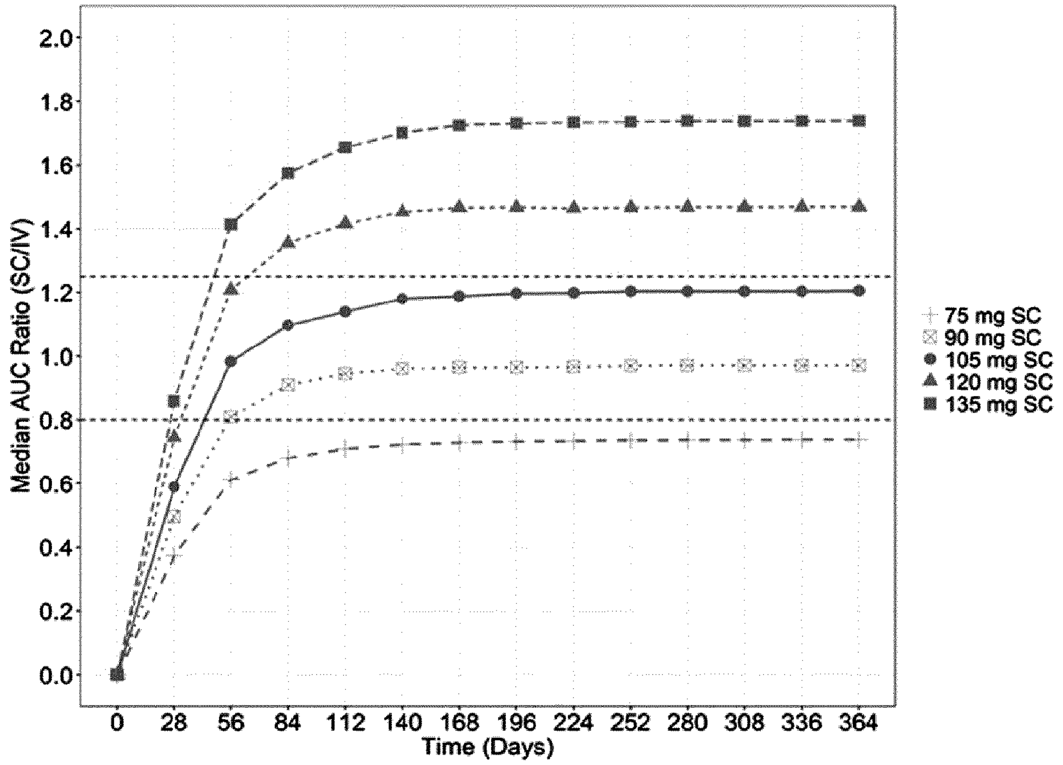


# Figure 19B

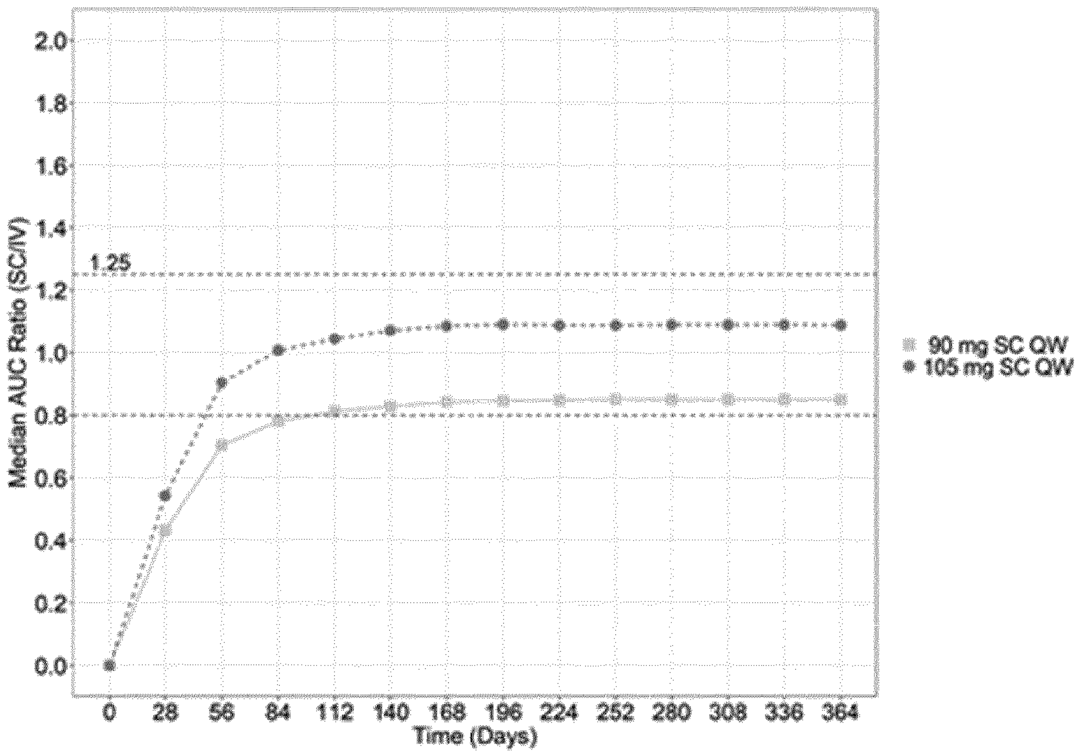

## Figure 20A

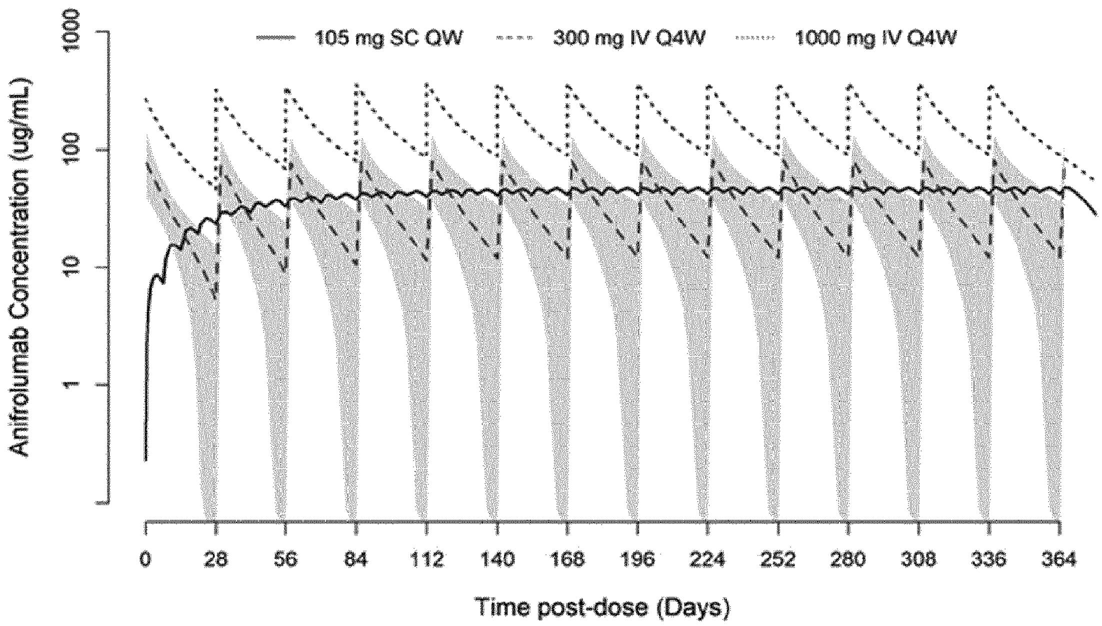


## Figure 20B

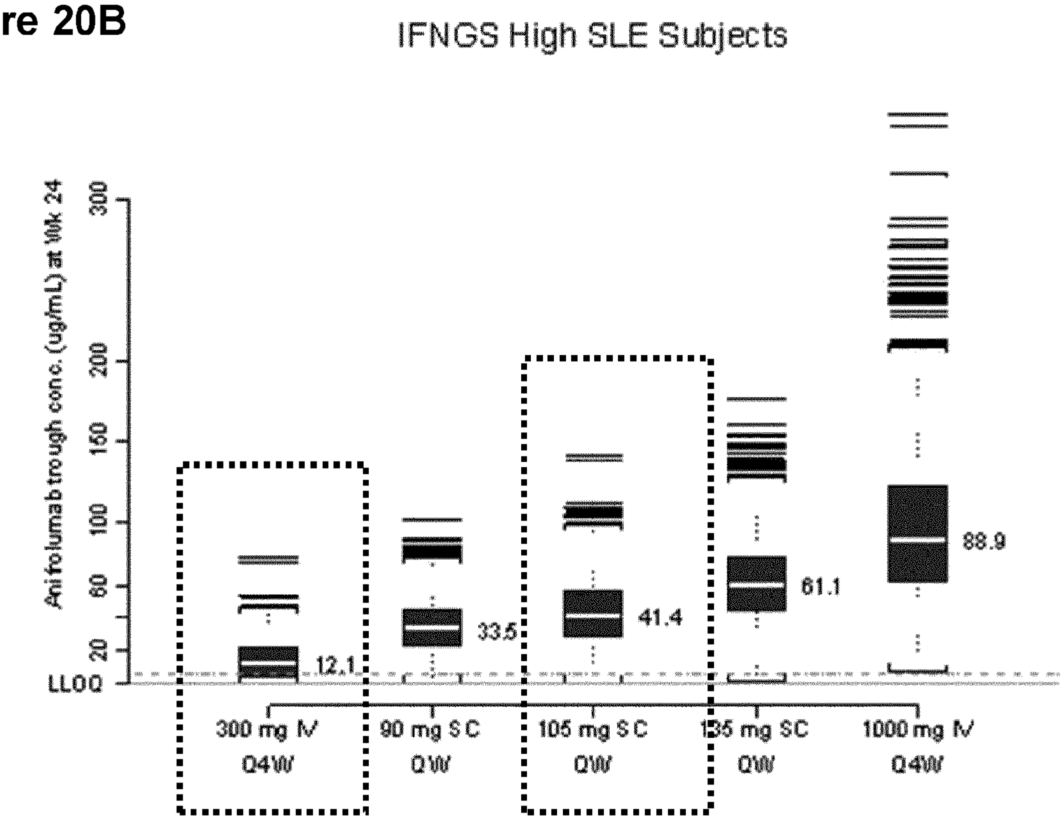

# Figure 21A

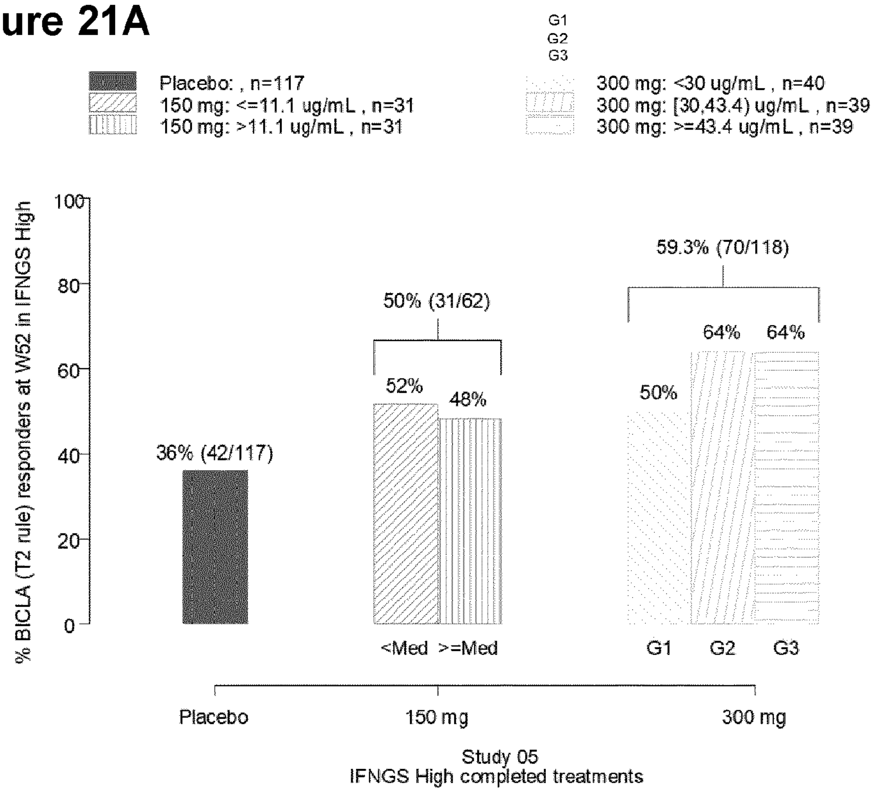


# Figure 21B

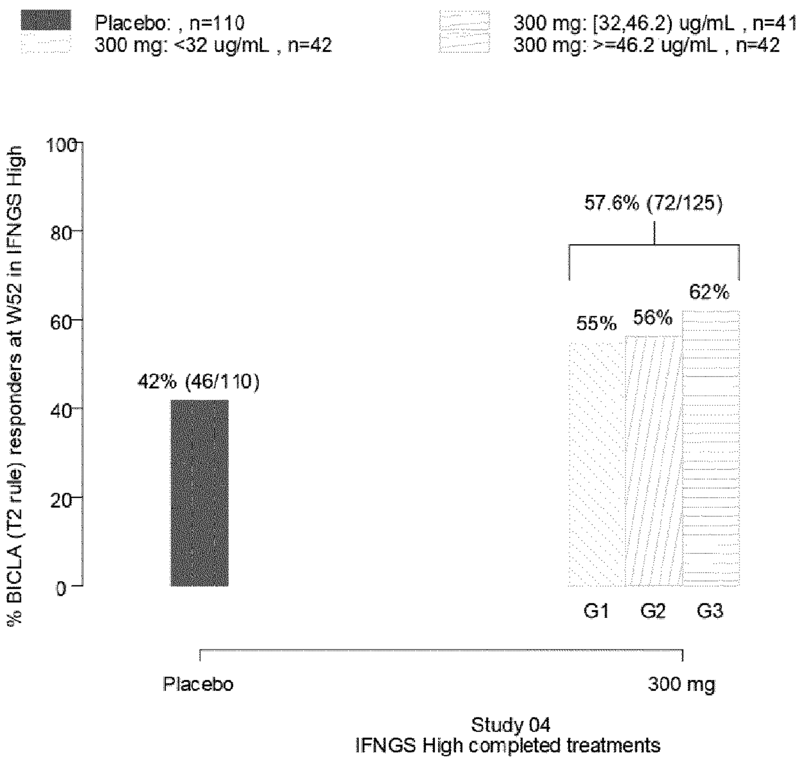

## Figure 22A

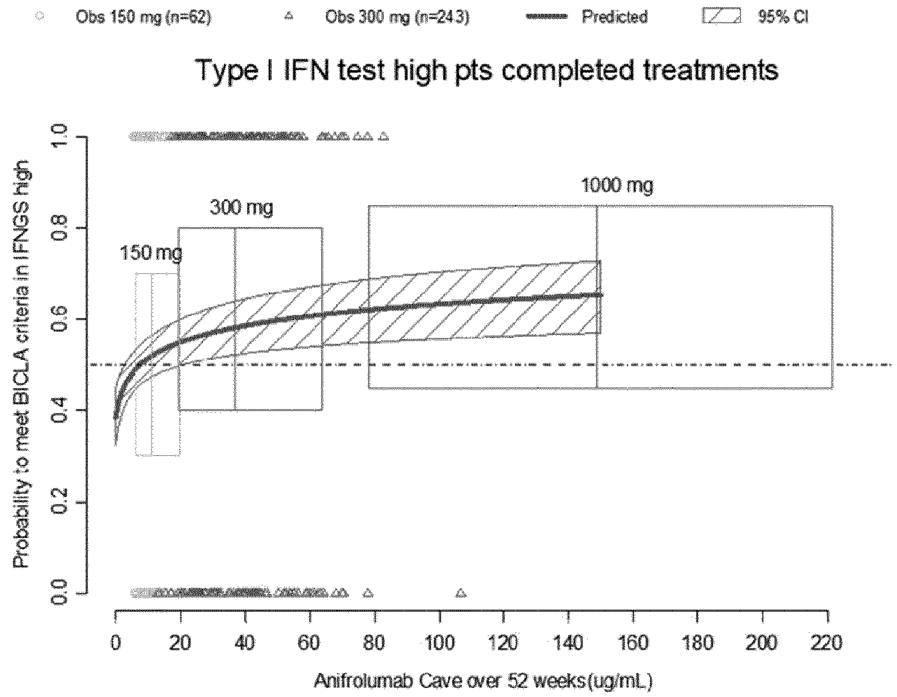


## Figure 22B

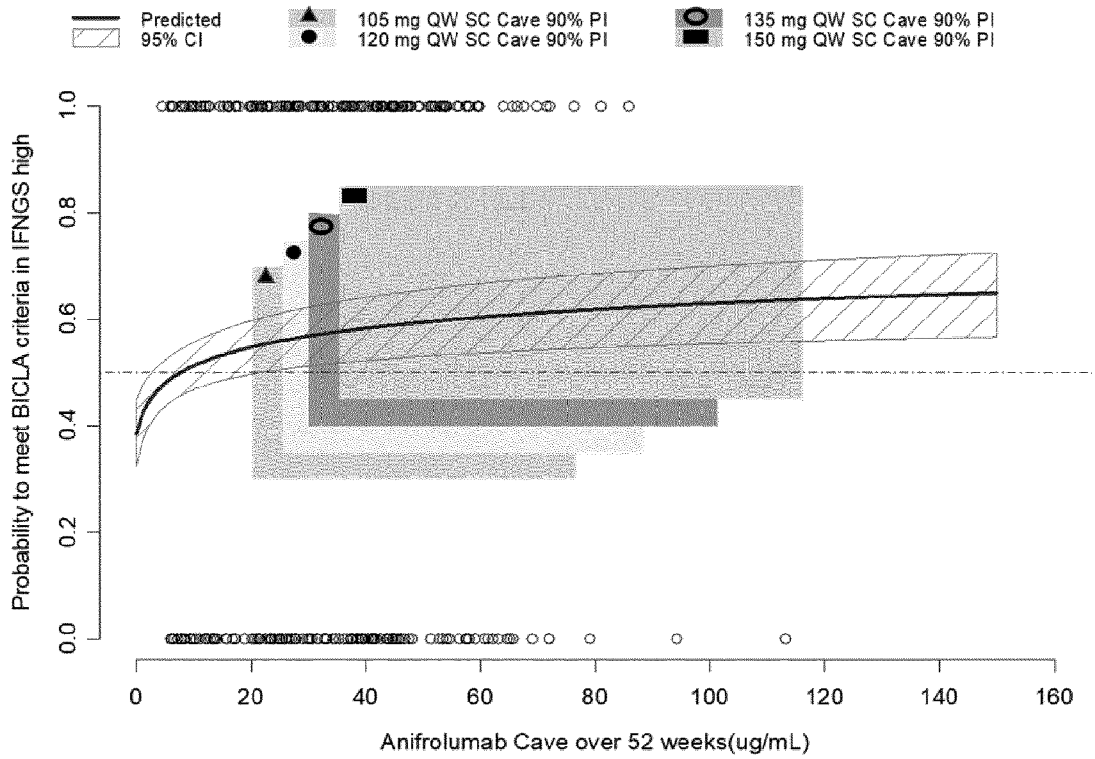

Figure 23A

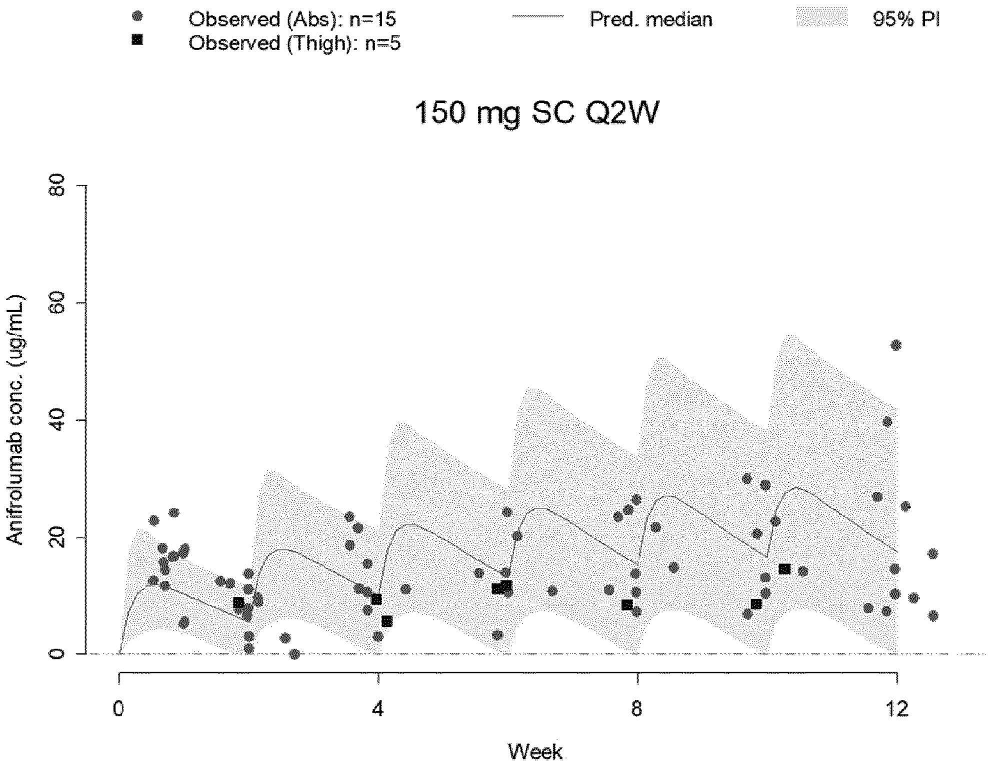


Figure 23B

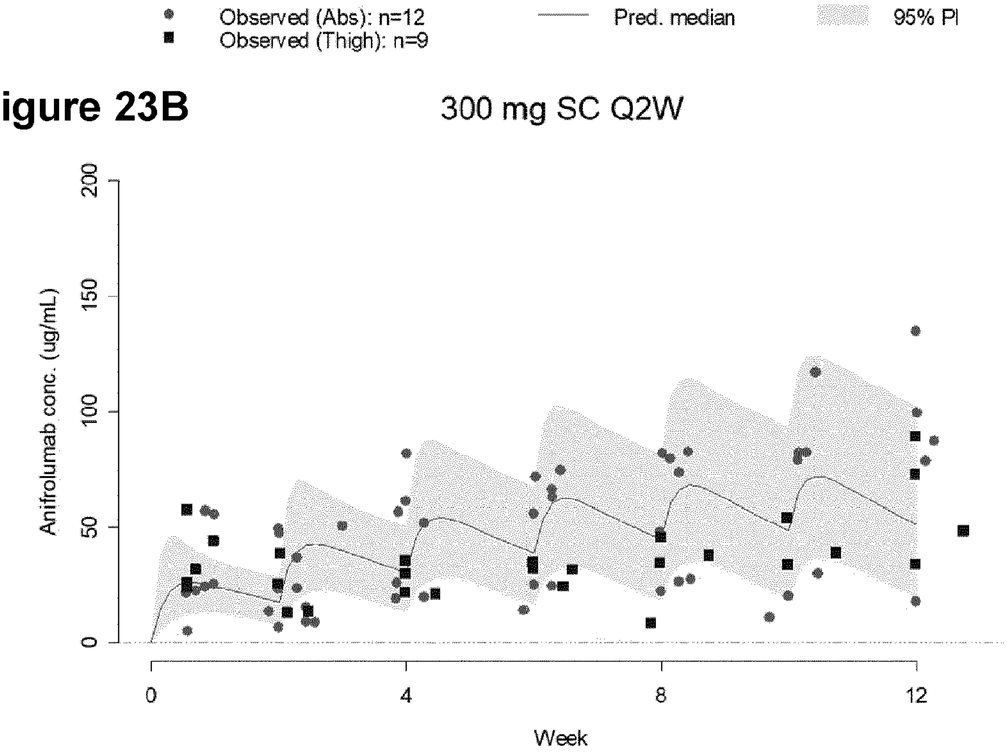

# Figure 24

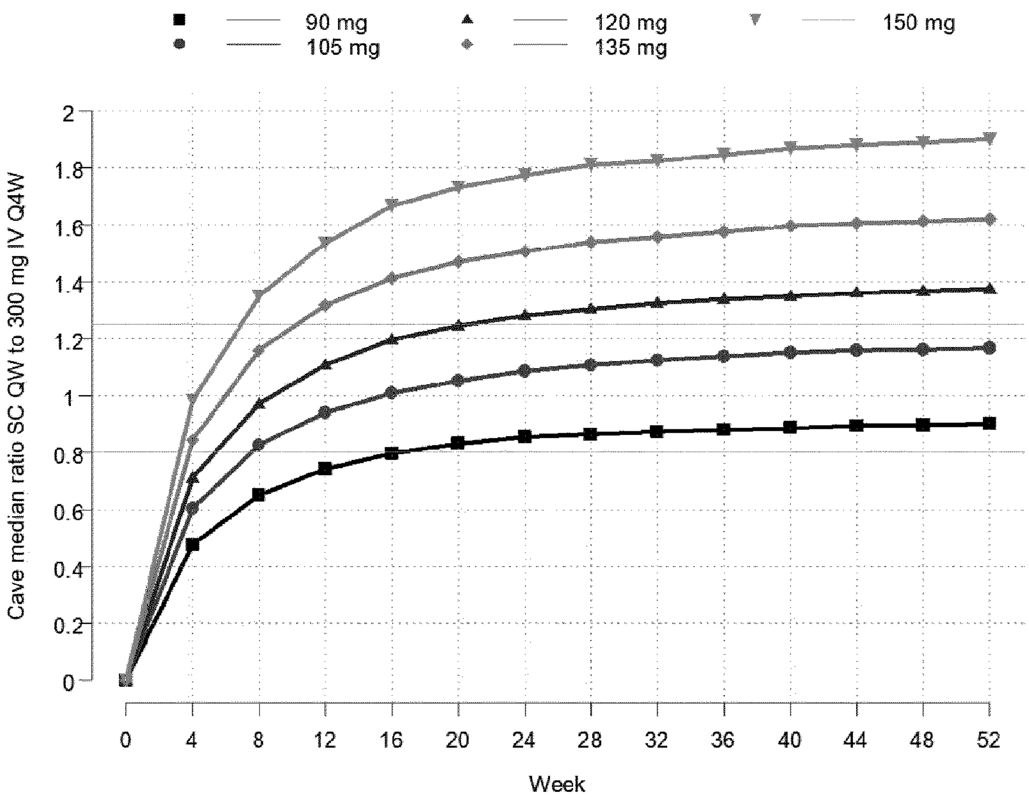
90 mg
105 mg
120 mg
135 mg
150 mg
Cave median ratio SC QW to 300 mg IV Q4W
Week
0
0.2
0.4
0.6
0.8
1
1.2
1.4
1.6
1.8
2
0 4 8 12 16 20 24 28 32 36 40 44 48 52

# Figure 25A

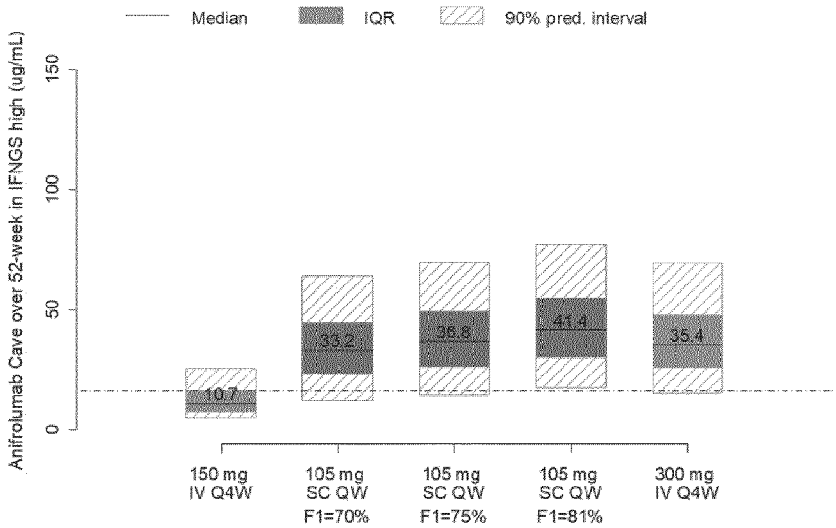


# Figure 25B

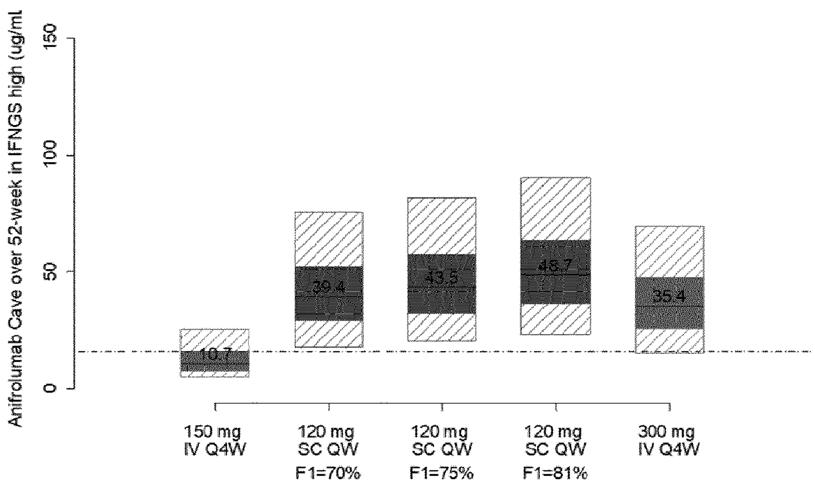


# Figure 25C

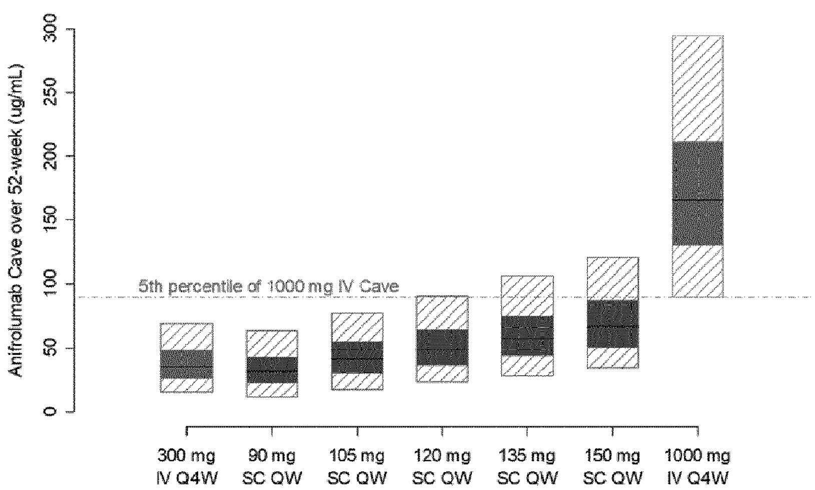

## Figure 26A

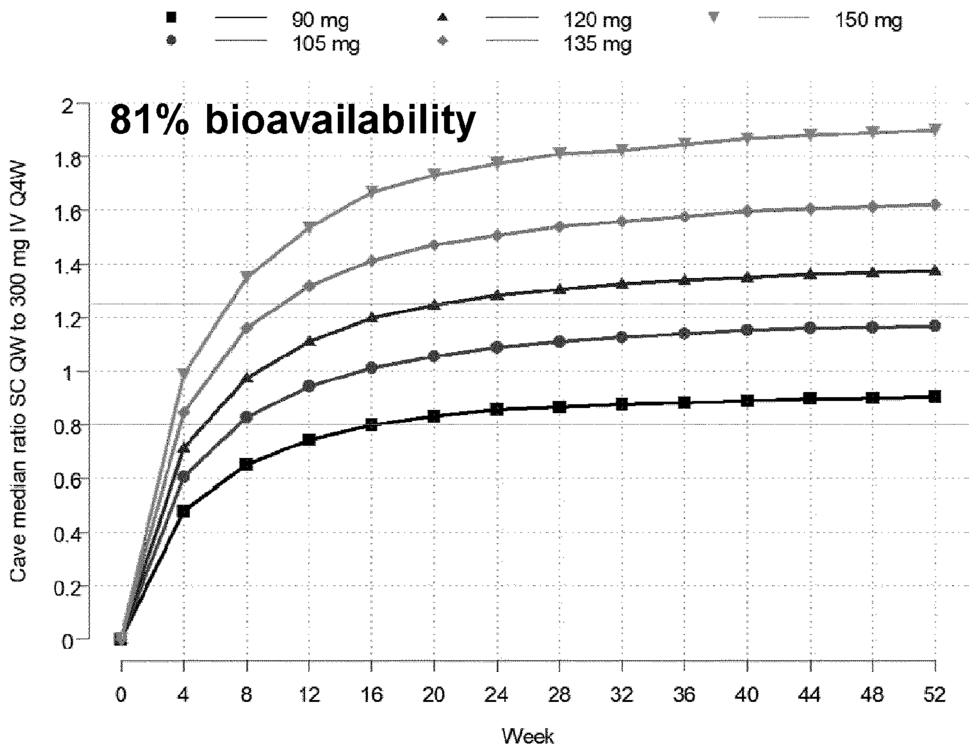


## Figure 26B

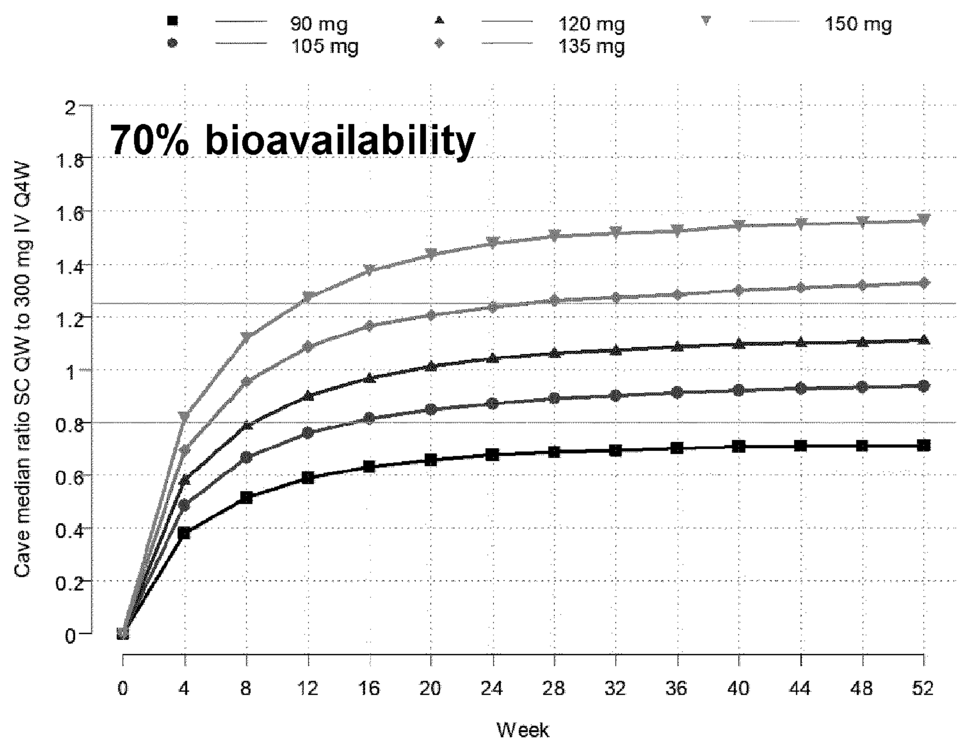

# Figure 27

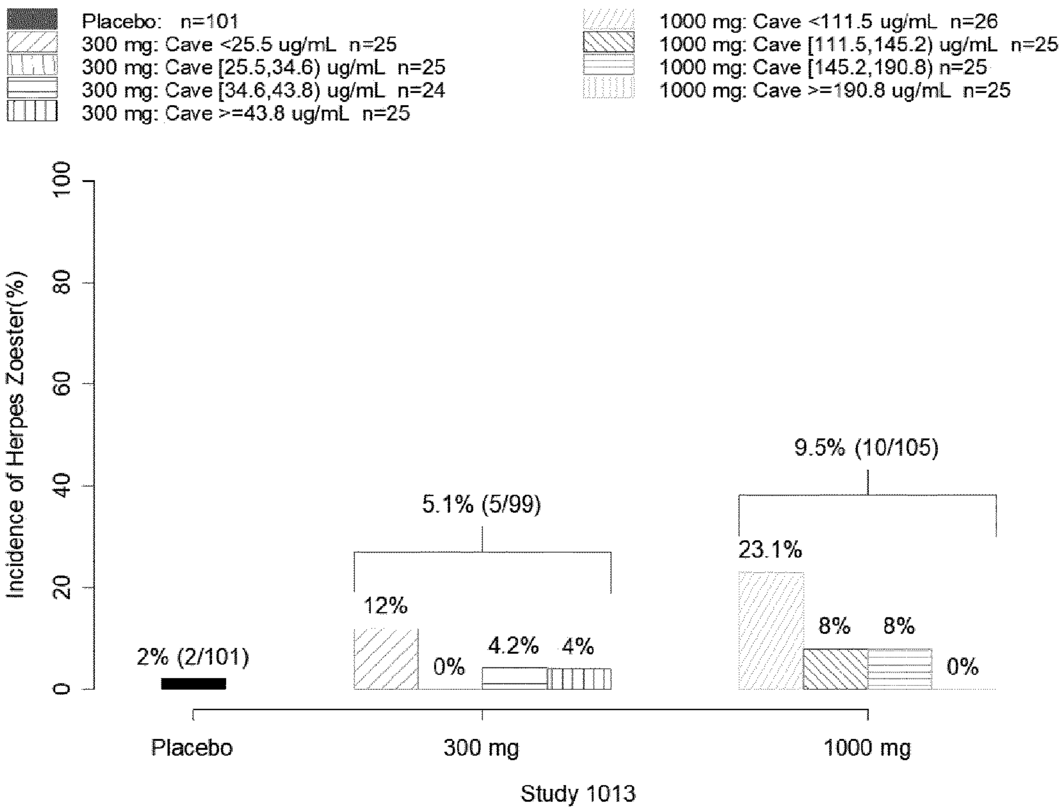
Placebo: n=101
300 mg: Cave <25.5 ug/mL n=25
300 mg: Cave [25.5,34.6) ug/mL n=25
300 mg: Cave [34.6,43.8) ug/mL n=24
300 mg: Cave >=43.8 ug/mL n=25
1000 mg: Cave <111.5 ug/mL n=26
1000 mg: Cave [111.5,145.2) ug/mL n=25
1000 mg: Cave [145.2,190.8) n=25
1000 mg: Cave >=190.8 ug/mL n=25
Incidence of Herpes Zoester(%)
0
20
40
60
80
100
2% (2/101)
5.1% (5/99)
12%
0%
4.2%
4%
9.5% (10/105)
23.1%
8%
8%
0%
Placebo
300 mg
1000 mg
Study 1013

Figure 28A

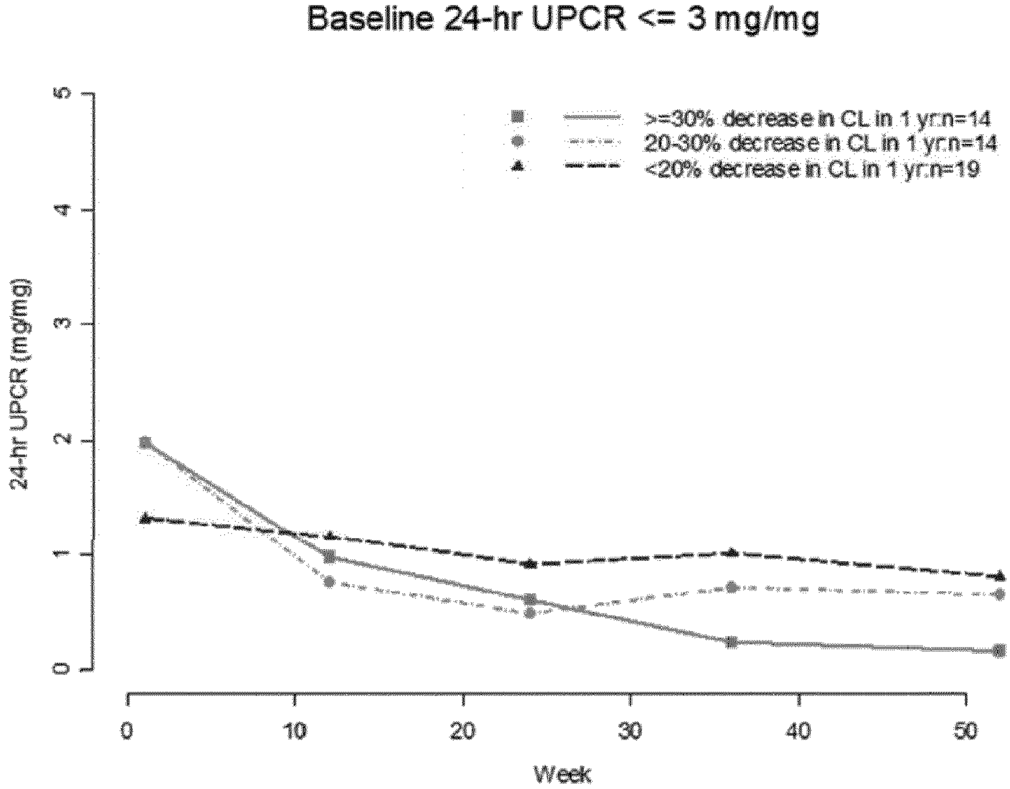


Figure 28B

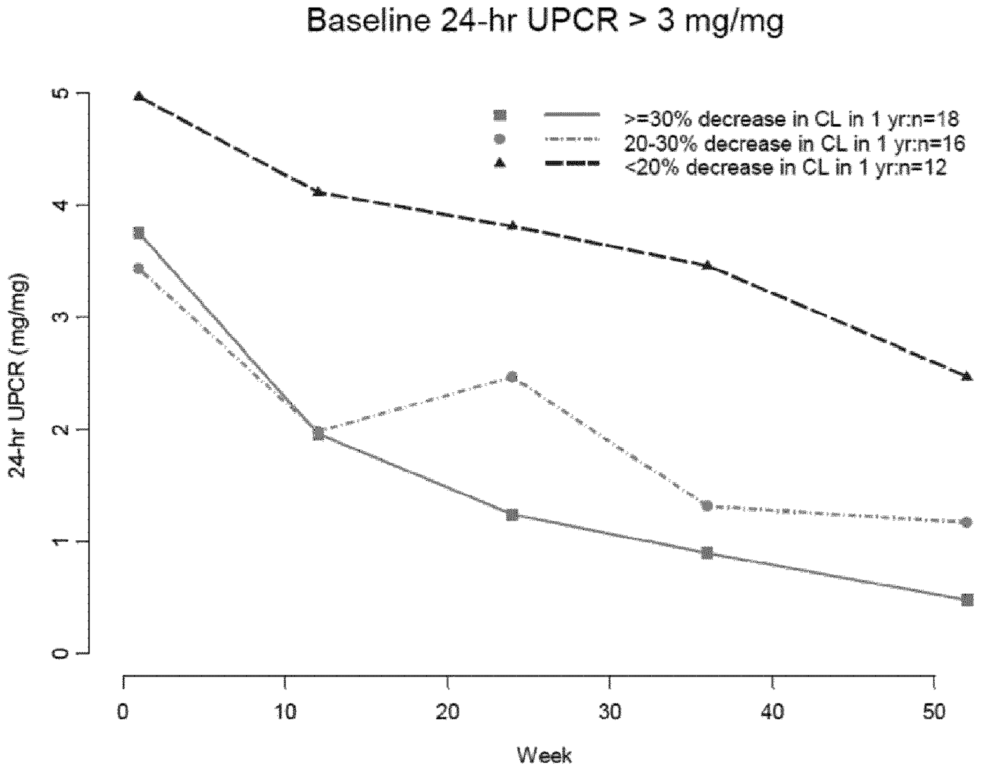

**Figure 29A**

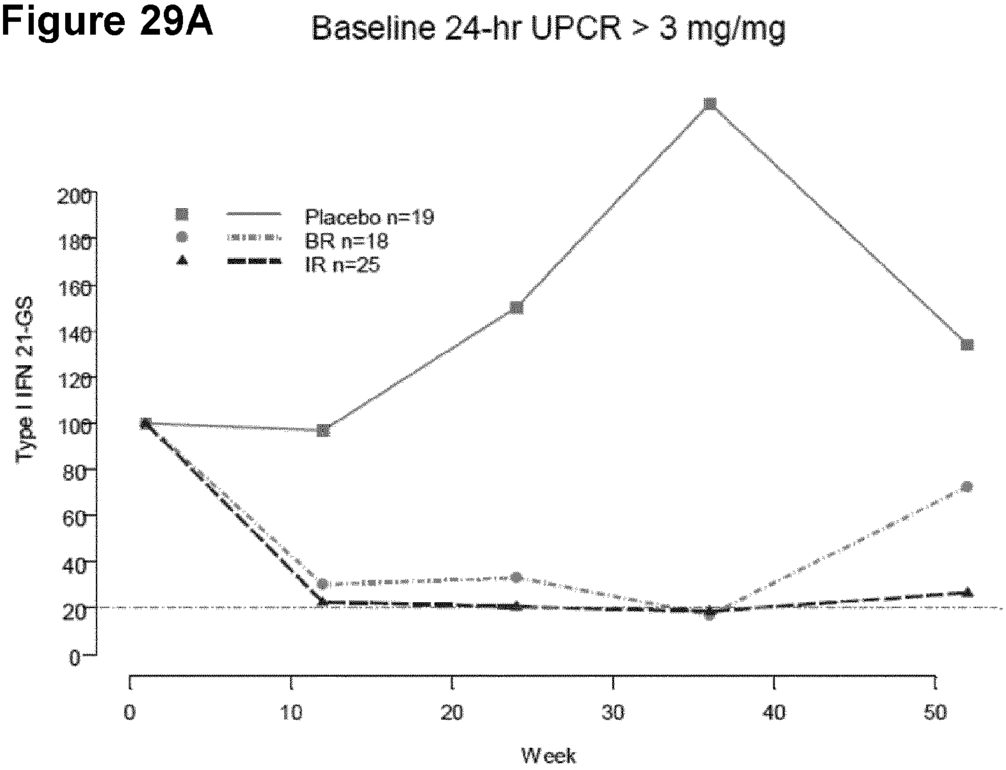


**Figure 29B**

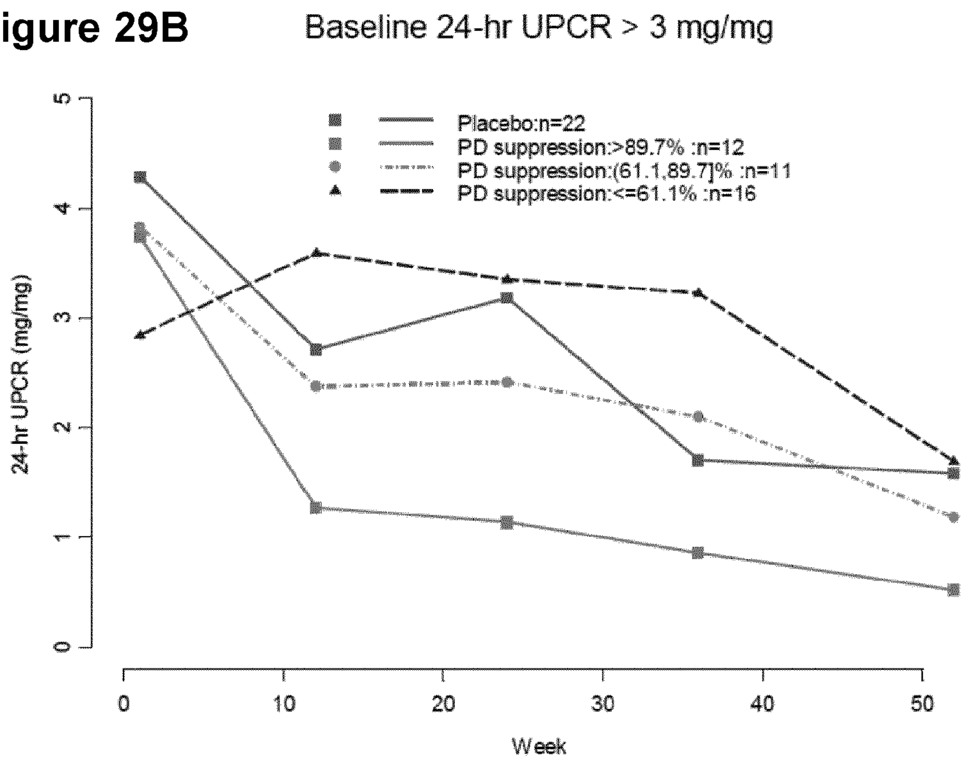

Figure 30

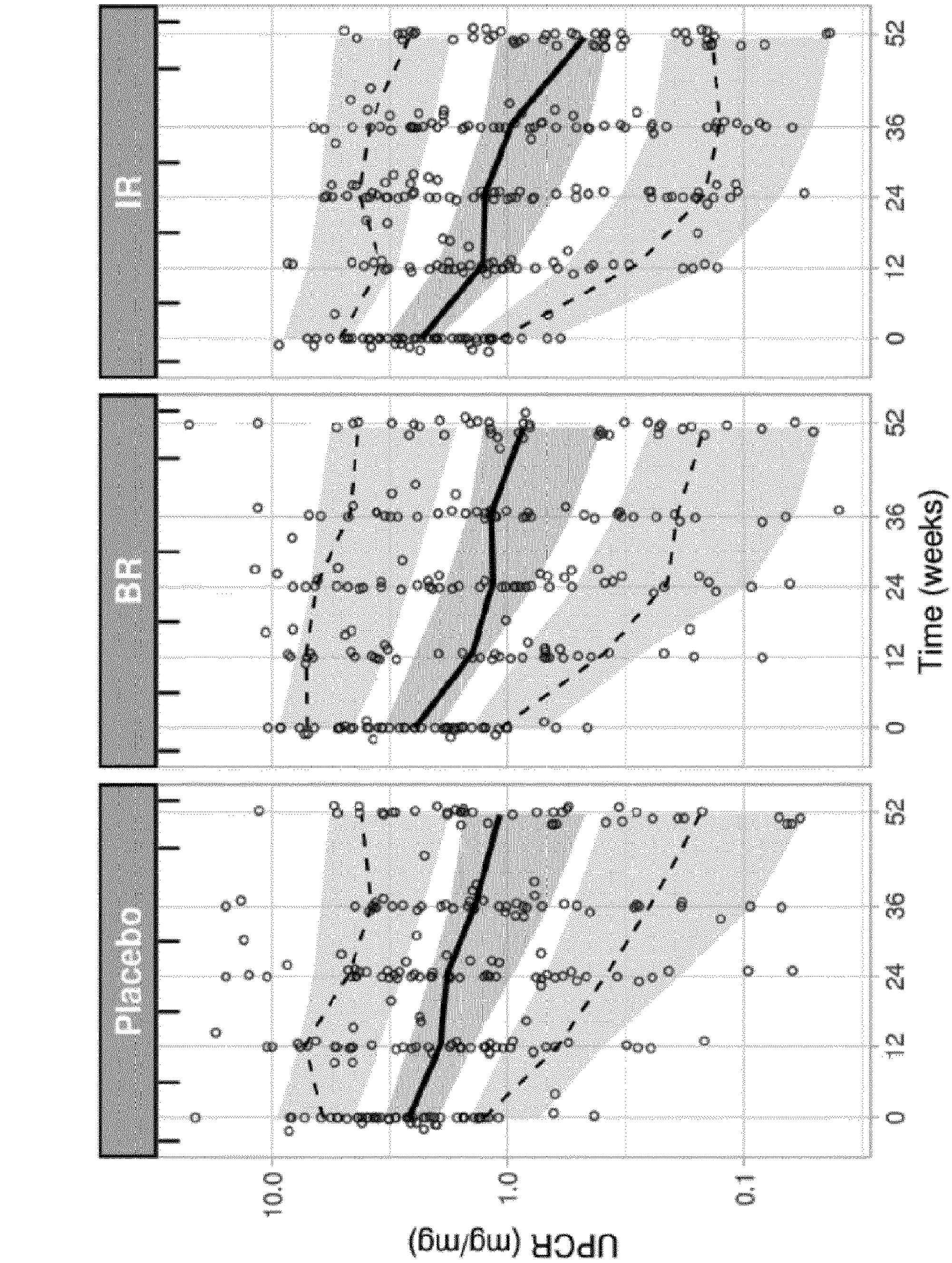

Figure 31

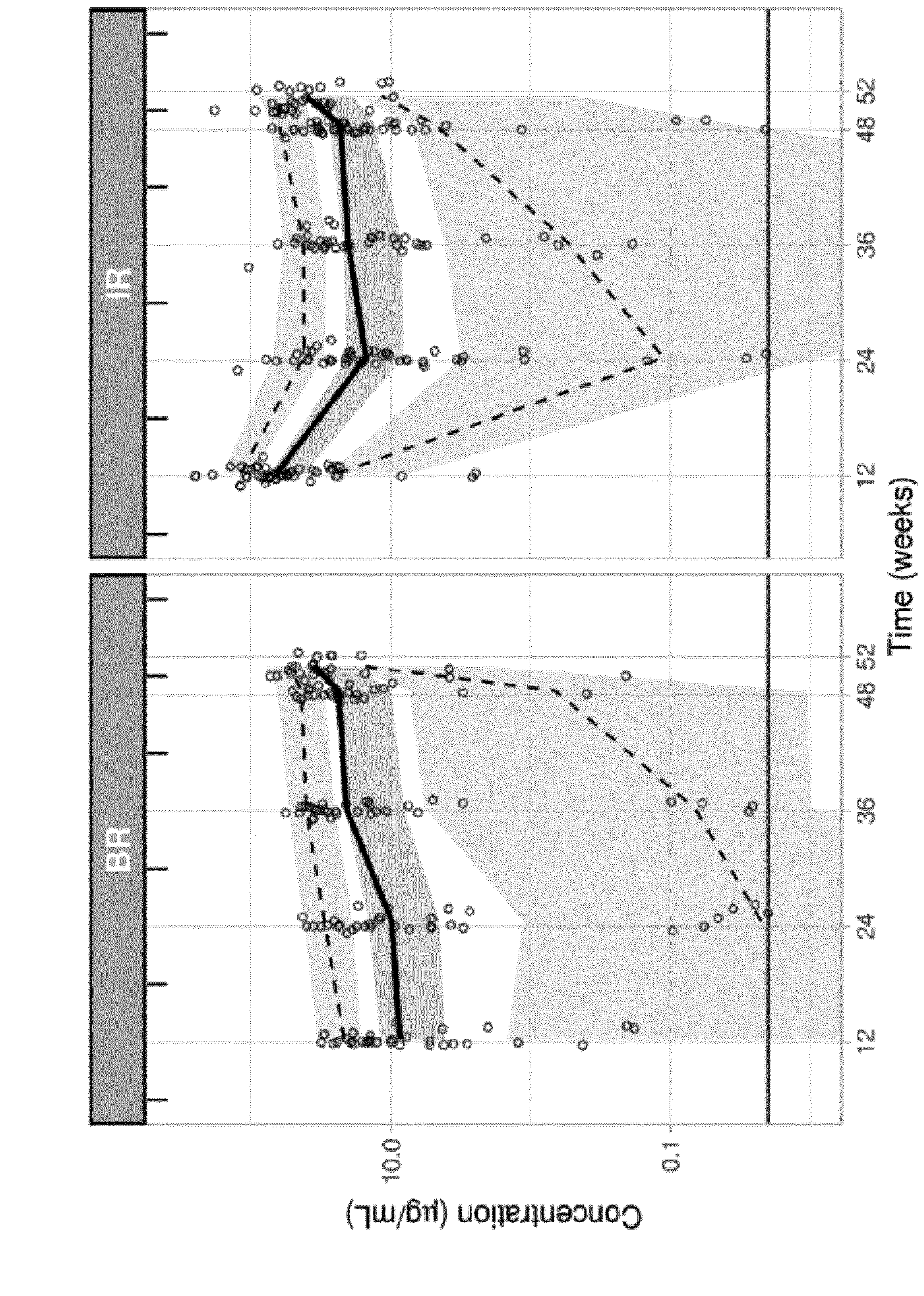

Figure 32

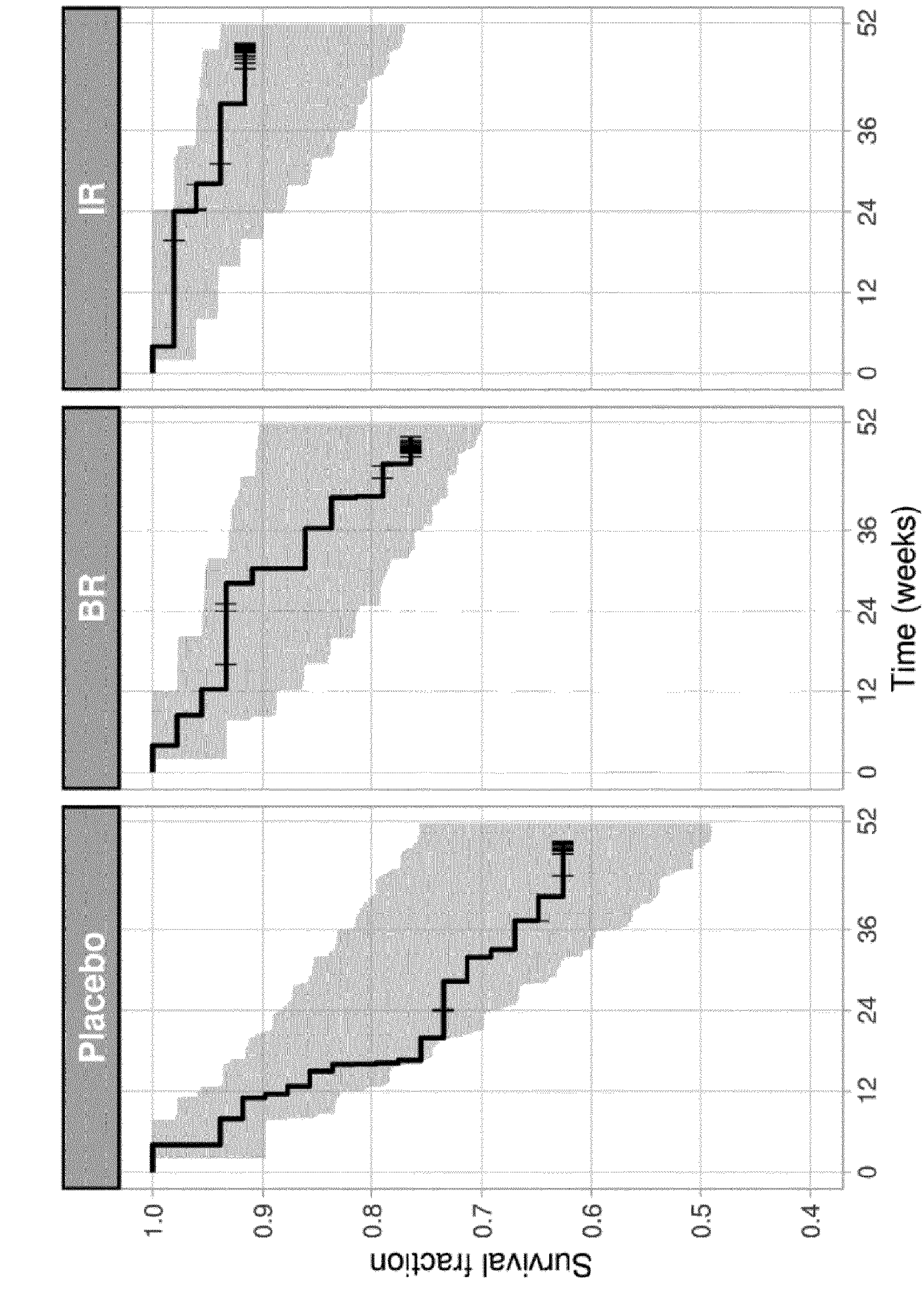

Placebo
BR
IR
Survival fraction
1.0
0.9
0.8
0.7
0.6
0.5
0.4
0
12
24
36
52
Time (weeks)

# Figure 33

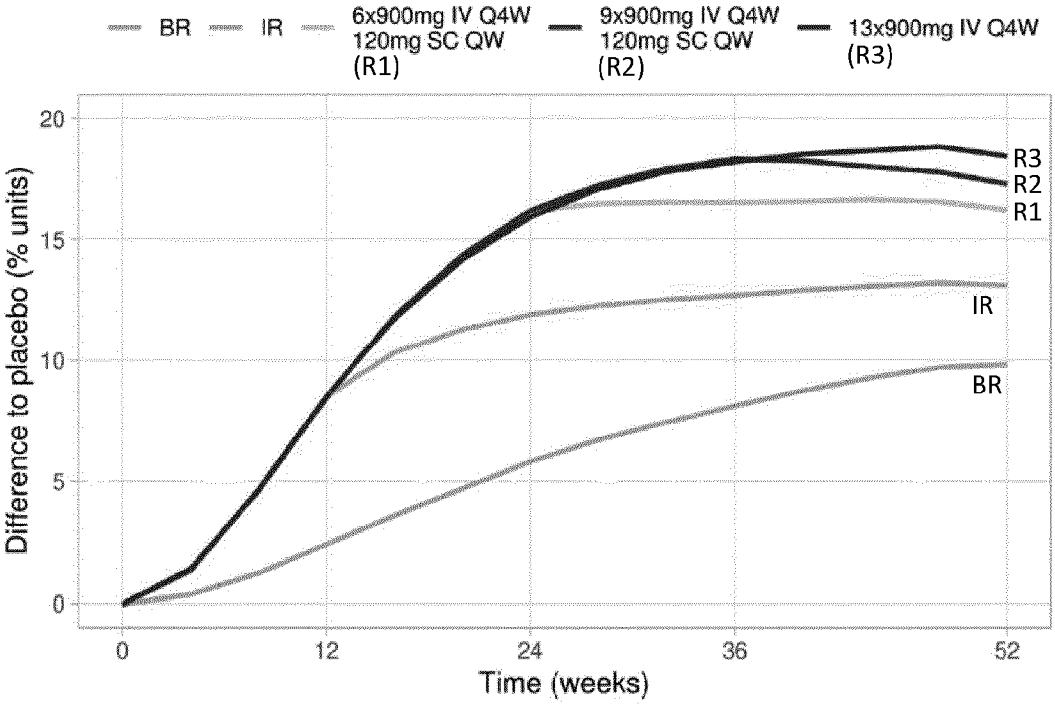
BR
IR
6x900mg IV Q4W
120mg SC QW
(R1)
9x900mg IV Q4W
120mg SC QW
(R2)
13x900mg IV Q4W
(R3)
Difference to placebo (% units)
20
15
10
5
0
0
12
24
36
52
Time (weeks)
R3
R2
R1
IR
BR

Figure 34

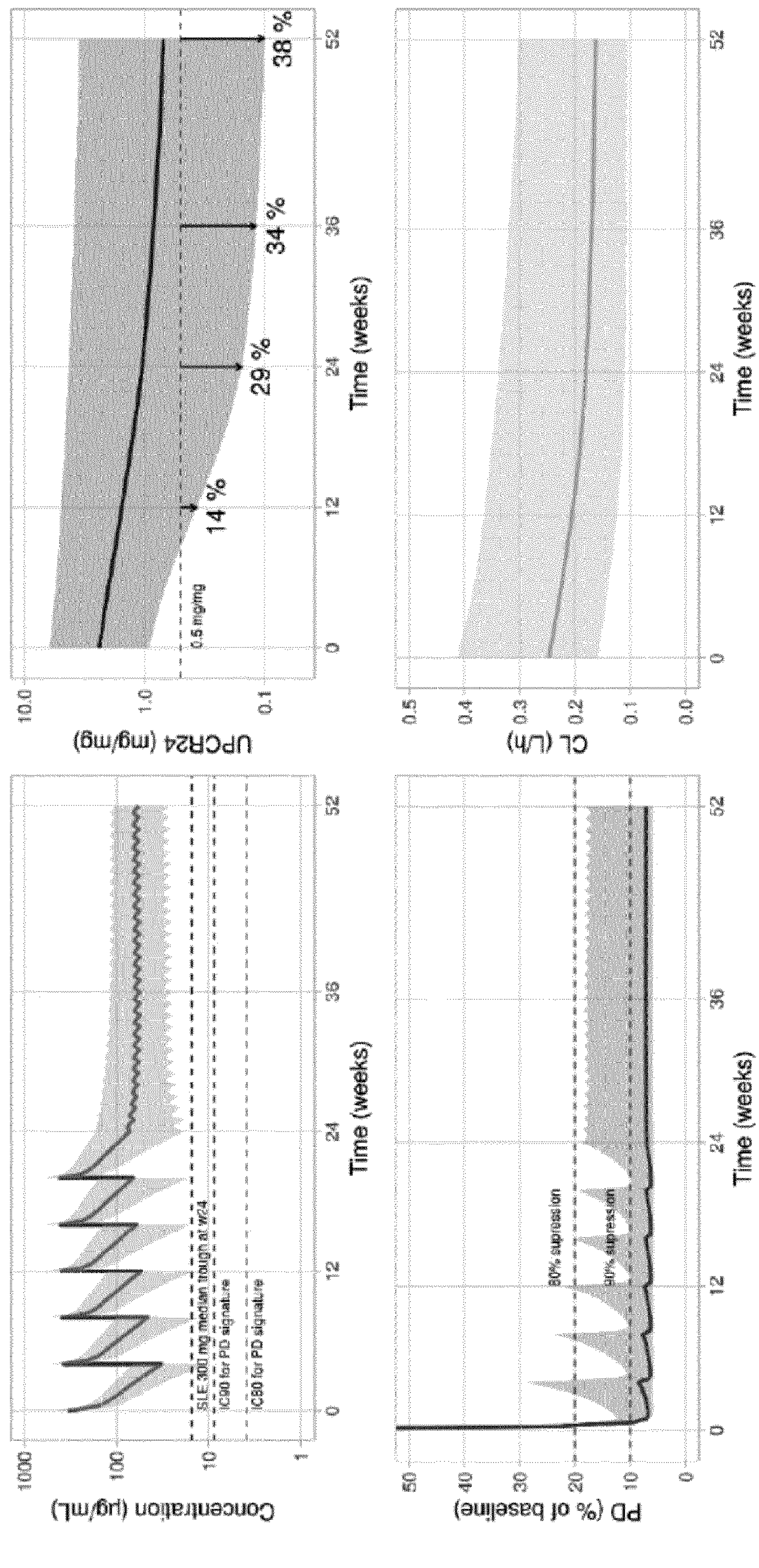
Concentration (µg/mL)
1000
100
10
1
SLE 300 mg median trough at w24
IC90 for PD signature
IC80 for PD signature
0
12
24
36
52
Time (weeks)
UPCR24 (mg/mg)
10.0
1.0
0.1
0.5 mg/mg
14 %
29 %
34 %
38 %
Time (weeks)
PD (% of baseline)
50
40
30
20
10
0
80% supression
90% supression
Time (weeks)
CL (L/h)
0.5
0.4
0.3
0.2
0.1
0.0
Time (weeks)

# Figure 35

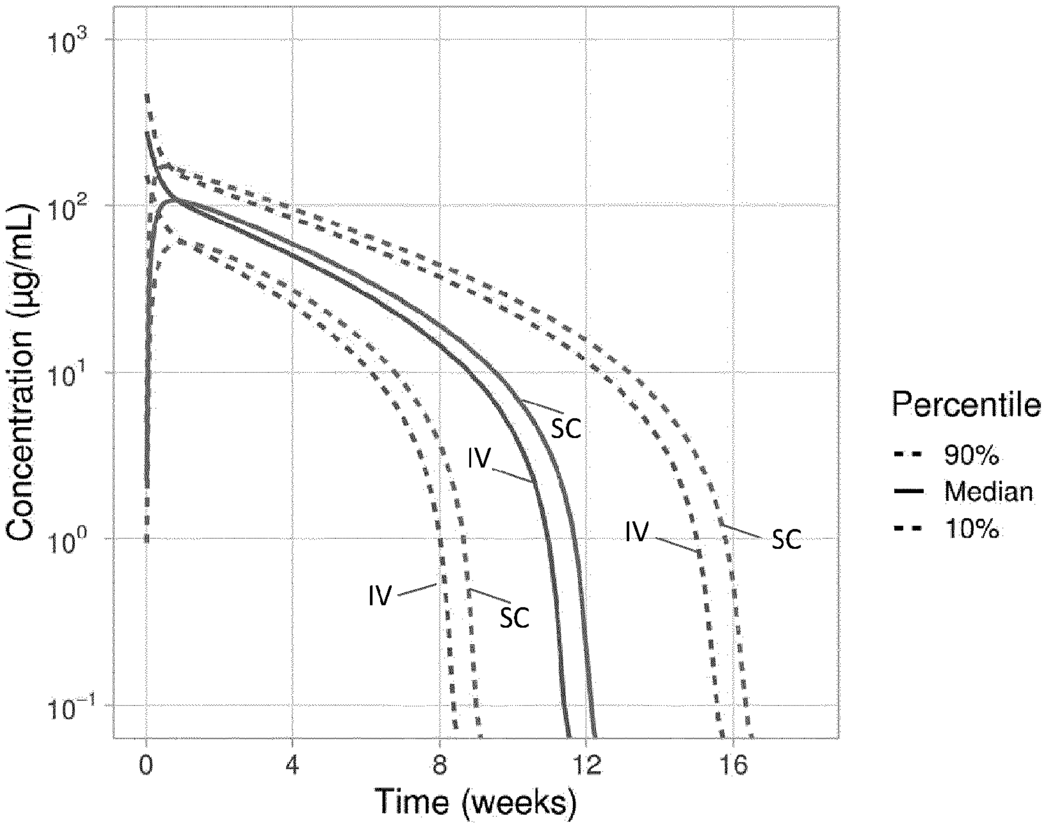

Concentration (µg/mL)
10^3
10^2
10^1
10^0
10^-1
0
4
8
12
16
Time (weeks)
SC
IV
IV
SC
IV
SC
IV
SC
Percentile
90%
Median
10%

Figure 36A

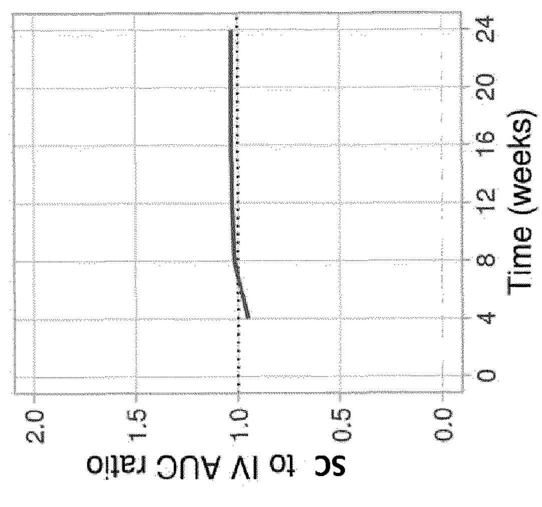


Figure 36B

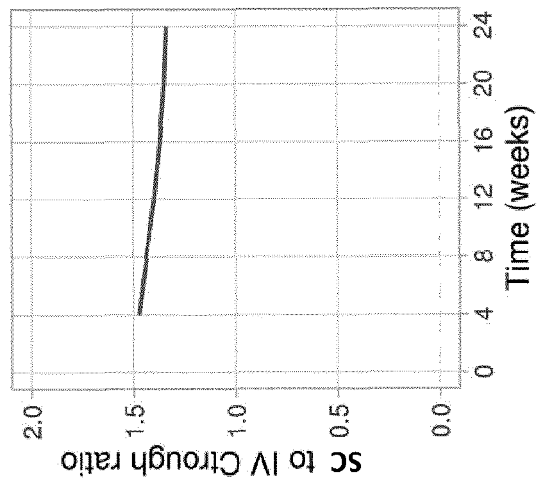


Figure 36C

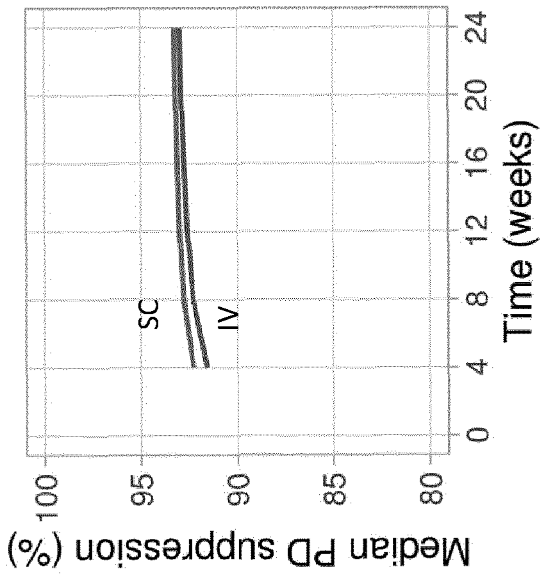

# Figure 37A

| Urinary protein | NIH-AI | NIH-CI |
|---|---|---|
| Cathepsin D | + | + |
| MCP-1 | + | + |
| OPG | + | + |
| TIMP-1 | + | + |
| Adiponectin | + | |
| A2Macro | + | |
| Apo A-I | + | |
| Apo B | + | |
| Apo C-I | + | |
| Apo C-III | + | |
| CLU | + | |
| FRTN | + | |
| IgM | + | |
| IL-6R β | + | |
| LTF | + | |
| Neuropilin-1 | + | |
| Omentin | + | |
| SAP | + | |
| RANTES | + | |
| vWF | + | |
| Apo A-II | | + |
| Apo H | | + |
| IGFBP-2 | | + |
| KIM-1 | | + |
| Vitronectin | | + |
| IL-6 (Simoa) | | + |

# Figure 37B

| Urinary protein | eGFR | SLEDAI | SLEDAI-R | C3 | C4 | NIH-AI | NIH-CI | IFNGS |
|---|---|---|---|---|---|---|---|---|
| Apo A-II | - | | + | | + | | + | |
| Apo B | - | + | + | | | + | | |
| Apo C-I | - | + | + | | | + | | |
| Cathepsin D | - | + | | | | + | + | |
| EN-RAGE | | + | + | - | - | | | + |
| Fibrinogen | - | + | + | | + | | | |
| LTF | - | + | + | | | + | | |
| MCP-1 | - | + | | | | + | + | |
| RANTES | | + | + | | | + | | + |
| IL-1β (Simoa) | | + | + | - | - | | | |

# Figure 38A

| Urinary protein | eGFR | SLEDAI | SLEDAI-R | C3 | C4 | NIH-AI | NIH-CI | IFNGS |
|---|---|---|---|---|---|---|---|---|
| EN-RAGE | | + | + | - | - | | | + |
| RANTES | | + | + | | | + | | + |
| BAFF | | | + | | | | | + |
| CD163 | | | + | | | | | + |
| VCAM-1 | - | | | | | | | + |
| MMP-3 | | | + | | | | | + |
| B2M | | | | | | | | + |
| GDF-15 | | | | | | | | + |
| ICAM-1 | | | | | | | | + |
| IL-1RA | | | | | | | | + |
| IL-6R | | | | | | | | + |
| LRG1 | | | | | | | | + |
| M-CSF | | | | | | | | + |
| PECAM-1 | | | | | | | | + |
| TNF RI | | | | | | | | + |
| TNFR2 | | | | | | | | + |
| uPAR | | | | | | | | + |

# Figure 38B

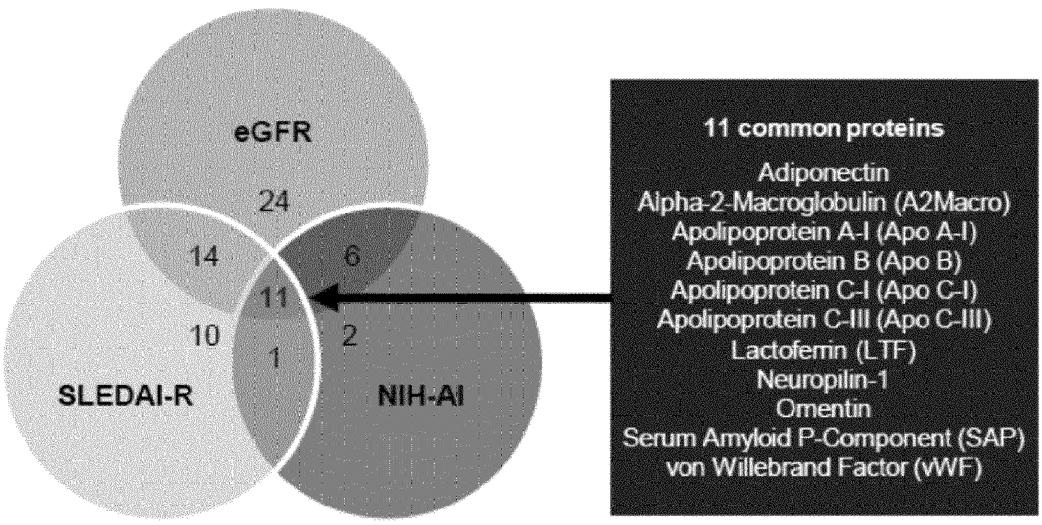

## Figure 39A

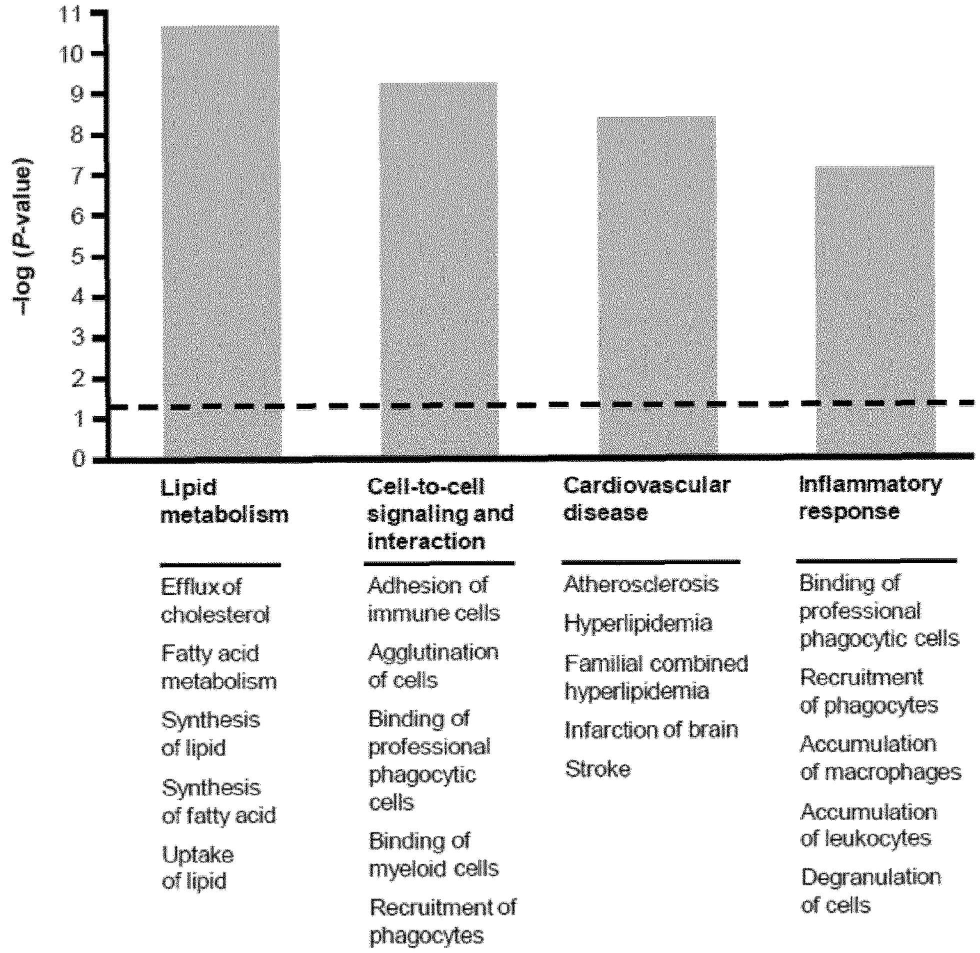


## Figure 39B

| eGFR | SLEDAI-R | NIH-AI | IFNGS |
|---|---|---|---|
| Angiogenin | IgA | FRTN | B2M |
| ALP | Transferrin | IgM | GDF-15 |
| Calbindin | | | ICAM-1 |
| CCL15 | | | IL-1ra |
| HCC-4 | | | IL-6r |
| FABP, adipocyte | | | LRG1 |
| IL-18 | | | M-CSF |
| MMP-7 | | | PECAM-1 |
| NGAL | | | TNF RI |
| PPP | | | TNFR2 |
| PARC | | | uPAR |
| TIMP-2 | | | |
| YKL-40 | | | |

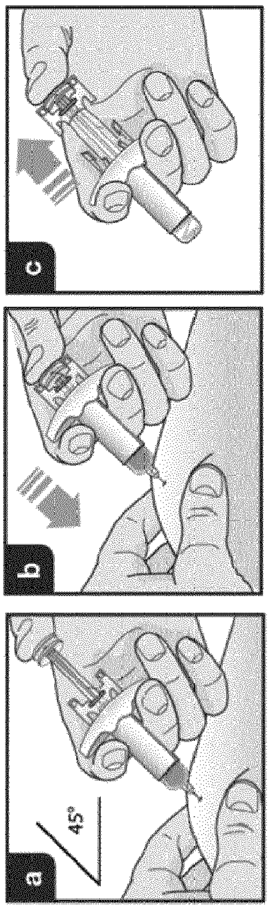


Figure 40A

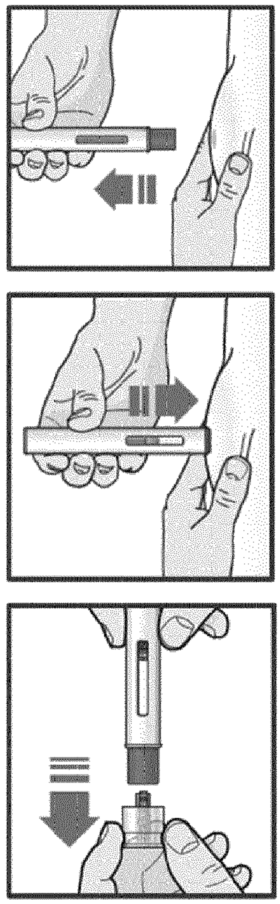

Figure 40B

Figure 41A

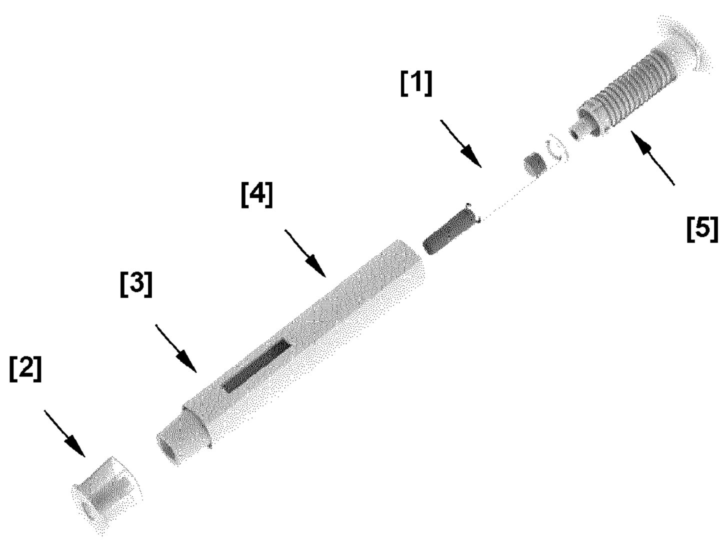


Figure 41B

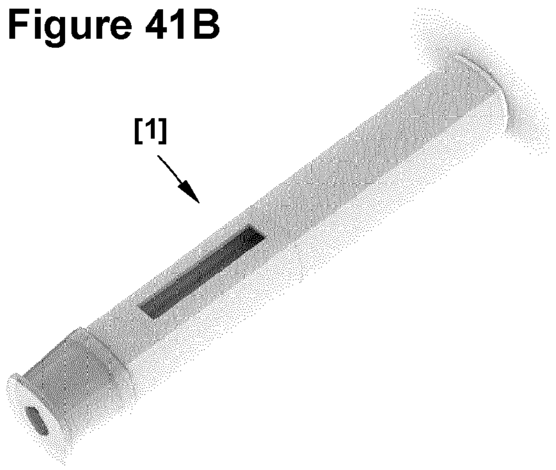


Figure 41C

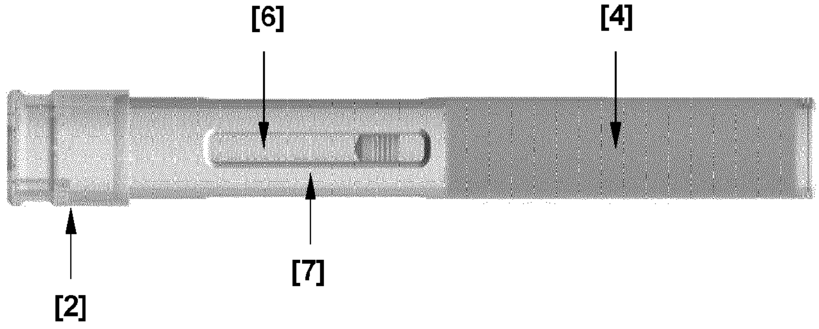

Figure 42A

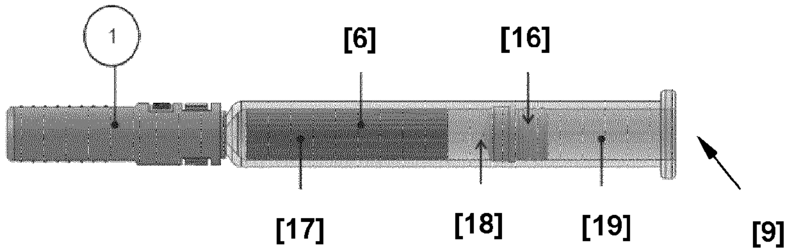


Figure 42B

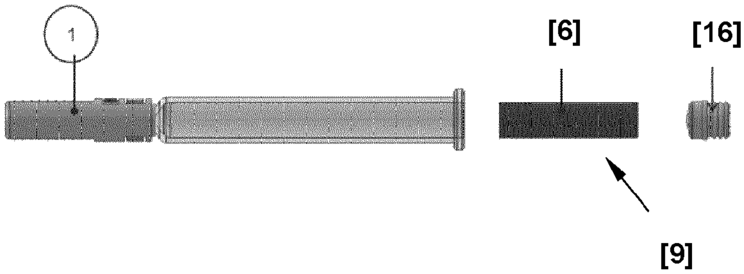


Figure 42C

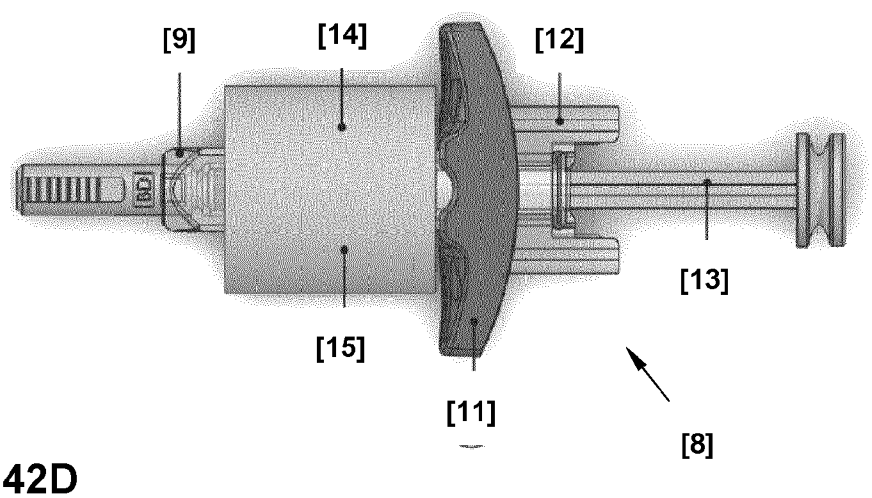


Figure 42D

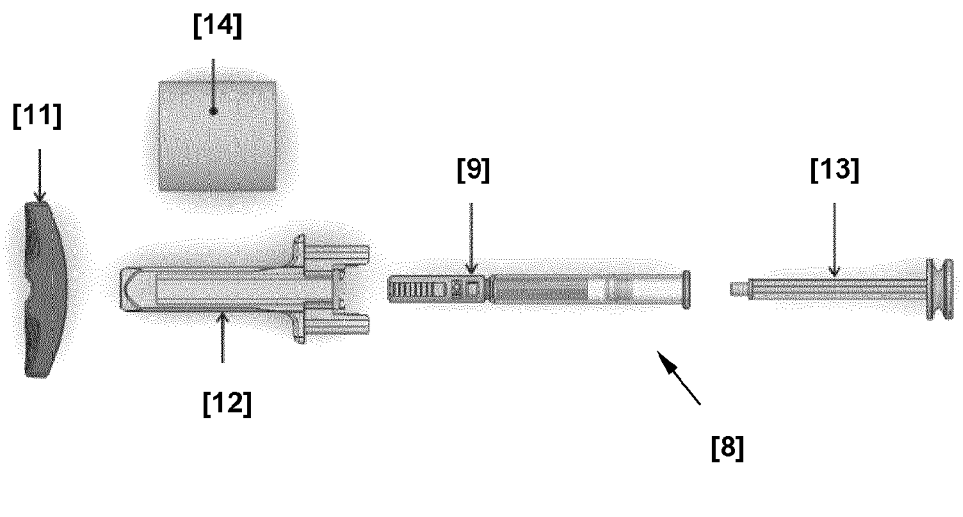

## Figure 43

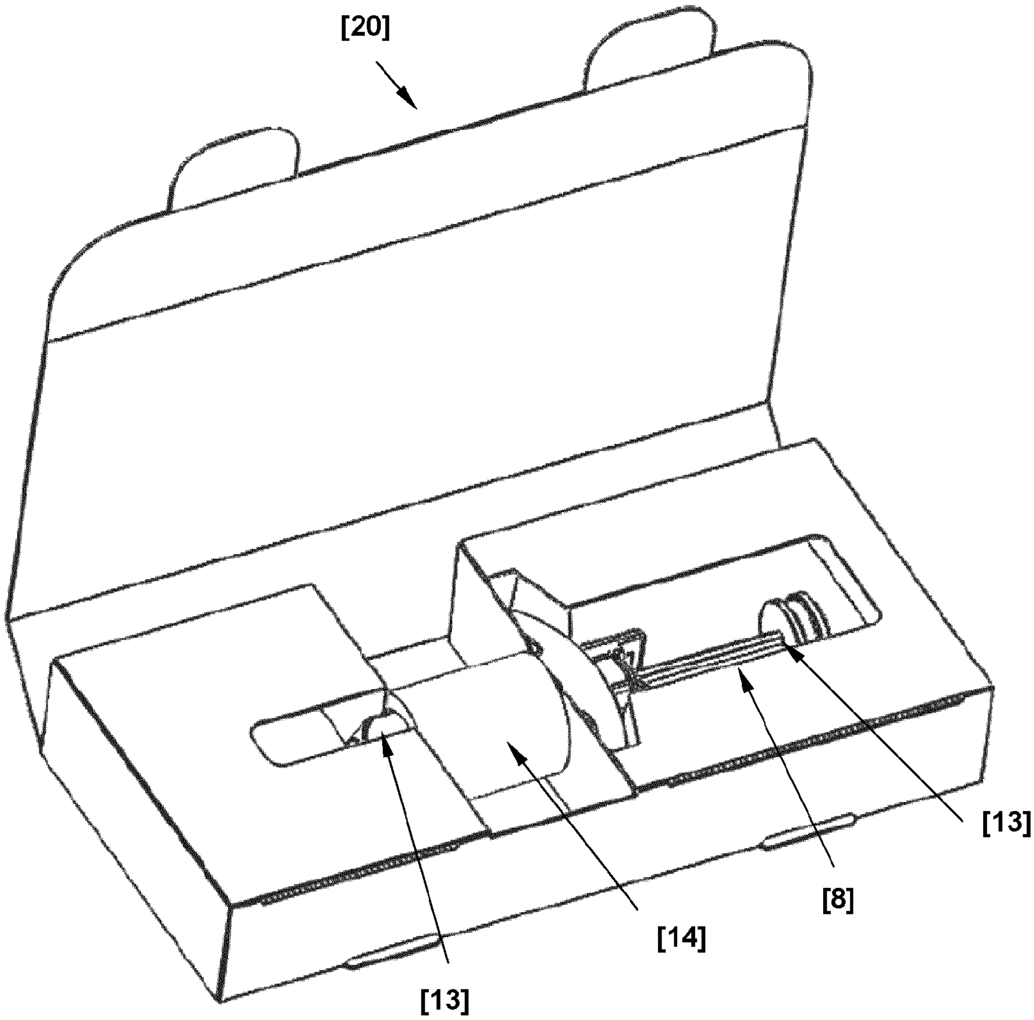

# Figure 44

```
Consensus                EVQLVQSGAEVKKPGESLKISCKGSGYIFTNYWIAWVRQMPGKGLESMGIIYPGDSDIRYSPSFQGQVTISADKSITTAY  80
SEQ ID NO: 1_anifro-VH   EVQLVQSGAEVKKPGESLKISCKGSGYIFTNYWIAWVRQMPGKGLESMGIIYPGDSDIRYSPSFQGQVTISADKSITTAY  80
SEQ ID NO: 3_anifro-HCDR1 ------------------------------NYWIA---------------------------------------------  50
SEQ ID NO: 4_anifro-HCDR2 -------------------------------------------------IIYPGDSDIRYSPSFQG--------------  31
SEQ ID NO: 5_anifro-HCDR2 --------------------------------------------------------------------------------
SEQ ID NO: 10_anifro-HFc --------------------------------------------------------------------------------
SEQ ID NO: 11_anifro-HC  EVQLVQSGAEVKKPGESLKISCKGSGYIFTNYWIAWVRQMPGKGLESMGIIYPGDSDIRYSPSFQGQVTISADKSITTAY  80
INN - anifro-HC          EVQLVQSGAEVKKPGESLKISCKGSGYIFTNYWIAWVRQMPGKGLESMGIIYPGDSDIRYSPSFQGQVTISADKSITTAY  80

Consensus                LQWSSLKASDTAMYYCARHDIEGFDYWGRGTLVTVSSASTKGPSVFPLAPSSKSTSGGTAALGCLVKDYFPEPVTVSWNS  160
SEQ ID NO: 1_anifro-VH   LQWSSLKASDTAMYYCARHDIEGFDYWGRGTLVTVSS-------------------------------------------  160
SEQ ID NO: 3_anifro-HCDR1 --------------------------------------------------------------------------------  130
SEQ ID NO: 4_anifro-HCDR2 --------------------------------------------------------------------------------  111
SEQ ID NO: 5_anifro-HCDR2 ------------------HDIEGFDY------------------------------------------------------  62
SEQ ID NO: 10_anifro-HFc -------------------------------------ASTKGPSVFPLAPSSKSTSGGTAALGCLVKDYFPEPVTVSWNS  43
SEQ ID NO: 11_anifro-HC  LQWSSLKASDTAMYYCARHDIEGFDYWGRGTLVTVSSASTKGPSVFPLAPSSKSTSGGTAALGCLVKDYFPEPVTVSWNS  160
INN - anifro-HC          LQWSSLKASDTAMYYCARHDIEGFDYWGRGTLVTVSSASTKGPSVFPLAPSSKSTSGGTAALGCLVKDYFPEPVTVSWNS  160

Consensus                GALTSGVHTFPAVLQSSGLYSLSSVVTVPSSSLGTQTYICNVNHKPSNTKVDKRVEPKSCDKTHTCPPCPAPEFEGGPSV  240
SEQ ID NO: 1_anifro-VH   --------------------------------------------------------------------------------  240
SEQ ID NO: 3_anifro-HCDR1 --------------------------------------------------------------------------------  210
SEQ ID NO: 4_anifro-HCDR2 --------------------------------------------------------------------------------  191
SEQ ID NO: 5_anifro-HCDR2 --------------------------------------------------------------------------------  142
SEQ ID NO: 10_anifro-HFc GALTSGVHTFPAVLQSSGLYSLSSVVTVPSSSLGTQTYICNVNHKPSNTKVDKRVEPKSCDKTHTCPPCPAPEFEGGPSV  123
SEQ ID NO: 11_anifro-HC  GALTSGVHTFPAVLQSSGLYSLSSVVTVPSSSLGTQTYICNVNHKPSNTKVDKRVEPKSCDKTHTCPPCPAPEFEGGPSV  240
INN - anifro-HC          GALTSGVHTFPAVLQSSGLYSLSSVVTVPSSSLGTQTYICNVNHKPSNTKVDKRVEPKSCDKTHTCPPCPAPEFEGGPSV  240

Consensus                FLFPPKPKDTLMISRTPEVTCVVVDVSHEDPEVKFNWYVDGVEVHNAKTKPREEQYNSTYRVVSVLTVLHQDWLNGKEYK  320
SEQ ID NO: 1_anifro-VH   --------------------------------------------------------------------------------  320
SEQ ID NO: 3_anifro-HCDR1 --------------------------------------------------------------------------------  290
SEQ ID NO: 4_anifro-HCDR2 --------------------------------------------------------------------------------  271
SEQ ID NO: 5_anifro-HCDR2 --------------------------------------------------------------------------------  222
SEQ ID NO: 10_anifro-HFc FLFPPKPKDTLMISRTPEVTCVVVDVSHEDPEVKFNWYVDGVEVHNAKTKPREEQYNSTYRVVSVLTVLHQDWLNGKEYK  203
SEQ ID NO: 11_anifro-HC  FLFPPKPKDTLMISRTPEVTCVVVDVSHEDPEVKFNWYVDGVEVHNAKTKPREEQYNSTYRVVSVLTVLHQDWLNGKEYK  320
INN - anifro-HC          FLFPPKPKDTLMISRTPEVTCVVVDVSHEDPEVKFNWYVDGVEVHNAKTKPREEQYNSTYRVVSVLTVLHQDWLNGKEYK  320

Consensus                CKVSNKALPASIEKTISKAKGQPREPQVYTLPPSREEMTKNQVSLTCLVKGFYPSDIAVEWESNGQPENNYKTTPPVLDS  400
SEQ ID NO: 1_anifro-VH   --------------------------------------------------------------------------------  400
SEQ ID NO: 3_anifro-HCDR1 --------------------------------------------------------------------------------  370
SEQ ID NO: 4_anifro-HCDR2 --------------------------------------------------------------------------------  351
SEQ ID NO: 5_anifro-HCDR2 --------------------------------------------------------------------------------  302
SEQ ID NO: 10_anifro-HFc CKVSNKALPASIEKTISKAKGQPREPQVYTLPPSREEMTKNQVSLTCLVKGFYPSDIAVEWESNGQPENNYKTTPPVLDS  283
SEQ ID NO: 11_anifro-HC  CKVSNKALPASIEKTISKAKGQPREPQVYTLPPSREEMTKNQVSLTCLVKGFYPSDIAVEWESNGQPENNYKTTPPVLDS  400
INN - anifro-HC          CKVSNKALPASIEKTISKAKGQPREPQVYTLPPSREEMTKNQVSLTCLVKGFYPSDIAVEWESNGQPENNYKTTPPVLDS  400

Consensus                DGSFFLYSKLTVDKSRWQQGNVFSCSVMHEALHNHYTQKSLSLSPGK  447
SEQ ID NO: 1_anifro-VH   -----------------------------------------------  117
SEQ ID NO: 3_anifro-HCDR1 -----------------------------------------------  5
SEQ ID NO: 4_anifro-HCDR2 -----------------------------------------------  17
SEQ ID NO: 5_anifro-HCDR2 -----------------------------------------------  8
SEQ ID NO: 10_anifro-HFc DGSFFLYSKLTVDKSRWQQGNVFSCSVMHEALHNHYTQKSLSLSPGK  330
SEQ ID NO: 11_anifro-HC  DGSFFLYSKLTVDKSRWQQGNVFSCSVMHEALHNHYTQKSLSLSPGK  447
INN - anifro-HC          DGSFFLYSKLTVDKSRWQQGNVFSCSVMHEALHNHYTQKSLSLSPGK  447
```

# Figure 45

```
Consensus                     EIVLTQSPGTLSLSPGERATLSCRASQSVSSSFFAWYQQKPGQAPRLLIYGASSRATGIPDRLSGSGSGTDFTLTITRLE   80
SEQ ID NO: 2_anifro-VK        EIVLTQSPGTLSLSPGERATLSCRASQSVSSSFFAWYQQKPGQAPRLLIYGASSRATGIPDRLSGSGSGTDFTLTITRLE   80
SEQ ID NO: 6_anifro-LCDR1     -----------------------RASQSVSSSFFA---------------------------------------------   57
SEQ ID NO: 7_anifro-LCDR2     --------------------------------------------------GASSRAT-----------------------   30
SEQ ID NO: 8_anifro-LCDR3     --------------------------------------------------------------------------------
SEQ ID NO: 09_anifro-LFc      --------------------------------------------------------------------------------
SEQ ID NO: 12_anifro-LC       EIVLTQSPGTLSLSPGERATLSCRASQSVSSSFFAWYQQKPGQAPRLLIYGASSRATGIPDRLSGSGSGTDFTLTITRLE   80

Consensus                     PEDFAVYYCQQYDSSAITFGQGTRLEIKRTVAAPSVFIFPPSDEQLKSGTASVVCLLNNFYPREAKVQWKVDNALQSGNS  160
SEQ ID NO: 2_anifro-VK        PEDFAVYYCQQYDSSAITFGQGTRLEIK----------------------------------------------------  160
SEQ ID NO: 6_anifro-LCDR1     --------------------------------------------------------------------------------  137
SEQ ID NO: 7_anifro-LCDR2     --------------------------------------------------------------------------------  110
SEQ ID NO: 8_anifro-LCDR3     ---------QQYDSSAIT--------------------------------------------------------------   71
SEQ ID NO: 09_anifro-LFc      ----------------------------RTVAAPSVFIFPPSDEQLKSGTASVVCLLNNFYPREAKVQWKVDNALQSGNS   52
SEQ ID NO: 12_anifro-LC       PEDFAVYYCQQYDSSAITFGQGTRLEIKRTVAAPSVFIFPPSDEQLKSGTASVVCLLNNFYPREAKVQWKVDNALQSGNS  160

Consensus                     QESVTEQDSKDSTYSLSSTLTLSKADYEKHKVYACEVTHQGLSSPVTKSFNRGEC                           215
SEQ ID NO: 2_anifro-VK        -------------------------------------------------------                           108
SEQ ID NO: 6_anifro-LCDR1     -------------------------------------------------------                            12
SEQ ID NO: 7_anifro-LCDR2     -------------------------------------------------------                             7
SEQ ID NO: 8_anifro-LCDR3     -------------------------------------------------------                             9
SEQ ID NO: 09_anifro-LFc      QESVTEQDSKDSTYSLSSTLTLSKADYEKHKVYACEVTHQGLSSPVTKSFNRGEC                           107
SEQ ID NO: 12_anifro-LC       QESVTEQDSKDSTYSLSSTLTLSKADYEKHKVYACEVTHQGLSSPVTKSFNRGEC                           215
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013188494 A
- US 10125195 B1
- US 7662381 B
- US 9988459 B
- WO 2018023976 A1
- CN 11327807
- WO 2011028933 A1
- WO 2011028933 A

### Non-patent literature cited in the description


- *WHO Drug information*, 2014, vol. 28 (1)
- **KABAT**. NIH Publication 91-3242. National Technical Information Service, 1991
- **J. G. HANLY et al.** *Rheumatol. Oxf. Engl.*, 2016, vol. 55, 252
- **E. C. BAECHLER et al.** *Proc. Natl. Acad. Sci. U. S. A.*, 2003, vol. 100, 2610
- **J. J. WEENING et al.** *Kidney Int.*, 2004, vol. 65, 521
- **N. MAROZ** ; **M. S. SEGAL**. *Am. J. Med. Sci.*, 2013, vol. 346, 319
- **A. FANOURIAKIS et al.** *Ann. Rheum. Dis.*, 2020, vol. 79, 713
- **E. M. GINZLER** ; **A. J. BOLLET** ; **E. A. FRIEDMAN**. *Annu. Rev. Med.*, 1980, vol. 31, 463
- **H.-J. ANDERS et al.** *Nat. Rev. Dis. Primer*, 2020, vol. 6, 7
- **R. FURIE et al.** *N. Engl. J. Med.*, 2020
- **C. ARRIENS et al.** *Ann. Rheum. Dis.*, 2020, vol. 79, 172
- **B. H. ROVIN et al.** *Kidney Int.*, 2019, vol. 95, 219
- **R. FURIE et al.** *Arthritis Rheumatol. Hoboken Nj*, 2017, vol. 69, 376
- **G. T. FERGUSON et al.** *J. Asthma Allergy*, 2018, vol. 11, 63
- **L. PENG et al.** *mAbs*, 2015, vol. 7, 428
- **I. M. BAJEMA et al.** *Kidney Int.*, 2018, vol. 93, 789
- **A. S. LEVEY et al.** *Ann. Intern. Med.*, 2009, vol. 150, 604
- **A. S. LEVEY et al.** *Ann. Intern. Med.*, 2006, vol. 145, 247
- **B. H. ROVIN et al.** *Lancet Lond. Engl.*, 2021, vol. 397, 2070
- **M. PETRI et al.** *Arthritis Rheum.*, 2008, vol. 58, 1784
- **R. TUMMALA et al.** *Lupus Sci. Med.*, 2018, vol. 5, e000252
- **B. W. HIGGS et al.** *Ann. Rheum. Dis.*, 2014, vol. 73, 256
- **R. A. FURIE et al.** *Lancet Rheumatol.*, 2019, vol. 1, e208
- **E. F. MORAND et al.** *N. Engl. J. Med.*, 2020, vol. 382, 211
- **Y. TANAKA** ; **R. TUMMALA**. *Mod. Rheumatol.*, 2020, 1
- **B. WANG et al.** *Clin. Pharmacol. Ther.*, 2013, vol. 93, 483
- **M. ARINGER et al.** *Arthritis Rheumatol. Hoboken NJ*, 2019, vol. 71, 1400
- **M. C. HOCHBERG**. *Arthritis Rheum.*, 1997, vol. 40, 1725
- **Y. LIN CHIA et al.** *Rheumatol. Oxf. Engl.*, 2021
- **Y. YAO et al.** *Arthritis Res. Ther.*, 2010, vol. 12, S6
- **D. D. GLADMAN** ; **D. IBAÑEZ** ; **M. B. UROWITZ**. *J. Rheumatol.*, 2002, vol. 29, 288
- **M. PETRI et al.** *Arthritis Rheum.*, 1991, vol. 34, 937
- **Y. YAO et al.** *Hum. Genomics Proteomics HGP*, 2009, vol. 2009
- **J. L. GORRIZ** ; **A. MARTINEZ-CASTELAO**. *Transplant. Rev. Orlando Fla*, 2012, vol. 26, 3
- **J. A. MORENO et al.** *Int. J. Mol. Sci.*, 2019, vol. 20
- **J.-Y. JUNG et al.** *Sci. Rep.*, 2019, vol. 9, 9704
- **I. N. BRUCE et al.** *Lancet Rheumatol.*, 2020
- **J. T. RYMAN** ; **B. MEIBOHM**. *CPT Pharmacomet. Syst. Pharmacol.*, 2017, vol. 6, 576